# EUROPEAN PATENT APPLICATION

(11) **EP 4 678 629 A1**
(43) Date of publication of application: **14.01.2026**
(21) Application number: 24766535.9
(22) Date of filing: 08.03.2024
(51) Int. Cl.: C07C 229/16, A61K 47/00

(54) **IONIZABLE LIPID MOLECULE, PREPARATION METHOD THEREFOR AND USE THEREOF**

(30) Priority: 09.03.2023 CN 202310234563
(71) Applicant: Maxirna (Shanghai) Pharmaceutical Co., Ltd., Songjiang District Shanghai 201601 (CN); Maxirna (Zhejiang) Technology Co., Ltd., Shaoxing, Zhejiang 312467 (CN); Shanghai Cell Therapy Group Co., Ltd, Jiading District Anting Town Shanghai 201805 (CN)
(72) Inventor: LI, Song, Shanghai 201805 (CN); HUANG, Ao, Shanghai 201805 (CN); JI, Jinshan, Shanghai 201805 (CN); TIAN, Zhidan, Shanghai 201805 (CN); GUO, Chuanxin, Shanghai 201805 (CN); SUN, Yan, Shanghai 201805 (CN); QIAN, Qijun, Shanghai 201805 (CN)
(74) Representative: Nederlandsch Octrooibureau
(86) International application number: PCT/CN2024/080821
(87) International publication number: WO 2024/183821

(57) **Abstract**

An ionizable lipid molecule, a preparation method therefor and a use thereof. Specifically, provided are a compound represented by formula I, and a stereoisomer, tautomer, pharmaceutically acceptable salt, prodrug or solvate thereof. The definition of each group in the formula is as described in the description. Also provided is a lipid nanoparticle, containing the ionizable lipid compound represented by formula (I). Further provided are a composition and a related use. The compound of formula I can be used to prepare the lipid nanoparticle for delivering a nucleic acid therapeutic agent or an active agent in vivo and in vitro.

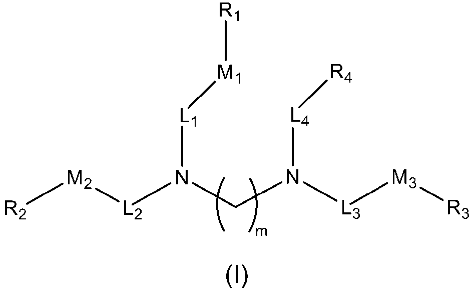

## Description

### TECHNICAL FIELD

The present invention relates to an ionizable lipid molecule, preparation method therefor and use thereof.

### TECHNICAL BACKGROUND

Nucleic acid drugs mainly include antisense nucleic acids, small interfering nucleic acids, microRNAs, messenger nucleic acids, and CRISPR systems. These nucleic acid drugs can effectively regulate and control the function of human genes in various ways, thereby achieving therapeutic effects. However, compared to traditional small molecules, nucleic acid drugs generally have a large molecular weight and carry strong negative charges, making it almost impossible for them to penetrate the cell membrane and enter the cell to exert their effects. And nucleic acids are highly susceptible to degradation and loss of function by various nucleases.

The successful application of ionizable lipid molecules has rapidly promoted the clinical application of nucleic acid drugs. Onpattro, as the first siRNA drug, utilizes DLin-MC3-DMA as an ionizable liposome for delivery to introduce the drug into liver cells for the treatment of nerve damage caused by transthyretin amyloidosis (hATTR, familial amyloidotic polyneuropathy). Moderna and BioNTech/Pfizer developed two COVID-19 vaccines using SM-102 and ALC-0315 respectively, which effectively responded to the COVID-19 in 2019. Generally speaking, nucleic acid drugs need to be composed of four components: ionizable lipids, phospholipids, cholesterol, and PEG. Among them, the ionizable lipids play the most important role in the process of delivering nucleic acid molecules into cells and releasing them into the cytoplasm to function. Up to now, various ionizable lipid molecules have been developed and various delivery functions have been achieved through structural modification and formulation optimization. However, there is still a need for improved ionizable lipid molecules for nucleic acid delivery.

### SUMMARY OF THE INVENTION

The present invention addresses the shortcomings of existing technology and provides a delivery vector for delivering genes into cells, a preparation method therefor, and application thereof.

The first aspect of the present invention provides an ionizable lipid molecule, which is a compound of chemical formula I, or stereoisomer, tautomer, pharmaceutically acceptable salt, prodrug or solvate thereof: 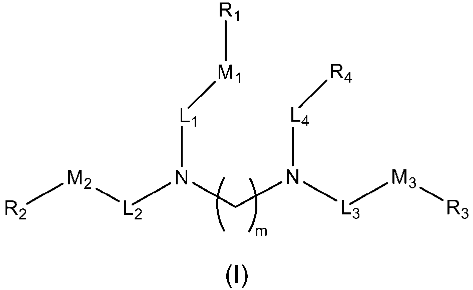 wherein,
L₁, L₂, and L₃ are each independently selected from optionally substituted alkylene, optionally substituted alkenylene, and optionally substituted alkynylene;
M₁, M₂, and M₃ are each independently selected from -C(O)O- and -OC(O)-;
R₁, R₂, and R₃ are each independently selected from optionally substituted alkyl, optionally substituted alkenyl, and optionally substituted alkynyl;
L₄ is selected from optionally substituted alkylene, optionally substituted alkenylene, and optionally substituted alkynylene;
R₄ is -OR₅, R₅ is selected from H, optionally substituted alkyl, optionally substituted alkenyl, and optionally substituted alkynyl; and
m is an integer from 3 to 6.

The second aspect of the present invention provides a lipid nanoparticle comprising an ionizable lipid compound of formula I.

The third aspect of the present invention provides a composition, which comprises the ionizable lipid compound of formula I, or comprises the lipid nanoparticle.

The fourth aspect of the present invention provides a method of treating or preventing a disease or condition in a subject, the method comprising administering the lipid nanoparticle containing a therapeutic agent or an active agent according to any embodiment of the present invention and the pharmaceutical composition according to any embodiment of the present invention.

The fifth aspect of the present description provides use of the compound of formula I according to any embodiment of the present invention in manufacture of a lipid nanoparticle or pharmaceutical composition.

The ionizable lipid molecule of the present invention can effectively deliver various forms of nucleic acid drugs such as ASO, siRNA, miRNA, mRNA, saRNA, sgRNA, etc., allowing different nucleic acid molecules to enter the cell and exert their functions, thereby achieving therapeutic effects.

### BRIEF DESCRIPTION OF THE DRAWINGS

FIG. 1 shows the molecular structure of positive control 1.
FIG. 2 shows the fluorescence intensity photo of mice after intramuscular injection for 6 hours.
FIG. 3 shows the fluorescence intensity photo of mice after intravenous injection for 6 hours.
FIG. 4 shows the fluorescence intensity photo of mice after intramuscular injection for 24 hours.
FIG. 5 shows the fluorescence intensity photo of mice after intravenous injection for 24 hours.
FIG. 6 shows the whole-body fluorescence intensity values of mice after intravenous injection for 6 and 24 hours.
FIG. 7 shows the whole-body fluorescence intensity values of mice after intramuscular injection for 6 and 24 hours.
FIG. 8 shows the fluorescence intensity values at the injection site of mice after intramuscular injection for 6 and 24 hours.
FIG. 9 shows the fluorescence intensity values of the liver abdomen of mice after intramuscular injection for 6 and 24 hours.
FIG. 10 shows the fluorescence intensity photo of mice after intramuscular injection for 6 hours.
FIG. 11 shows the fluorescence intensity photo of mice after intramuscular injection for 24 hours.
FIG. 12 shows the whole-body fluorescence intensity values of mice after intramuscular injection for 6 and 24 hours.
FIG. 13 shows the fluorescence intensity values at the injection site of mice after intramuscular injection for 6 and 24 hours.
FIG. 14 shows the fluorescence intensity photo of mice after intramuscular injection for 6 hours.
FIG. 15 shows the fluorescence intensity photo of mice after intramuscular injection for 24 hours.
FIG. 16 shows the whole-body fluorescence intensity values of mice after intramuscular injection for 6 and 24 hours.
FIG. 17 shows the fluorescence intensity values at the injection site of mice after intramuscular injection for 6 and 24 hours.

### DETAILED DESCRIPTION

It should be understood that within the scope of the present invention, the above-mentioned technical features of the present invention and the technical features specifically described in the following (such as the examples) can be combined with each other to form a preferred technical solution.

### I. Terms

Unless otherwise indicated, the following terms used herein have the following meanings:
As used herein, "includes" and "comprises" and the like are to be construed in an open and inclusive sense, and throughout the specification and claims it means "including but not limited to" unless the context requires otherwise.

In the present invention, reference to "one embodiment" means that a particular feature, structure, or characteristic described in connection with the embodiment is included in at least one embodiment of the present invention. Thus, appearances of the phrase "in one or more embodiments" in various places throughout this specification are not necessarily all referring to the same embodiment. Furthermore, the particular features, structures or characteristics may be combined in any suitable manner in one or more embodiments.

Unless defined otherwise, all technical and scientific terms used herein have the same meaning as commonly understood by one of ordinary skill in the art to which this invention belongs. As used in the specification and claims, the singular forms "a", "said" and "the" include plural referents unless the context clearly dictates otherwise.

An "effective amount" or "therapeutically effective amount" of an active agent or therapeutic agent, such as a therapeutic nucleic acid, is sufficient amount to produce a desired effect (such as an increase or inhibition of expression of a target sequence compared to normal expression levels detected in the absence of the nucleic acid).

The "nucleic acid" described herein refers to a polymer comprising at least two deoxyribonucleotides or ribonucleotides in single- or double-stranded form, including DNA, RNA, and hybrids thereof. The DNA can be in the form of an antisense molecule, plasmid DNA, cDNA, PCR product, or vector. RNA can be in the form of small hairpin RNA (shRNA), messenger RNA (mRNA), antisense RNA, small interfering RNA (siRNA), microRNA (miRNA), self-replicating RNA (saRNA), small guide RNA (sgRNA), multivalent RNA, Dicer substrate RNA, or viral RNA (vRNA) and combination thereof.

The "gene" described herein refers to a nucleic acid (such as DNA or RNA) sequence that comprises a partial or entire length coding sequence necessary to produce a polypeptide or precursor polypeptide.

The "lipid" described herein refers to a group of organic compounds that include, but are not limited to, esters of fatty acids, and are generally characterized as being poorly soluble in water but soluble in many organic solvents. They are generally divided into at least three categories: (1) a "simple lipid", which includes a fat and an oil as well as a wax; (2) a "complex lipid", which includes phospholipid and glycolipid; and (3) a "derived lipid", which like steroid.

The "steroid" is a compound that contains the following carbon skeleton:

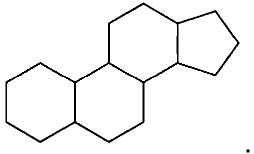

Non-limiting examples of the steroid include cholesterol and the like.

The "cholesterol derivative" may be a cholesterol derivative known in the art for preparing a lipid nanoparticle, and an exemplary cholesterol derivative includes a commonly used cholesterol, CAS: 57-88-5.

The "polymer-conjugated lipid" described herein refers to a molecule comprising a lipid moiety and a polymer moiety. An example of the polymer-conjugated lipid is a PEGylated (PEGed) lipid. The term "PEGylated lipid" refers to a molecule comprising a lipid moiety and a polyethylene glycol moiety. The PEGylated lipid is known in the art and includes 1-(monomethoxy-polyethylene glycol)-2,3-dimyristoylglycerol (PEG-DMG), PEG-DAG (diacylglycerol), PEG-PE (phosphatidylethanolamine), PEG-S-DAG, PEG-DSPE (-distearoylphosphatidylethanolamine), PEG-cer (ceramide) and PEG dialkoxypropyl carbamate, etc. In a preferred embodiment of the present invention, the polymer-conjugated lipid is PEG2000-DMG.

The "neutral lipid" described herein refers to a lipid substance that exists in an uncharged or neutral zwitterionic form at a selected pH. At physiological pH, the lipids include, but are not limited to: phosphatidylcholines such as 1,2-distearoyl-sn-glyceryl-3-phosphocholine (DSPC), 1,2-dipalmitoyl-sn-glyceryl-3-phosphocholine (DPPC), 1,2-dimyristoyl-sn-glyceryl-3-phosphocholine (DMPC), 1-palmitoyl-2-oleoyl-sn-g lyceryl-3-phosphocholine (POPC), 1,2-dioleoyl-sn-glyceryl-3-phosphocholine (DOPC), phosphatidylethanolamine such as 1,2-dioleoyl-sn-glyceryl-3-phosphoethanolamine (DOPE), sphingomyelin (SM) and ceramide. The neutral lipid can be of synthetic or natural origin.

The "lipid nanoparticle" described herein refers to a particle having at least one nanometer-scale size (e.g., 1-1000 nm). The lipid nanoparticle can be comprised in a preparation for delivering an active agent or therapeutic agent (e.g., a nucleic acid) to target sites of interest (e.g., cells, tissues (e.g., diseased tissues such as tumor tissue), organs). In some embodiments, the lipid nanoparticle of the present invention comprises a nucleic acid. The lipid nanoparticle generally comprises one or more compounds of formula I according to the present invention, one or more auxiliary lipid molecules, one or more cholesterol or cholesterol derivatives and/or one or more polymer-conjugated lipid molecules. The auxiliary lipid molecule can be one or more neutral lipid molecules. The active agent or therapeutic agent can be encapsulated in the lipid portion of the lipid nanoparticle or in an aqueous space encapsulated by some or all of the lipid portion of the lipid nanoparticle, thereby protecting it from enzymatic degradation, or from other undesired effects induced by the host organism's or cell's mechanisms, such as adverse immune responses.

As is known in the art, the lipid nanoparticle can have an average diameter of about 30 nm to about 200 nm, about 30 nm to about 150 nm, about 40 nm to about 150 nm, about 50 nm to about 150 nm, about 60 nm to about 130 nm, about 70 nm to about 110 nm, about 70 nm to about 100 nm, about 80 nm to about 100 nm, about 90 nm to about 100 nm, about 70 nm to about 90 nm, about 80 nm to about 90 nm, about 70 nm to about 80 nm, or about 30 nm, about 35 nm, about 40 nm, about 45 nm, about 50 nm, about 55 nm, about 60 nm , about 65 nm, about 70 nm, about 75 nm, about 80 nm, about 85 nm, about 90 nm, about 95 nm, about 100 nm, about 105 nm, about 110 nm, about 115 nm, about 120 nm, about 125 nm, about 130 nm, about 135 nm, about 140 nm, about 145 nm or about 150 nm, and the lipid nanoparticle is essentially non-toxic. In certain embodiments, the nucleic acid, when present in the lipid nanoparticle, is resistant to degradation by a nuclease in an aqueous solution. The lipid nanoparticle comprising a nucleic acid and a preparation method therefor are known in the prior art, for example, see CN102712935A or related patents, etc., the entire disclosure of which is incorporated herein by reference in its entirety for all purposes.

The form of the lipid nanoparticle of the present invention is not particularly limited, but examples of forms in which the ionizable lipid of the present invention is dispersed in an aqueous solvent include unilamellar liposomes, multilamellar liposomes, or unspecified layered structures, etc.

Herein, "halogen" means fluorine, chlorine, bromine or iodine.

"Hydroxyl" means an -OH group.

"Oxo" means an -C(=O)- group.

"Carboxy" refers to -COOH.

"Nitro" refers to -NO₂.

"Cyano" refers to -CN.

"Amino" refers to -NH₂.

"Alkyl" refers to a straight-chain or branched saturated aliphatic hydrocarbon group containing 1-24, 1-18, 1-15, 1-12, 3-10, or 1-8 carbon atoms, such as methyl, ethyl, propyl, butyl, pentyl, heptyl, undecyl, tridecyl, pentadecyl, heptadecyl, heneicosyl, triacontanyl, methylpentadecyl, hexylnonyl, etc., and the alkyl is attached to the rest of the molecule by a single bond.

"Alkylene" is a saturated, branched or straight chain hydrocarbon group having 1-24, 1-18, 1-15, 1-12, 3-10, or 1-8 carbon atoms and having two monovalent radical centers obtained by removal of two hydrogen atoms from the same or two different carbon atoms of the parent alkane, such as -CH₂-, -CH₂CH₂-, -CH₂CH₂CH₂-, -CH₂CH₂CH₂CH₂-, and -CH₂CH(CH₃)CH₂-, etc.

"Alkoxy" refers to alkyl-O-, and the alkyl is as defined herein. An exemplary alkoxy includes methoxy, ethoxy, n-propoxy, isopropoxy, etc. Alkyleneoxy refers to an alkylene with O attached to one end. The alkylene is as defined herein. An exemplary alkyleneoxy is -CH₂CH₂-O-.

"Alkoxyoxo" refers to a group with an oxo group attached to the oxygen of an alkoxy, such as CH₃OC(=O)-, CH₃CH₂OC(=O)- and the like.

"Alkylsulfinyl" means an alkyl-S(=O)- group.

"Alkenyl" means a straight or branched chain aliphatic hydrocarbon group containing 2-24, 2-18, 2-15, 2-12, 3-10, or 2-8 carbon atoms, which contains one or more unsaturated carbon-carbon double bonds, such as vinyl, propenyl, tridecene group, tetradecadienyl, octadecatrienyl, etc., and is attached to the rest of the molecule by a single bond.

"Alkenylene" means a divalent straight-chain or branched alkenyl, usually having 2-24, 2-18, 2-15, 2-12, 3-10, or 2-8 carbon atoms, containing one or more unsaturated carbon-carbon double bonds and connected to the rest of the molecule through two single bonds. An exemplary alkenylene can be -CHCH=CHCH-.

"Cycloalkenyl" means an unsaturated cyclic alkenyl containing three to ten ring carbon atoms.

"Alkynyl" refers to a straight-chain or branched aliphatic hydrocarbon group containing 2-24, 2-18, 2-15, 2-12, 3-10, or 2-8 carbon atoms, which contains one or more unsaturated carbon-carbon triple bonds, such as ethynyl, propynyl, etc., and connected to the rest of the molecule through a single bond.

"Cycloalkynyl" refers to an unsaturated cyclic alkynyl containing three to ten ring carbon atoms.

"Alkynylene" refers to a divalent straight-chain or branched alkynyl, usually having 3-24, 3-18, 3-15, 3-12, 3-10, or 3-8 carbon atoms, containing one or more unsaturated carbon-carbon triple bonds and connected to the rest of the molecule through two single bonds. An exemplary alkenylene can be -CHC=CCH-.

"Acyl" refers to an alkyl-C(O)- group such as acetyl, propionyl, and the like.

"Aryl" refers to a carbocyclic ring system group comprising hydrogen, 6 to 18 carbon atoms (preferably 6-14 carbon atoms), and at least one aromatic ring. Aryl can be monocyclic, bicyclic, tricyclic or tetracyclic ring systems, which can include fused or bridged ring systems. Aryls include, but are not limited to, aryl groups derived from aceanthrylene, acenaphthylene, acephenanthrylene, anthracene, azulene, benzene, fluoranthene, fluorene, asymmetric indacene, symmetric indacene, indane, indene, naphthalene, phenacene, phenanthrene, heptene, pyrene, and triphenylene.

"Aryloxy" means -OR, where R is aryl.

"Carbocyclyl" refers to a stable saturated or unsaturated non-aromatic monocyclic or polycyclic hydrocarbon group composed only of carbon and hydrogen atoms, and the number of ring carbon atoms may be 3-18. Carbocyclyl can include fused or bridged ring systems having 3 to 18 carbon atoms, are saturated and are attached to the rest of the molecule by a single bond. In some embodiments, the carbocyclyl is a cycloalkyl, representative cycloalkyls include, but are not limited to, those having three to fifteen carbon atoms (C₃-C₁₅ cycloalkyl), three to ten carbon atoms (C₃-C₁₀ cycloalkyl), three to eight carbon atoms (C₃-C₈ cycloalkyl), three to six carbon atoms (C₃-C₆ cycloalkyl), three to five carbon atoms (C₃-C₅ cycloalkyl) or three to four carbon atoms (C₃-C₄ cycloalkyl). Monocyclic cycloalkyls include, for example, cyclopropyl, cyclobutyl, cyclopentyl, cyclohexyl, cycloheptyl and cyclooctyl. Polycyclic cycloalkyls include, for example, adamantyl, norbornyl, decalinyl, bicyclo[3.3.0]octane, bicyclo[4.3.0]nonane, cis-decalin, trans-decalin, bicyclo[2.1.1]hexane, bicyclo[2.2.1]heptane, bicyclo[2.2.2]octane, bicyclo[3.2.2]nonane and bicyclo[3.3.2]decane and 7,7- dimethyl-bicyclo[2.2.1]heptanyl. In a preferred embodiment, the cycloalkyl is a 3-8 membered cycloalkyl, such as cyclopropyl, cyclopentyl and cyclohexyl, etc.

"Heterocyclyl" refers to a stable 3-membered to 20-membered non-aromatic cyclic group consisting of 2 to 14 carbon atoms and 1 to 6 heteroatoms selected from nitrogen, phosphorus, oxygen and sulfur. The heterocyclyl can be a monocyclic, bicyclic, tricyclic or multicyclic ring system, which can include fused, bridged or spirocyclic systems. A heterocyclyl can be partially or fully saturated. A heterocyclyl can be attached to the rest of the compound via a carbon atom or a heteroatom and by a single bond. The heterocyclyl is preferably a stable 4-membered to 11-membered non-aromatic monocyclic, bicyclic, bridged or spirocyclic group containing 1 to 3 heteroatoms selected from nitrogen, oxygen and sulfur, more preferably a stable 4-membered to 8-membered non-aromatic monocyclic, bicyclic, bridged or spirocyclic group containing 1 to 3 heteroatoms selected from nitrogen, oxygen and sulfur. Examples of heterocyclyls include, but are not limited to: pyrrolidinyl, morpholinyl, piperazinyl, homopiperazinyl, piperidinyl, thiomorpholinyl, 2,7-diaza-spiro[3.5]nonane-7-yl, 2-oxa-6-aza-spiro[3.3]heptane-6-yl, 2,5-diaza-bicyclo[2.2.1]heptane-2-yl, azetidinyl, pyranyl, tetrahydropyranyl, thiopyranyl, tetrahydrofuryl, oxazinyl, dioxolyl, tetrahydroisoquinolyl, decahydroisoquinolyl, imidazolinyl, imidazolidinyl, quinazinyl, thiazolidinyl, isothiazolidinyl, isoxazolidinyl, indolinyl, octahydroindolyl, octahydroisoindolyl, pyrrolidinyl, pyrazolidinyl , phthalimide, etc.

"Heteroaryl" refers to a 5-membered to 16-membered conjugated ring system group having 1 to 15 carbon atoms (preferably 1 to 10 carbon atoms) and 1 to 6 heteroatoms selected from nitrogen, oxygen and sulfur. Heteroaryls can be monocyclic, bicyclic, tricyclic or multicyclic ring systems. Heteroaryl is preferably a stable 5-membered to 12-membered aromatic group comprising 1 to 5 heteroatoms selected from nitrogen, oxygen and sulfur, more preferably a stable 5-membered to 10-membered aromatic group comprising 1 to 4 heteroatoms selected from nitrogen, oxygen and sulfur, or a 5-membered to 6-membered aromatic group comprising 1 to 3 heteroatoms selected from nitrogen, oxygen and sulfur. Examples of heteroaryls include, but are not limited to, thienyl, imidazolyl, pyrazolyl, thiazolyl, oxazolyl, oxadiazolyl, isoxazolyl, pyridyl, pyrimidinyl, pyrazinyl, pyridazinyl, benzimidazolyl, benzopyrazolyl, indolyl, furyl, pyrrolyl, triazolyl, tetrazolyl, triazinyl, indolyl, isoindolyl, indazolyl, isoindazolyl , purinyl, quinolinyl, isoquinolinyl, diazinyl, naphthyridinyl, quinoxalinyl, pteridinyl, carbazolyl, carbolinyl, phenanthridinyl, phenanthrolinyl, acridinyl, phenazinyl, isothiazolyl, benzothiazolyl, benzothienyl, oxatriazolyl, cinnolinyl, quinazolinyl, phenylthio, indolizyl, o-phenanthrenyl, isoxazolyl, phenoxazinyl, phenothiazinyl, 4,5,6,7-tetrahydrobenzo[b]thienyl, naphthopyridyl, [1,2,4]triazolo[4,3-b]pyridazine, [1,2,4]triazolo[4,3-a]pyrazine, [1,2,4]triazolo[4,3-c]pyrimidine, [1,2,4]triazolo[4,3-a]pyridine, imidazo[1,2-a]pyridine, imidazo[1,2-b]pyridazine, imidazo[1,2-a]pyrazine, etc.

Herein, when a group is "optionally substituted", it may be optionally substituted with 1-5 substituents selected from: alkyl, alkenyl, alkynyl, halogen, haloalkyl, haloalkenyl, haloalkynyl, cyano, nitro, optionally substituted aryl, optionally substituted heteroaryl, optionally substituted cycloalkyl, optionally substituted heterocyclyl. Each of these aryl, heteroaryl, cycloalkyl and heterocyclic groups as substituents may be optionally substituted by groups such as alkyl, alkenyl, alkynyl, halogen, haloalkyl, haloalkenyl, haloalkynyl, cyano, nitro, aryl, heteroaryl, cycloalkyl and heterocyclyl. It is understood that the number of substituents is influenced by the molecular structure of the compound. For example, when the substituent is aryl, heteroaryl, cycloalkyl or heterocyclyl, the number of substituents is usually one; when the substituent is halogen, the number of halogen atoms can be 2-5, according to the chain length of the substituted group or the number of atoms of ring carbon.

Herein, "ionizable lipid compound" refers to a lipid compound that exists in a charged form within a specific pH value or pH range, including positively charged or negatively charged, preferably a positively charged lipid compound (i.e, a cationic lipids compound). The specific pH value or pH range refers to the pH value or pH range of the environment in which the lipid compound is stored or intended to be used, including but not limited to physiological pH.

Herein, "pharmaceutically acceptable salts" include acid addition salts and base addition salts.

"Pharmaceutically acceptable acid addition salt" refers to a salt formed with an inorganic acid or an organic acid while retaining the biological efficacy and properties of the free base. The inorganic acid includes hydrochloric acid, hydrobromic acid, sulfuric acid, nitric acid, phosphoric acid and the like. The organic acids include acetic acid, 2,2-dichloroacetic acid, adipic acid, alginic acid, ascorbic acid, aspartic acid, benzenesulfonic acid, benzoic acid, 4-acetylaminobenzoic acid, camphoric acid, camphor-10-sulfonic acid, capric acid, caproic acid, caprylic acid, carbonic acid, cinnamic acid, citric acid, cyclamate, lauryl sulfate, ethane-1,2-disulfonic acid, ethanesulfonic acid, 2-hydroxyethanesulfonic acid , formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid , hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxyl-2-naphthoic acid, niacin, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid and undecylenic acid, etc.

"Pharmaceutically acceptable base addition salts" refer to those salts that retain the biological effectiveness and properties of the free acids. These salts are prepared by adding an inorganic or organic base to the free acid. Salts derived from inorganic bases include, but are not limited to, sodium salts, potassium salts, lithium salts, ammonium salts, calcium salts, magnesium salts, iron salts, zinc salts, copper salts, manganese salts, aluminum salts, and the like. Preferred inorganic salts are ammonium salts, sodium salts, potassium salts, calcium salts and magnesium salts. Salts derived from organic bases include, but are not limited to, salts of primary, secondary, and tertiary amines, substituted amines, including naturally occurring substituted amines, cyclic amines, and basic ion exchange resins such as ammonia, isopropylamine, trimethylamine, diethylamine, triethylamine (TEA), tripropylamine, diethanolamine, ethanolamine, deanol, 2-dimethylaminoethanol, 2-diethylaminoethanol, dicyclohexylamine, lysine, arginine, histidine, caffeine, procaine, hybamine, choline, betaine, pheniramine, benzathine, ethylenediamine, glucosamine, methylglucosamine, theobromine, triethanolamine, tromethamine, purine, piperazine, piperidine, N-ethylpiperidine, polyamine resin, etc. Particularly preferred organic bases are isopropylamine, diethylamine, ethanolamine, trimethylamine, dicyclohexylamine, choline and caffeine.

Herein, "pharmaceutical composition" refers to a preparation containing an active pharmaceutical ingredient and the compound of formula I of the present invention. Pharmaceutical compositions generally contain a therapeutically effective amount of a therapeutic agent or active agent, that is, the therapeutic agent or active agent. A "therapeutically effective amount" refers to an amount sufficient to achieve treatment in a mammal when administered to a mammal, preferably a human. The amount of the therapeutic agent or active agent which constitutes a "therapeutically effective amount" will vary depending on the therapeutic agent or active agent used, the condition and its severity, the route of administration and the age of the mammal to be treated, but can be routinely determined by one of ordinary skill in the art based on their knowledge and the present disclosure.

The "pharmaceutically acceptable carrier or excipient" described herein includes, but is not limited to, any adjuvant, carrier, excipient, glidant, sweetener, diluent, preservative, dye/colorant, flavor enhancer, surfactant, wetting agent, dispersant, suspending agent, stabilizer, isotonic agent, solvent or emulsifier that has been approved by FDA or CFDA for use in humans or domesticated animals.

The "treatment" as described herein encompasses treatment for the disease or condition of interest in a mammal, preferably a human, suffering from the disease or condition of interest, and includes:
(1) preventing the disease or condition from occurring in a mammal, especially when such mammal is susceptible to the condition but has not been diagnosed as having the condition;
(2) inhibiting the disease or condition, that is, prevent its development;
(3) amelioration of the disease or condition, that is, causing regression of the disease or condition; or
(4) relief of symptoms caused by the disease or condition, i.e., pain relief without curing the underlying disease or condition.

Herein, "disease" and "condition" may be used interchangeably, or may be different, because a particular disease or condition may not have a known causative agent (so that the cause has not yet been identified), and therefore it has not been recognized as a disease and is only considered as an undesired condition or syndrome in which a more or less specific group of symptoms has been identified by a clinician.

Herein, "mammal" includes humans as well as domestic animals such as laboratory animals and household pets (e.g., cats, dogs, pigs, cows, sheep, goats, horses, rabbits) and non-domestic animals (eg wild animals, etc.).

Compounds of formula I, or pharmaceutically acceptable salts thereof, may contain one or more asymmetric centers and thus give rise to enantiomers, diastereomers and other stereoisomeric forms defined as (R)- or (S)- or (D)- or (L)-(for amino acids) in terms of absolute stereochemistry. The present invention includes all such possible isomers, as well as their racemic and optically pure forms. Optically active (+) and (-), (R)- and (S)-, or (D)- and (L)-isomers can be prepared using chiral synthons or chiral reagents, or by resolution using conventional techniques such as chromatography and fractional crystallization. Conventional techniques for the preparation/isolation of individual enantiomers include chiral synthesis from suitable optically pure precursors or resolution of racemates (or racemates of salts or derivatives) by, for example, chiral high pressure liquid chromatography (HPLC). When the compound described herein contains an olefinic double bond or other geometric asymmetric centers, and unless otherwise stated, it is intended that the compound includes both E and Z geometric isomers. Likewise, all tautomeric forms are also intended to be included.

A "stereoisomer" refers to a compound made up of the same atoms bonded by the same bonds but having different three-dimensional structures, which are not interchangeable. The present invention encompasses various stereoisomers and mixtures thereof, including "enantiomers", which refer to two stereoisomers whose molecules are nonsuperimposeable mirror images of each other.

A "tautomer" refers to a proton shift from one atom of a molecule to another atom of the same molecule. The present invention includes tautomers of any said compounds.

### II. Ionizable Lipid Compounds

The ionizable lipid compound described herein has the structural formula of the following formula I: 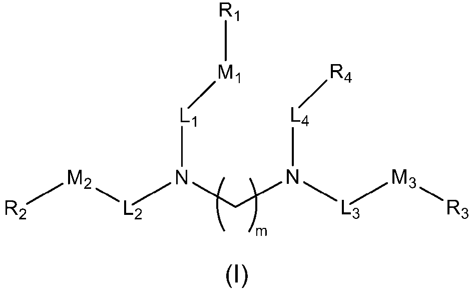 wherein, L₁, L₂, and L₃ are each independently selected from optionally substituted alkylene, optionally substituted alkenylene, and optionally substituted alkynylene; M₁, M₂, and M₃ are each independently selected from -C(O)O- and - OC(O)-; R₁, R₂, and R₃ are each independently selected from optionally substituted alkyl, optionally substituted alkenyl, and optionally substituted alkynyl; L₄ is selected from optionally substituted alkylene, optionally substituted alkenylene, and optionally substituted alkynylene; R₄ is -OR₅; R₅ is selected from H, optionally substituted alkyl, optionally substituted alkenyl, and optionally substituted alkynyl; and m is an integer from 3 to 6.

Stereoisomers, tautomers, pharmaceutically acceptable salts, prodrugs and solvates of the compounds of formula I are also included herein.

In formula I, when a group is "optionally substituted", its substituents can be selected from hydroxyl, alkoxy, halogen, alkyl, haloalkyl, hydroxyl-substituted alkyl, alkenyl, haloalkenyl, hydroxyl-substituted alkenyl, -NR'R", optionally substituted cycloalkyl, optionally substituted aryl, optionally substituted heteroaryl, or optionally substituted heterocyclyl. The number of substituents can be 1-6. The R' and R" are each independently selected from H and C₁₋₄ alkyl. The alkyl of alkyl, alkoxy, haloalkyl and hydroxyl-substituted alkyl can be C₁₋₆ alkyl or C₁₋₄ alkyl; the alkenyl of alkenyl, haloalkenyl, hydroxyl-substituted alkenyl can be C₂₋₆ alkenyl or C₂₋₄ alkenyl; the cycloalkyl can be C₃₋₈ cycloalkyl, optionally substituted by 1-4 substituents selected from halogen, C₁₋₄ alkyl, hydroxyl and -NR'R"; the aryl can be 6-14 membered aryl, such as phenyl and naphthyl, the aryl can be optionally substituted by 1-4 substituents selected from halogen, C₁₋₄ alkyl, hydroxyl and -NR'R"; the heteroaryl can be 5-10 membered heteroaryl, such as 5-10 membered heteroaryl containing nitrogen and/or oxygen, such as pyridyl, pyrazolyl and imidazolyl, etc., the heteroaryl can be optionally substituted by 1-4 substituents selected from halogen, C₁₋₄ alkyl, hydroxyl and -NR'R"; the heterocyclyl can be a 4-10 membered heterocyclyl, preferably a 4-10 membered heterocyclyl containing nitrogen and/or oxygen, such as morpholinyl, piperidinyl, piperazinyl, pyrrolidinyl, etc., and the heterocyclyl can be optionally substituted by 1-4 substituents selected from halogen, C₁₋₄ alkyl, hydroxyl and -NR'R".

In a preferred embodiment, in formula I, L₁, L₂, and L₃ are each independently selected from an optionally substituted C₁₋₂₄ alkylene, an optionally substituted C₂₋₂₄ alkenylene, and an optionally substituted C₂₋₂₄ alkynylene. Preferably, L₁, L₂, and L₃ are each independently selected from -(CH₂)ₙ-, wherein n is an integer from 1 to 24, preferably from 2 to 24, more preferably from 3 to 15, and further preferably from 3 to 11. In some embodiments, n in L₁, L₂, and L₃ are each independently an integer from 3 to 8. In some embodiments, L₁, L₂, and L₃ are the same group.

In a preferred embodiment, in formula I, R₁, R₂, and R₃ are each independently selected from an optionally substituted C₁₋₂₄ alkyl, an optionally substituted C₂₋₂₄ alkenyl, and an optionally substituted C₂₋₂₄ alkynyl. Preferably, R₁, R₂, and R₃ are each independently selected from a C₁₋₂₄ alkyl optionally substituted with one or more C₁₋₂₄ alkyls, and a C₂₋₂₄ alkenyl optionally substituted with one or more C₁₋₂₄ alkyls. In one or more embodiments, the C₂₋₂₄ alkenyl contains 1-4 carbon-carbon double bonds. The carbon-carbon double bonds can be in Z and/or E configuration, preferably in Z configuration.

In a preferred embodiment, in formula I, R₁, R₂, and R₃ are each independently selected from

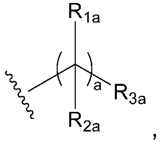

wherein, a is an integer from 1 to 24, preferably from 2 to 20, and more preferably from 3 to 15; R₁ₐ and R₂ₐ are independently H or a C₁₋₂₄ alkyl at each occurrence; alternatively, each R₁ₐ is independently H or a C₁₋₂₄ alkyl, and at least one R₂ₐ, together with the carbon atom to which it is bound is taken together with an adjacent R₂ₐ and the carbon atom to which it is bound to form a carbon-carbon double bond; and R₃ₐ is H or methyl.

In some embodiments, a, R₁ₐ, R₂ₐ, and R₃ₐ are each selected such that R₁, R₂, and R₃ each independently contain 1 to 40, preferably 1-30, more preferably 1-24, and further preferably 3-24 carbon atoms.

In some embodiments, at least one R₁ₐ is H, for example, all R₁ₐ are H; and at least one R₂ₐ is H, for example, all R₂ₐ are H. In other embodiments, at least one R₁ₐ is H, for example, all R₁ₐ are H, and at least one R₂ₐ is a C₁₋₂₄ alkyl, preferably a C₃₋₂₀ alkyl, and more preferably a C₃₋₁₅ alkyl.

In some embodiments, R₁, R₂, and R₃ are each independently selected from 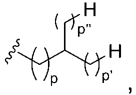 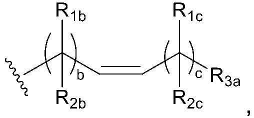 and

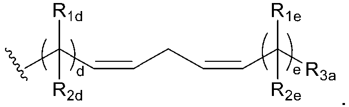

Among them, p is an integer from 0-2, p' is an integer from 1-24, and p" is an integer from 0-24; b, c, d, and e are each independent integers from 1 to 22; R_{1b} and R_{2b} are independently H or a C₁₋₁₂ alkyl at each occurrence, or each R_{1b} is independently H or a C₁₋₁₂ alkyl, and at least one R_{2b}, together with the carbon atom to which it is bound is taken together with an adjacent R_{2b} and the carbon atom to which it is bound to form a carbon-carbon double bond; R_{1c} and R_{2c} are independently H or a C₁₋₁₂ alkyl at each occurrence, or each R_{1c} is independently H or a C₁₋₁₂ alkyl, and at least one R_{2c}, together with the carbon atom to which it is bound is taken together with an adjacent R_{2c} and the carbon atom to which it is bound to form a carbon-carbon double bond; R_{1d} and R_{2d} are independently H or a C₁₋₁₂ alkyl at each occurrence, or each R_{1d} is independently H or a C₁₋₁₂ alkyl, and at least one R_{2d}, together with the carbon atom to which it is bound is taken together with an adjacent R_{2d} and the carbon atom to which it is bound to form a carbon-carbon double bond; R₁ₑ and R₂ₑ are independently H or a C₁₋₁₂ alkyl at each occurrence, or each R₁ₑ is independently H or a C₁₋₁₂ alkyl, and at least one R₂ₑ, together with the carbon atom to which it is bound is taken together with an adjacent R₂ₑ and the carbon atom to which it is bound to form a carbon-carbon double bond; and R₃ₐ is H or methyl.

In some embodiments, p' and p" are each selected such that R₁, R₂, and R₃ each independently contain 1 to 40, preferably 1-30, more preferably 1-24, and further preferably 3-24 carbon atoms.

In some embodiments, p' and p" are the same integer.

In some embodiments, b, c, d, e, R_{1b}, R_{2b}, R_{1c}, R_{2c}, R_{1d}, R_{2d}, R₁ₑ, and R₂ₑ are each selected such that R₁, R₂, and R₃ each independently contain 1 to 40, preferably 1-30, more preferably 1-24, and further preferably 3-24 carbon atoms.

In some embodiments, at least one R_{1b} is H, for example, all R_{1b} are H; at least one R_{2b} is H, for example, all R_{2b} are H; at least one R_{1c} is H, for example, all R_{1c} are H; at least one R_{2c} is H, for example, all R_{2c} are H; at least one R_{1d} is H, for example, all R_{1d} are H; at least one R_{2d} is H, for example, all R_{2d} are H; at least one R₁ₑ is H, for example, all R₁ₑ are H; and at least one R₂ₑ is H, for example, all R₂ₑ are H.

In some embodiments, b, c, d, and e are each independently an integer from 1 to 10.

In some embodiments,
examples of 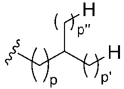 include but are not limited to:
-(CH₂)_{p*}CH₃, P* is an integer from 1 to 23, preferably from 3 to 14, 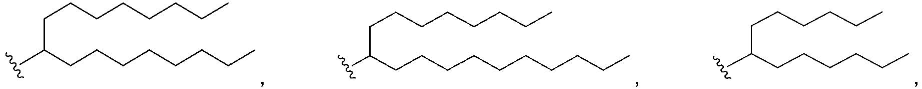 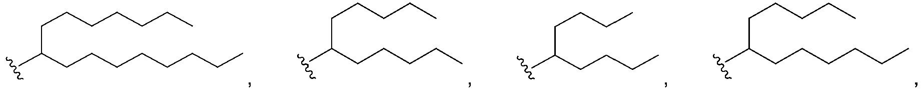 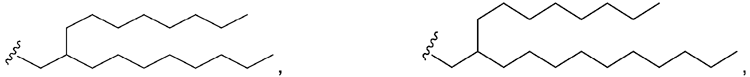 and

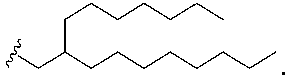

In some embodiments,
examples of 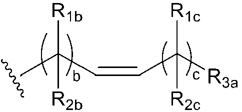 include but are not limited to:

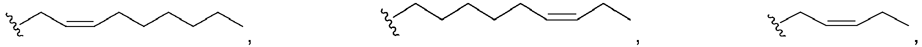

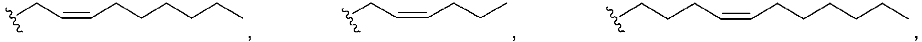

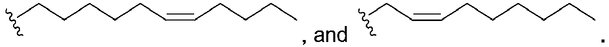

In some embodiments,
examples of 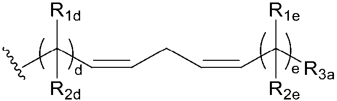 include but are not limited to: 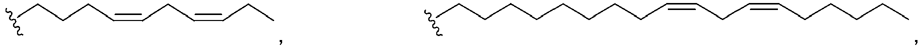 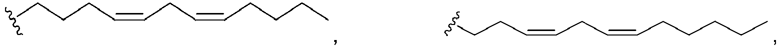 and

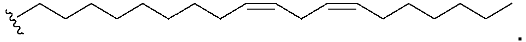

In some embodiments, R₁, R₂, and R₃ are each independently selected from a C₂₋₂₄ alkenyl, which can be substituted with 1-4 C₁₋₆ alkyls, preferably methyl, and the number of carbon-carbon double bonds can be 1-4, preferably 1-2, and the carbon-carbon double bonds can be in E configuration. Examples of the alkenyl include but are not limited to: 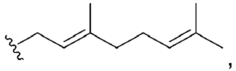 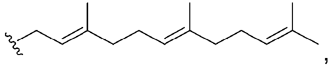 and

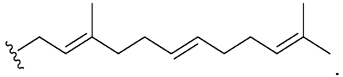

In one or more embodiments, R₁, R₂, and R₃ are the same group.

In a preferred embodiment, in formula I, L₄ is selected from an optionally substituted C₁₋₈ alkylene, an optionally substituted C₂₋₆ alkenylene, and an optionally substituted C₂₋₆ alkynylene, and preferably, L₄ is selected from -(CH₂)ₒ-, wherein o is an integer from 1 to 8. In some embodiments, L₄ is selected from an optionally substituted C₁₋₆ alkylene, an optionally substituted C₂₋₆ alkenylene, and an optionally substituted C₂₋₆ alkynylene, and preferably, L₄ is selected from -(CH₂)ₒ-, wherein o is an integer from 1 to 6, preferably an integer from 2 to 5, and more preferably 2, 3, or 4.

In a preferred embodiment, in formula I, R₅ is selected from H, an optionally substituted C₁₋₆ alkyl, an optionally substituted C₂₋₆ alkenyl, and an optionally substituted C₂₋₆ alkynyl, and preferably, R₅ is selected from H, and C₁₋₆ alkyl.

In a preferred embodiment, the compound of formula I has the following structure: 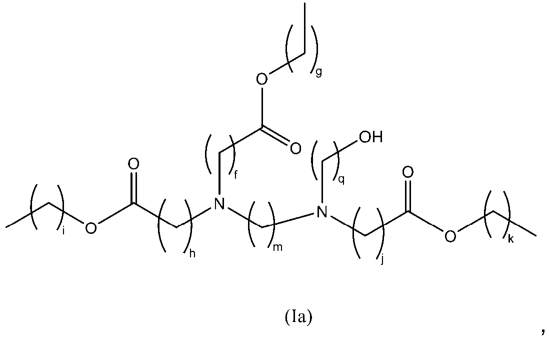 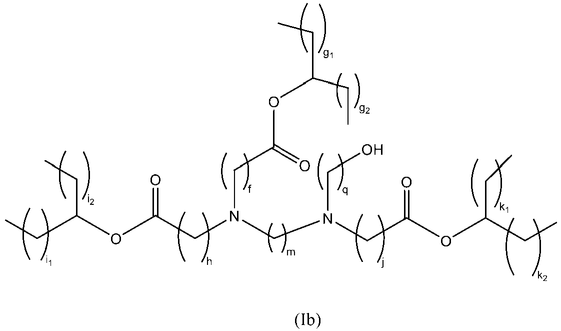 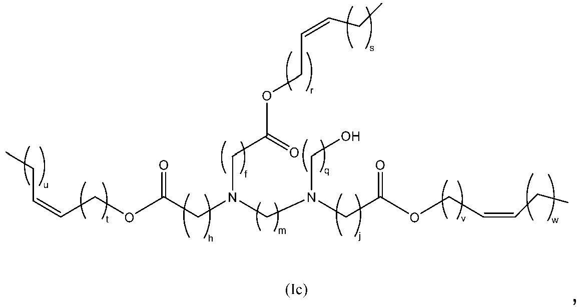 or 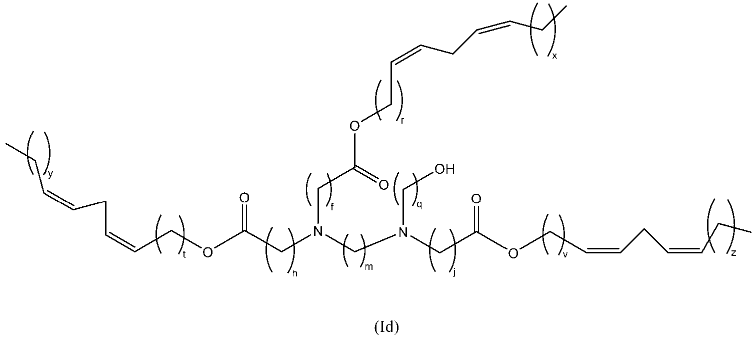 wherein, f, g, h, i, j, k, g1, g2, i1, i2, k1, k2, r, s, t, u, v, w, x, y, and z are each independently an integer from 1 to 24.

In some embodiments, f, h, and j are each independently an integer from 2 to 24, preferably an integer from 3 to 15, and more preferably an integer from 3 to 11.

In some embodiments, g, g1, g2, i, i1, i2, k, k1, and k2 are each independently an integer from 1 to 15, and preferably an integer from 3 to 15.

In some embodiments, r, s, t, u, v, w, x, y, and z are each independently an integer from 1 to 15, and preferably an integer from 1 to 10.

In some embodiments, q is an integer from 1 to 6, preferably an integer from 2 to 5, and more preferably an integer from 2 to 4.

In some embodiments, f, h, and j are the same integer, g, i, and k are the same integer, g1, g2, i1, i2, k1, and k2 are the same integer, r, t, and v are the same integer, s, u, and w are the same integer, and x, y, and z are the same integer.

In formulas I, la, Ib, Ic, and Id, m is an integer from 3 to 6, such as 3, 4, 5, and 6.

In some embodiments, the compound of formula I described in the present invention is a compound of formula la, wherein, i and k are each independently an integer from 1 to 15, preferably each independently an integer from 3 to 15, and preferably, i and k are the same integer; h and j are each independently an integer from 2 to 11, preferably each independently an integer from 3 to 7, and preferably, h and j are the same integer; m is 3, 4, 5, or 6, preferably 3 or 4; f is an integer from 3 to 11, preferably an integer from 3 to 6; g is an integer from 3 to 15, preferably an integer from 6 to 12; q is an integer from 2 to 5.

In some embodiments, the compound of formula I described in the present invention is a compound of formula la, wherein, i, k, and g are each independently an integer from 1 to 15, preferably each independently an integer from 3 to 15, more preferably each independently an integer from 8 to 12, and preferably, i, k, and g are the same integer; f, h, and j are each independently an integer from 2 to 11, preferably each independently an integer from 3 to 7, and preferably, f, h, and j are the same integer; m is 3, 4, 5, or 6, preferably 3 or 4; q is an integer from 2 to 5, preferably 2 or 3.

In some embodiments, the compound of formula I described in the present invention is a compound of formula Id, wherein, h and j are each independently an integer from 2 to 11, preferably each independently an integer from 3 to 8, and preferably, h and j are the same integer; m is 3, 4, 5, or 6, preferably 3 or 4; t and v are each independently an integer from 1 to 10, preferably each independently an integer from 5 to 10, and preferably, t and v are the same integer; y and z are each independently an integer from 1 to 10, preferably each independently an integer from 3 to 8, and preferably, y and z are the same integer; f is an integer from 3 to 11, preferably an integer from 3 to 8; r is an integer from 1 to 10, preferably an integer from 5 to 10; x is an integer from 1 to 10, preferably an integer from 3 to 8; q is an integer from 2 to 5.

In some embodiments, the compound of formula I described in the present invention is a compound of formula Id, wherein, f, h, and j are each independently an integer from 2 to 11, preferably each independently an integer from 3 to 7, and preferably, f, h, and j are the same integer; m is 3, 4, 5, or 6, preferably 3 or 4; t, r, and v are each independently an integer from 1 to 10, preferably each independently an integer from 6 to 10, and preferably, t, r, and v are the same integer; x, y, and z are each independently an integer from 1 to 10, preferably each independently an integer from 3 to 7, and preferably, x, y, and z are the same integer; q is an integer from 2 to 5.

### III. Lipid Nanoparticle

The compound of formula I can be used for preparing a lipid nanoparticle for delivering a nucleic acid therapeutic agent or active agents *in vivo* and *in vitro.*

In addition to the compounds of formula I of the present invention, the lipid nanoparticle of the present invention may also contain one or more auxiliary lipid molecules, one or more cholesterol or cholesterol derivatives and/or one or more polymer conjugated lipid molecules.

Preferably, the auxiliary lipid molecule is a neutral lipid molecule, preferably selected from: DSPC, DPPC, DMPC, DOPC, POPC, DOPE and SM. In a preferred embodiment, the lipid nanoparticle comprises DOPE.

Preferably, the cholesterol derivative is cholesterol (chol for short, CAS: 57-88-5).

Preferably, in the polymer-conjugated lipid molecule, the polymer is polyethylene glycol. In a preferred embodiment, the polymer-conjugated lipid molecule is selected from PEG-DAG, PEG-PE, PEG-S-DAG, PEG-DSPE, PEG-cer and PEG dialkoxypropyl carbamate. Preferably, the lipid nanoparticle contains PEG2000-DSPE.

In a preferred embodiment, in the lipid nanoparticle, when contained, a molar ratio of the compound of formula I, stereoisomer, racemate or pharmaceutically acceptable salt thereof to the auxiliary lipid molecule, the cholesterol or cholesterol derivative, and the polymer-conjugated lipid molecule is (60 to 5):(60 to 5):(50 to 5):(10 to 1), preferably (60 to 30):(30 to 10):(50 to 30):(5 to 1).

The average particle size of the lipid nanoparticle of the present invention can generally be 50 nm to 200 nm, preferably 80 nm to 120 nm.

The lipid nanoparticle of the present invention can be used to express a protein encoded by an mRNA, wherein the protein is a protein with therapeutic, preventive, or improved physiological functions of an organism, including an antigen, an antibody, and a protein with biological functions known in the art; or used to upregulate endogenous protein expression by delivering an miRNA inhibitor targeting a specific miRNA or regulating a set of miRNAs targeting one or several mRNAs; or used to downregulate (such as silence) the protein and/or mRNA levels of a target gene; or used for delivering an mRNA and a plasmid to express a transgene; or used to induce pharmacological effects generated by protein expression, such as increasing red blood cell production by delivering an appropriate erythropoietin mRNA, or protecting against infection by delivering an mRNA encoding an antigen or antibody of interest.

In a preferred embodiment, the lipid nanoparticle of the present invention contains a therapeutic agent or an active agent, preferably, the therapeutic agent or active agent is a nucleic acid therapeutic agent or active agent. More preferably, the nucleic acid therapeutic agent or active agent is selected from: messenger RNA (mRNA), antisense oligonucleotide (ASO), small interfering RNA (siRNA), microRNA (miRNA), self-replicating RNA (saRNA), small guide RNA (sgRNA), ribozyme and aptamer.

The lipid nanoparticle of the present invention can be prepared by conventional preparation methods in the art. For example, compound I is mixed with the auxiliary lipid molecule, the cholesterol and the polymer-conjugated lipid molecule according to a certain molar ratio and dissolved in ethanol to obtain an ethanol lipid solution. The mRNA was dissolved in citrate buffer to obtain an aqueous mRNA solution. The ethanol lipid solution and the mRNA aqueous solution were mixed in a certain volume ratio using a microfluidic device. The medium was replaced by ultrafiltration, ethanol was removed and volumed with Dulbecco's phosphate buffered saline (DPBS). Finally, the lipid nanoparticle was filtered through a 0.2 µm sterile filter to obtain an LNP preparation using an ionizable lipid.

### IV. Composition

The present invention also provides a composition containing the compound of formula I of the present invention. In some embodiments, the composition is a pharmaceutical composition, which contains the compound of formula I of the present invention, a therapeutic agent or an active agent, and one or more auxiliary lipid molecules, one or more cholesterol or cholesterol derivatives and/or one or more polymer-conjugated lipid molecules.

Preferably, the auxiliary lipid molecule is a neutral lipid molecule, preferably selected from: DSPC, DPPC, DMPC, DOPC, POPC, DOPE and SM. In a preferred embodiment, the lipid nanoparticle comprises DOPE or DSPC.

Preferably, the cholesterol derivative is cholesterol.

Preferably, in the polymer-conjugated lipid molecule, the polymer is polyethylene glycol. In a preferred embodiment, the polymer-conjugated lipid molecule is selected from PEG-DMG, PEG-DAG, PEG-PE, PEG-S-DAG, PEG-DSPE, PEG-cer and PEG dialkoxypropyl carbamate. Preferably, the lipid nanoparticle contains PEG2000-DMG.

Preferably, the therapeutic agent or active agent is a nucleic acid therapeutic agent or active agent. More preferably, the nucleic acid therapeutic agent or active agent is selected from: messenger RNA (mRNA), antisense oligonucleotide (ASO), small interfering RNA (siRNA), microRNA (miRNA), self-replicating RNA (saRNA), small guide RNA (sgRNA), ribozyme and aptamer.

Preferably, the pharmaceutical composition contains a therapeutically or prophylactically effective amount of the lipid nanoparticle according to any embodiment of the present invention and a pharmaceutically acceptable carrier or excipient. In particular, the compound of formula I of the present invention is present in the composition in an amount effective to form a lipid nanoparticle and deliver a therapeutically or prophylactically effective amount of a therapeutic or active agent, e.g., for the treatment of a particular target disease or condition. Appropriate concentrations and dosages can be readily determined by those skilled in the art.

The pharmaceutical composition of the present invention can be formulated into preparations in solid, semi-solid, liquid or gaseous form, such as tablets, capsules, powders, granules, ointments, solutions, suspensions, suppositories, injections, inhalants, gels, microspheres and aerosols. Routes of administration of such pharmaceutical compositions include, but are not limited to, oral, topical, transdermal, inhalation, intraperitoneal, sublingual, buccal, rectal, vaginal and intranasal. The term "intraperitoneal" as used herein includes subcutaneous injection, intravenous, intramuscular, intradermal, intrasternal injection or infusion.

An appropriate pharmaceutically acceptable carrier and excipient can be selected according to specific dosage forms and administration routes. For example, as a solid composition for oral administration, the pharmaceutical composition can be formulated into powders, granules, compressed tablets, pills, capsules, chewing gum, flakes and the like. Such solid compositions generally contain one or more inert diluents or edible carriers. In addition, one or more of the following may be present: binders such as carboxymethylcellulose, ethylcellulose, microcrystalline cellulose, tragacanth or gelatin; excipients such as starch, lactose or dextrin, disintegrants such as alginic acid, sodium alginate, Primogel, corn starch, etc.; lubricants such as magnesium stearate or Sterotex; glidants such as colloidal silicon dioxide; sweeteners such as sucrose or saccharin; flavoring agents such as peppermint , methyl salicylate, or orange flavor; and coloring agent.

When the pharmaceutical composition is in the form of a capsule such as a gelatin capsule, it may contain, in addition to the above materials, a liquid carrier such as polyethylene glycol or oil.

The pharmaceutical composition of the present invention can be prepared by methods well known in the field of pharmacy. For example, a pharmaceutical composition intended to be administered by injection can be prepared by combining the lipid nanoparticle of the present invention with sterile distilled water or other carriers to form a solution.

The pharmaceutical compositions of the present invention are administered in a therapeutically effective amount which will vary according to a number of factors including the activity of the particular therapeutic agent employed; the metabolic stability and duration of action of the therapeutic agent; the patient's age, weight, overall health, sex, and diet; route and timing of administration; excretion rates; drug combinations; severity of a particular disorder or condition; and subjects being treated.

### V. Methods of Treatment and Uses

The lipid nanoparticle and pharmaceutical composition of the present invention can be used to deliver a therapeutic agent or active agent, such as a nucleic acid, *in vivo* and *in vitro,* for the treatment or prevention of a disease or condition in a subject.

Therefore, the present invention provides use of the compound of formula I according to any embodiment of the present invention in manufacture of a lipid nanoparticle or pharmaceutical composition for treatment or prevention of a disease or condition in a subject, use of the lipid nanoparticle according to any embodiment of the present invention in manufacture of drugs for treatment or prevention of a disease or condition in a subject, and a compound of formula I, lipid nanoparticle or pharmaceutical composition according to any embodiment of the present invention for treatment or prevention of a disease or condition in a subject.

The present invention also provides a method of treating or preventing a disease or condition in a subject, comprising administering a therapeutically or prophylactically effective amount of a therapeutic or active agent, such as a nucleic acid, to a subject in need, wherein the therapeutic or active agent is encapsulated within the lipid nanoparticle containing the compound of formula I according to any of the embodiments herein; or comprising administering a therapeutically or prophylactically effective amount of the pharmaceutical composition according to any of the embodiments herein to a subject in need.

The diseases and conditions described herein can be various diseases and conditions known in the art that are suitable for the treatment and prevention by the therapeutic agent or active agent (such as the nucleic acid) described herein, and depend on the specific biological functions of the therapeutic agent or active agent (such as the nucleic acid) delivered by the lipid nanoparticle. In some embodiments, the pharmaceutical composition is a vaccine, and the method includes immunizing an individual so that the individual is immune to a corresponding disease or condition. In some embodiments, the disease or condition includes but is not limited to infectious diseases, such as infectious diseases caused by viruses and/or bacteria. Exemplary diseases or conditions include infection caused by influenza virus, hepatitis B virus, hepatitis C virus, COVID-19 virus, etc. In some embodiments, the diseases or conditions also include, for example, tumors, including solid tumors and hematologic tumors, various inflammatory conditions, and the like.

Appropriate routes and methods of administration herein are well known in the art and described above, such as including but not limited to oral, topical, transdermal, inhalation, intraperitoneal, sublingual, buccal, rectal, vaginal and intranasal. In some embodiments, the intraperitoneal route of administration includes: subcutaneous injection, intravenous, intramuscular, intradermal, intrasternal injection or infusion.

The present invention also includes the use of a compound of formula I of the present invention for the delivery of a therapeutic agent or active agent, such as a nucleic acid, or for the preparation of a reagent for the delivery of a therapeutic agent or active agent, such as a nucleic acid. The present invention also provides a method of delivering a therapeutic agent or active agent such as a nucleic acid *in vivo* or *in vitro,* the method comprising encapsulating the therapeutic agent or active agent in a lipid nanoparticle made of a compound of formula I, and delivering the therapeutic agent or active agent via the lipid nanoparticle or a composition containing the lipid nanoparticle. The nucleic acid may be as described in any of the embodiments herein. In some embodiments, the therapeutic agent or active agent (such as a nucleic acid) is delivered to a target site of interest (such as a cell, a tissue (diseased tissues such as tumor tissue, etc.), organs) via the lipid nanoparticle or a composition containing the lipid nanoparticle. In some embodiments, the cells are immune cells, including but not limited to T cells, dendritic cells (DC cells) or tumor infiltrating lymphocytes (TIL) and the like.

### VI. The preparation method of the compound of formula I

The compound of formula I of the present invention can be prepared by following general synthetic routes 1 or 2:

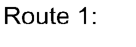

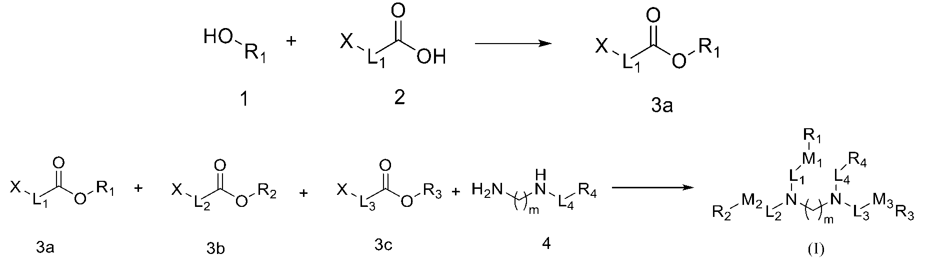

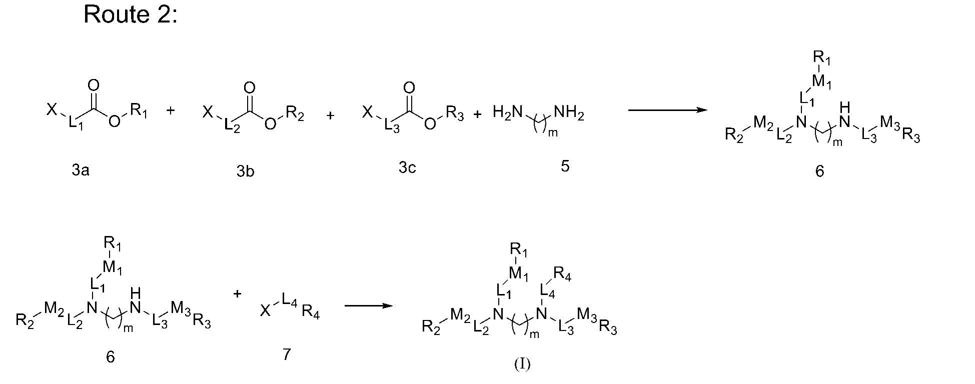

Among them, R₁, R₂, R₃, R₄, L₁, L₂, L₃, L₄, M₁, M₂, M₃, and m are defined as any of the embodiments herein; X can be a halogen such as Br, Cl, etc. Firstly, compound 1 and compound 2 react to form compound 3a, which can be carried out under conditions suitable for condensation. Suitable conditions for condensation include one of the following: ① H₂SO₄; ② EDC·HCl and DMAP; and ③ oxalyl chloride. 3b and 3c can be synthesized using the same method as 3a. Then, 3a, 3b, 3c, and 4 react under a condition suitable for a nucleophilic substitution reaction (such as alkaline) to form the compound of formula I. Alternatively, 3a, 3b, 3c, and 5 can react under a condition suitable for a nucleophilic substitution reaction (such as alkaline) to form compound 6, and then compound 6 and compound 7 can react under a condition suitable for a nucleophilic substitution reaction (such as alkaline) to form the compound of formula I.

The present invention will be further described below in the form of specific examples. It should be understood that these examples are merely illustrative and are not intended to limit the scope of the present invention. Unless otherwise specified, the methods and reagents used in the examples are conventional methods and reagents in the art.

### Examples

### Example I : Synthesis of ionizable lipid molecules

### Example 1

### Diundecyl 8,8'-((3-((2-hydroxyethyl)(8-oxo-8-(undecyloxy)octyl)amino)propyl)azanediyl)dioctanoate

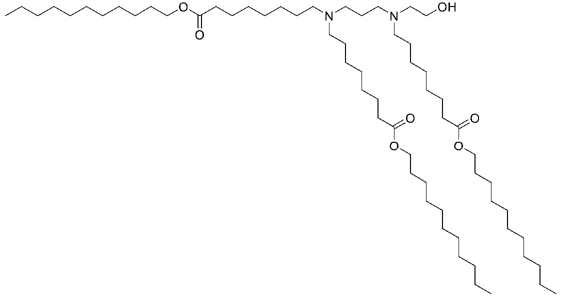

### Step 1: Synthesis of undecyl 8-bromooctanoate

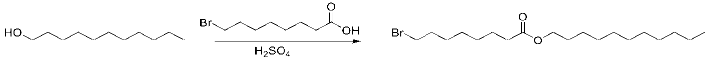

1-undecanol (1.0 g, 5.8 mmol) was mixed with 8-bromooctanoic acid (1.6 g, 7.5 mmol). The mixture was heated to 60 °C, then concentrated sulfuric acid (0.1 mL) was added, and stirred at this temperature for 3 hours. Thin layer chromatography (TLC) showed complete consumption of raw materials, the mixture was mixed directly, and purified by column chromatography with PE:EA=15:1 to obtain 1.8 g of a colorless oily product with a yield of 82.2%.

¹H-NMR (400 MHz, CDCl₃) δ 4.06 (t, J = 6.7 Hz, 2H), 3.40 (t, J = 6.8 Hz, 2H), 2.29 (t, J = 7.5 Hz, 2H), 1.90 - 1.80 (m, 2H), 1.67 - 1.58 (m, 4H), 1.48 - 1.39 (m, 2H), 1.37 - 1.31 (m, 7H), 1.30 - 1.27 (m, 4H), 1.26 (s, 9H), 0.88 (t, J = 6.7 Hz, 3H).

### Step 2: Synthesis of diundecyl 8,8'-((3-((2-hydroxyethyl)(8-oxo-8-(undecyloxy)octyl)amino)propyl)azanediyl)dioctanoate

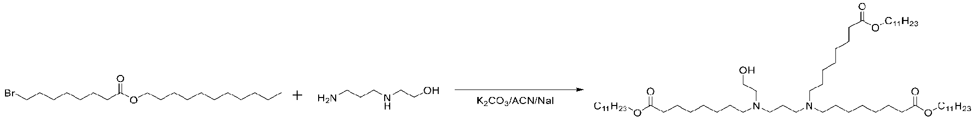

2-((3-aminopropyl)amino)ethane-1-ol (135 mg, 1.1 mmol) was dissolved in acetonitrile (16 mL), and undecyl 8-bromooctanoate (1.4 g, 3.8 mmol), K₂CO₃ (0.7 g, 5.1 mmol), and KI (13 mg) were added respectively, and stirred at 85 °C for 16 hours. An appropriate amount of water was added to the mixture, then EA (50 mL * 3) was added for extraction. The combined organic phases were dried and concentrated under reduced pressure. The crude product was purified by column chromatography with DCM:MeOH=50:1 to obtain 0.5 g of a colorless oily product with a yield of 43.5%.

ESI-MS *m*/*z:* cald for C₆₂H₁₂₃N₂O₇ [M+H]⁺: 1007.9, found 1007.9.

¹H-NMR (400 MHz, CDCl₃) δ 4.05 (t, J = 6.7 Hz, 6H), 3.57 - 3.50 (m, 2H), 2.58 - 2.42 (m, 5H), 2.42 - 2.35 (m, 6H), 2.28 (t, J = 7.5 Hz, 6H), 1.65 - 1.52 (m, 13H), 1.49 - 1.41 (m, 5H), 1.40 - 1.33 (m, 5H), 1.33 - 1.23 (m, 64H), 0.88 (t, J = 6.8 Hz, 9H).

### Example 2

### Di(heptadecane-9-yl)8,8'-((3-((8-(heptadecane-9-oxy)-8-oxooctyl)(2-hydroxyethyl)amino)propyl)azanediyl)dioctanoate

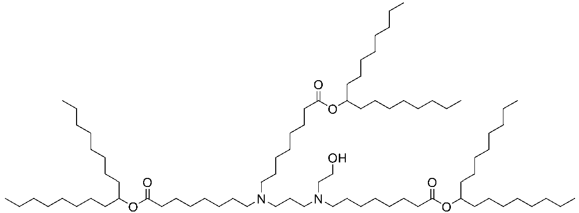

### Step 1: Synthesis of heptadecan-9-yl 8-bromooctanoate

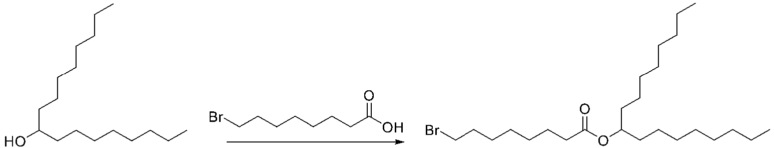

Heptadecane-9-ol (9.0 g, 35.1 mmol) and 8-bromooctanoic acid (8.6 g, 38.6 mmol) were dissolved in dichloromethane (DCM) (100 mL), EDC·HCl (13.5 g, 70.2 mmol), DMAP (8.6 g, 70.2 mmol), and triethylamine (14.2 g, 140.4 mmol) were added, and stirred at room temperature for 16 hours. TLC showed complete consumption of raw materials, an appropriate amount of water was added, then DCM (100 mL x 3) was added for extraction. The combined organic phases were dried and concentrated under reduced pressure. The crude product was purified by column chromatography with PE:EA=20:1 to obtain 5.6 g of a light yellow oily product with a yield of 34.3%.

¹H-NMR (400 MHz, CDCl₃) δ 4.92 - 4.80 (m, 1H), 3.40 (t, J = 6.8 Hz, 2H), 2.28 (t, J = 7.4 Hz, 2H), 1.91 - 1.72 (m, 2H), 1.68 - 1.59 (m, 2H), 1.54 - 1.47 (m, 4H), 1.46 - 1.42 (m, 1H), 1.42 - 1.27 (m, 12H), 1.26 (s, 17H), 0.88 (t, J = 6.7 Hz, 6H).

### Step 2: di(heptadecane-9-yl)8,8'-((3-((8-(heptadecane-9-oxy)-8-oxooctyl)(2-hydroxyethyl)amino)propyl)azanediyl)dioctanoate

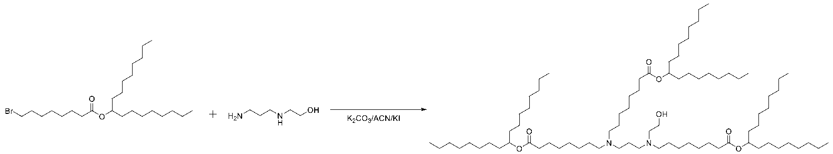

2-((3-aminopropyl)amino)ethane-1-ol (60 mg, 0.5 mmol) was dissolved in acetonitrile (6 mL), and heptadecan-9-yl 8-bromooctanoate (1.4 g, 3.0 mmol), K₂CO₃ (0.4 g, 3.0 mmol), and KI (10 mg) were added respectively, and stirred at 85 °C for 16 hours. An appropriate amount of water was added to the mixture, then EA (50 mL * 3) was added for extraction. The combined organic phases were dried and concentrated under reduced pressure. The crude product was purified by column chromatography with DCM:MeOH=40:1 to obtain 73 mg of a colorless oily product with a yield of 11.6 %.

ESI-MS *m*/*z:* cald for C₈₀H₁₅₉N₂O₇ [M+H]⁺: 1260.1, found 1260.1.

¹H-NMR (400 MHz, CDCl₃) δ 4.91 - 4.80 (m, 3H), 3.54 (t, J = 5.2 Hz, 2H), 2.57 - 2.35 (m, 14H), 2.27 (t, J = 7.5 Hz, 8H), 1.67 - 1.55 (m, 10H), 1.50 (d, J = 6.1 Hz, 14H), 1.45 - 1.36 (m, 11H), 1.31 - 1.25 (m, 78H), 0.87 (t, J = 6.7 Hz, 18H).

### Example 3

### Di((E)-3,7-dimethyloctan-2,6-dien-1-yl)8,8'-((3-((8-(((E)-3,7-dimethyloctan-2,6-dien-1-yl)oxo)-8-oxooctyl)(2-hydroxyethyl)amino)propyl)azanediyl)dioctanoate

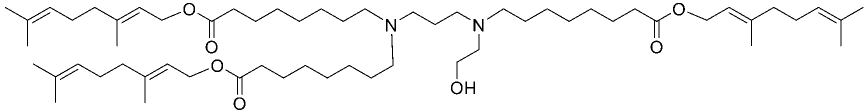

### Step 1: Synthesis of (E)-3,7-dimethyloctan-2,6-dien-1-yl 8-bromooctanoate

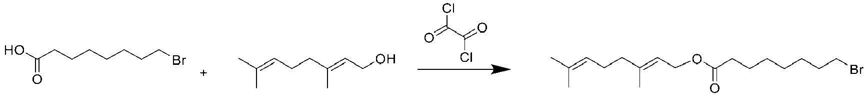

Under nitrogen protection, 8-bromooctanoic acid (5 g, 22.41 mmol) was dissolved in 100 mL of anhydrous DCM. Oxalyl chloride (3.41 g, 28.89 mmol, 1.2 eq) was added to the solution in an ice bath. Then the mixture was reacted at 40 °C for one hour, concentrated under reduced pressure , and dissolved again in 100 mL of anhydrous DCM. (E)-3,7-dimethyloctan-2,6-dien-1-ol (2.77 g, 7.93 mmol, 0.8 eq) was dissolved in 20 mL of Et3N, and the mixture was added dropwise to the aforementioned reaction solution, and stirred at room temperature for 1 hour. After the reaction was complete, the solution was separated, the organic phase was washed with saturated saline solution, dried with anhydrous sodium sulfate, and then concentrated under reduced pressure and purified by column chromatography to obtain 2.3 g of a target substance with a yield of 28.6%.

### Step 2: Synthesis of di((E)-3,7-dimethyloctan-2,6-dien-1-yl)8,8'-((3-((8-(((E)-3,7-dimethyloctan-2,6-dien-1-yl)oxo)-8-oxooctyl)(2-hydroxyethyl)amino)propyl)azanediyl)dioctanoate

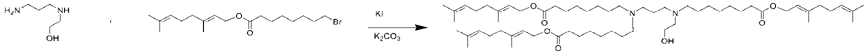

2-((3-aminopropyl)amino)ethane-1-ol (0.1 g, 0.846 mmol) was dissolved in 20 mL of anhydrous acetonitrile, and (E)-3,7-dimethyloctan-2,6-dien-1-yl 8-bromooctanoate (1.06 g, 2.96 mmol), K₂CO₃ (0.584 g, 4.23 mmol, 5 eq), and KI (14 mg, 0.084 mmol, 0.1 eq) were added and heated to 85 °C overnight. After the reaction was complete, 20 mL of DCM was added and filtered to remove insoluble substances. The mixture was washed with 20 mL of saturated saline solution, dried with anhydrous sodium sulfate, and then concentrated under reduced pressure and purified by column chromatography. Finally, 0.54 g of a target substance was obtained with a yield of 67%.

### ESI-MS m/z: cald for C₅₉H₁₀₅N₂O₇ [M+H]⁺: 953.8, found 953.8.

¹H NMR (400 MHz, CDCl₃) δ 5.40 - 5.32 (m, 3H), 5.15 - 5.07 (m, 3H), 4.61 (d, J = 7.1 Hz, 6H), 3.77 - 3.64 (m, 4H), 2.91 - 2.87 (m, 1H), 2.83 - 2.75 (m, 4H), 2.73 - 2.64 (m, 4H), 2.59 - 2.53 (m, 2H), 2.37 - 2.28 (m, 7H), 2.17 - 2.03 (m, 12H), 1.92 - 1.85 (m, 2H), 1.72 (d, J = 8.0 Hz, 17H), 1.68 - 1.61 (m, 17H), 1.51 (d, J = 8.5 Hz, 2H), 1.35 (d, J = 8.5 Hz, 16H), 1.31 - 1.24 (m, 4H).

### Example 4

### Diheptyl 8,8'-((3-((8-(heptyloxy)-8-oxooctyl)(2-hydroxyethyl)amino)propyl)azanediyl)dioctanoate

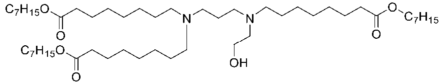

### Step 1: Synthesis of heptyl 8-bromooctanoate

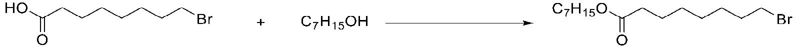

Under nitrogen protection, 8-bromooctanoic acid (5 g, 22.41 mmol) was dissolved in anhydrous DCM (100 mL). Oxalyl chloride (3.41 g, 28.89 mmol, 1.2 eq) was added to the solution in an ice bath. Then the mixture was reacted at 40 °C for one hour, concentrated under reduced pressure , and dissolved again in 100 mL of anhydrous DCM. N-heptanol (2.08 g, 17.93 mmol, 0.8 eq) was dissolved in 20 mL of Et3N, added dropwise to the aforementioned reaction solution, and stirred at room temperature for 1 hour. After the reaction was complete, the solution was separated, the organic phase was washed with saturated saline solution, dried with anhydrous sodium sulfate, and then concentrated under reduced pressure and purified by column chromatography. 1.39 g of a target substance was obtained with a yield of 19.3%.

### Step 2: Synthesis of diheptyl 8,8'-((3-((8-(heptyloxy)-8-oxooctyl)(2-hydroxyethyl)amino)propyl)azanediyl)dioctanoate:

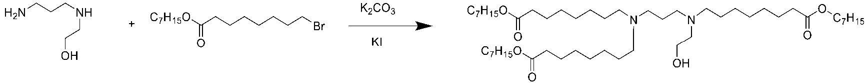

2-((3-aminopropyl)amino)ethane-1-ol (0.1 g, 0.846 mmol) was dissolved in 20 mL of anhydrous acetonitrile. Heptyl 8-bromooctanoate (0.95g, 2.96 mmol, 3.5 eq), K₂CO₃ (0.584 g, 4.23 mmol, 5 eq), and KI (14 mg, 0.084 mmol, 0.1 eq) were added and heated to 85 °C overnight. After the reaction was complete, 20 mL of DCM was added and filtered to remove insoluble substances. The mixture was washed with 20 mL of saturated saline solution, dried with anhydrous sodium sulfate, and then concentrated under reduced pressure and purified by column chromatography. Finally, 0.21 g of a target substance was obtained with a yield of 29.6%.

ESI-MS m/z: cald for C₅₀H₉₉N₂O₇ [M+H]⁺: 839.7, found 839.7.

¹H NMR (400 MHz, CDCl₃) δ 4.08 (t, J = 6.8 Hz, 6H), 3.80 - 3.69 (m, 4H), 2.93 (t, J = 7.3 Hz, 2H), 2.83 (t, J = 8.2 Hz, 4H), 2.75 - 2.65 (m, 4H), 2.61 - 2.53 (m, 2H), 2.37 - 2.30 (m, 5H), 1.64 (t, J = 6.9 Hz, 16H), 1.40 - 1.25 (m, 42H), 0.95 - 0.87 (m, 9H).

### Example 5

### Dipentyl 8,8'-((3-((2-hydroxyethyl)(8-oxo-8-(pentyloxy)octyl)amino)propyl)azanediyl)dioctanoate

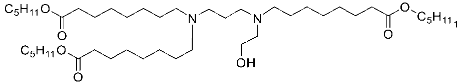

### Step 1: Synthesis of pentyl 8-bromooctanoate

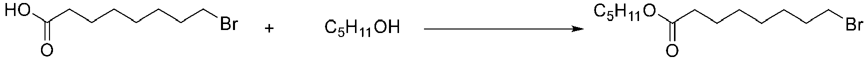

Under nitrogen protection, bromooctanoic acid (5 g, 22.41 mmol) was dissolved in 100 mL of anhydrous DCM. Oxalyl chloride (3.41 g, 28.89 mmol, 1.2 eq) was added to the solution in an ice bath. Then the mixture was reacted at 40 °C for one hour, concentrated under reduced pressure, and dissolved again in 100 mL of anhydrous DCM. Amyl alcohol (1.58 g, 17.93 mmol, 0.8 eq) was dissolved in 20 mL of Et3N, added dropwise to the aforementioned reaction solution, and stirred at room temperature for 1 hour. After the reaction was complete, the solution was separated, the organic phase was washed with saturated saline solution, dried with anhydrous sodium sulfate, and then concentrated under reduced pressure and purified by column chromatography. 1.39 g of a target substance was obtained with a yield of 19.3%.

### Step 2: Synthesis of dipentyl 8,8'-((3-((2-hydroxyethyl)(8-oxo-8-(pentyloxy)octyl)amino)propyl)azanediyl)dioctanoate:

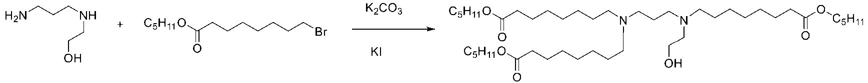

2-((3-aminopropyl)amino)ethane-1-ol (0.1 g, 0.846 mmol) was dissolved in 20 mL of anhydrous acetonitrile. Pentyl 8-bromooctanoate (0.95g, 2.96 mmol, 3.5 eq), K₂CO₃ (0.584 g, 4.23 mmol, 5 eq), and KI (14 mg, 0.084 mmol, 0.1 eq) were added and heated to 85 °C overnight. After the reaction was complete, 20 mL of DCM was added and filtered to remove insoluble substances. The mixture was washed with 20 mL of saturated saline solution, dried with anhydrous sodium sulfate, and then concentrated under reduced pressure and purified by column chromatography. Finally, 0.21 g of a target substance was obtained with a yield of 29.6%.

ESI-MS *m*/*z:* cald for C₄₄H₈₇N₂O₇ [M+H]⁺: 755.6, found 755.6.

¹H NMR (400 MHz, CDCl₃) δ 4.12 - 4.02 (m, 6H), 3.64 - 3.57 (m, 2H), 2.70 (s, 1H), 2.66 - 2.52 (m, 8H), 2.52 - 2.45 (m, 2H), 2.33 - 2.25 (m, 6H), 1.82 - 1.72 (m, 2H), 1.67 - 1.50 (m, 16H), 1.50 - 1.40 (m, 2H), 1.39 - 1.30 (m, 27H), 1.30 - 1.22 (m, 4H), 0.96 - 0.87 (m, 9H).

### Example 6

### Di((Z)-non-2-en-1-yl)8,8'-((3-((2-hydroxyethyl)(8-(((Z)-non-2-en-1-yl)oxo)-8-oxooctyl)amino)propyl)azanediyl)dioctanoate

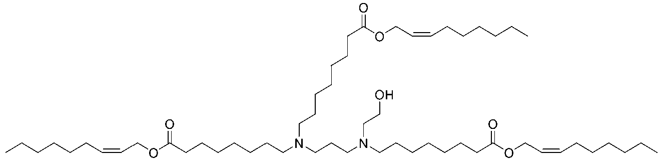

### Step 1: Synthesis of (Z)-non-2-en-1-yl 8-bromooctanoate

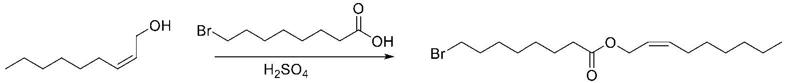

Cis-2-nonen-1-ol (1.0 g, 7.0 mmol) was mixed with 8-bromooctanoic acid (1.9 g, 8.4 mmol). The mixture was heated to 60 °C, concentrated sulfuric acid (0.1 mL) was added, and stirred at this temperature for 5 hours. TLC showed complete consumption of raw materials, the mixture was mixed directly, and purified by column chromatography with PE:EA=50:1 to obtain 2.2 g of a colorless oily product with a yield of 91.7%.

¹H-NMR (400 MHz, CDCl₃) δ 5.70 - 5.59 (m, 1H), 5.58 - 5.46 (m, 1H), 3.43 - 3.36 (m, 2H), 3.40 (t, J = 6.8 Hz, 2H), 2.31 (t, J = 7.5 Hz, 2H), 2.15 - 2.05 (m, 2H), 1.90 - 1.80 (m, 2H), 1.68 - 1.59 (m, 2H), 1.47 - 1.39 (m, 2H), 1.38 - 1.25 (m, 12H), 0.92 - 0.84 (m, 3H).

### Step 2: Synthesis of di((Z)-non-2-en-1-yl)8,8'-((3-((2-hydroxyethyl)(8-(((Z)-non-2-en-1-yl)oxo)-8-oxooctyl)amino)propyl)azanediyl)dioctanoate

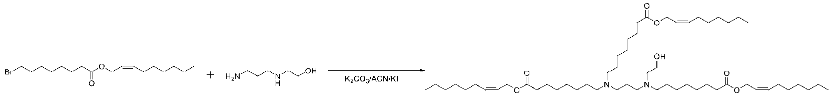

N-(2-hydroxyethyl)-1,3-propanediamine (60 mg, 0.5 mmol) was dissolved in acetonitrile (6 mL), and (Z)-non-2-en-1-yl 8-bromooctanoate (0.6 g, 1.7 mmol), K₂CO₃ (0.3 g, 2.3 mmol), and Kl (10 mg) were added respectively, and stirred at 85 °C for 16 hours. An appropriate amount of water was added to the mixture, then EA (50 mL * 3) was added for extraction. The combined organic phases were dried and concentrated under reduced pressure. The crude product was purified by column chromatography with DCM:MeOH=40:1 to obtain 180 mg of a colorless oily product with a yield of 38.5 %.

### ESI-MS m/z: cald for C₅₆H₁₀₅N₂O₇ [M+H]⁺: 917.5, found 917.5.

¹H-NMR (400 MHz, CDCl₃) δ 5.68 - 5.58 (m, 3H), 5.56 - 5.46 (m, 3H), 4.61 (d, J = 6.8 Hz, 6H), 3.53 (t, J = 5.2 Hz, 2H), 2.57 - 2.36 (m, 10H), 2.35 (d, J = 5.1 Hz, 2H), 2.29 (t, J = 7.5 Hz, 6H), 2.14 - 2.04 (m, 6H), 1.66 - 1.50 (m, 8H), 1.44 - 1.24 (m, 48H), 0.91 - 0.83 (m, 9H).

### Example 7

### Di((4Z,7Z)-dec-4,7-dien-1-yl)8,8'-((3-((8-(((4Z,7Z)-dec-4,7-dien-1-yl)oxo)-8-oxooctyl)(2-hydroxyethyl)amino)propyl)azanediyl)dioctanoate

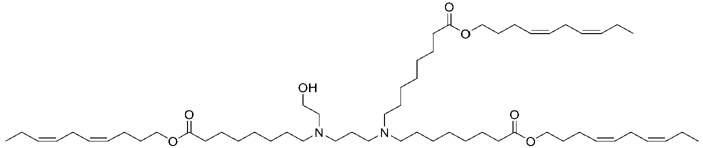

### Step 1: Synthesis of (4Z,7Z)-dec-4,7-dien-1-yl 8-bromooctanoate

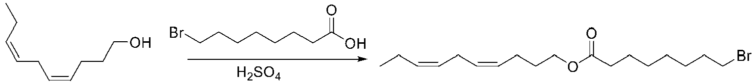

(4Z, 7Z)-decadienol (0.9 g, 5.8 mmol) was mixed with 8-bromooctanoic acid (1.6 g, 7.0 mmol). The mixture was heated to 60 °C, concentrated sulfuric acid (0.1 mL) was added, and stirred at this temperature for 4 hours. TLC showed complete consumption of raw materials, the mixture was mixed directly, and purified by column chromatography with PE: EA=30:1 to obtain 1.3 g of a colorless oily product with a yield of 61.9%.

¹H-NMR (400 MHz, CDCl₃) δ 5.47 - 5.23 (m, 4H), 4.07 (t, J = 6.6 Hz, 2H), 3.40 (t, J = 6.8 Hz, 2H), 2.77 (t, J = 6.6 Hz, 2H), 2.30 (t, J = 7.5 Hz, 2H), 2.19 - 2.01 (m, 4H), 1.90 - 1.80 (m, 2H), 1.74 - 1.58 (m, 4H), 1.48 - 1.39 (m, 2H), 1.39 - 1.23 (m, 4H), 0.97 (t, J = 7.5 Hz, 3H).

### Step 2: Synthesis of di((4Z,7Z)-dec-4,7-dien-1-yl)8,8'-((3-((8-(((4Z,7Z)-dec-4,7-dien-1-yl)oxo)-8-oxooctyl)(2-hydroxyethyl)amino)propyl)azanediyl)dioctanoate

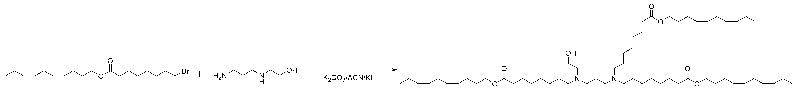

N-(2-hydroxyethyl)-1,3-propanediamine (120 mg, 1.0 mmol) was dissolved in acetonitrile (8 mL), and (4Z,7Z)-dec-4,7-dien-1-yl 8-bromooctanoate (1.3 g, 3.6 mmol), K₂CO₃ (0.6 g, 4.6 mmol), and KI (10 mg) were added respectively, and stirred at 85 °C for 16 hours. An appropriate amount of water was added to the mixture, then DCM (50 mL * 3) was added for extraction. The combined organic phases were dried and concentrated under reduced pressure. The crude product was purified by column chromatography with DCM:MeOH=30:1 to obtain 340 mg of a colorless oily product with a yield of 35.0 %.

ESI-MS *m*/*z:* cald for C59H105N2O7 [M+H]⁺: 953.5, found 953.5.

¹H-NMR (400 MHz, CDCl₃) δ 5.48 - 5.18 (m, 12H), 4.07 (t, J = 6.6 Hz, 6H), 3.57 - 3.50 (m, 2H), 2.77 (t, J = 6.6 Hz, 6H), 2.57 - 2.25 (m, 19H), 2.17 - 1.97 (m, 13H), 1.73 - 1.65 (m, 7H), 1.63 - 1.53 (m, 4H), 1.41 (s, 5H), 1.35 - 1.23 (m, 21H), 0.97 (t, J = 7.5 Hz, 9H).

### Example 8

### Di((Z)-non-6-en-1-yl)8,8'-((3-((2-hydroxyethyl)(8-(((Z)-non-6-en-1-yl)oxo)-8-oxooctyl)amino)propyl)azanediyl)dioctanoate

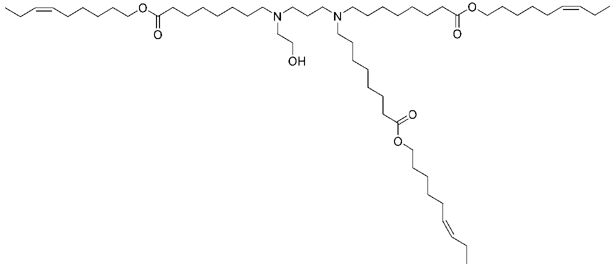

### Step 1: Synthesis of (Z)-non-6-en-1-yl 8-bromooctanoate

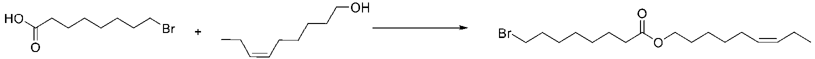

Under nitrogen protection, bromooctanoic acid (5 g, 22.41 mmol) was dissolved in 100 mL of anhydrous DCM. Oxalyl chloride (3.41 g, 28.89 mmol, 1.2 eq) was added to the solution in an ice bath. Then the mixture was reacted at 40 °C for one hour, and then concentrated under reduced pressure and purified through the column, and dissolved again in 100 mL of anhydrous DCM. (Z)-non-6-en-1-ol (2.55 g, 17.93 mmol, 0.8 eq) was dissolved in 20 mL of Et3N, added dropwise to the aforementioned reaction solution, and stirred at room temperature for 1 hour. After the reaction was complete, the solution was separated, the organic phase was washed with saturated saline solution, dried with anhydrous sodium sulfate, and then concentrated under reduced pressure and purified by the column to obtain 2.78 g of a target substance with a yield of 35.7%.

### Step 2: Synthesis of di(Z)-non-6-en-1-yl)8,8'-((3-((2-hydroxyethyl)(8-(((Z)-non-6-en-1-yl)oxo)-8-oxooctyl)amino)propyl)azanediyl)dioctanoate:

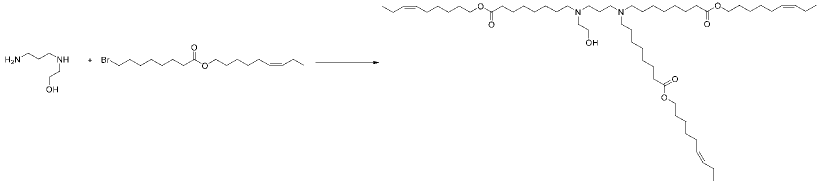

2-((3-aminopropyl)amino)ethane-1-ol (0.15g, 1.27 mmol) was dissolved in 20 mL of anhydrous acetonitrile. (Z)-non-6-en-1-yl 8-bromooctanoate (1.54 g, 4.44 mmol, 3.5 eq), K₂CO₃ (0.877 g, 6.35 mmol, 5 eq), and Kl (0.21 g, 0.127 mmol, 0.1 eq) were added and heated to 85 °C overnight. After the reaction was complete, 20 mL of DCM was added and filtered to remove insoluble substances. The mixture was washed with 20 mL of saturated saline solution, dried with anhydrous sodium sulfate, and then concentrated under reduced pressure and purified by column chromatography. Finally, 0.32 g of a target substance was obtained with a yield of 27.6%.

ESI-MS *m*/*z:* cald for C₅₆H₁₀₅N₂O₇ [M+H]⁺: 917.8, found 917.8.

¹H NMR (400 MHz, CDCl₃) δ 5.46 - 5.29 (m, 6H), 4.09 (t, J = 6.7 Hz, 6H), 3.81 - 3.71 (m, 1H), 3.59 (t, J = 5.2 Hz, 2H), 2.64 - 2.54 (m, 4H), 2.54 - 2.43 (m, 8H), 2.32 (t, J = 7.5 Hz, 6H), 2.13 - 1.99 (m, 13H), 1.69 - 1.59 (m, 13H), 1.50 - 1.47 (m, 3H), 1.45 - 1.38 (m, 12H), 1.36 - 1.25 (m, 21H), 0.99 (t, J = 7.5 Hz, 9H).

### Example 9

### Dinonyl 8,8'-((3-((2-hydroxyethyl)(8-(nonoxy)-8-oxooctyl)amino)propyl)azanediyl)dioctanoate

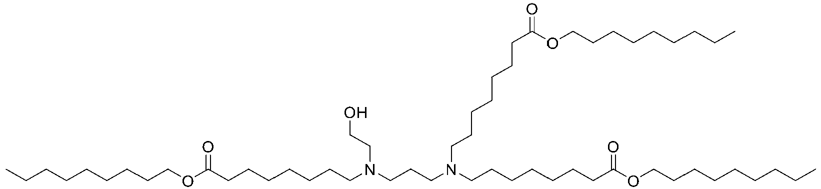

### Step 1: Synthesis of nonyl 8-bromooctanoate

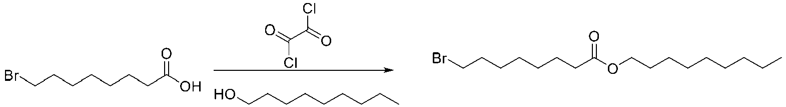

8-bromooctanoic acid (7.23 g, 32.4 mmol) was dissolved in 100 mL of DCM, and oxalyl chloride (4.94 g, 38.89 mmol) was slowly added to the mixture, refluxed for 4 hours, and then concentrated under reduced pressure to obtain 8 g of crude product. Half of the mixture was dissolved in DCM (150 mL) and TEA (3.28 g, 32.4 mmol), 1-nonanol (2.34 g, 16.2 mmol) was added, and stirred at 40 °C for 4 hours. TLC showed complete consumption of raw materials, the mixture was mixed directly, and purified by column chromatography with PE:EA=15:1 to obtain 4.5 g of a colorless oily product with a yield of 79.5%.

### Step 2: Synthesis of dinonyl 8,8'-((3-((2-hydroxyethyl)(8-(nonoxy)-8-oxooctyl)amino)propyl)azanediyl)dioctanoate

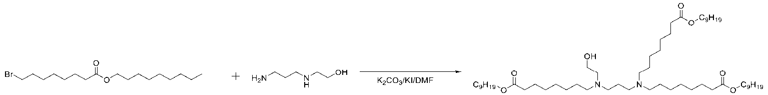

2-((3-aminopropyl)amino)ethane-1-ol (59.1 mg, 0.5 mmol) was dissolved in N,N-dimethylformamide (DMF) (5 mL), and nonyl 8-bromooctanoate (611.36 mg, 1.75 mmol), K₂CO₃ (345.53 mg, 2.5 mmol), and Kl (13 mg) were added respectively, and stirred at 85 °C for 16 hours. An appropriate amount of water was added to the mixture, then EA (50 mL * 3) was added for extraction. The combined organic phases were dried and concentrated under reduced pressure. The crude product was purified by column chromatography with DCM (containing 5% EA):MeOH=50:1 to 5:1 to obtain 0.3 g of a colorless oily product with a yield of 65 %.

ESI-MS *m*/*z:* cald for C₅₆H₁₁₁N₂O₇ [M+H]⁺: 923.7, found 923.7.

¹H NMR (400 MHz, CDCl₃) δ 4.05 (t, J = 6.8 Hz, 6H), 3.67 (t, J = 5.1 Hz, 2H), 3.00-2.84 (m, 3H), 2.84-2.74 (m, 4H), 2.73-2.63 (m, 4H), 2.56 (t, J = 7.8 Hz, 2H), 2.29 (t, J = 7.5 Hz, 6H), 2.02-1.88 (m, 2H), 1.71-1.56 (m, 16H), 1.54-1.45 (m, 2H), 1.42 - 1.13 (m, 54H), 0.88 (t, J = 6.7 Hz, 9H).

### Example 10

### Di((Z)-pent-2-en-1-yl)8,8'-((3-((2-hydroxyethyl)(8-oxo-8-(((Z)-pent-2-en-1-yl)oxo)octyl)amino)propyl)azanediyl)dioctanoate

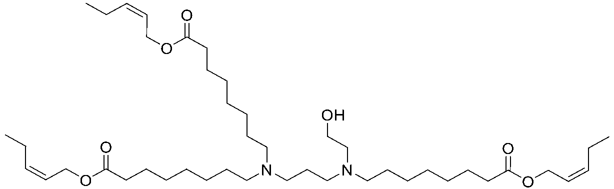

### Step 1: Synthesis of (Z)-pent-2-en-1-yl 8-bromooctanoate

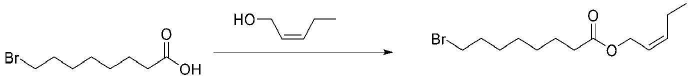

8-bromooctanoic acid (7.23 g, 32.4 mmol) was dissolved in 100 mL of DCM, oxalyl chloride (4.94 g, 38.89 mmol) was slowly added to the mixture, refluxed for 4 hours, and then concentrated under reduced pressure to obtain 8 g of crude product. Half of the mixture was dissolved in DCM (150 mL) and TEA (3.28 g, 32.4 mmol), (Z)-pent-2-en-1-ol (1.395 g, 16.2 mmol) was added, and stirred at 40 °C for 4 hours. TLC showed complete consumption of raw materials, , the mixture was mixed directly, and purified by column chromatography with PE:EA=15:1 to obtain 4.0 g of a colorless oily product with a yield of 84.78%.

### Step 2: Synthesis of di((Z)-pent-2-en-1-yl)8,8'-((3-((2-hydroxyethyl)(8-oxo-8-(((Z)-pent-2-en-1-yl)oxo)octyl)amino)propyl)azanediyl)dioctanoate

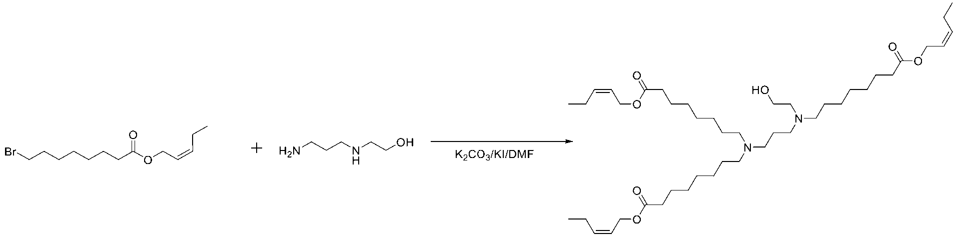

2-((3-aminopropyl)amino)ethane-1-ol (59.1 mg, 0.5 mmol) was dissolved in DMF (5 mL), and (Z)-pent-2-en-1-yl 8-bromooctanoate (509.65 mg, 1.75 mmol), K₂CO₃ (345.53 mg, 2.5 mmol), and Kl (13 mg) were added respectively, and stirred at 85 °C for 16 hours. An appropriate amount of water was added to the mixture, then EA (50 mL * 3) was added for extraction. The combined organic phases were dried and concentrated under reduced pressure. The crude product was purified by column chromatography with DCM (containing 5% EA):MeOH=50:1 to 5:1 to obtain 0.1 g of a colorless oily product with a yield of 26.7 %.

ESI-MS *m*/*z:* cald for C₄₄H₈₁N₂O₇ [M+H]⁺: 949.4, found 949.4.

¹H NMR (400 MHz, CDCl₃) δ 5.71 - 5.59 (m, 3H), 5.55 - 5.43 (m, 3H), 4.62 (dd, J = 6.9, 1.3 Hz, 6H), 3.67 (t, J = 5.0 Hz, 2H), 2.99-2.90 (m, 1H), 2.87-2.78 (m, 4H), 2.74 - 2.65 (m, 4H), 2.61 - 2.51 (m, 2H), 2.31 (t, J = 7.5 Hz, 6H), 2.17-2.07 (m, 6H), 2.02-1.88 (m, 2H), 1.70-1.56 (m, 10H), 1.54-1.44 (m, 2H), 1.39-1.24 (m, 20H), 1.00 (t, J = 7.5 Hz, 9H).

### Example 11

### Diundecyl 6,6'-((3-((2-hydroxyethyl)(6-oxo-6-(undecyloxy)hexyl)amino)propyl)azanediyl)dihexanoate

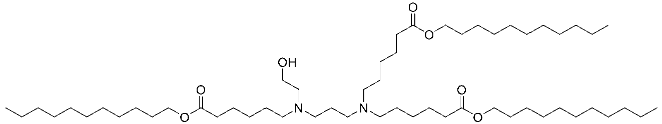

### Step 1: Synthesis of undecyl 6-bromohexanoate

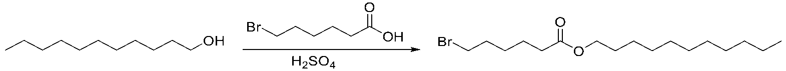

1-undecanol (2.0 g, 11.6 mmol) was mixed with 6-bromohexanoic acid (2.7 g, 13.9 mmol). The mixture was heated to 60°C, concentrated sulfuric acid (0.1 mL) was added, and stirred at this temperature for 6 hours. TLC showed complete consumption of raw materials, the mixture was mixed directly, and purified by column chromatography with PE:EA=20:1 to obtain 3.5 g of a colorless oily product with a yield of 86.4%.

1H-NMR (400 MHz, CDCl₃) δ 4.06 (t, J = 6.7 Hz, 2H), 3.40 (t, J = 6.8 Hz, 2H), 2.31 (t, J = 7.4 Hz, 2H), 1.95 - 1.80 (m, 2H), 1.70 - 1.59 (m, 4H), 1.54 - 1.40 (m, 2H), 1.27 (d, J = 7.7 Hz, 16H), 0.88 (t, J = 6.7 Hz, 3H).

### Step 2: Synthesis of diundecyl 6,6'-((3-((2-hydroxyethyl)(6-oxo-6-(undecyloxy)hexyl)amino)propyl)azanediyl)dihexanoate

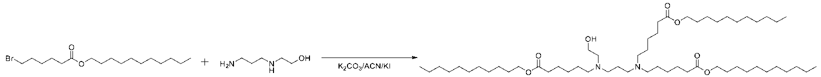

2-((3-aminopropyl)amino)ethane-1-ol (60 mg, 0.5 mmol) was dissolved in acetonitrile (6 mL), and undecyl 6-bromohexanoate (0.6 g, 1.8 mmol), K₂CO₃ (0.3 g, 2.3 mmol), and KI (10 mg) were added respectively, and stirred at 85 °C for 16 hours. An appropriate amount of water was added to the mixture, then DCM (30 mL * 3) was added for extraction. The combined organic phases were dried and concentrated under reduced pressure. The crude product was purified by column chromatography with DCM: MeOH=40:1 to obtain 160 mg of a colorless oily product with a yield of 34.0 %.

ESI-MS *m*/*z:* cald for C₅₆H₁₁₁N₂O₇ [M+H]⁺: 923.5, found 923.5.

1H-NMR (400 MHz, CDCl₃) δ 4.05 (t, J = 6.8 Hz, 6H), 3.53 (t, J = 5.2 Hz, 2H), 2.56 - 2.34 (m, 12H), 2.29 (t, J = 7.5 Hz, 6H), 1.68 - 1.57 (m, 12H), 1.49 - 1.39 (m, 6H), 1.28 (d, J = 17.3 Hz, 57H), 0.88 (t, J = 6.8 Hz, 9H).

### Example 12

### Diundecyl 4,4'-((3-((2-hydroxyethyl)(4-oxo-4-(undecyloxy)butyl)amino)propyl)azanediyl)dibutanoate

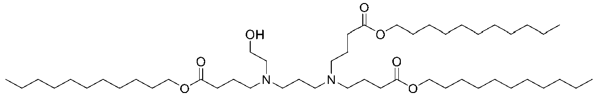

### Step 1: Synthesis of undecyl 4-bromobutanoate

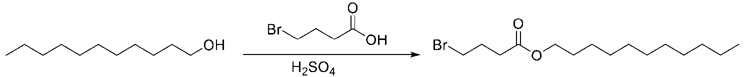

1-undecanol (2.0 g, 11.6 mmol) was mixed with 4-bromobutyric acid (2.3 g, 13.9 mmol). The mixture was heated to 60°C, concentrated sulfuric acid (0.1 mL) was added, and stirred at this temperature for 6 hours. TLC showed complete consumption of raw materials, the mixture was mixed directly, and purified by column chromatography with PE:EA=20:1 to obtain 3.3 g of a colorless oily product with a yield of 88.5%.

¹H-NMR (400 MHz, CDCl₃) δ 4.07 (t, J = 6.7 Hz, 2H), 3.46 (t, J = 6.4 Hz, 2H), 2.49 (t, J = 7.2 Hz, 2H), 2.22 - 2.11 (m, 2H), 1.66 - 1.60 (m, 2H), 1.35 - 1.23 (m, 16H), 0.88 (t, J = 6.7 Hz, 3H).

### Step 2: Synthesis of diundecyl 4,4'-((3-((2-hydroxyethyl)(4-oxo-4-(undecyloxy)butyl)amino)propyl)azanediyl)dibutanoate

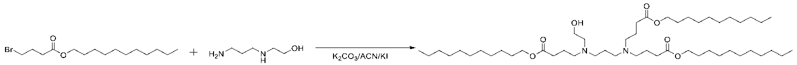

2-((3-aminopropyl)amino)ethane-1-ol (60 mg, 0.5 mmol) was dissolved in acetonitrile (8 mL), and undecyl 4-bromobutanoate (0.6 g, 1.8 mmol), K₂CO₃ (0.3 g, 2.3 mmol), and Kl (10 mg) were added respectively, and stirred at 85 °C for 16 hours. An appropriate amount of water was added to the mixture, then DCM (30 mL * 3) was added for extraction. The combined organic phases were dried and concentrated under reduced pressure. The crude product was purified by column chromatography with DCM:MeOH=30:1 to obtain 180 mg of a colorless oily product with a yield of 42.0 %.

ESI-MS *m*/*z:* cald for C₅₀H₉₉N₂O₇ [M+H]⁺: 839.3, found 839.3.

¹H-NMR (400 MHz, CDCl₃) δ 4.09 - 4.01 (m, 6H), 3.54 (t, J = 5.2 Hz, 2H), 2.57 (t, J = 5.2 Hz, 2H), 2.48 (t, J = 7.2 Hz, 4H), 2.41 (t, J = 7.2 Hz, 6H), 2.35 - 2.26 (m, 6H), 2.02 (s, 1H), 1.82 - 1.68 (m, 6H), 1.66 - 1.49 (m, 8H), 1.27 (d, J = 8.8 Hz, 48H), 0.88 (t, J = 6.7 Hz, 9H).

### Example 13

### Ditridecyl 8,8'-((3-((2-hydroxyethyl)(8-oxo-8-(tridecyloxy)octyl)amino)propyl)azanediyl)dioctanoate

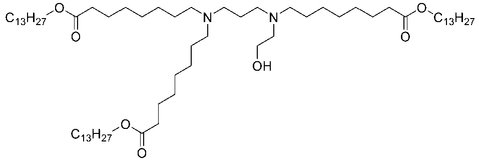

### Step 1: Synthesis of tridecyl 8-bromooctanoate

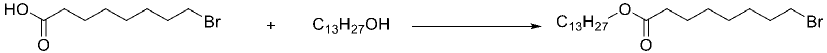

Under nitrogen protection, bromooctanoic acid (5 g, 22.41 mmol) was dissolved in 100 mL of anhydrous DCM. Oxalyl chloride (3.41g, 28.89 mmol, 1.2 eq) was added to the solution in an ice bath. Then the mixture was reacted at 40 °C for one hour, concentrated under reduced pressure, and dissolved again in 100 mL of anhydrous DCM. Tridecyl alcohol (2.55 g, 17.93mmol, 0.8 eq) was dissolved in 20 mL of Et3N, added dropwise to the aforementioned reaction solution, and stirred at room temperature for 1 hour. After the reaction was complete, the solution was separated, the organic phase was washed with saturated saline solution, dried with anhydrous sodium sulfate, and then concentrated under reduced pressure and purified by column chromatography. 2.44 g of a target substance was obtained with a yield of 26.8%.

### Step 2: Synthesis of ditridecyl 8,8'-((3-((2-hydroxyethyl)(8-oxo-8-(tridecyloxy)octyl)amino)propyl)azanediyl)dioctanoate:

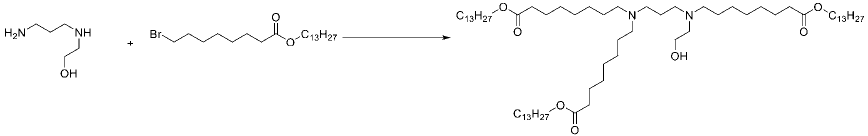

2-((3-aminopropyl)amino)ethane-1-ol (0.1 g, 0.846 mmol) was dissolved in 20 mL of anhydrous acetonitrile. Tridecyl 8-bromooctanoate (1.2 g, 2.96 mmol, 3.5 eq), K₂CO₃ (0.584 g, 6.35 mmol, 5 eq), and KI (0.21 g, 0.127 mmol, 0.1 eq) were added and heated to 85 °C overnight. After the reaction was complete, 20 mL of DCM was added and filtered to remove insoluble substances. The mixture was washed with 20 mL of saturated saline solution, dried with anhydrous sodium sulfate, and then concentrated under reduced pressure and purified by column chromatography. Finally, 0.34 g of a target substance was obtained with a yield of 36.8%.

ESI-MS *m*/*z:* cald for C₆₈H₁₃₅N₂O₇ [M+H]⁺: 1092.0, found 1092.0.

¹H NMR (400 MHz, CDCl₃) δ 4.09 (t, J = 6.8 Hz, 6H), 3.79 - 3.72 (m, 1H), 2.76 - 2.57 (m, 8H), 2.52 (t, J = 7.6 Hz, 2H), 2.32 (t, J = 7.5 Hz, 6H), 1.71 - 1.55 (m, 16H), 1.45 - 1.20 (m, 85H), 0.92 (t, J = 6.8 Hz, 9H).

### Example 14

### Diundecyl 10,10'-((3-((2-hydroxyethyl)(10-oxo-10-(undecyloxy)decyl)amino)propyl)azanediyl)dicaprate

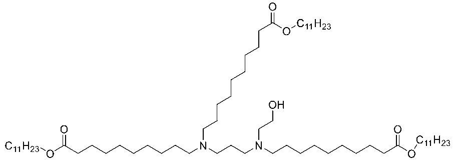

### Step 1: Synthesis of undecyl 10-bromocaprate

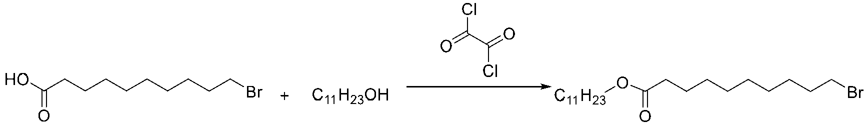

Under nitrogen protection, bromodecanoic acid (5 g, 19.9 mmol) was dissolved in 100 mL of anhydrous DCM. Oxalyl chloride (3.03 g, 23.89 mmol, 1.2 eq) was added to the solution in an ice bath. Then the mixture was reacted at 40 °C for one hour, concentrated under reduced pressure, and dissolved again in 100 mL of anhydrous DCM. 1-undecanol (2.74 g, 15.93 mmol, 0.8 eq) was dissolved in 20 mL of Et3N, added dropwise to the aforementioned reaction solution, and stirred at room temperature for 1 hour. After the reaction was complete, the solution was separated, the organic phase was washed with saturated saline solution, dried with anhydrous sodium sulfate, and then concentrated under reduced pressure and purified by column chromatography. 1.96 g of a target substance was obtained with a yield of 24.3%.

### Step 2: Synthesis of diundecyl 10,10'-((3-((2-hydroxyethyl)(10-oxo-10-(undecyloxy)decyl)amino)propyl)azanediyl)dicaprate:

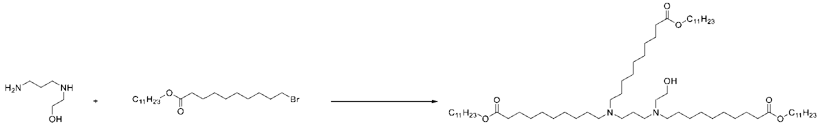

2-((3-aminopropyl)amino)ethane-1-ol (0.1 g, 0.846 mmol) was dissolved in 20 mL of anhydrous acetonitrile. Undecyl 10-bromocaprate (1.2 g, 2.96 mmol, 3.5 eq), K₂CO₃ (0.584 g, 6.35 mmol, 5 eq), and KI (0.21 g, 0.127 mmol, 0.1 eq) were added and heated to 85 °C overnight. After the reaction was complete, 20 mL of DCM was added and filtered to remove insoluble substances. The mixture was washed with 20 mL of saturated saline solution, dried with anhydrous sodium sulfate, and then concentrated under reduced pressure and purified by column chromatography. Finally, 0.34 g of a target substance was obtained with a yield of 36.8%.

ESI-MS *m*/*z:* cald for C₆₈H₁₃₅N₂O₇ [M+H]⁺: 1092.0, found 1092.0.

¹H NMR (400 MHz, CDCl₃) δ 4.09 (t, J = 6.8 Hz, 6H), 3.83 - 3.76 (m, 1H), 3.24 - 3.17 (m, 1H), 3.09 (t, J = 8.5 Hz, 3H), 2.88 - 2.77 (m, 2H), 2.65 (d, J = 9.1 Hz, 1H), 2.33 (t, J = 7.5 Hz, 7H), 2.12 - 1.98 (m, 2H), 1.80 - 1.54 (m, 16H), 1.46 - 1.16 (m, 85H), 1.01 - 0.83 (m, 10H).

### Example 15

### Di((Z)-non-2-en-1-yl)10,10'-((3-((2-hydroxyethyl)(10-(((Z)-non-2-en-1-yl)oxo)-10-oxodecyl)amino)propyl)azanediyl)dicaprate

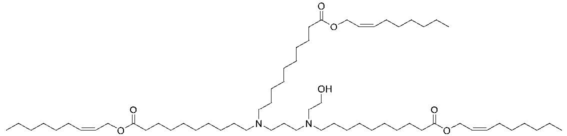

### Step 1: Synthesis of (Z)-non-2-en-1-yl 10-bromocaprate

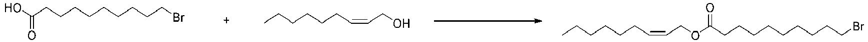

Under nitrogen protection, 10-bromodecanoic acid (5 g, 19.9 mmol) was dissolved in 100 mL of anhydrous DCM. Oxalyl chloride (3.03 g, 23.89 mmol, 1.2 eq) was added to the solution in an ice bath. Then the mixture was reacted at 40 °C for one hour, concentrated under reduced pressure, and dissolved again in 100 mL of anhydrous DCM. (Z)-non-2-en-1-ol (2.74 g, 15.93 mmol, 0.8 eq) was dissolved in 20 mL of Et3N, added dropwise to the aforementioned reaction solution, and stirred at room temperature for 1 hour. After the reaction was complete, the solution was separated, the organic phase was washed with saturated saline solution, dried with anhydrous sodium sulfate, and then concentrated under reduced pressure and purified by column chromatography. 3.93 g of a target substance was obtained with a yield of 52.6%.

### Step 2: Synthesis of di((Z)-non-2-en-1-yl)10,10'-((3-((2-hydroxyethyl)(10-(((Z)-non-2-en-1-yl)oxo)-10-oxodecyl)amino)propyl)azanediyl)dicaprate:

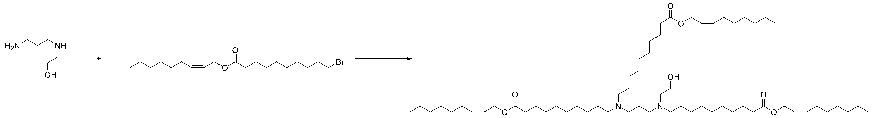

2-((3-aminopropyl)amino)ethane-1-ol (0.1 g, 0.846 mmol) was dissolved in 20 mL of anhydrous acetonitrile. (Z)-non-2-en-1-yl 10-bromocaprate (1.11 g, 2.96 mmol, 3.5 eq), K₂CO₃ (0.584 g, 4.23 mmol, 5 eq), and KI (0.014 g, 0.084 mmol, 0.1 eq) were added and heated to 85 °C overnight. After the reaction was complete, 20 mL of DCM was added and filtered to remove insoluble substances. The mixture was washed with 20 mL of saturated saline solution, dried with anhydrous sodium sulfate, and then concentrated under reduced pressure and purified by column chromatography. Finally, 0.319 g of a target substance was obtained with a yield of 22.4%.

ESI-MS *m*/*z:* cald for C₆₂H₁₁₇N₂O₇ [M+H]⁺: 1001.9, found 1001.9.

¹H NMR (400 MHz, CDCl₃) δ 5.73 - 5.62 (m, 2H), 5.61 - 5.50 (m, 2H), 4.73 - 4.62 (m, 6H), 4.21 - 4.12 (m, 1H), 3.69 - 3.61 (m, 1H), 2.83 - 2.63 (m, 6H), 2.53 - 2.48 (m, 2H), 2.34 (t, J = 7.6 Hz, 6H), 2.18 - 2.06 (m, 8H), 1.88 - 1.75 (m, 2H), 1.69 - 1.62 (m, 6H), 1.45 - 1.21 (m, 64H), 0.97 - 0.88 (m, 9H).

### Example 16

### Di((Z)-non-2-en-1-yl)4,4'-((3-((2-hydroxyethyl)(4-(((Z)-non-2-en-1-yl)oxo)-4-oxobutyl)amino)propyl)azanediyl)dibutanoate

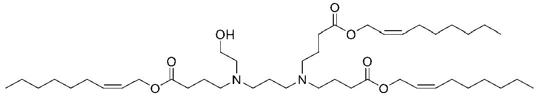

### Step 1: Synthesis of (Z)-non-2-en-1-yl 4-bromobutanoate

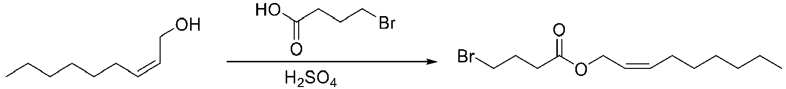

(Z)-non-2-en-1-ol (1.0 g, 7.0 mmol) was mixed with 4-bromobutyric acid (1.4 g, 8.4 mmol). The mixture was heated to 60 °C, concentrated sulfuric acid (0.1 mL) was added, and stirred at this temperature for 6 hours. TLC showed complete consumption of raw materials, the mixture was mixed directly, and purified by column chromatography with PE:EA=20:1 to obtain 1.9 g of a colorless oily product with a yield of 95.0%.

¹H-NMR (400 MHz, CDCl3) δ 5.70 - 5.60 (m, 1H), 5.57 - 5.46 (m, 1H), 4.67 - 4.60 (m, 2H), 3.46 (t, J = 6.5 Hz, 2H), 2.51 (t, J = 7.2 Hz, 2H), 2.23 - 2.04 (m, 4H), 1.38 - 1.22 (m, 8H), 0.92 - 0.84 (m, 3H).

### Step 2: Synthesis of di((Z)-non-2-en-1-yl)4,4'-((3-((2-hydroxyethyl)(4-(((Z)-non-2-en-1-yl)oxo)-4-oxobutyl)amino)propyl)azanediyl)dibutanoate

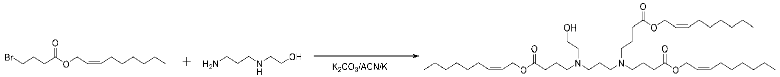

2-((3-aminopropyl)amino)ethane-1-ol (60 mg, 0.5 mmol) was dissolved in acetonitrile (8 mL), and (Z)-non-2-en-1-yl 4-bromobutanoate (0.6 g, 2.0 mmol), K₂CO₃ (0.4 g, 2.6 mmol), and KI (10 mg) were added respectively, and stirred at 85 °C for 16 hours. An appropriate amount of water was added to the mixture, then DCM (30 mL * 3) was added for extraction. The combined organic phases were dried and concentrated under reduced pressure. The crude product was purified by column chromatography with DCM:MeOH=30:1 to obtain 58 mg of a colorless oily product with a yield of 15.2 %.

ESI-MS *m*/*z:* cald for C₄₄H₈₁N₂O₇ [M+H]⁺: 749.1, found 749.1.

1H-NMR (400 MHz, CDCl₃) δ 5.69 - 5.57 (m, 3H), 5.56 - 5.46 (m, 3H), 4.65 - 4.58 (m, 6H), 3.54 (t, J = 5.2 Hz, 2H), 2.56 (t, J = 5.2 Hz, 2H), 2.48 (t, J = 7.2 Hz, 4H), 2.41 (t, J = 7.2 Hz, 6H), 2.36 - 2.27 (m, 6H), 2.14 - 2.04 (m, 6H), 1.82 - 1.68 (m, 6H), 1.61 - 1.49 (m, 2H), 1.38 - 1.25 (m, 25H), 0.92 - 0.84 (m, 9H).

### Example 17

### Di((Z)-hexyl-2-en-1-yl)8,8'-((3-((8-(((Z)-hexyl-2-en-1-yl)oxo)-8-oxooctyl)(2-hydroxyethyl)amino)propyl)azanediyl)dioctanoate

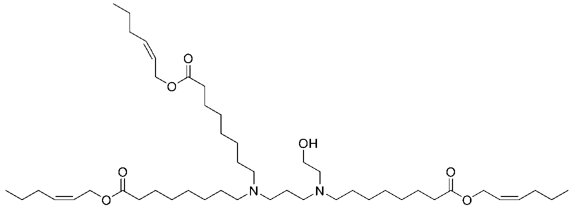

### Step 1: Synthesis of (Z)-hexyl-2-en-1-yl 8-bromooctanoate

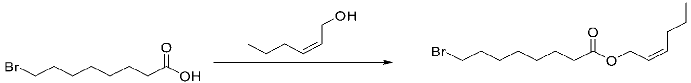

8-bromooctanoic acid (10.1 g, 45.27 mmol) was dissolved in 150 mL of DCM, oxalyl chloride (6.9 g, 54.33 mmol) was slowly added to the mixture, refluxed for 4 hours, and then concentrated under reduced pressure to obtain 10 g of crude product. 5 g of the crude product was dissolved in DCM (200 ml) and TEA (4.09 g, 40.4 mmol), cis(Z)-hexyl-2-en-1-ol (2.02 g, 20.19 mmol) was added, and stirred at 40 °C for 4 hours. TLC showed complete consumption of raw materials, the mixture was mixed directly, and purified by column chromatography with PE:EA=15:1 to obtain 4.0 g of a colorless oily product with a yield of 64.9%.

### Step 2: Di((Z)-hexyl-2-en-1-yl)8,8'-((3-((8-(((Z)-hexane-2-en-2-yl)oxy)-8-oxooctyl)(2-hydroxyethyl)amino)propyl)azadiyl)dioic acid

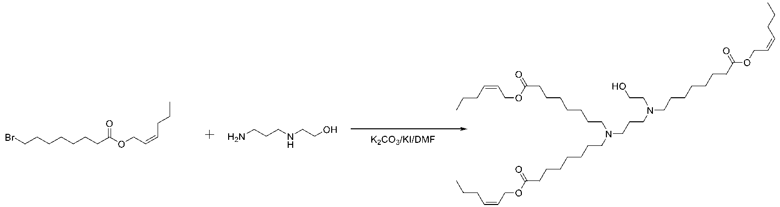

N-(2-hydroxyethyl)-1,3-propanediamine (108.6 mg, 0.92 mmol) was dissolved in DMF (5 mL), (Z)-hexyl-2-en-1-yl 8-bromooctanoate (981.8 mg, 3.22 mmol), K₂CO₃ (762.8 mg, 5.52 mmol), and KI (30 mg) were added respectively, and stirred at 85 °C for 16 hours. An appropriate amount of water was added to the mixture, then EA (80 mL X 3) was added for extraction. The combined organic phases were dried and concentrated under reduced pressure. The crude product was purified by column chromatography with DCM (containing 5% EA):MeOH=50:1 to 5:1 to obtain 0.1 g of a colorless oily product with a yield of 13.74 %.

ESI-MS *m*/*z:* cald for C₄₇H₈₇N₂O₇ [M+H]⁺: 791.7, found 791.7.

¹H NMR (400 MHz, CDCl₃) δ 5.74-5.62 (m, 3H), 5.62 - 5.52 (m, 3H), 4.66 (d, J = 6.8 Hz, 6H), 3.59 (t, J = 5.2 Hz, 2H), 2.60 (t, J = 5.2 Hz, 2H), 2.56-2.42 (m, 9H), 2.34 (t, J = 7.5 Hz, 6H), 2.17 - 2.07 (m, 6H), 1.71-1.58 (m, 8H), 1.54-1.37 (m, 13H), 1.37-1.24 (m, 19H), 0.94 (t, J = 7.4 Hz, 9H).

### Example 18

### Diundecyl 8,8'-((3-((3-hydroxypropyl)(8-oxo-8-(undecyloxy)octyl)amino)propyl)azanediyl)dioctanoate

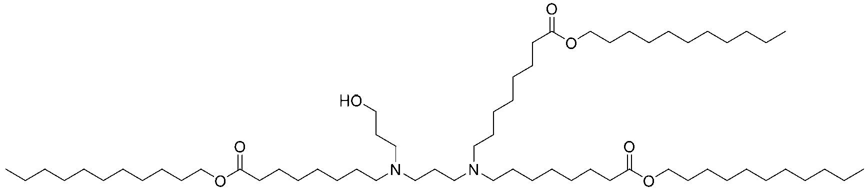

### Step 1: Synthesis of undecyl 8-bromooctanoate

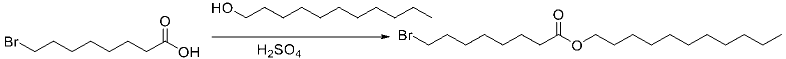

8-bromooctanoic acid (9 g, 40.34 mmol) and 1-undecanol (5 g, 29 mmol) were dissolved after stirring at 60 °C for 0.5 hours, and 3 drops of concentrated sulfuric acid were added. The mixture was reacted at 60 °C for 6 hours. TLC showed complete consumption of raw materials, the mixture was mixed directly, and eluted by column chromatography with PE:EA=15:1 to obtain 8.2 g of a colorless oily product with a yield of 74.9%.

### Step 2: Synthesis of diundecyl 8,8'-((3-((8-oxo-8-(undecyloxy)octyl)amino)propyl)azanediyl)dioctanoate

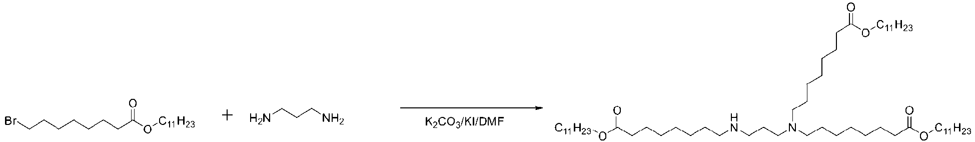

Propane-1,3-diamine (80 mg, 1.08 mmol) was dissolved in DMF (5 mL), and undecyl 8-bromooctanoate (1.22 g, 3.24 mmol), K₂CO₃ (895.54 mg, 6.48 mmol), and KI (30 mg) were added respectively, and stirred at 60 °C for 6 hours. An appropriate amount of water was added to the mixture, then EA (80 mL * 3) was added for extraction. The combined organic phases were dried and concentrated under reduced pressure. The crude product was purified by column chromatography with DCM (containing 5% EA):MeOH=50:1 to 5:1 to obtain 0.32 g of a colorless oily product with a yield of 30.75 %.

### Step 3: Synthesis of diundecyl 8,8'-((3-((3-hydroxypropyl)(8-oxo-8-(undecyloxy)octyl)amino)propyl)azanediyl)dioctanoate

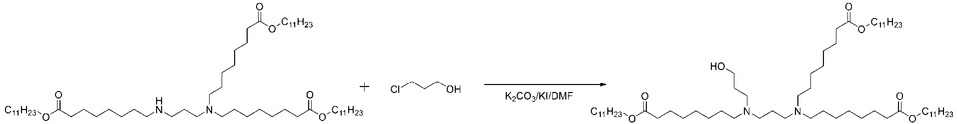

Diundecyl 8,8'-((3-((8-oxo-8-(undecyloxy)octyl)amino)propyl)azanediyl)dioctanoate (320 mg, 0.332 mmol) was dissolved in DMF (5 mL), and 3-chloro-1-propanol (62.77 mg, 0.664 mmol), K₂CO₃(138.2 mg, 1 mmol), and KI (30 mg) were added respectively, and the mixture stirred at 90°C for 16 hours. Then EA (80 mL * 3) was added for extraction. The combined organic phases were dried and concentrated under reduced pressure. The crude product was purified by column chromatography with DCM (containing 5% EA):MeOH=50:1 to 5:1 to obtain 0.13 g of a colorless oily product with a yield of 38.33 %.

ESI-MS *m*/*z:* cald for C₆₃H₁₂₅N₂O₇ [M+H]⁺: 1021.9, found 1021.9.

¹H NMR (400 MHz, CDCl₃) δ 4.09 (t, J = 6.8 Hz, 6H), 3.81 (t, J = 5.2 Hz, 2H), 3.02-2.94 (m, 2H), 2.93-2.81 (m, 5H), 2.79 - 2.58 (m, 4H), 2.33 (t, J = 7.5 Hz, 6H), 2.13-2.01 (m, 2H), 1.85 - 1.77 (m, 2H), 1.77-1.68 (m, 4H), 1.68-1.60 (m, 12H), 1.59-1.51 (m, 2H), 1.43 - 1.23 (m, 68H), 0.92 (t, J = 6.7 Hz, 9H).

### Example 19

### Di((Z)-non-2-en-1-yl)8,8'-((3-((3-hydroxypropyl)(8-(((Z)-non-2-en-1-yl)oxo)-8-oxooctyl)amino)propyl)azanediyl)dioctanoate

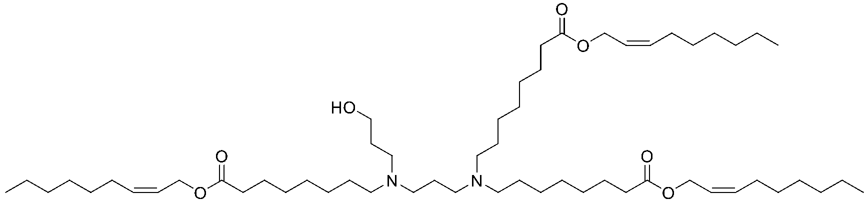

### Step 1: Synthesis of (Z)-non-2-en-1-yl 8-bromooctanoate

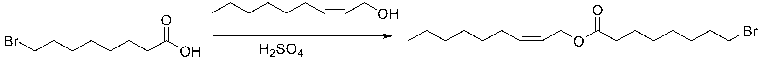

8-bromooctanoic acid (5 g, 22.41 mmol) and (Z)-non-2-en-1-ol (3.188 g, 22.41 mmol) were dissolved after stirring at 60 °C for 0.5 hours, 3 drops of concentrated sulfuric acid were added, and reacted at 60 °C for 6 hours. TLC showed complete consumption of raw materials, the mixture was mixed directly, and purified by column chromatography with PE:EA=15:1 to obtain 6 g of a colorless oily product with a yield of 77%.

### Step 2: Synthesis of di((Z)-non-2-en-1-yl)8,8'-((3-((8-(((Z)-non-2-en-1-yl)oxo)-8-oxooctyl)amino)propyl)azanediyl)dioctanoate

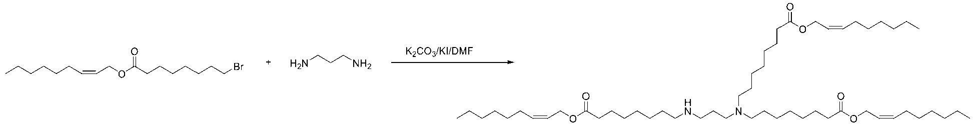

Propane-1,3-diamine (128 mg, 1.73 mmol) was dissolved in DMF (5 mL), and (Z)-non-2-en-1-yl 8-bromooctanoate (1.8 g, 5.18 mmol), K₂CO₃ (956.34 mg, 6.92 mmol), and KI (30 mg) were added respectively, and stirred at 60 °C for 6 hours. An appropriate amount of water was added to the mixture, then EA (80 mL * 3) was added for extraction. The combined organic phases were dried and concentrated under reduced pressure. The crude product was purified by column chromatography with DCM (containing 5% EA):MeOH=50:1 to 5:1 to obtain 0.67 g of a colorless oily product with a yield of 44.34 %.

### Step 3: Synthesis of di((Z)-non-2-en-1-yl)8,8'-((3-((3-hydroxypropyl)(8-(((Z)-non-2-en-1-yl)oxo)-8-oxooctyl)amino)propyl)azanediyl)dioctanoate

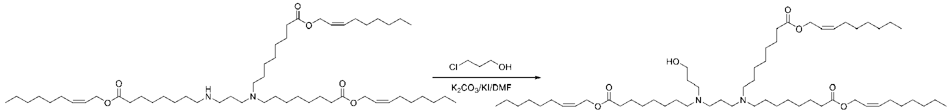

Di((Z)-non-2-en-1-yl)8,8'-((3-((8-(((Z)-non-2-en-1-yl)oxo)-8-oxooctyl)amino)propyl)azanediyl)dioctanoate (420 mg, 0.48 mmol) was dissolved in DMF (5 mL), and 3-chloro-1-propanol (90.76 mg, 0.96 mmol), K₂CO₃ (276.4 mg, 2 mmol), and KI (30 mg) were added respectively, and stirred at 90 °C for 16 hours. Then EA (80 mL * 3) was added for extraction. The combined organic phases were dried and concentrated under reduced pressure. The crude product was purified by column chromatography with DCM (containing 5% EA):MeOH=50:1 to 5:1 to obtain 0.1 g of a colorless oily product with a yield of 22.36 %.

ESI-MS *m*/*z:* cald for C₅₇H₁₀₇N₂O₇ [M+H]⁺: 931.6, found 931.6.

¹H NMR (400 MHz, CDCl₃) δ 5.75 - 5.64 (m, 3H), 5.61 - 5.48 (m, 3H), 4.66 (dd, J = 6.8, 1.3 Hz, 6H), 3.81 (t, J = 5.2 Hz, 2H), 3.07-2.71 (m, 9H), 2.68 - 2.48 (m, 5H), 2.34 (t, J = 7.5 Hz, 6H), 2.19 - 2.10 (m, 6H), 2.05-1.91 (m, 2H), 1.84 - 1.60 (m, 12H), 1.58-1.48 (m, 2H), 1.48 - 1.27 (m, 42H), 0.96 - 0.88 (m, 9H).

### Example 20

### Diundecyl 8,8'-((3-((4-hydroxybutyl)(8-oxo-8-(undecyloxy)octyl)amino)propyl)azanediyl)dioctanoate

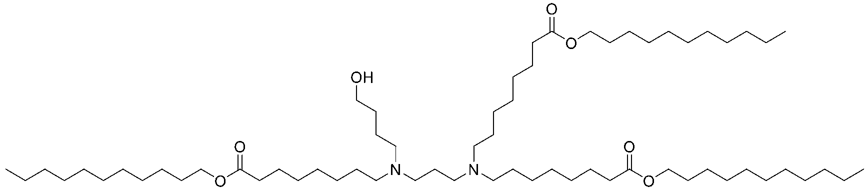

### Step 1: Synthesis of diundecyl 8,8'-((3-((4-hydroxybutyl)(8-oxo-8-(undecyloxy)octyl)amino)propyl)azanediyl)dioctanoate

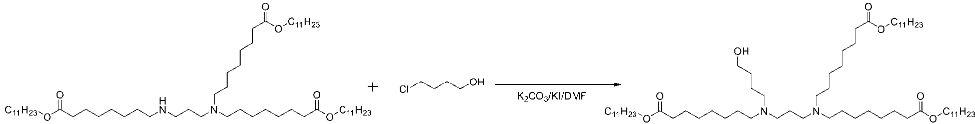

Di((Z)-non-2-en-1-yl)8,8'-((3-((8-(((Z)-non-2-en-1-yl)oxo)-8-oxooctyl)amino)propyl)azanediyl)dioctanoate (660 mg, 0.685 mmol) was dissolved in DMF (5 mL), and 4-chloro-1-butanol (148.74 mg, 1.37 mmol), K₂CO₃ (207.3 mg, 1.5 mmol), and KI (30 mg) were added respectively, and stirred at 90 °C for 16 hours. Then EA (80 mL * 3) was added for extraction. The combined organic phases were dried and concentrated under reduced pressure. The crude product was purified by column chromatography with DCM (containing 5% EA):MeOH=50:1 to 5:1 to obtain 0.07 g of a colorless oily product with a yield of 9.87 %.

ESI-MS *m*/*z:* cald for C₆₄H₁₂₇N₂O₇ [M+H]⁺: 1036.0, found 1036.0.

¹H NMR (400 MHz, CDCl₃) δ 4.08 (t, J = 6.8 Hz, 6H), 3.65 (t, J = 5.3 Hz, 2H), 3.52-3.46 (m, 1H), 3.01 - 2.62 (m, 13H), 2.32 (t, J = 7.5 Hz, 6H), 2.2-2.05 (m, 2H), 1.82-1.54 (m, 21H), 1.44 - 1.18 (m, 66H), 0.91 (t, J = 6.7 Hz, 9H).

### Example 21

### Tetra((Z)-non-2-en-1-yl)8,8',8",8‴-(propane-1,3-diyldi(azanetriyl))tetraoctanoate

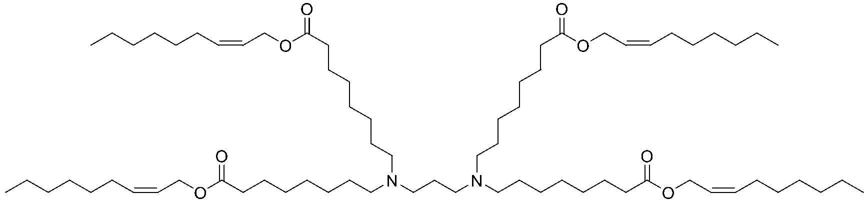

### Step 1: Synthesis of tetra((Z)-non-2-en-1-yl)8,8',8",8"'-(propane-1,3-diyldi(azanetriyl))tetraoctanoate

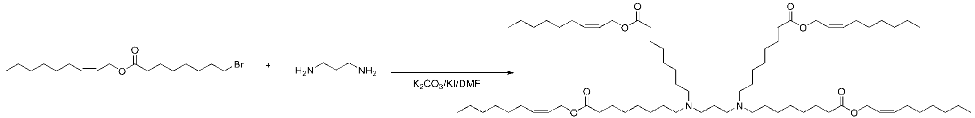

Propane-1,3-diamine (154 mg, 2.077 mmol) was dissolved in DMF (5 mL), and (Z)-non-2-en-1-yl 8-bromooctanoate (2.17 g, 6.25 mmol), K₂CO₃ (1 g, 7.25 mmol), and KI (30 mg) were added respectively, and stirred at 60 °C for 6 hours. An appropriate amount of water was added to the mixture, then EA (80 mL * 3) was added for extraction. The combined organic phases were dried and concentrated under reduced pressure. The crude product was purified by column chromatography with DCM (containing 5% EA):MeOH=50:1 to 5:1 to obtain 0.6 g of a colorless oily product with a yield of 25.34 %.

ESI-MS *m*/*z:* cald for C₇₁H₁₃₁N₂O₈ [M+H]⁺: 1140.0, found 1140.0.

¹H NMR (400 MHz, CDCl₃) δ 5.80 - 5.62 (m, 4H), 5.61 - 5.49 (m, 4H), 4.66 (dd, J = 6.8, 1.3 Hz, 8H), 2.72-2.40 (m, 10H), 2.34 (t, J = 7.5 Hz, 8H), 2.21 - 2.03 (m, 8H), 1.70-1.59 (m, 10H), 1.59-1.45 (m, 7H), 1.44 - 1.24 (m, 59H), 0.96 - 0.81 (m, 12H).

### Example 22

### Dipentadecyl 8,8'-((3-((2-hydroxyethyl)(8-oxo-8-(pentadecyloxy)octyl)amino)propyl)azanediyl)dioctanoate

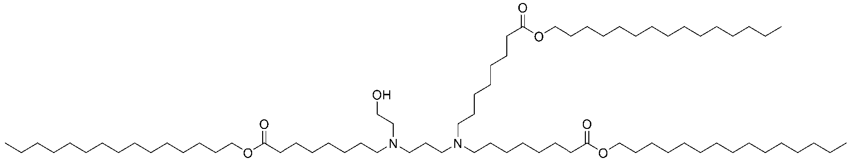

### Step 1: Synthesis of pentadecyl 8-bromooctanoate

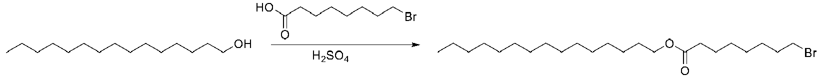

1-pentadecanol (1.0 g, 4.4 mmol) was mixed with 8-bromooctanoic acid (1.2 g, 5.3 mmol). The mixture was heated to 60 °C, concentrated sulfuric acid (0.1 mL) was added, and stirred at this temperature for 5 hours. TLC showed complete consumption of raw materials, the mixture was mixed directly, and purified by column chromatography with PE:EA=10:1 to obtain 1.8 g of a colorless oily product with a yield of 94.7%.

¹H-NMR (400 MHz, CDCl₃) δ 4.05 (t, J = 6.7 Hz, 2H), 3.40 (t, J = 6.8 Hz, 2H), 2.29 (t, J = 7.5 Hz, 2H), 1.90 - 1.79 (m, 2H), 1.66 - 1.58 (m, 4H), 1.43 (t, J = 6.8 Hz, 2H), 1.36 - 1.24 (m, 28H), 0.88 (t, J = 6.8 Hz, 3H).

### Step 2: Synthesis of dipentadecyl 8,8'-((3-((2-hydroxyethyl)(8-oxo-8-(pentadecyloxy)octyl)amino)propyl)azanediyl)dioctanoate:

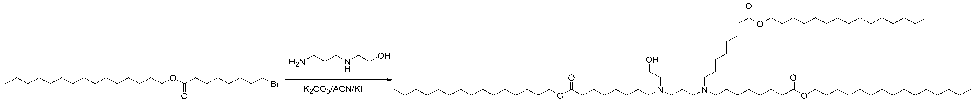

2-((3-aminopropyl)amino)ethane-1-ol (60 mg, 0.5 mmol) was dissolved in acetonitrile (8 mL), and pentadecyl 8-bromooctanoate (0.9 g, 2.0 mmol), K₂CO₃ (0.4 g, 2.6 mmol), and KI (10 mg) were added respectively, and stirred at 85 °C for 16 hours. An appropriate amount of water was added to the mixture, then DCM (30 mL * 3) was added for extraction. The combined organic phases were dried and concentrated under reduced pressure. The crude product was purified by column chromatography with DCM:MeOH=30:1 to obtain 120 mg of a colorless oily product with a yield of 20.0 %.

ESI-MS *m*/*z:* cald for C₇₄H₁₄₇N₂O₇ [M+H]⁺: 1176.0, found 1176.0.

¹H-NMR (400 MHz, CDCl₃) δ 4.05 (t, J = 6.8 Hz, 6H), 3.54 (t, J = 5.2 Hz, 2H), 2.57 - 2.33 (m, 11H), 2.28 (t, J = 7.5 Hz, 6H), 1.66 - 1.58 (m, 12H), 1.46 - 1.38 (m, 6H), 1.33 - 1.23 (m, 94H), 0.88 (t, J = 6.8 Hz, 9H).

### Example 23

### Diundecyl 12,12'-((3-((2-hydroxyethyl)(12-oxo-12-(undecyloxy)dodecyl)amino)propyl)azanediyl)didodecanoate

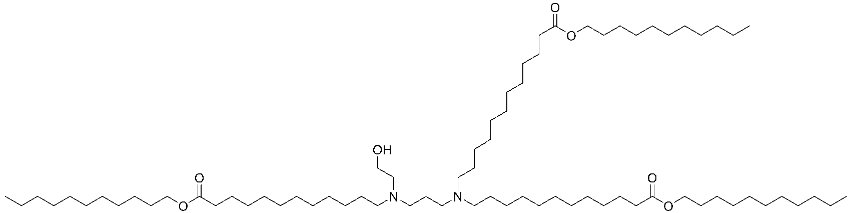

### Step 1: Synthesis of undecyl 12-bromododecanoate

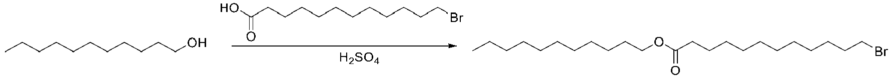

1-undecanol (1.0 g, 5.8 mmol) was mixed with 12-bromododecanoic acid (1.9 g, 7.0 mmol). The mixture was heated to 60 °C, concentrated sulfuric acid (0.1 mL) was added, and stirred at this temperature for 5 hours. TLC showed complete consumption of raw materials, the mixture was mixed directly, and purified by column chromatography with PE:EA=30:1 to obtain 2.2 g of a colorless oily product with a yield of 88.0%.

¹H-NMR (400 MHz, CDCl₃) δ 4.05 (t, J = 6.7 Hz, 2H), 3.40 (t, J = 6.9 Hz, 2H), 2.29 (t, J = 7.5 Hz, 2H), 1.90 - 1.79 (m, 2H), 1.66 - 1.57 (m, 4H), 1.46 - 1.38 (m, 2H), 1.34 - 1.24 (m, 28H), 0.88 (t, J = 6.8 Hz, 3H).

### Step 2: Synthesis of diundecyl 12,12'-((3-((2-hydroxyethyl)(12-oxo-12-(undecyloxy)dodecyl)amino)propyl)azanediyl)didodecanoate

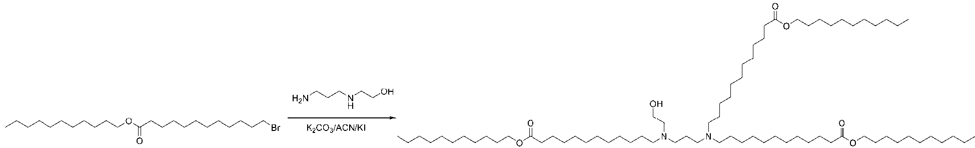

2-((3-aminopropyl)amino)ethane-1-ol (60 mg, 0.5 mmol) was dissolved in acetonitrile (10 mL), and undecyl 12-bromododecanoate (0.9 g, 2.0 mmol), K2CO3 (0.4 g, 2.6 mmol), and KI (10 mg) were added respectively, and stirred at 85 °C for 16 hours. An appropriate amount of water was added to the mixture, then DCM (30 mL * 3) was added for extraction. The combined organic phases were dried and concentrated under reduced pressure. The crude product was purified by column chromatography with DCM:MeOH=25:1 to obtain 220 mg of a colorless oily product with a yield of 36.7 %.

ESI-MS *m*/*z:* cald for C₇₄H₁₄₇N₂O₇ [M+H]⁺: 1176.0, found 1176.0.

1H-NMR (400 MHz, CDCl₃) δ 4.05 (t, J = 6.7 Hz, 6H), 3.54 (t, J = 5.2 Hz, 2H), 2.59 - 2.34 (m, 12H), 2.28 (t, J = 7.5 Hz, 6H), 1.61 (t, J = 7.2 Hz, 12H), 1.45 - 1.39 (m, 6H), 1.27 (d, J = 9.1 Hz, 92H), 0.88 (t, J = 6.8 Hz, 9H).

### Example 24

### Di((9Z,12Z)-octadeca-9,12-dien-1-yl)8,8'-((3-((2-hydroxyethyl)(8-(((9Z,12Z)-octadeca-9,12-dien-1-yl)oxo)-8-oxooctyl)amino)propyl)azanediyl)dioctanoate

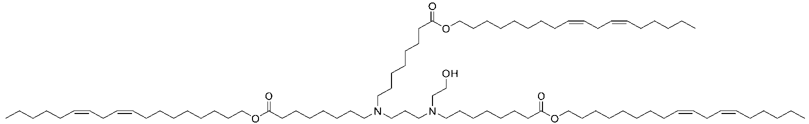

### Step 1: Synthesis of (9Z,12Z)-octadeca-9,12-dien-1-yl 8-bromooctanoate

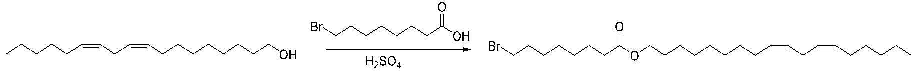

(9Z,12Z)-octadeca-9,12-dien-1-ol (1.0 g, 3.6 mmol) was mixed with 8-bromooctanoic acid (1.0 g, 4.5 mmol). The mixture was heated to 60 °C, concentrated sulfuric acid (0.1 mL) was added, and stirred at this temperature for 6 hours. TLC showed complete consumption of raw materials, the mixture was mixed directly, and purified by column chromatography with PE: EA=30:1 to obtain 1.6 g of a colorless oily product with a yield of 92.1%.

¹H-NMR (400 MHz, CDCl₃) δ5.43 - 5.27 (m, 4H), 4.05 (t, J = 6.7 Hz, 2H), 3.40 (t, J = 6.8 Hz, 2H), 2.77 (t, J = 6.4 Hz, 2H), 2.29 (t, J = 7.5 Hz, 2H), 2.09 - 1.99 (m, 4H), 1.90 - 1.79 (m, 2H), 1.67 - 1.58 (m, 4H), 1.48 - 1.39 (m, 2H), 1.37 - 1.26 (m, 20H), 0.89 (t, J = 6.8 Hz, 3H).

### Step 2: Synthesis of di((9Z,12Z)-octadeca-9,12-dien-1-yl)8,8'-((3-((2-hydroxyethyl)(8-(((9Z,12Z)-octadeca-9,12-dien-1-yl)oxo)-8-oxooctyl)amino)propyl)azanediyl)dioctanoate

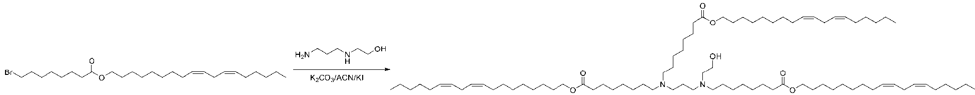

2-((3-aminopropyl)amino)ethane-1-ol (60 mg, 0.5 mmol) was dissolved in acetonitrile (10 mL), and (9Z,12Z)-octadeca-9,12-dien-1-yl 8-bromooctanoic acid (1.0 g, 2.0 mmol), K₂CO₃ (0.4 g, 2.6 mmol), and KI (10 mg) were added respectively, and stirred at 85 °C for 16 hours. An appropriate amount of water was added to the mixture, then DCM (30 mL * 3) was added for extraction. The combined organic phases were dried and concentrated under reduced pressure. The crude product was purified by column chromatography with DCM:MeOH=30:1 to obtain 220 mg of a colorless oily product with a yield of 33.5 %.

ESI-MS *m*/*z:* cald for C₈₃H₁₅₃N₂O₇ [M+H]⁺: 1290.1, found 1290.1.

¹H-NMR (400 MHz, CDCl₃) δ 5.43 - 5.27 (m, 12H), 4.05 (t, J = 6.8 Hz, 6H), 3.54 (t, J = 5.2 Hz, 2H), 2.77 (t, J = 6.4 Hz, 6H), 2.57 - 2.34 (m, 11H), 2.28 (t, J = 7.6 Hz, 6H), 2.08 - 2.01 (m, 12H), 1.61 (t, J = 7.1 Hz, 12H), 1.47 - 1.39 (m, 6H), 1.36 - 1.24 (m, 70H), 0.89 (t, J = 6.8 Hz, 9H).

### Example 25

### Di((Z)-non-2-en-1-yl)6,6'-((3-((2-hydroxyethyl)(6-(((Z)-non-2-en-1-yl)oxo)-6-oxohexyl)amino)propyl)azanediyl)dihexanoate

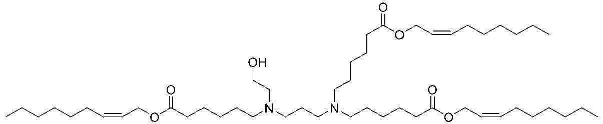

### Step 1: Synthesis of (Z)-non-2-en-1-yl 6-bromohexanoate

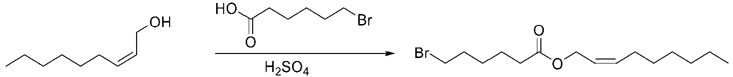

Cis-2-nonen-1-ol (1.0 g, 7.0 mmol) was mixed with 6-bromohexanoic acid (1.6 g, 8.4 mmol). The mixture was heated to 60 °C, concentrated sulfuric acid (0.1 mL) was added, and stirred at this temperature for 5 hours. TLC showed complete consumption of raw materials, the mixture was mixed directly, and purified by column chromatography with PE:EA=15:1 to obtain 2.1 g of a colorless oily product with a yield of 95.5%.

¹H-NMR (400 MHz, CDCl₃) δ 5.70 - 5.59 (m, 1H), 5.57 - 5.46 (m, 1H), 4.66 - 4.59 (m, 2H), 3.40 (t, J = 6.8 Hz, 2H), 2.33 (t, J = 7.4 Hz, 2H), 2.14 - 2.04 (m, 2H), 1.93 - 1.81 (m, 2H), 1.71 - 1.61 (m, 2H), 1.53 - 1.41 (m, 2H), 1.39 - 1.22 (m, 8H), 0.92 - 0.84 (m, 3H).

### Step 2: Synthesis of di((Z)-non-2-en-1-yl)6,6'-((3-((2-hydroxyethyl)(6-(((Z)-non-2-en-1-yl)oxo)-6-oxohexyl)amino)propyl)azanediyl)dihexanoate

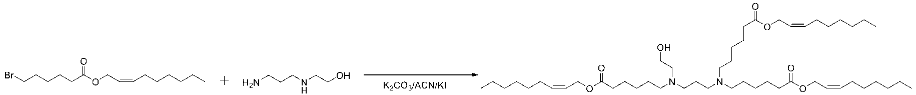

2-((3-aminopropyl)amino)ethane-1-ol (60 mg, 0.5 mmol) was dissolved in acetonitrile (8 mL), and (Z)-non-2-en-1-yl-6-bromohexanoate (0.6 g, 2.0 mmol), K₂CO₃ (0.4 g, 2.6 mmol), and KI (10 mg) were added respectively, and stirred at 85 °C for 16 hours. An appropriate amount of water was added to the mixture, then DCM (30 mL * 3) was added for extraction. The combined organic phases were dried and concentrated under reduced pressure. The crude product was purified by column chromatography with DCM:MeOH=30:1 to obtain 150 mg of a colorless oily product with a yield of 35.5 %.

ESI-MS *m*/*z:* cald for C₅₀H₉₃N₂O₇ [M+H]⁺: 833.3, found 833.3.

1H-NMR (400 MHz, CDCl₃) δ 5.69 - 5.58 (m, 3H), 5.57 - 5.46 (m, 3H), 4.65 - 4.58 (m, 6H), 3.53 (t, J = 5.2 Hz, 2H), 2.58 - 2.34 (m, 11H), 2.31 (t, J = 7.5 Hz, 6H), 2.15 - 2.04 (m, 6H), 1.69 - 1.59 (m, 7H), 1.50 - 1.40 (m, 6H), 1.39 - 1.23 (m, 32H), 0.92 - 0.84 (m, 9H).

### Example 26

### Ditridecyl 6,6'-((3-((2-hydroxyethyl)(6-oxo-6-(tridecyloxy)hexyl)amino)propyl)azanediyl)dihexanoate

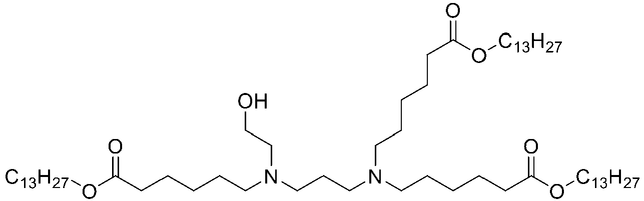

### Step 1: Synthesis of tridecyl 6-bromohexanoate

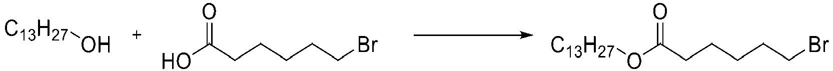

Using trideca-1-ol and 6-bromohexanoic acid as raw materials and according to step 1 of Example 3, tridecyl 6-bromohexanoate was obtained. The crude product was purified by column chromatography to obtain 16.5 g of a target molecule with a yield of 94.8%.

¹H NMR (400 MHz, CDCl₃) δ 4.06 (t, J = 6.8 Hz, 2H), 3.40 (t, J = 6.8 Hz, 2H), 2.31 (t, J = 7.4 Hz, 2H), 1.92 - 1.83 (m, 2H), 1.70 - 1.59 (m, 4H), 1.53 - 1.42 (m, 2H), 1.26 (d, J = 4.3 Hz, 20H), 0.88 (t, J = 6.8 Hz, 3H).

### Step 2: Synthesis of ditridecyl 6,6'-((3-((2-hydroxyethyl)(6-oxo-6-(tridecyloxy)hexyl)amino)propyl)azanediyl)dihexanoate

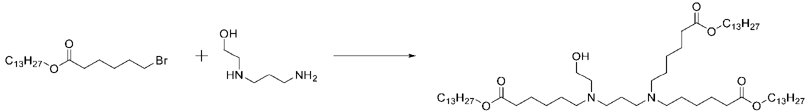

Using tridecyl 6-bromohexanoate and 2-((3-aminopropyl)amino)ethane-1-ol as raw materials and according to step 2 of Example 3, ditridecyl 6,6'-((3-((2-hydroxyethyl)(6-oxo-6-(tridecyloxy)hexyl)amino)propyl)azanediyl)dihexanoate was obtained. The crude product was purified by column chromatography to obtain 252 mg of a target molecule with a yield of 49.0%.

ESI-MS *m*/*z:* cald for C₆₂H₁₂₃N₂O₇ [M+H]⁺: 1008.0, found 1008.0.

¹H NMR (400 MHz, CDCl₃) δ 4.05 (t, J = 6.8 Hz, 6H), 3.53 (t, J = 5.2 Hz, 2H), 2.54 (t, J = 5.2 Hz, 2H), 2.50 - 2.34 (m, 10H), 2.29 (t, J = 7.5 Hz, 6H), 1.67 - 1.56 (m, 13H), 1.47 - 1.39 (m, 6H), 1.34 - 1.24 (m, 68H), 0.87 (t, J = 6.8 Hz, 9H).

### Example 27

### Diundecyl 6,6'-((3-((3-hydroxypropyl)(6-oxo-6-(undecyloxy)hexyl)amino)propyl)azanediyl)dihexanoate

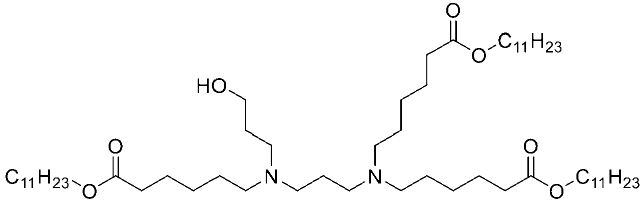

### Step 1: Synthesis of undecyl 6-bromohexanoate

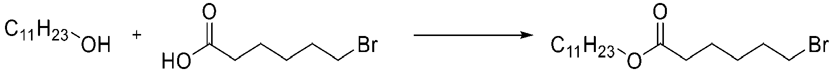

Using undeca-1-ol and 6-bromohexanoic acid as raw materials and according to step 1 of Example 19, undecyl 6-bromohexanoate was obtained. The crude product was purified by column chromatography to obtain 11.6 g of a target molecule with a yield of 86.6%.

¹H NMR (400 MHz, CDCl₃) δ 4.06 (t, J = 6.7 Hz, 2H), 3.40 (t, J = 6.8 Hz, 2H), 2.31 (t, J = 7.4 Hz, 2H), 1.93 - 1.81 (m, 2H), 1.69 - 1.59 (m, 4H), 1.55 - 1.40 (m, 2H), 1.33 - 1.22 (m, 16H), 0.88 (t, J = 6.7 Hz, 3H).

### Step 2: Synthesis of 6,6'-(3-((6-oxo-6-(undecyloxy)hexyl)amino)propyl)azahexyl)diundecyl dihexanoate

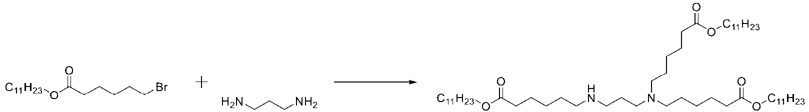

Using undecyl 6-bromohexanoate and 1,3-propanediamine as raw materials and according to step 2 of Example 19, 6,6'-(3-((6-oxo-6-(undecyloxy)hexyl)amino)propyl)azahexyl)diundecyl dihexanoate was obtained. The crude product was purified by column chromatography to obtain 1.1 g of a target molecule with a yield of 26.2%.

ESI-MS *m*/*z:* cald for C₅₄H₁₀₇N₂O₆ [M+H]⁺: 880.0, found 880.0.

¹H NMR (400 MHz, CDCl₃) δ 4.04 (t, J = 6.8 Hz, 6H), 2.66 - 2.55 (m, 4H), 2.44 (t, J = 7.1 Hz, 2H), 2.40 - 2.34 (m, 4H), 2.32 - 2.25 (m, 6H), 1.68 - 1.58 (m, 14H), 1.54 - 1.39 (m, 6H), 1.35 - 1.23 (m, 55H), 0.90 - 0.85 (m, 9H).

### Step 3: Synthesis of diundecyl 6,6'-((3-((3-hydroxypropyl)(6-oxo-6-(undecyloxy)hexyl)amino)propyl)azanediyl)dihexanoate

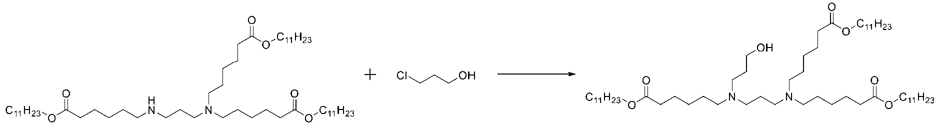

Using 6,6'-(3-((6-oxo-6-(undecyloxy)hexyl)amino)propyl)azahexyl)diundecyl dihexanoate and 3-chloro-1-propanol as raw materials and according to step 3 of Example 19, diundecyl 6,6'-((3-((3-hydroxypropyl)(6-oxo-6-(undecyloxy)hexyl)amino)propyl)azanediyl)dihexanoate was obtained. The crude product was purified by column chromatography to obtain 320 mg of a target molecule with a yield of 27.4%.

ESI-MS *m*/*z:* cald for C₅₇H₁₁₃N₂O₇ [M+H]⁺: 938.0, found 938.0.

¹H NMR (400 MHz, CDCl₃) δ 4.04 (t, J = 6.8 Hz, 6H), 3.77 (t, J = 5.1 Hz, 2H), 2.62 (t, J = 5.6 Hz, 2H), 2.52 - 2.34 (m, 10H), 2.32 - 2.25 (m, 6H), 1.66 - 1.57 (m, 13H), 1.52 - 1.39 (m, 6H), 1.36 - 1.20 (m, 58H), 1.00 - 0.78 (m, 9H).

### Example 28

### Diundecyl 6,6'-((3-((4-hydroxybutyl)(6-oxo-6-(undecyloxy)hexyl)amino)propyl)azanediyl)dihexanoate

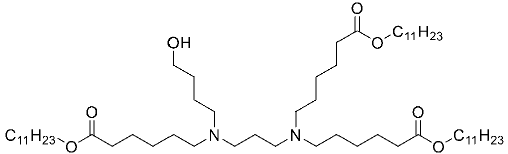

### Step 1: Synthesis of diundecyl 6,6'-((3-((4-hydroxybutyl)(6-oxo-6-(undecyloxy)hexyl)amino)propyl)azanediyl)dihexanoate

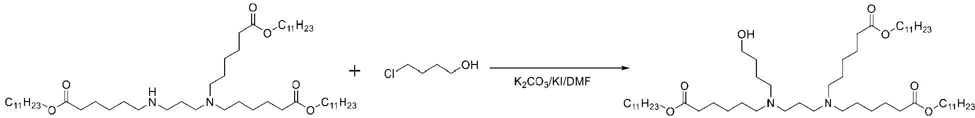

Using 6,6'-(3-((6-oxo-6-(undecyloxy)hexyl)amino)propyl)azahexyl)diundecyl dihexanoate and 4-chloro-1-butanol as raw materials and according to step 3 of Example 19, diundecyl 6,6'-((3-((4-hydroxybutyl)(6-oxo-6-(undecyloxy)hexyl)amino)propyl)azanediyl)dihexanoate was obtained. The crude product was purified by column chromatography to obtain 104 mg of a target molecule with a yield of 12.4%.

ESI-MS *m*/*z:* cald for C₅₈H₁₁₅N₂O₇ [M+H]⁺: 952.0, found 952.0.

¹H NMR (400 MHz, CDCl₃) δ 4.05 (t, J = 6.8 Hz, 6H), 3.54 (t, J = 4.6 Hz, 2H), 2.48 - 2.34 (m, 11H), 2.33 - 2.25 (m, 6H), 1.66 - 1.55 (m, 17H), 1.51 - 1.39 (m, 6H), 1.35 - 1.24 (m, 57H), 0.88 (t, J = 6.7 Hz, 9H).

### Example 29

### 8,8'-((3-((3-hydroxypropyl)(8-oxo-8-(tridecyloxy)octyl)amino)propyl)azahexyl)distearate

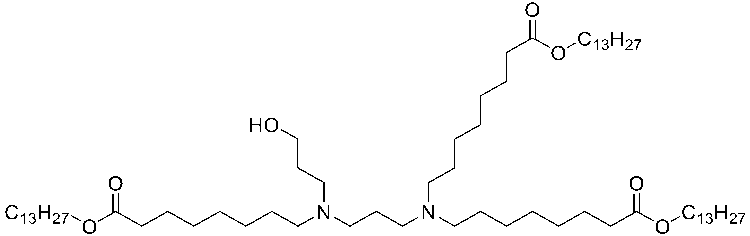

### Step 1: Synthesis of tridecyl 8-bromooctanoate

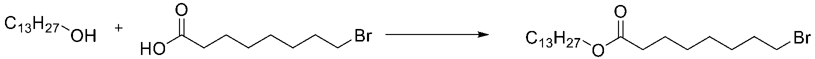

Using trideca-1-ol and 8-bromooctanoic acid as raw materials and according to step 1 of Example 19, tridecyl 8-bromohexyloctanoate was obtained. The crude product was purified by column chromatography to obtain 7.2 g of a target molecule with a yield of 79.23%.

### Step 2: Synthesis of 8,8'-((3-((8-oxo-8-(tridecyloxy)octyl)amino)propyl)azahexyl)distearate

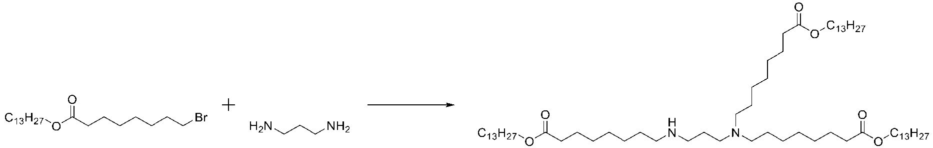

Using tridecyl 8-bromooctanoate and 1,3-propanediamine as raw materials and according to step 2 of Example 19, 8,8'-((3-((8-oxo-8-(tridecyloxy)octyl)amino)propyl)azahexyl)distearate was obtained. The crude product was purified by column chromatography to obtain 1.2 g of a target molecule with a yield of 46.65%.

ESI-MS *m*/*z:* cald for C₆₆H₁₃₁N₂O₆ [M+H]⁺: 1048.0, found 1048.0.

### Step 3: Synthesis of 8,8'-((3-((3-hydroxypropyl)(8-oxo-8-(tridecyloxy)octyl)amino)propyl)azahexyl)distearate

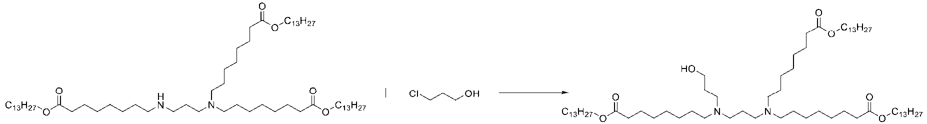

Using 8,8'-((3-((8-oxo-8-(tridecyloxy)octyl)amino)propyl)azahexyl)distearate and 3-chloro-1-propanol as raw materials and according to step 3 of Example 19, 8,8'-((3-((3-hydroxypropyl)(8-oxo-8-(tridecyloxy)octyl)amino)propyl)azahexyl)distearate was obtained. The crude product was purified by column chromatography to obtain 0.15 g of a target molecule with a yield of 23.67%.

ESI-MS *m*/*z:* cald for C₆₉H₁₃₇N₂O₇ [M+H]⁺: 1106.0, found 1106.0.

¹H NMR (400 MHz, CDCl₃) δ 4.04 (t, J = 6.8 Hz, 6H), 3.72-3.81 (m, 2H), 2.97 - 2.68 (m, 8H), 2.67-2.47 (m, 5H),2.28 (t, J = 7.5 Hz, 6H), 2.07-1.90 (m, 2H), 1.84 - 1.71 (m, 2H), 1.70 - 1.55 (m, 16H), 1.53 - 1.43 (m, 2H), 1.46 - 1.09 (m, 78H), 0.87 (t, J = 6.9 Hz, 9H).

### Example 30

### 6,6'-((3-((3-hydroxypropyl)(6-oxo-6-(tridecyloxy)hexyl)amino)propyl)azadiyl)ditridecyl dihexanoate

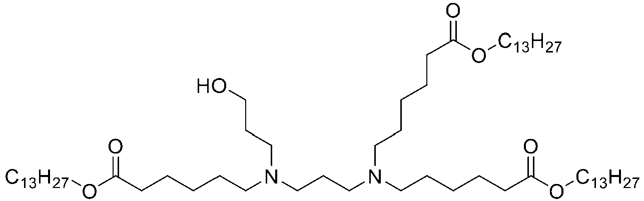

### Step 1: Synthesis of tridecyl 6-bromohexanoate

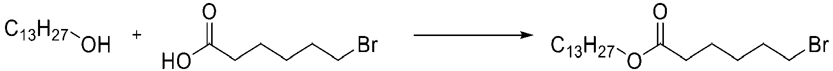

Using trideca-1-ol and 6-bromohexanoic acid as raw materials and according to step 1 of Example 19, tridecyl 6-bromohexanoate was obtained. The crude product was purified by column chromatography to obtain 7 g of a target molecule with a yield of 72.37%.

### Step 2: Synthesis of 6,6'-((3-((6-oxo-6-(tridecyloxy)hexyl)amino)propyl)azahexyl)ditridecyl dihexanoate

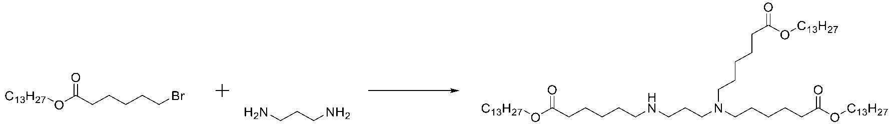

Using tridecyl 6-bromohexanoate and 1,3-propanediamine as raw materials and according to step 2 of Example 19, 6,6'-((3-((6-oxo-6-(tridecyloxy)hexyl)amino)propyl)azahexyl)ditridecyl dihexanoate was obtained. The crude product was purified by column chromatography to obtain 1.5 g of a target molecule with a yield of 35.38%.

ESI-MS *m*/*z:* cald for C₆₀H₁₁₉N₂O₆ [M+H]⁺: 964.0, found 964.0.

### Step 3: Synthesis of 6,6'-((3-((3-hydroxypropyl)(6-oxo-6-(tridecyloxy)hexyl)amino)propyl)azadiyl)ditridecyl dihexanoate

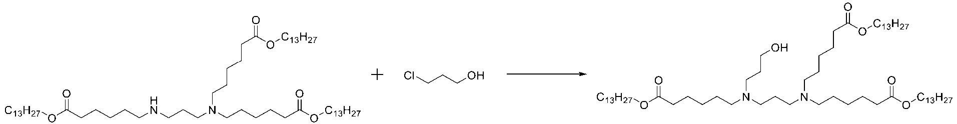

Using 6,6'-((3-((6-oxo-6-(tridecyloxy)hexyl)amino)propyl)azahexyl)ditridecyl dihexanoate and 3-chloro-1-propanol as raw materials and according to step 3 of Example 19, 6,6'-((3-((3-hydroxypropyl)(6-oxo-6-(tridecyloxy)hexyl)amino)propyl)azadiyl)ditridecyl dihexanoate was obtained. The crude product was purified by column chromatography to obtain a target molecule.

ESI-MS *m*/*z:* cald for C₆₃H₁₂₅N₂O₇ [M+H]⁺: 1022.0, found 1022.0.

¹H NMR (400 MHz, CDCl₃) δ 4.04 (t, J = 6.8 Hz, 6H), 3.76 (t, J = 5.3 Hz, 2H), 2.94 - 2.76 (m, 8H), 2.75 - 2.55 (m, 5H), 2.36-2.24 (m, 6H), 2.09-1.95 (m, 2H), 1.88 - 1.74 (m, 2H), 1.74-1.51 (m, 18H), 1.44-1.12 (m, 66H), 0.87 (t, J = 6.7 Hz, 9H).

### Example 31

### 6,6'-((3-((4-hydroxybutyl)(6-oxo-6-(tridecyloxy)hexyl)amino)propyl)azadiyl)ditridecyl dihexanoate

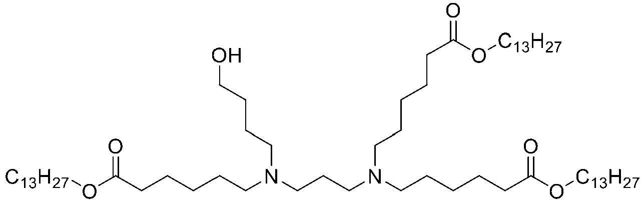

### Step 1: Synthesis of 6,6'-((3-((4-hydroxybutyl)(6-oxo-6-(tridecyloxy)hexyl)amino)propyl)azadiyl)ditridecyl dihexanoate

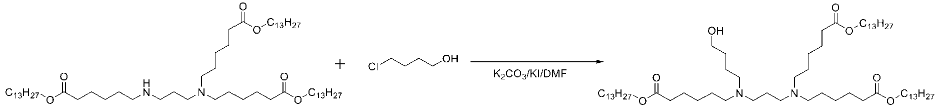

Using 6,6'-((3-((6-oxo-6-(tridecyloxy)hexyl)amino)propyl)azahexyl)ditridecyl dihexanoate and 4-chloro-1-butanol as raw materials and according to step 3 of Example 19, 6,6'-((3-((4-hydroxybutyl)(6-oxo-6-(tridecyloxy)hexyl)amino)propyl)azadiyl)ditridecyl dihexanoate was obtained. The crude product was purified by column chromatography to obtain 0.09 g of a target molecule with a yield of 9.3%.

ESI-MS *m*/*z:* cald for C₆₄H₁₂₇N₂O₇ [M+H]⁺: 1036.0, found 1036.0.

¹H NMR (400 MHz, CDCl₃) δ 4.05 (t, J = 6.8 Hz, 6H), 3.64 (t, J = 5.4 Hz, 2H), 3.02-2.59 (m, 12H), 2.31 (m, 6H), 2.15-1.96 (m, 2H), 1.86-1.50 (m, 22H), 1.45-1.16 (m, 66H), 0.88 (t, J = 6.8 Hz, 9H).

### Example 32

### 7,7'-(3-((2-hydroxyethyl)(7-oxo-7-(undecyloxy)heptyl)amino)propyl)azadiyl)diheptanoate

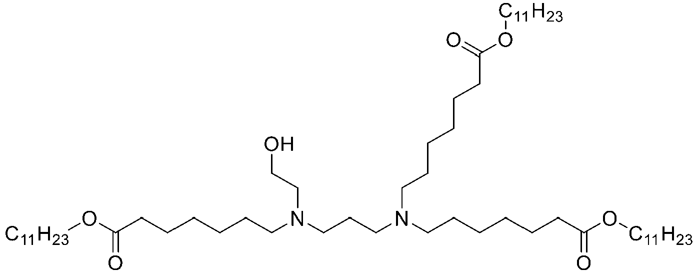

### Step 1: Synthesis of undecyl 7-bromoheptanoate

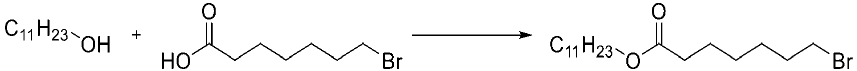

Using undeca-1-ol and 7-bromoheptanoic acid as raw materials and according to step 1 of Example 19, undecyl 7-bromoheptanoate was obtained. The crude product was purified by column chromatography to obtain a target molecule.

### Step 2: Synthesis of 7,7'-(3-((2-hydroxyethyl)(7-oxo-7-(undecyloxy)heptyl)amino)propyl)azadiyl)diheptanoate

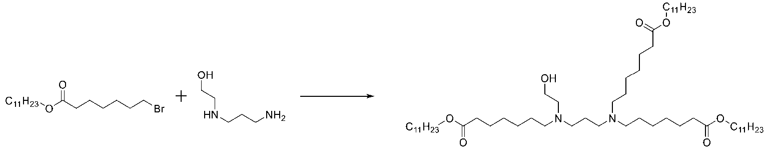

Using undecyl 7-bromoheptanoate and 2-((3-aminopropyl)amino)ethane-1-ol as a raw material and according to step 2 of Example 3, 7,7'-(3-((2-hydroxyethyl)(7-oxo-7-(undecyloxy)heptyl)amino)propyl)azadiyl)diheptanoate was obtained. The crude product was purified by column chromatography to obtain a target molecule.

ESI-MS *m*/*z:* cald for C₅₉H₁₁₇N₂O₇ [M+H]⁺: 966.0, found 966.0.

### Example 33

### 7,7'-(3-((2-hydroxyethyl)(7-oxo-7-(tridecyloxy)heptyl)amino)propyl)azadiyl)diheptanoate

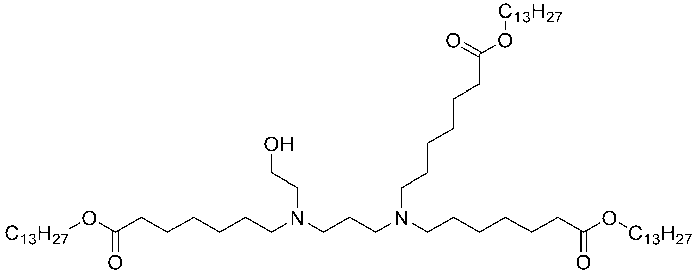

### Step 1: Synthesis of tridecyl 7-bromoheptanoate

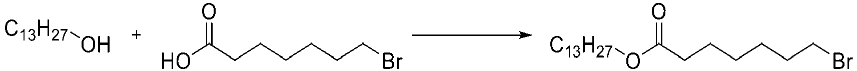

Using trideca-1-ol and 7-bromoheptanoic acid as raw materials and according to step 1 of Example 19, tridecyl 7-bromoheptanoate was obtained. The crude product was purified by column chromatography to obtain a target molecule.

### Step 2: 7,7'-(3-((2-hydroxyethyl)(7-oxo-7-(tridecyloxy)heptyl)amino)propyl)azadiyl)diheptanoate

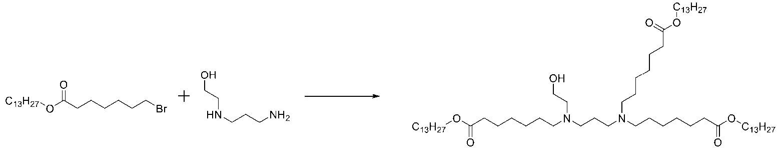

Using tridecyl 7-bromoheptanoate and 2-((3-aminopropyl)amino)ethane-1-ol as raw materials and according to step 2 of Example 3, 7,7'-(3-((2-hydroxyethyl)(7-oxo-7-(tridecyloxy)heptyl)amino)propyl)azadiyl)diheptanoate was obtained. The crude product was purified by column chromatography to obtain a target molecule.

ESI-MS *m*/*z:* cald for C₆₅H₁₂₉N₂O₇ [M+H]⁺: 1050.0, found 1050.0.

### Example 34

### 7,7'-(3-((3-hydroxypropyl)(7-oxo-7-(tridecyloxy)heptyl)amino)propyl)azadiyl)diheptanoate

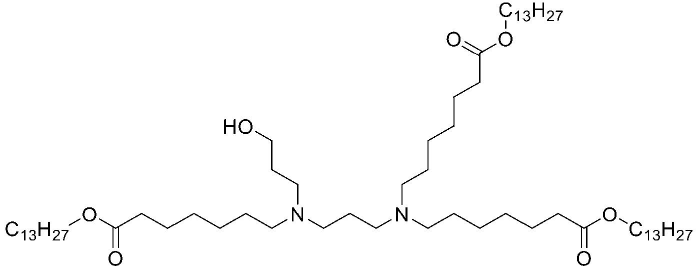

### Step 1: Synthesis of 7,7'-((3-((7-oxo-7-(tridecyloxy)heptyl)amino)propyl)azahexyl)diheptanoate

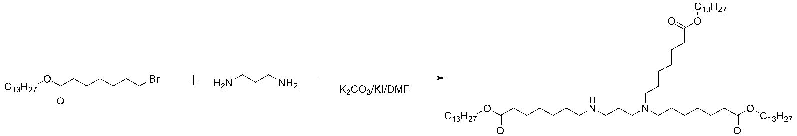

Using tridecyl 7-bromoheptanoate and 1,3-propanediamine as raw materials and according to step 2 of Example 19, the synthesis of 7,7'-((3-((7-oxo-7-(tridecyloxy)heptyl)amino)propyl)azahexyl)diheptanoate was carried out. The crude product was purified by column chromatography to obtain a target molecule.

ESI-MS *m*/*z:* cald for C₆₃H₁₂₅N₂O₆ [M+H]⁺: 1006.0, found 1006.0.

### Step 2: Synthesis of 7,7'-(3-((3-hydroxypropyl)(7-oxo-7-(tridecyloxy)heptyl)amino)propyl)azadiyl)diheptanoate

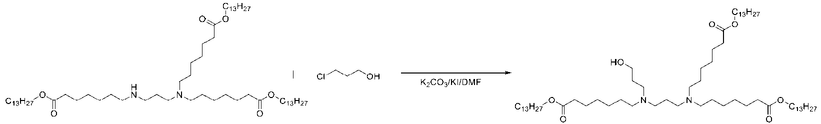

Using 7,7'-((3-((7-oxo-7-(tridecyloxy)heptyl)amino)propyl)azahexyl)diheptanoate and 3-chloro-1-propanol as raw materials and according to step 3 of Example 19, 7,7'-(3-((3-hydroxypropyl)(7-oxo-7-(tridecyloxy)heptyl)amino)propyl)azadiyl)diheptanoate was obtained. The crude product was purified by column chromatography to obtain a target molecule.

ESI-MS *m*/*z:* cald for C₆₆H₁₃₁N₂O₇ [M+H]⁺: 1064.0, found 1064.0.

### Example 35

### 7,7'-(3-((4-hydroxybutyl)(7-oxo-7-(tridecyloxy)heptyl)amino)propyl)azadiyl)diheptanoate

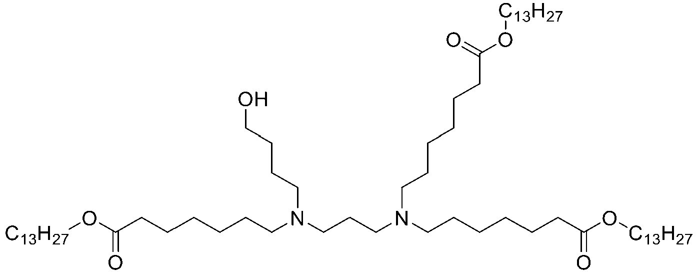

### Step 1: Synthesis of 7,7'-(3-((4-hydroxybutyl)(7-oxo-7-(tridecyloxy)heptyl)amino)propyl)azadiyl)diheptanoate

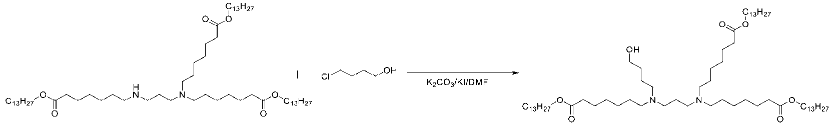

Using 7,7'-((3-((7-oxo-7-(tridecyloxy)heptyl)amino)propyl)azahexyl)diheptanoate and 4-chloro-1-butanol as raw materials and according to step 3 of Example 19, 7,7'-(3-((4-hydroxybutyl)(7-oxo-7-(tridecyloxy)heptyl)amino)propyl)azadiyl)diheptanoate was obtained. The crude product was purified by column chromatography to obtain a target molecule.

ESI-MS *m*/*z:* cald for C₆₇H₁₃₃N₂O₇ [M+H]⁺: 1078.0, found 1078.0.

### Example 36

### 7,7'-(3-((3-hydroxypropyl)(7-oxo-7-(undecyloxy)heptyl)amino)propyl)azadiyl)diheptanoate

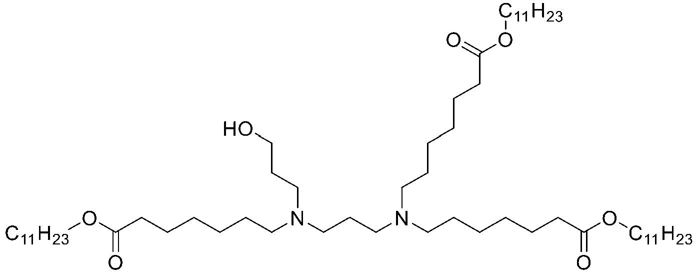

### Step 1: Synthesis of 7,7'-(3-((7-oxo-7-(undecyloxy)heptyl)amino)propyl)azahexyl)diheptanoate

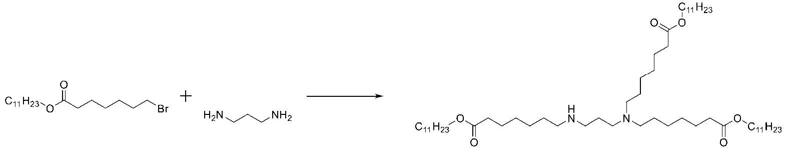

Using undecyl 7-bromoheptanoate and 1,3-propanediamine as raw materials and according to step 2 of Example 3, 7,7'-(3-((7-oxo-7-(undecyloxy)heptyl)amino)propyl)azahexyl)diheptanoate was obtained. The crude product was purified by column chromatography to obtain a target molecule.

ESI-MS *m*/*z:* cald for C₅₇H₁₁₃N₂O₆ [M+H]⁺: 922.0, found 922.0.

### Step 3: Synthesis of 7,7'-(3-((3-hydroxypropyl)(7-oxo-7-(undecyloxy)heptyl)amino)propyl)azadiyl)diheptanoate

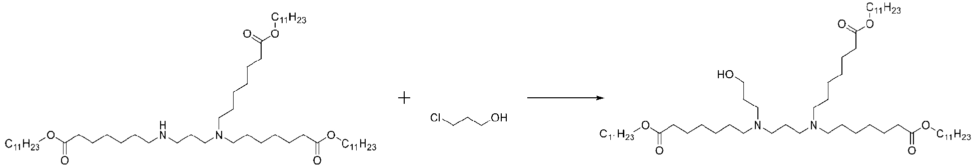

Using 7,7'-(3-((7-oxo-7-(undecyloxy)heptyl)amino)propyl)azahexyl)diheptanoate and 3-chloro-1-propanol as raw materials and according to step 2 of Example 3, 7,7'-(3-((3-hydroxypropyl)(7-oxo-7-(undecyloxy)heptyl)amino)propyl)azadiyl)diheptanoate was obtained. The crude product was purified by column chromatography to obtain a target molecule.

ESI-MS *m*/*z:* cald for C₆₀H₁₁₉N₂O₇ [M+H]⁺: 980.0, found 980.0.

### Example 37

### 7,7'-(3-((4-hydroxybutyl)(7-oxo-7-(undecyloxy)heptyl)amino)propyl)azadiyl)diheptanoate

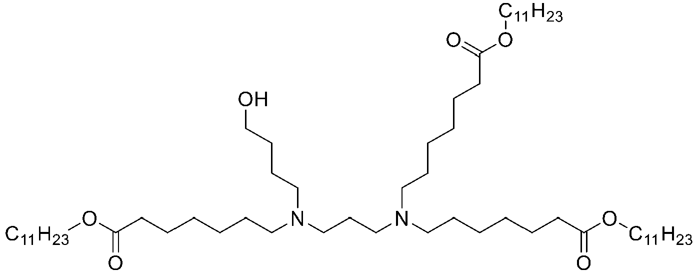

### Step 1: Synthesis of 7,7'-(3-((4-hydroxybutyl)(7-oxo-7-(undecyloxy)heptyl)amino)propyl)azadiyl)diheptanoate

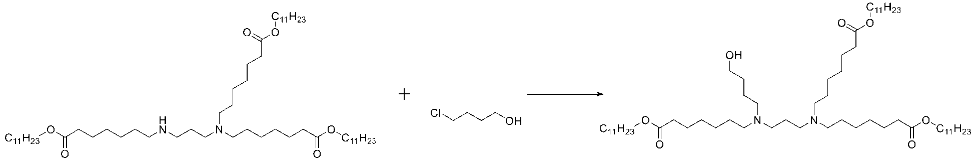

Using 7,7'-(3-((7-oxo-7-(undecyloxy)heptyl)amino)propyl)azahexyl)diheptanoate and 4-chloro-1-butanol as raw materials and according to step 3 of Example 19, 7,7'-(3-((4-hydroxybutyl)(7-oxo-7-(undecyloxy)heptyl)amino)propyl)azadiyl)diheptanoate was obtained. The crude product was purified by column chromatography to obtain a target molecule.

ESI-MS *m*/*z:* cald for C₆₁H₁₂₁N₂O₇ [M+H]⁺: 994.0, found 994.0.

### Example 38

### Diundecyl 5,5'-((3-((2-hydroxyethyl)(5-oxo-5-(undecyloxy)pentyl)amino)propyl)azadiyl)dipentanoate

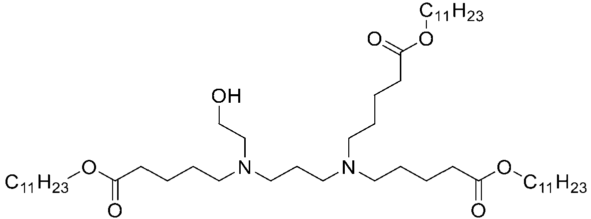

### Step 1: Synthesis of undecyl 5-bromopentanoate

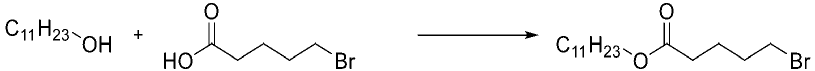

Using undeca-1-ol and 5-bromovaleric acid as raw materials and according to step 1 of Example 3, undecyl 5-bromopentanoate was obtained. The crude product was purified by column chromatography to obtain a target molecule.

### Step 2: Synthesis of diundecyl 5,5'-((3-((2-hydroxyethyl)(5-oxo-5-(undecyloxy)pentyl)amino)propyl)azadiyl)dipentanoate

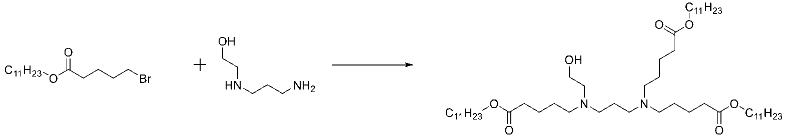

Using undecyl 5-bromopentanoate and 2-((3-aminopropyl)amino)ethane-1-ol as raw materials and according to step 2 of Example 3, diundecyl 5,5'-((3-((2-hydroxyethyl)(5-oxo-5-(undecyloxy)pentyl)amino)propyl)azadiyl)dipentanoate was obtained. The crude product was purified by column chromatography to obtain a target molecule.

ESI-MS *m*/*z:* cald for C₅₃H₁₀₅N₂O₇ [M+H]⁺: 882.0, found 882.0.

### Example 39

### Ditridecyl 5,5'-((3-((2-hydroxyethyl)(5-oxo-5-(tridecyloxy)pentyl)amino)propyl)azadiyl)dipentanoate

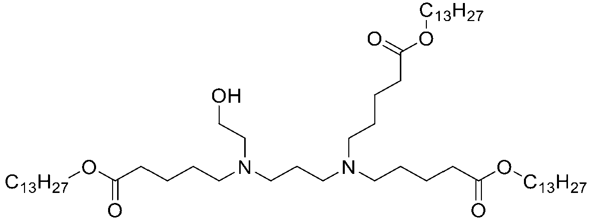

### Step 1: Synthesis of tridecyl 5-bromopentanoate

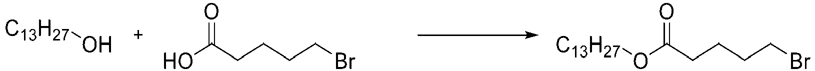

Using trideca-1-ol and 5-bromovaleric acid as raw materials and according to step 1 of Example 3, tridecyl 5-bromopentanoate was obtained. The crude product was purified by column chromatography to obtain a target molecule.

### Step 2: Synthesis of ditridecyl 5,5'-((3-((2-hydroxyethyl)(5-oxo-5-(tridecyloxy)pentyl)amino)propyl)azadiyl)dipentanoate

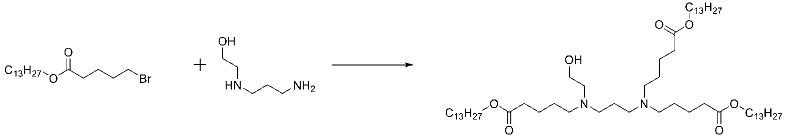

Using tridecyl 5-bromopentanoate and 2-((3-aminopropyl)amino)ethane-1-ol as raw materials and according to step 2 of Example 3, ditridecyl 5,5'-((3-((2-hydroxyethyl)(5-oxo-5-(tridecyloxy)pentyl)amino)propyl)azadiyl)dipentanoate was obtained. The crude product was purified by column chromatography to obtain a target molecule.

ESI-MS *m*/*z:* cald for C₅₉H₁₁₇N₂O₇ [M+H]⁺: 966.0, found 966.0.

### Example 40

### 5,5'-((3-((2-hydroxyethyl)(5-oxo-5-(tridecyloxy)pentyl)amino)propyl)azadiyl)ditridecyl dipentanoate

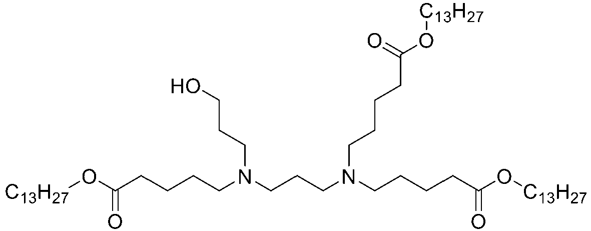

### Step 1: Synthesis of 5,5'-((3-((5-oxo-5-(tridecyloxy)pentyl)amino)propyl)azadiyl)ditridecyl dipentanoate

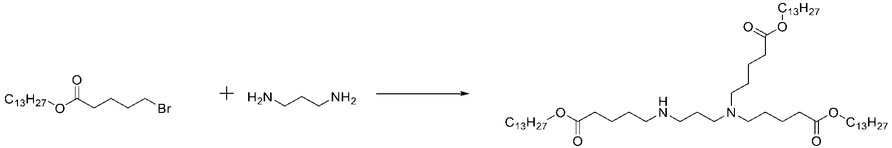

Using tridecyl 5-bromopentanoate and 1,3-propanediamine as raw materials and according to step 2 of Example 19, 5,5'-((3-((5-oxo-5-(tridecyloxy)pentyl)amino)propyl)azadiyl)ditridecyl dipentanoate was obtained. The crude product was purified by column chromatography to obtain a target molecule.

ESI-MS *m*/*z:* cald for C₅₇H₁₁₃N₂O₆ [M+H]⁺: 922.0, found 922.0.

### Step 2: Synthesis of 5,5'-((3-((2-hydroxyethyl)(5-oxo-5-(tridecyloxy)pentyl)amino)propyl)azadiyl)ditridecyl dipentanoate

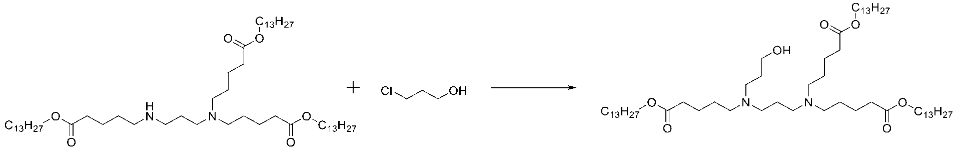

Using 5,5'-((3-(5-oxo-5-(tridecyloxy)pentyl)amino)propyl)azadiyl)ditridecyl dipentanoate and 3-chloro-1-propanol as raw materials and according to step 3 of Example 19, 5,5'-((3-((2-hydroxyethyl)(5-oxo-5-(tridecyloxy)pentyl)amino)propyl)azadiyl)ditridecyl dipentanoate was obtained. The crude product was purified by column chromatography to obtain a target molecule.

ESI-MS *m*/*z:* cald for C₆₀H₁₁₉N₂O₇ [M+H]⁺: 980.0, found 980.0.

### Example 41

### Ditridecyl 5,5'-((3-((4-hydroxybutyl)(5-oxo-5-(tridecyloxy)pentyl)amino)propyl)azadiyl)dipentanoate

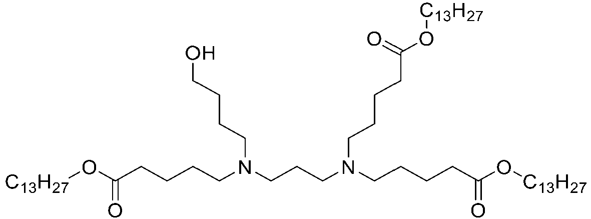

### Step 1: Synthesis of ditridecyl 5,5'-((3-((4-hydroxybutyl)(5-oxo-5-(tridecyloxy)pentyl)amino)propyl)azadiyl)dipentanoate

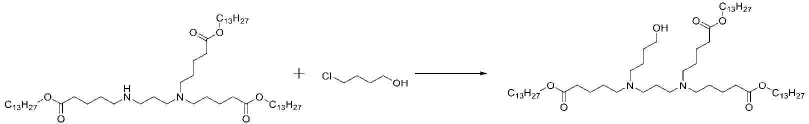

Using ditridecyl 5,5'-((3-(5-oxo-5-(tridecyloxy)pentyl)amino)propyl)azadiyl)dipentanoate and 4-chloro-1-butanol as raw materials and according to step 3 of Example 19, ditridecyl 5,5'-((3-((4-hydroxybutyl)(5-oxo-5-(tridecyloxy)pentyl)amino)propyl)azadiyl)dipentanoate was obtained. The crude product was purified by column chromatography to obtain a target molecule.

ESI-MS *m*/*z:* cald for C₆₁H₁₂₁N₂O₇ [M+H]⁺: 994.0, found 994.0.

### Example 42

### Diundecyl 5,5'-((3-((3-hydroxypropyl)(5-oxo-5-(undecyloxy)pentyl)amino)propyl)azadiyl)dipentanoate

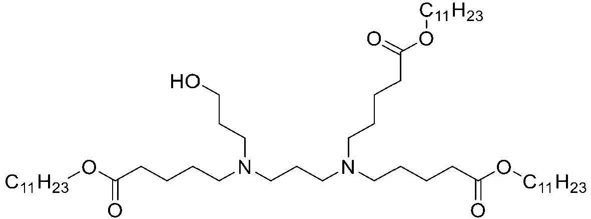

### Step 1: Synthesis of diundecyl 5,5'-((3-((5-oxo-5-(undecyloxy)pentyl)amino)propyl)azahexyl)dipentanoate

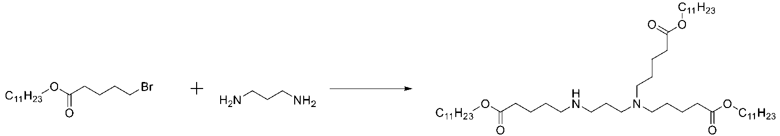

Using undecyl 5-bromopentanoate and 1,3-propanediamine as raw materials and according to step 2 of Example 19, diundecyl 5,5'-((3-((5-oxo-5-(undecyloxy)pentyl)amino)propyl)azahexyl)dipentanoate was obtained. The crude product was purified by column chromatography to obtain a target molecule.

ESI-MS *m*/*z:* cald for C₅₁H₁₀₁N₂O₆ [M+H]⁺: 838.0, found 838.0.

### Step 2: Synthesis of diundecyl 5,5'-((3-((3-hydroxypropyl)(5-oxo-5-(undecyloxy)pentyl)amino)propyl)azadiyl)dipentanoate

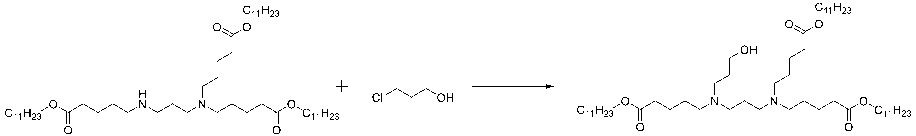

Using diundecyl 5,5'-((3-((5-oxo-5-(undecyloxy)pentyl)amino)propyl)azahexyl)dipentanoate and 3-chloro-1-propanol as raw materials and according to step 3 of Example 19, diundecyl 5,5'-((3-((3-hydroxypropyl)(5-oxo-5-(undecyloxy)pentyl)amino)propyl)azadiyl)dipentanoate was obtained. The crude product was purified by column chromatography to obtain 130 mg of a target molecule with a yield of 52.0%.

ESI-MS *m*/*z:* cald for C₅₄H₁₀₇N₂O₇ [M+H]⁺: 896.0, found 896.0.

¹H NMR (400 MHz, CDCl₃) δ 4.05 (t, J = 6.8 Hz, 6H), 3.77 (t, J = 5.1 Hz, 2H), 2.62 (t, J = 5.6 Hz, 2H), 2.47 - 2.36 (m, 10H), 2.34 - 2.28 (m, 6H), 1.63 (tt, J = 13.8, 6.1 Hz, 18H), 1.54 - 1.39 (m, 7H), 1.28 (d, J = 17.4 Hz, 46H), 0.88 (t, J = 6.7 Hz, 9H).

### Example 43

### Diundecyl 5,5'-((3-((4-hydroxybutyl)(5-oxo-5-(undecyloxy)pentyl)amino)propyl)azahexyl)dipentanoate

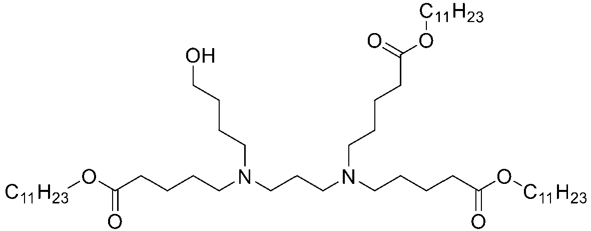

### Step 1: Synthesis of diundecyl 5,5'-((3-((4-hydroxybutyl)(5-oxo-5-(undecyloxy)pentyl)amino)propyl)azahexyl)dipentanoate

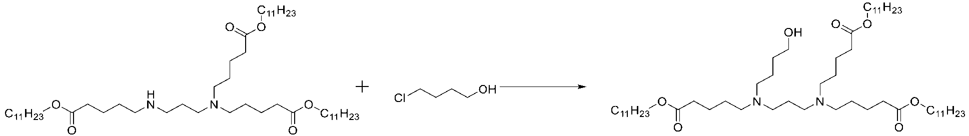

Using diundecyl 5,5'-((3-((5-oxo-5-(undecyloxy)pentyl)amino)propyl)azahexyl)dipentanoate and 4-chloro-1-butanol as raw materials and according to step 3 of Example 19, diundecyl 5,5'-((3-((4-hydroxybutyl)(5-oxo-5-(undecyloxy)pentyl)amino)propyl)azahexyl)dipentanoate was obtained. The crude product was purified by column chromatography to obtain a target molecule.

ESI-MS *m*/*z:* cald for C₅₅H₁₀₉N₂O₇ [M+H]⁺: 910.0, found 910.0.

### Example 44

### Didodecyl 6,6'-((3-((6-(dodecoxy)-6-oxohexyl)(2-hydroxyethyl)amino)propyl)azahexyl)dihexanoate

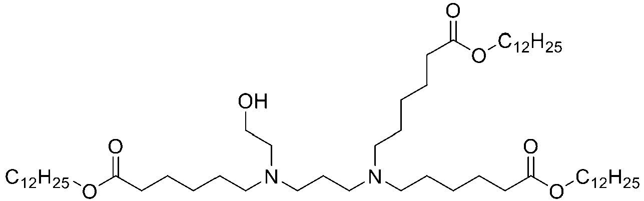

### Step 1: Synthesis of dodecyl 6-bromohexanoate

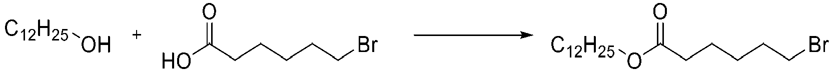

Using dodeca-1-ol and 6-bromohexanoic acid as raw materials and according to step 1 of Example 19, dodecyl 6-bromohexanoate was obtained. The crude product was purified by column chromatography to obtain a target molecule.

### Step 2: Synthesis of didodecyl 6,6'-((3-((6-(dodecoxy)-6-oxohexyl)(2-hydroxyethyl)amino)propyl)azahexyl)dihexanoate

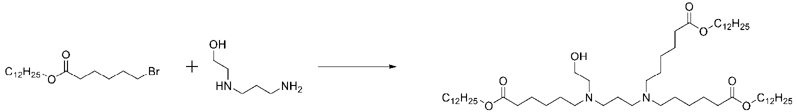

Using dodecyl 6-bromohexanoate and 2-((3-aminopropyl)amino)ethane-1-ol as raw materials and according to step 2 of Example 3, didodecyl 6,6'-((3-((6-(dodecoxy)-6-oxohexyl)(2-hydroxyethyl)amino)propyl)azahexyl)dihexanoate was obtained. The crude product was purified by column chromatography to obtain a target molecule.

ESI-MS *m*/*z:* cald for C₅₉H₁₁₇N₂O₇ [M+H]⁺: 966.0, found 966.0.

### Example 45

### Didodecyl 6,6'-((3-((6-(dodecoxy)-6-oxohexyl)(3-hydroxypropyl)amino)propyl)azahexyl)dihexanoate

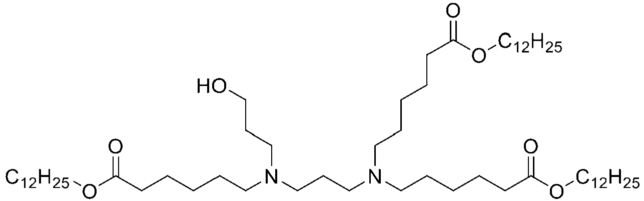

### Step 1: Synthesis of didodecyl 6,6'-((3-((6-(dodecoxy)-6-oxohexyl)amino)propyl)azahexyl)dihexanoate

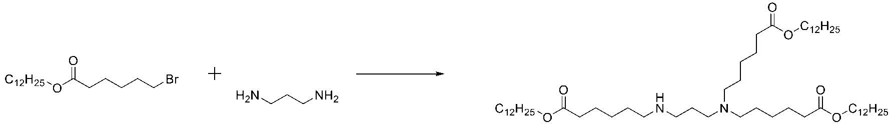

Using dodecyl 6-bromohexanoate and 1,3-propanediamine as raw materials and according to step 2 of Example 19, didodecyl 6,6'-((3-((6-(dodecoxy)-6-oxohexyl)amino)propyl)azahexyl)dihexanoate was obtained. The crude product was purified by column chromatography to obtain a target molecule.

ESI-MS *m*/*z:* cald for C₅₇H₁₁₃N₂O₆ [M+H]⁺: 922.0, found 922.0.

### Step 3: Synthesis of 5,5'-((3-((4-hydroxybutyl)(5-oxo-5-(undecyloxy)pentyl)amino)propyl)azahexyl)diundecyl dipentanoate

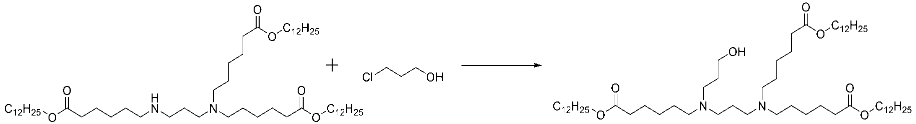

Using didodecyl 6,6'-((3-((6-(dodecoxy)-6-oxohexyl)amino)propyl)azahexyl)dihexanoate and 3-chloro-1-propanol as raw materials and according to step 3 of Example 19, 5,5'-((3-((4-hydroxybutyl)(5-oxo-5-(undecyloxy)pentyl)amino)propyl)azahexyl)diundecyl dipentanoate was obtained. The crude product was purified by column chromatography to obtain a target molecule.

ESI-MS *m*/*z:* cald for C₆₀H₁₁₉N₂O₇ [M+H]⁺: 980.0, found 980.0.

### Example 46

### Didodecyl 6,6'-((3-((6-(dodecyloxy)-6-oxohexyl)(4-hydroxybutyl)amino)propyl)azanediyl)dihexanoate

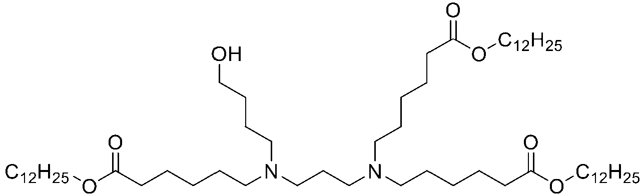

### Step 1: Synthesis of dodecyl 6-bromohexanoate

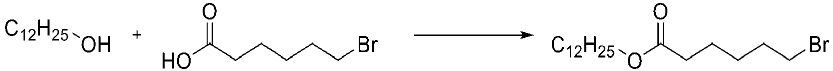

Using dodeca-1-ol and 6-bromohexanoic acid as raw materials and according to step 1 of Example 3, dodecyl 6-bromohexanoate was obtained. The crude product was purified by column chromatography to obtain a target molecule.

### Step 2: Synthesis of didodecyl 6,6'-((3-((6-(dodecyloxy)-6-oxohexyl)amino)propyl)azanediyl)dihexanoate

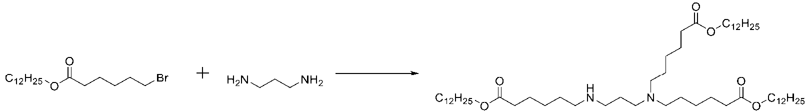

Using dodecyl 6-bromohexanoate and 1,3-propanediamine as raw materials and according to step 2 of Example 18, didodecyl 6,6'-((3-((6-(dodecyloxy)-6-oxohexyl)amino)propyl)azanediyl)dihexanoate was obtained. The crude product was purified by column chromatography to obtain a target molecule.

ESI-MS *m*/*z:* cald for C₅₇H₁₁₃N₂O₆ [M+H]⁺: 922.5, found 922.5.

### Step 3: Synthesis of didodecyl 6,6'-((3-((6-(dodecyloxy)-6-oxohexyl)(4-hydroxybutyl)amino)propyl)azanediyl)dihexanoate

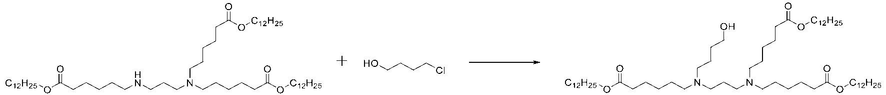

Using didodecyl 6,6'-((3-((6-(dodecyloxy)-6-oxohexyl)amino)propyl)azanediyl)dihexanoate as raw material and according to step 3 of Example 18, didodecyl 6,6'-((3-((6-(dodecyloxy)-6-oxohexyl)(4-hydroxybutyl)amino)propyl)azanediyl)dihexanoate was obtained. The crude product was purified by column chromatography to obtain a target molecule.

ESI-MS *m*/*z:* cald for C₆₁H₁₂₁N₂O₇ [M+H]⁺: 922.5, found 922.5.

### Example 47

### Didodecyl 7,7'-((3-((7-(dodecyloxy)-7-oxoheptyl)(2-hydroxyethyl)amino)propyl)azanediyl)diheptanoate

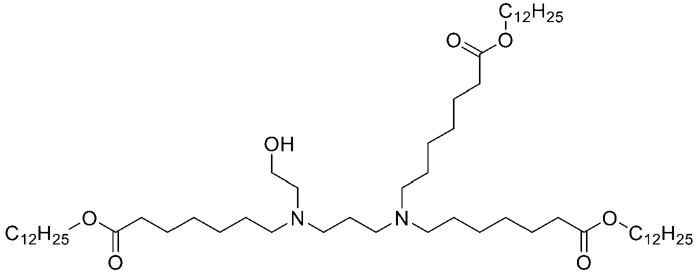

### Step 1: Synthesis of dodecyl 7-bromoheptanoate

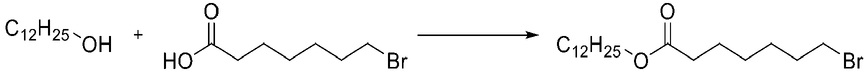

Using dodeca-1-ol and 7-bromoheptanoic acid as raw materials and according to step 1 of Example 3, dodecyl 7-bromoheptanoate was obtained. The crude product was purified by column chromatography to obtain a target molecule.

### Step 2: Synthesis of didodecyl 7,7'-((3-((7-(dodecyloxy)-7-oxoheptyl)(2-hydroxyethyl)amino)propyl)azanediyl)diheptanoate

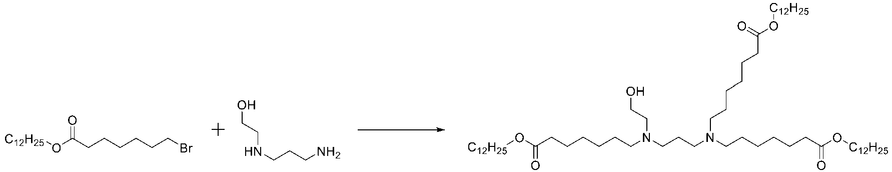

Using dodecyl 7-bromoheptanoate as the raw material and according to step 2 of Example 3, didodecyl 7,7'-((3-((7-(dodecyloxy)-7-oxoheptyl)(2-hydroxyethyl)amino)propyl)azanediyl)diheptanoate was obtained. The crude product was purified by column chromatography to obtain a target molecule.

ESI-MS *m*/*z:* cald for C₆₂H₁₂₃N₂O₇ [M+H]⁺: 1008.7, found 1008.7.

### Example 48

### Didodecyl 7,7'-((3-((7-(dodecyloxy)-7-oxoheptyl)(3-hydroxypropyl)amino)propyl)azanediyl)diheptanoate

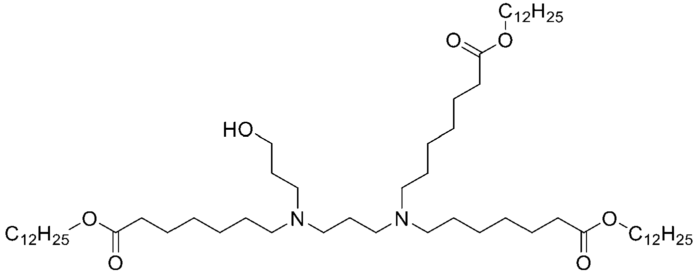

### Step 1: Synthesis of didodecyl 7,7'-((3-((7-(dodecyloxy)-7-oxoheptyl)amino)propyl)azanediyl)diheptanoate

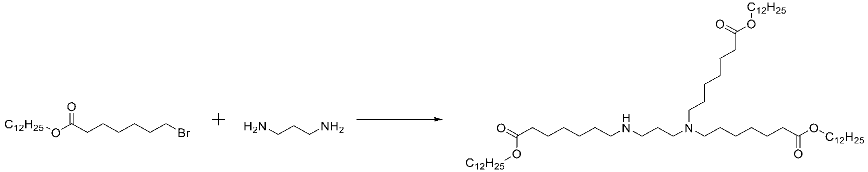

Using dodecyl 7-bromoheptanoate and 1,3-propanediamine as raw materials and according to step 2 of Example 18, didodecyl 7,7'-((3-((7-(dodecyloxy)-7-oxoheptyl)amino)propyl)azanediyl)diheptanoate was obtained. The crude product was purified by column chromatography to obtain a target molecule.

ESI-MS *m*/*z:* cald for C₆₀H₁₁₉N₂O₆ [M+H]⁺: 964.6, found 964.6.

### Step 3: Synthesis of didodecyl 7,7'-((3-((7-(dodecyloxy)-7-oxoheptyl)(3-hydroxypropyl)amino)propyl)azanediyl)diheptanoate

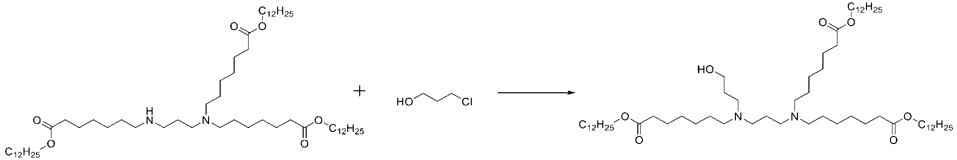

Using didodecyl 7,7'-((3-((7-(dodecyloxy)-7-oxoheptyl)amino)propyl)azanediyl)diheptanoate and 3-chloro-1-propanol as raw materials and according to step 3 of Example 18, didodecyl 7,7'-((3-((7-(dodecyloxy)-7-oxoheptyl)(3-hydroxypropyl)amino)propyl)azanediyl)diheptanoate was obtained. The crude product was purified by column chromatography to obtain a target molecule.

ESI-MS *m*/*z:* cald for C₆₃H₁₂₅N₂O₇ [M+H]⁺: 1022.7, found 1022.7.

### Example 49

### Didodecyl 7,7'-((3-((7-(dodecyloxy)-7-oxoheptyl)(4-hydroxybutyl)amino)propyl)azanediyl)diheptanoate

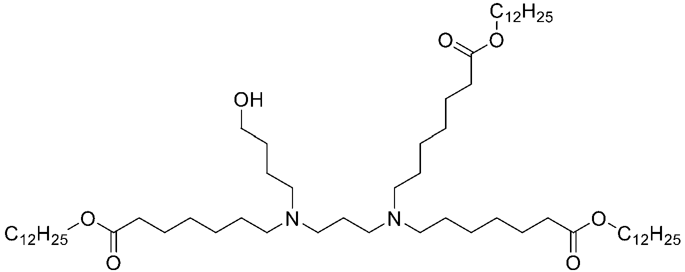

### Step 1: Synthesis of didodecyl 7,7'-((3-((7-(dodecyloxy)-7-oxoheptyl)(4-hydroxybutyl)amino)propyl)azanediyl)diheptanoate

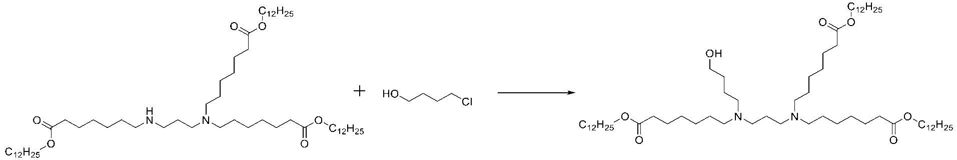

Using didodecyl 7,7'-((3-((7-(dodecyloxy)-7-oxoheptyl)amino)propyl)azanediyl)diheptanoate and 4-chloro-1-butanol as raw materials and according to step 3 of Example 18, didodecyl 7,7'-((3-((7-(dodecyloxy)-7-oxoheptyl)(4-hydroxybutyl)amino)propyl)azanediyl)diheptanoate was obtained. The crude product was purified by column chromatography to obtain a target molecule.

ESI-MS *m*/*z:* cald for C₆₄H₁₂₇N₂O₇ [M+H]⁺: 1036.7, found 1036.7.

### Example 50

### Didodecyl 8,8'-((3-((8-(dodecyloxy)-8-oxooctyl)(2-hydroxyethyl)amino)propyl)azanediyl)dioctanoate

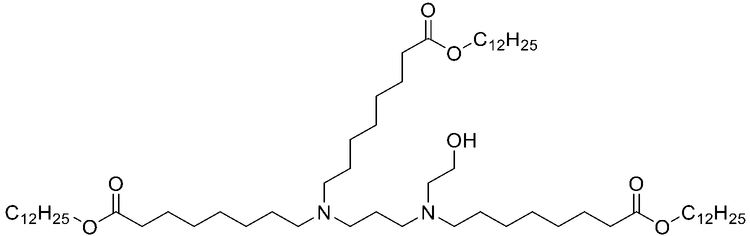

### Step 1: Synthesis of dodecyl 8-bromooctanoate

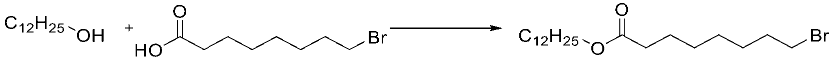

Using dodeca-1-ol and 8-bromooctanoic acid as raw materials and according to step 1 of Example 3, dodecyl 8-bromooctanoate was obtained. The crude product was purified by column chromatography to obtain a target molecule.

### Step 2: Synthesis of didodecyl 8,8'-((3-((8-(dodecyloxy)-8-oxooctyl)(2-hydroxyethyl)amino)propyl)azanediyl)dioctanoate

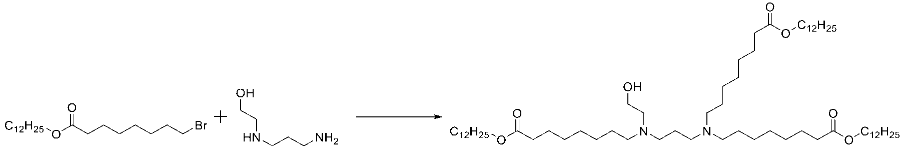

Using dodecyl 8-bromooctanoate as raw material and according to step 2 of Example 3, didodecyl 8,8'-((3-((8-(dodecyloxy)-8-oxooctyl)(2-hydroxyethyl)amino)propyl)azanediyl)dioctanoate was obtained. The crude product was purified by column chromatography to obtain a target molecule.

ESI-MS *m*/*z:* cald for C₆₅H₁₂₉N₂O₇ [M+H]⁺: 1050.7, found 1050.7.

### Example 51

### Didodecyl 8,8'-((3-((8-(dodecyloxy)-8-oxooctyl)(3-hydroxypropyl)amino)propyl)azanediyl)dioctanoate

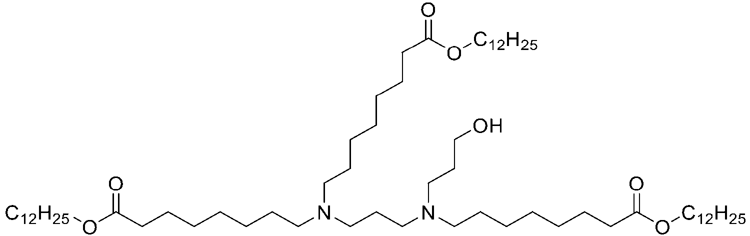

### Step 1: Synthesis of didodecyl 8,8'-((3-((8-(dodecyloxy)-8-oxooctyl)amino)propyl)azanediyl)dioctanoate

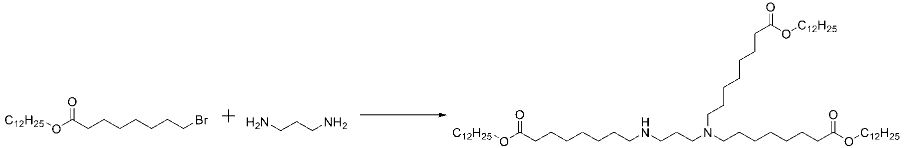

Using dodecyl 8-bromooctanoate and 1,3-propanediamine as raw materials and according to step 2 of Example 18, didodecyl 8,8'-((3-((8-(dodecyloxy)-8-oxooctyl)amino)propyl)azanediyl)dioctanoate was obtained. The crude product was purified by column chromatography to obtain a target molecule.

ESI-MS *m*/*z:* cald for C₆₃H₁₂₅N₂O₆ [M+H]⁺: 1006.7, found 1006.7.

### Step 3: Synthesis of didodecyl 8,8'-((3-((8-(dodecyloxy)-8-oxooctyl)(3-hydroxypropyl)amino)propyl)azanediyl)dioctanoate

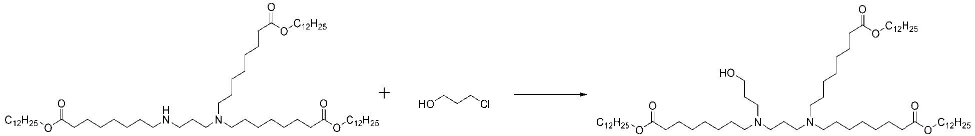

Using didodecyl 8,8'-((3-((8-(dodecyloxy)-8-oxooctyl)amino)propyl)azanediyl)dioctanoate and 3-chloro-1-propanol as raw materials and according to step 3 of Example 18, didodecyl 8,8'-((3-((8-(dodecyloxy)-8-oxooctyl)(3-hydroxypropyl)amino)propyl)azanediyl)dioctanoate was obtained. The crude product was purified by column chromatography to obtain a target molecule.

ESI-MS *m*/*z:* cald for C₆₆H₁₃₁N₂O₇ [M+H]⁺: 1064.8, found 1064.8.

### Example 52

### Didodecyl 8,8'-((3-((8-(dodecyloxy)-8-oxooctyl)(4-hydroxybutyl)amino)propyl)azanediyl)dioctanoate

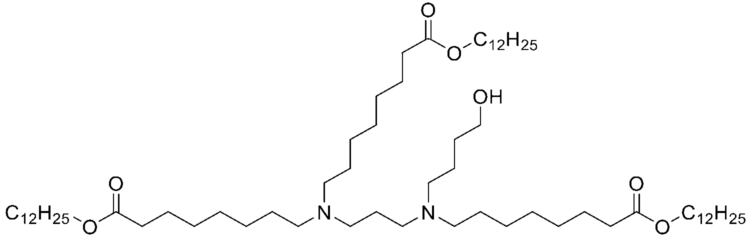

### Step 1: Synthesis of didodecyl 8,8'-((3-((8-(dodecyloxy)-8-oxooctyl)(4-hydroxybutyl)amino)propyl)azanediyl)dioctanoate

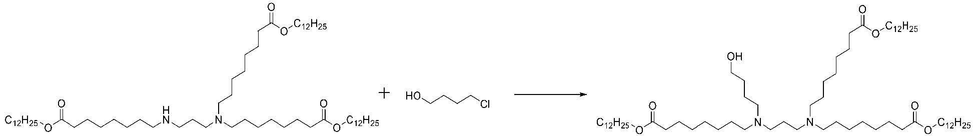

Using didodecyl 8,8'-((3-((8-(dodecyloxy)-8-oxooctyl)amino)propyl)azanediyl)dioctanoate and 4-chloro-1-butanol as raw materials and according to step 3 of Example 18, didodecyl 8,8'-((3-((8-(dodecyloxy)-8-oxooctyl)(4-hydroxybutyl)amino)propyl)azanediyl)dioctanoate was obtained. The crude product was purified by column chromatography to obtain a target molecule.

ESI-MS *m*/*z:* cald for C₆₇H₁₃₃N₂O₇ [M+H]⁺: 1078.8, found 1078.8.

### Example 53

### Ditetradecyl 6,6'-((3-((2-hydroxyethyl)(6-oxo-6-(tetradecyloxy)hexyl)amino)propyl)azanediyl)dihexanoate

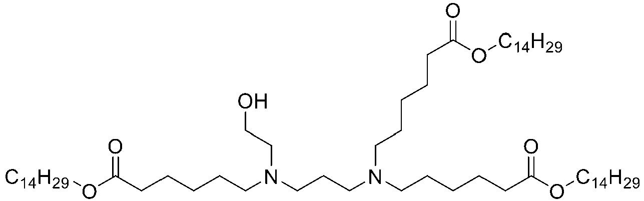

### Step 1: Synthesis of tetradecyl 6-bromohexanoate

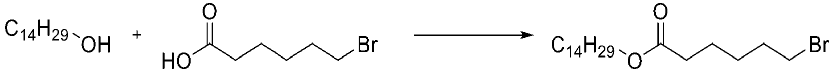

Using tetradeca-1-ol and 6-bromohexanoic acid as raw materials and according to step 1 of Example 3, tetradecyl 6-bromohexanoate was obtained. The crude product was purified by column chromatography to obtain a target molecule.

### Step 2: Synthesis of ditetradecyl 6,6'-((3-((2-hydroxyethyl)(6-oxo-6-(tetradecyloxy)hexyl)amino)propyl)azanediyl)dihexanoate

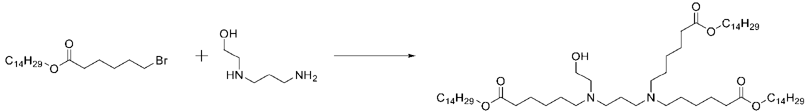

Using tetradecyl 6-bromohexanoate and 2-((3-aminopropyl)amino)ethane-1-ol as raw materials and according to step 2 of Example 3, ditetradecyl 6,6'-((3-((2-hydroxyethyl)(6-oxo-6-(tetradecyloxy)hexyl)amino)propyl)azanediyl)dihexanoate was obtained. The crude product was purified by column chromatography to obtain a target molecule.

ESI-MS *m*/*z:* cald for C₆₅H₁₂₉N₂O₇ [M+H]⁺: 1050.8, found 1050.8.

### Example 54

### Ditetradecyl 6,6'-((3-((3-hydroxypropyl)(6-oxo-6-(tetradecyloxy)hexyl)amino)propyl)azanediyl)dihexanoate

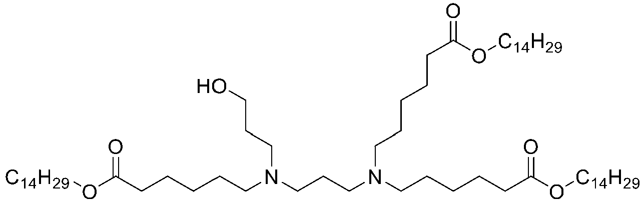

### Step 1: Synthesis of ditetradecyl 6,6'-((3-((6-oxo-6-(tetradecyloxy)hexyl)amino)propyl)azanediyl)dihexanoate

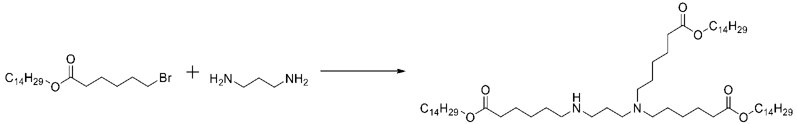

Using tetradecyl 6-bromohexanoate and 1,3-propanediamine as raw materials and according to step 2 of Example 18, ditetradecyl 6,6'-((3-((6-oxo-6-(tetradecyloxy)hexyl)amino)propyl)azanediyl)dihexanoate was obtained. The crude product was purified by column chromatography to obtain a target molecule.

ESI-MS *m*/*z:* cald for C₆₃H₁₂₅N₂O₆ [M+H]⁺: 1006.7, found 1006.7.

### Step 3: Synthesis of ditetradecyl 6,6'-((3-((3-hydroxypropyl)(6-oxo-6-(tetradecyloxy)hexyl)amino)propyl)azanediyl)dihexanoate

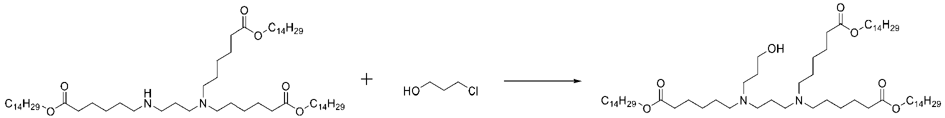

Using ditetradecyl 6,6'-((3-((6-oxo-6-(tetradecyloxy)hexyl)amino)propyl)azanediyl)dihexanoate and 3-chloro-1-propanol as raw materials and according to step 3 of Example 18, ditetradecyl 6,6'-((3-((3-hydroxypropyl)(6-oxo-6-(tetradecyloxy)hexyl)amino)propyl)azanediyl)dihexanoate was obtained. The crude product was purified by column chromatography to obtain a target molecule.

ESI-MS *m*/*z:* cald for C₆₆H₁₃₁N₂O₇ [M+H]⁺: 1064.8, found 1064.8.

### Example 55

### Ditetradecyl 6,6'-((3-((4-hydroxybutyl)(6-oxo-6-(tetradecyloxy)hexyl)amino)propyl)azanediyl)dihexanoate

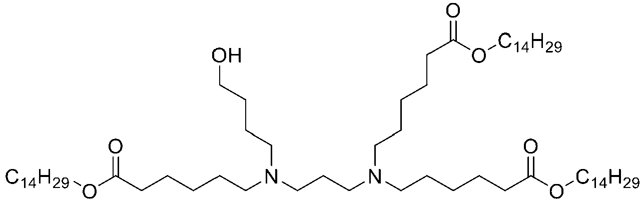

### Step 1: Synthesis of ditetradecyl 6,6'-((3-((4-hydroxybutyl)(6-oxo-6-(tetradecyloxy)hexyl)amino)propyl)azanediyl)dihexanoate

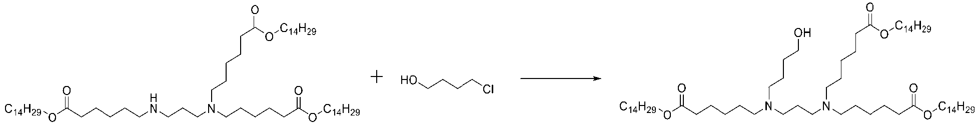

Using ditetradecyl 6,6'-((3-((6-oxo-6-(tetradecyloxy)hexyl)amino)propyl)azanediyl)dihexanoate and 4-chloro-1-butanol as raw materials and according to step 3 of Example 18, ditetradecyl 6,6'-((3-((4-hydroxybutyl)(6-oxo-6-(tetradecyloxy)hexyl)amino)propyl)azanediyl)dihexanoate was obtained. The crude product was purified by column chromatography to obtain a target molecule.

ESI-MS *m*/*z:* cald for C₆₇H₁₃₃N₂O₇ [M+H]⁺: 1078.8, found 1078.8.

### Example 56

### Ditetradecyl 7,7'-((3-((2-hydroxyethyl)(7-oxo-7-(tetradecyloxy)heptyl)amino)propyl)azanediyl)diheptanoate

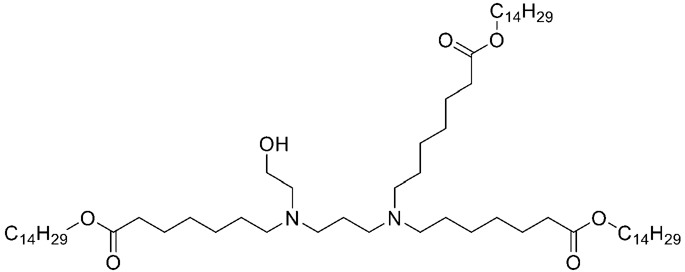

### Step 1: Synthesis of tetradecyl 7-bromoheptanoate

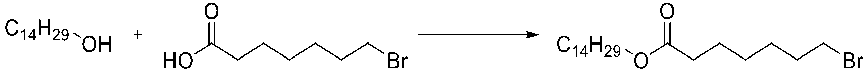

Using tetradeca-1-ol and 7-bromoheptanoic acid as raw materials and according to step 1 of Example 3, tetradecyl 7-bromoheptanoate was obtained. The crude product was purified by column chromatography to obtain a target molecule.

### Step 2: Synthesis of ditetradecyl 7,7'-((3-((2-hydroxyethyl)(7-oxo-7-(tetradecyloxy)heptyl)amino)propyl)azanediyl)diheptanoate

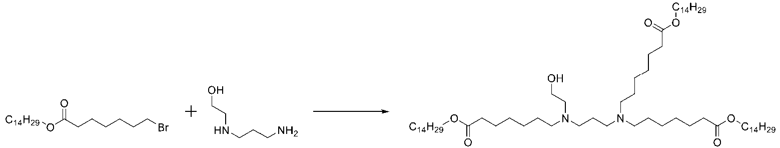

Using tetradecyl 7-bromoheptanoate as raw material and according to step 2 of Example 3, ditetradecyl 7,7'-((3-((2-hydroxyethyl)(7-oxo-7-(tetradecyloxy)heptyl)amino)propyl)azanediyl)diheptanoate was obtained. The crude product was purified by column chromatography to obtain a target molecule.

ESI-MS *m*/*z:* cald for C₆₈H₁₃₅N₂O₇ [M+H]⁺: 1092.8, found 1092.8.

### Example 57

### Ditetradecyl 7,7'-((3-((3-hydroxypropyl)(7-oxo-7-(tetradecyloxy)heptyl)amino)propyl)azanediyl)diheptanoate

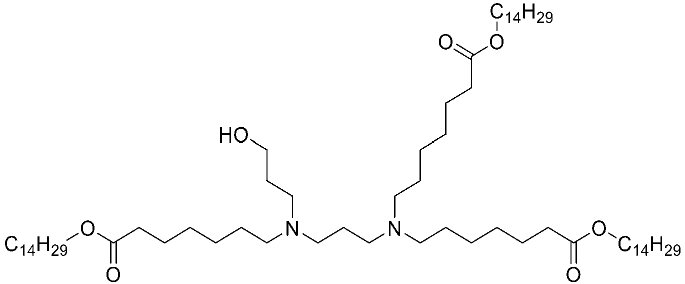

### Step 1: Synthesis of ditetradecyl 7,7'-((3-((7-oxo-7-(tetradecyloxy)heptyl)amino)propyl)azanediyl)diheptanoate

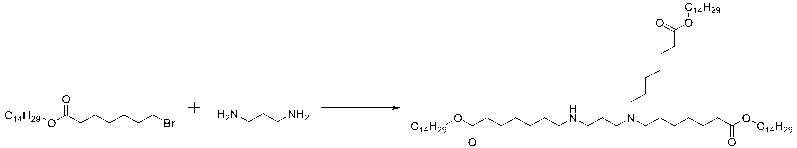

Using dodecyl 7-bromoheptanoate and 1,3-propanediamine as raw materials and according to step 2 of Example 18, ditetradecyl 7,7'-((3-((7-oxo-7-(tetradecyloxy)heptyl)amino)propyl)azanediyl)diheptanoate was obtained. The crude product was purified by column chromatography to obtain a target molecule.

ESI-MS *m*/*z:* cald for C₆₆H₁₃₁N₂O₆ [M+H]⁺: 1048.8, found 1048.8.

### Step 2: Synthesis of ditetradecyl 7,7'-((3-((3-hydroxypropyl)(7-oxo-7-(tetradecyloxy)heptyl)amino)propyl)azanediyl)diheptanoate

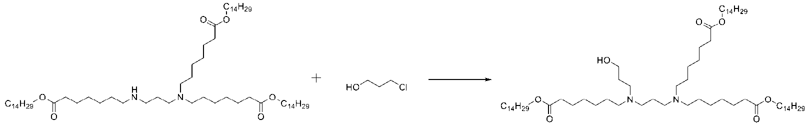

Using ditetradecyl 7,7'-((3-((7-oxo-7-(tetradecyloxy)heptyl)amino)propyl)azanediyl)diheptanoate and 3-chloro-1-propanol as raw materials and according to step 3 of Example 18, ditetradecyl 7,7'-((3-((3-hydroxypropyl)(7-oxo-7-(tetradecyloxy)heptyl)amino)propyl)azanediyl)diheptanoate was obtained. The crude product was purified by column chromatography to obtain a target molecule.

ESI-MS *m*/*z:* cald for C₆₆H₁₃₇N₂O₇ [M+H]⁺: 1106.9, found 1106.9.

### Example 58

### Ditetradecyl 7,7'-((3-((4-hydroxybutyl)(7-oxo-7-(tetradecyloxy)heptyl)amino)propyl)azanediyl)diheptanoate

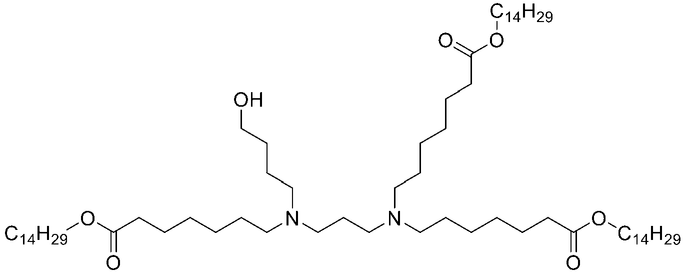

### Step 1: Synthesis of ditetradecyl 7,7'-((3-((4-hydroxybutyl)(7-oxo-7-(tetradecyloxy)heptyl)amino)propyl)azanediyl)diheptanoate

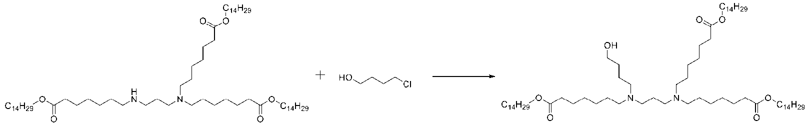

Using ditetradecyl 7,7'-((3-((7-oxo-7-(tetradecyloxy)heptyl)amino)propyl)azanediyl)diheptanoate and 4-chloro-1-butanol as raw materials and according to step 3 of Example 18, ditetradecyl 7,7'-((3-((4-hydroxybutyl)(7-oxo-7-(tetradecyloxy)heptyl)amino)propyl)azanediyl)diheptanoate was obtained. The crude product was purified by column chromatography to obtain a target molecule.

ESI-MS *m*/*z:* cald for C₇₀H₁₃₉N₂O₇ [M+H]⁺: 1120.9, found 1120.9.

### Example 59

### Ditetradecyl 8,8'-((3-((2-hydroxyethyl)(8-oxo-8-(tetradecyloxy)octyl)amino)propyl)azanediyl)dioctanoate

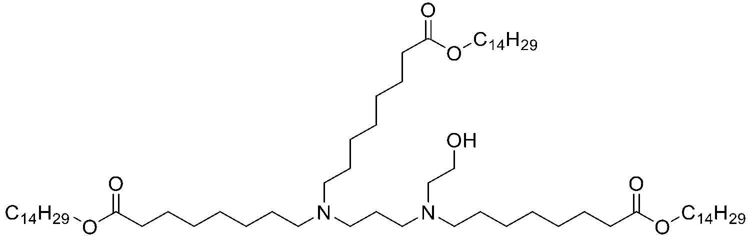

### Step 1: Synthesis of tetradecyl 8-bromooctanoate

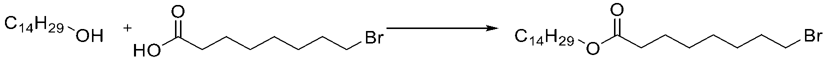

Using tetradeca-1-ol and 8-bromooctanoic acid as raw materials and according to step 1 of Example 3, tetradecyl 8-bromooctanoate was obtained. The crude product was purified by column chromatography to obtain a target molecule.

### Step 2: Synthesis of ditetradecyl 8,8'-((3-((2-hydroxyethyl)(8-oxo-8-(tetradecyloxy)octyl)amino)propyl)azanediyl)dioctanoate

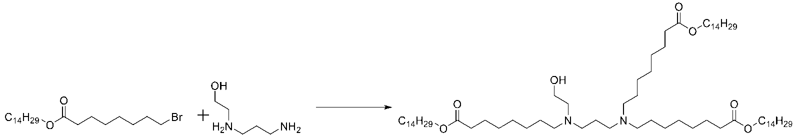

Using tetradecyl 8-bromooctanoate as raw material and according to step 2 of Example 3, ditetradecyl 8,8'-((3-((2-hydroxyethyl)(8-oxo-8-(tetradecyloxy)octyl)amino)propyl)azanediyl)dioctanoate was obtained. The crude product was purified by column chromatography to obtain a target molecule.

ESI-MS *m*/*z:* cald for C₇₁H₁₄₁N₂O₇ [M+H]⁺: 1134.9, found 1134.9.

### Example 60

### Ditetradecyl 8,8'-((3-((3-hydroxypropyl)(8-oxo-8-(tetradecyloxy)octyl)amino)propyl)azanediyl)dioctanoate

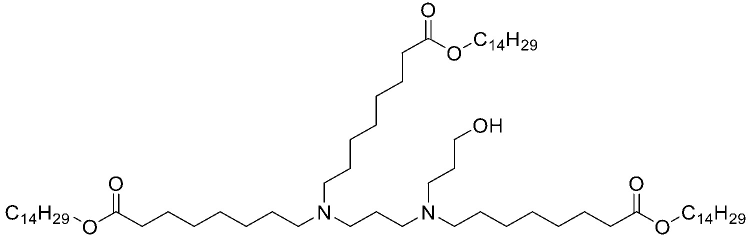

### Step 1: Synthesis of ditetradecyl 8,8'-((3-((8-oxo-8-(tetradecyloxy)octyl)amino)propyl)azanediyl)dioctanoate

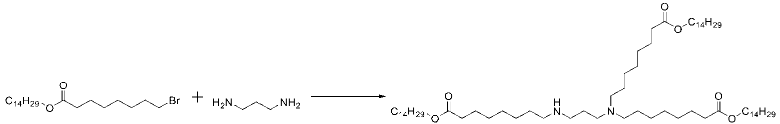

Using tetradecyl 8-bromooctanoate and 1,3-propanediamine as raw materials and according to step 2 of Example 18, ditetradecyl 8,8'-((3-((8-oxo-8-(tetradecyloxy)octyl)amino)propyl)azanediyl)dioctanoate was obtained. The crude product was purified by column chromatography to obtain a target molecule.

ESI-MS *m*/*z:* cald for C₆₉H₁₃₇N₂O₆ [M+H]⁺: 1090.9, found 1090.9.

### Step 3: Synthesis of ditetradecyl 8,8'-((3-((3-hydroxypropyl)(8-oxo-8-(tetradecyloxy)octyl)amino)propyl)azanediyl)dioctanoate

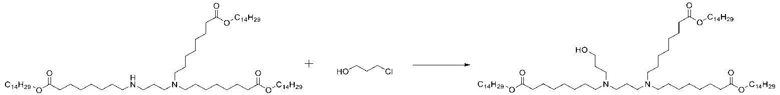

Using ditetradecyl 8,8'-((3-((8-oxo-8-(tetradecyloxy)octyl)amino)propyl)azanediyl)dioctanoate and 3-chloro-1-propanol as raw materials and according to step 3 of Example 18, ditetradecyl 8,8'-((3-((3-hydroxypropyl)(8-oxo-8-(tetradecyloxy)octyl)amino)propyl)azanediyl)dioctanoate was obtained. The crude product was purified by column chromatography to obtain a target molecule.

ESI-MS *m*/*z:* cald for C₇₂H₁₄₃N₂O₇ [M+H]⁺: 1148.9, found 1148.9.

### Example 61

### Ditetradecyl 8,8'-((3-((4-hydroxybutyl)(8-oxo-8-(tetradecyloxy)octyl)amino)propyl)azanediyl)dioctanoate

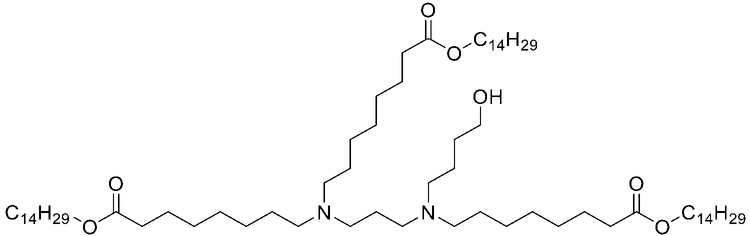

### Step 1: Synthesis of ditetradecyl 8,8'-((3-((4-hydroxybutyl)(8-oxo-8-(tetradecyloxy)octyl)amino)propyl)azanediyl)dioctanoate

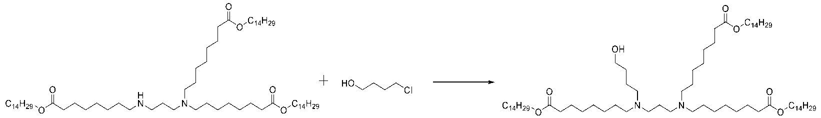

Using ditetradecyl 8,8'-((3-((8-oxo-8-(tetradecyloxy)octyl)amino)propyl)azanediyl)dioctanoate and 4-chloro-1-butanol as raw materials and according to step 3 of Example 18, ditetradecyl 8,8'-((3-((4-hydroxybutyl)(8-oxo-8-(tetradecyloxy)octyl)amino)propyl)azanediyl)dioctanoate was obtained. The crude product was purified by column chromatography to obtain a target molecule.

ESI-MS *m*/*z:* cald for C₇₃H₁₄₅N₂O₇ [M+H]⁺: 1163.0, found 1163.0.

### Example 62

### Ditridecyl 4,4'-((3-((2-hydroxyethyl)(4-oxo-4-(tridecyloxy)butyl)amino)propyl)azanediyl)dibutanoate

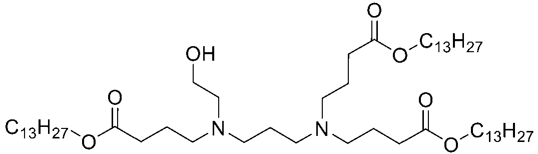

### Step 1: Synthesis of tridecyl 4-bromobutanoate

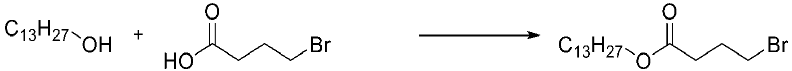

Using trideca-1-ol and 4-bromobutyric acid as raw materials and according to step 1 of Example 3, tridecyl 4-bromobutanoate was obtained. The crude product was purified by column chromatography to obtain 8.3 g of a target molecule with a yield of 95.4%.

¹H NMR (400 MHz, CDCl₃) δ 4.07 (t, J = 6.8 Hz, 2H), 3.47 (t, J = 6.5 Hz, 2H), 2.50 (t, J = 7.2 Hz, 2H), 2.22 - 2.12 (m, 2H), 1.67 - 1.57 (m, 2H), 1.33 - 1.22 (m, 20H), 0.88 (t, J = 6.7 Hz, 3H).

### Step 2: Synthesis of ditridecyl 4,4'-((3-((2-hydroxyethyl)(4-oxo-4-(tridecyloxy)butyl)amino)propyl)azanediyl)dibutanoate

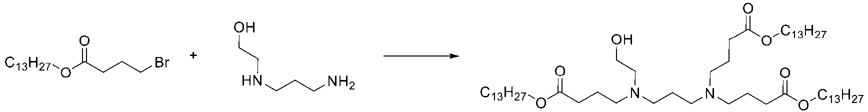

Using tridecyl 4-bromobutanoate and 2-((3-aminopropyl)amino)ethane-1-ol as raw material and according to step 2 of Example 3, ditridecyl 4,4'-((3-((2-hydroxyethyl)(4-oxo-4-(tridecyloxy)butyl)amino)propyl)azanediyl)dibutanoate was obtained. The crude product was purified by column chromatography to obtain 110 mg of a target molecule with a yield of 23.4%.

ESI-MS *m*/*z:* cald for C₅₆H₁₁₁N₂O₇ [M+H]⁺: 923.8, found 923.8.

¹H NMR (400 MHz, CDCl₃) δ4.11 - 4.01 (m, 6H), 3.55 (t, J = 5.2 Hz, 2H), 2.57 (t, J = 5.2 Hz, 2H), 2.52 - 2.38 (m, 10H), 2.35 - 2.27 (m, 6H), 1.81 - 1.69 (m, 6H), 1.61 (m, 8H), 1.32 - 1.23 (m, 61H), 0.88 (t, J = 6.7 Hz, 9H).

### Example 63

### Ditridecyl 4,4'-((3-((3-hydroxypropyl)(4-oxo-4-(tridecyloxy)butyl)amino)propyl)azanediyl)dibutanoate

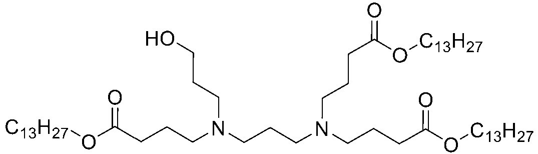

### Step 1: Synthesis of tridecyl 4-bromobutanoate

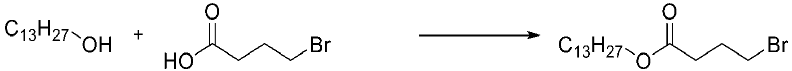

Using trideca-1-ol and 4-bromobutyric acid as raw materials and according to step 1 of Example 3, tridecyl 4-bromobutanoate was obtained. The crude product was purified by column chromatography to obtain a target molecule.

### Step 2: ditridecyl 4,4'-((3-((3-hydroxypropyl)(4-oxo-4-(tridecyloxy)butyl)amino)propyl)azanediyl)dibutanoate

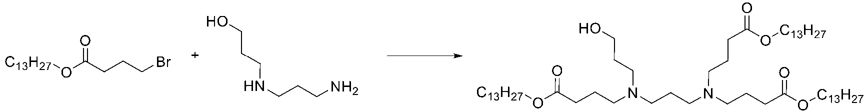

Using tridecyl 4-bromobutanoate and 3-((3-aminopropyl)amino)propan-1-ol as raw materials and according to step 2 of Example 3, ditridecyl 4,4'-((3-((3-hydroxypropyl)(4-oxo-4-(tridecyloxy)butyl)amino)propyl)azanediyl)dibutanoate was obtained. The crude product was purified by column chromatography to obtain 106 mg of a target molecule with a yield of 16.6%.

### ESI-MS m/z: cald for C₅₇H₁₁₃N₂O₇ [M+H]⁺: 937.8, found 937.8.

¹H NMR (400 MHz, CDCl₃) δ 4.04 (t, J = 6.8 Hz, 6H), 3.78 (t, J = 5.2 Hz, 2H), 2.67 - 2.60 (m, 2H), 2.50 - 2.36 (m, 14H), 2.34 - 2.28 (m, 8H), 1.74 - 1.58 (m, 21H), 1.26 (m, 50H), 0.88 (s, 9H).

### Example 64

### Diundecyl 4,4'-((3-((3-hydroxypropyl)(4-oxo-4-(undecyloxy)butyl)amino)propyl)azanediyl)dibutanoate

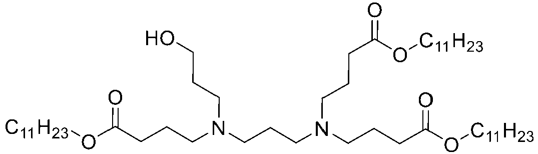

### Step 1: Synthesis of undecyl 4-bromobutanoate

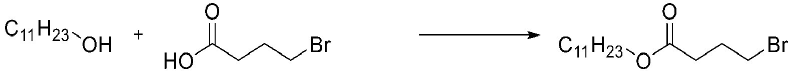

Using undeca-1-ol and 4-bromobutyric acid as raw materials and according to step 1 of Example 3, undecyl 4-bromobutanoate was obtained. The crude product was purified by column chromatography to obtain a target molecule.

### Step 2: Synthesis of diundecyl 4,4'-((3-((3-hydroxypropyl)(4-oxo-4-(undecyloxy)butyl)amino)propyl)azanediyl)dibutanoate

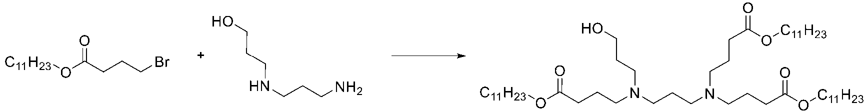

Using undecyl 4-bromobutanoate and 3-((3-aminopropyl)amino)propan-1-ol as raw materials and according to step 2 of Example 3, diundecyl 4,4'-((3-((3-hydroxypropyl)(4-oxo-4-(undecyloxy)butyl)amino)propyl)azanediyl)dibutanoate was obtained. The crude product was purified by column chromatography to obtain 93 mg of a target molecule with a yield of 13.9%.

ESI-MS *m*/*z:* cald for C₅₁H₁₀₁N₂O₇ [M+H]⁺: 853.7, found 853.7.

¹H NMR (400 MHz, CDCl₃) δ 4.05 (t, J = 6.8 Hz, 6H), 3.78 (t, J = 5.2 Hz, 2H), 2.64 (t, J = 5.6 Hz, 2H), 2.50 - 2.36 (m, 10H), 2.31 (t, J = 7.5 Hz, 6H), 1.78 - 1.57 (m, 16H), 1.33 - 1.22 (m, 49H), 0.88 (t, J = 6.8 Hz, 9H).

### Example 65

### Ditridecyl 4,4'-((3-((4-hydroxybutyl)(4-oxo-4-(tridecyloxy)butyl)amino)propyl)azanediyl)dibutanoate

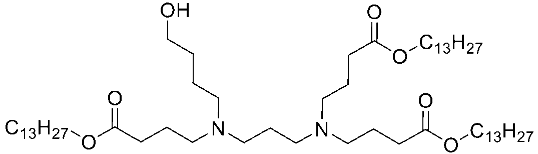

### Step 1: Synthesis of tridecyl 4-bromobutanoate

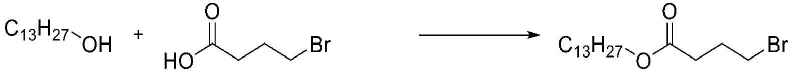

Using trideca-1-ol and 4-bromobutyric acid as raw materials and according to step 1 of Example 3, tridecyl 4-bromobutanoate was obtained. The crude product was purified by column chromatography to obtain a target molecule.

### Step 2: Synthesis of ditridecyl 4,4'-((3-((4-hydroxybutyl)(4-oxo-4-(tridecyloxy)butyl)amino)propyl)azanediyl)dibutanoate

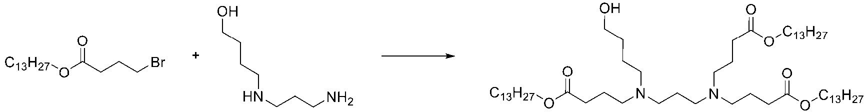

Using tridecyl 4-bromobutanoate and 4-((3-aminopropyl)amino)butan-1-ol as raw materials and according to step 2 of Example 3, ditridecyl 4,4'-((3-((4-hydroxybutyl)(4-oxo-4-(tridecyloxy)butyl)amino)propyl)azanediyl)dibutanoate was obtained. The crude product was purified by column chromatography to obtain a target molecule.

ESI-MS *m*/*z:* cald for C₅₈H₁₁₅N₂O₇ [M+H]⁺: 951.8, found 951.8.

### Example 66

### Diundecyl 4,4'-((3-((4-hydroxybutyl)(4-oxo-4-(undecyloxy)butyl)amino)propyl)azanediyl)dibutanoate

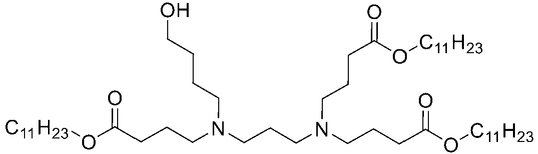

### Step 1: Synthesis of undecyl 4-bromobutanoate

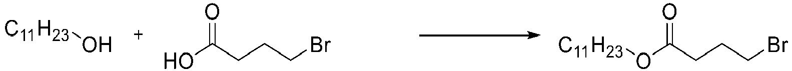

Using undeca-1-ol and 4-bromobutyric acid as raw materials and according to step 1 of Example 3, undecyl 4-bromobutanoate was obtained. The crude product was purified by column chromatography to obtain a target molecule.

### Step 2: diundecyl 4,4'-((3-((4-hydroxybutyl)(4-oxo-4-(undecyloxy)butyl)amino)propyl)azanediyl)dibutanoate

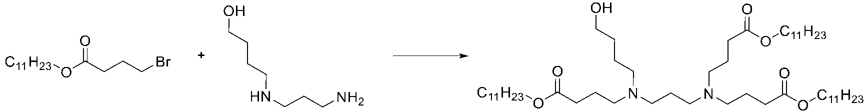

Using undecyl 4-bromobutanoate and 4-((3-aminopropyl)amino)butan-1-ol as raw materials and according to step 2 of Example 3, diundecyl 4,4'-((3-((4-hydroxybutyl)(4-oxo-4-(undecyloxy)butyl)amino)propyl)azanediyl)dibutanoate was obtained. The crude product was purified by column chromatography to obtain a target molecule.

ESI-MS *m*/*z:* cald for C₅₂H₁₀₃N₂O₇ [M+H]⁺: 868.4, found 869.4.

### Example 67

### Diundecyl 3,3'-((3-((2-hydroxyethyl)(3-oxo-3-(undecyloxy)propyl)amino)propyl)azanediyl)dipropionate

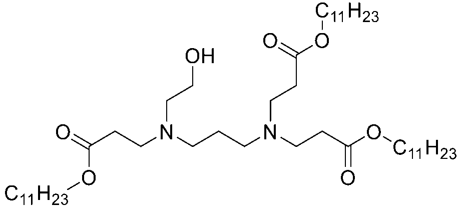

### Step 1: Synthesis of undecyl 3-bromopropionate

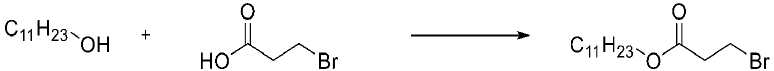

Using undeca-1-ol and 3-bromopropionic acid as raw materials and according to step 1 of Example 3, undecyl 3-bromopropionate was obtained. The crude product was purified by column chromatography to obtain 4 g of a target molecule with a yield of 44.89%.

### Step 2: Synthesis of diundecyl 3,3'-((3-((2-hydroxyethyl)(3-oxo-3-(undecyloxy)propyl)amino)propyl)azanediyl)dipropionate

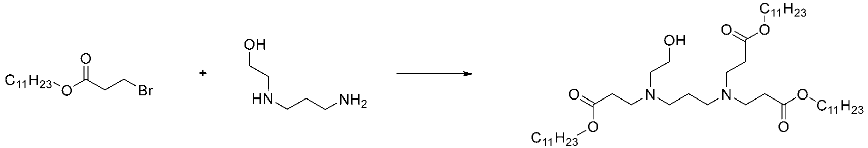

Using undecyl 3-bromopropionate and 2-((3-aminopropyl)amino)ethane-1-ol as raw materials and according to step 2 of Example 3, diundecyl 3,3'-((3-((2-hydroxyethyl)(3-oxo-3-(undecyloxy)propyl)amino)propyl)azanediyl)dipropionate was obtained. The crude product was purified by column chromatography to obtain 0.15 g of a target molecule with a yield of 14.7%.

ESI-MS *m*/*z:* cald for C₄₇H₉₃N₂O₇ [M+H]⁺: 797.7, found 797.7.

¹H NMR (400 MHz, CDCl₃) δ 4.06 (m, 6H), 3.56 (t, J = 5.1 Hz, 2H), 2.85-2.69 (m, 6H), 2.61-2.54 (m, 2H), 2.54-2.35 (m, 10H), 1.72-1.52 (m, 8H), 1.41 - 1.18 (m, 49H), 0.88 (t, J = 6.8 Hz, 9H).

### Example 68

### Diundecyl 3,3'-((3-((3-hydroxypropyl)(3-oxo-3-(undecyloxy)propyl)amino)propyl)azanediyl)dipropionate

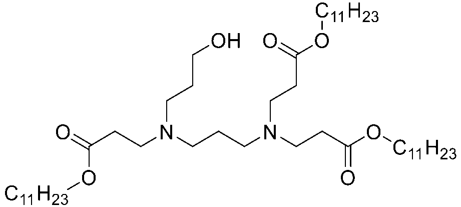

### Step 1: Synthesis of undecyl 3-bromopropionate

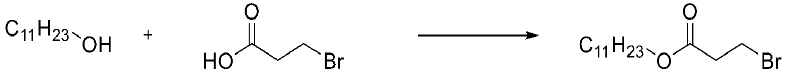

Using undeca-1-ol and 3-bromopropionic acid as raw materials and according to step 1 of Example 3, undecyl 3-bromopropionate was obtained. The crude product was purified by column chromatography to obtain a target molecule.

### Step 2: Synthesis of diundecyl 3,3'-((3-((3-hydroxypropyl)(3-oxo-3-(undecyloxy)propyl)amino)propyl)azanediyl)dipropionate

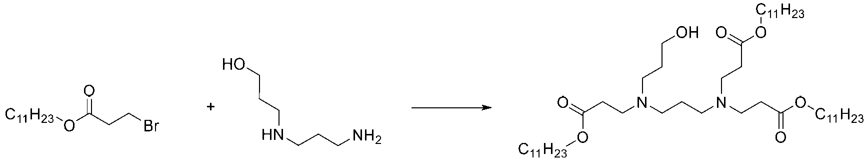

Using undecyl 3-bromopropionate and 3-((3-aminopropyl)amino)propan-1-ol as raw materials and according to step 2 of Example 3, diundecyl 3,3'-((3-((3-hydroxypropyl)(3-oxo-3-(undecyloxy)propyl)amino)propyl)azanediyl)dipropionate was obtained. The crude product was purified by column chromatography to obtain a target molecule.

ESI-MS *m*/*z:* cald for C₄₈H₉₅N₂O₇ [M+H]⁺: 811.7, found 811.7.

### Example 69

### Diundecyl 3,3'-((3-((4-hydroxybutyl)(3-oxo-3-(undecyloxy)propyl)amino)propyl)azanediyl)dipropionate

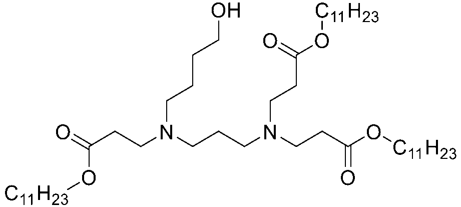

### Step 1: Synthesis of undecyl 3-bromopropionate

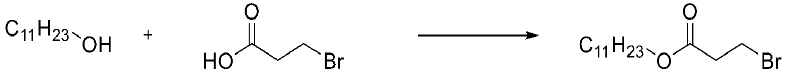

Using undeca-1-ol and 3-bromopropionic acid as raw materials and according to step 1 of Example 3, undecyl 3-bromopropionate was obtained. The crude product was purified by column chromatography to obtain a target molecule.

### Step 4: Synthesis of diundecyl 3,3'-((3-((4-hydroxybutyl)(3-oxo-3-(undecyloxy)propyl)amino)propyl)azanediyl)dipropionate

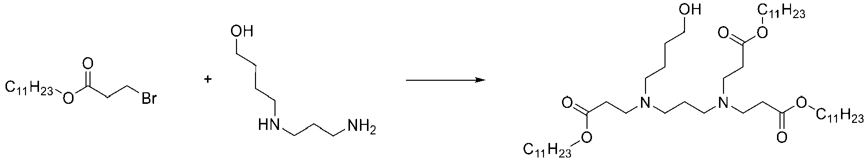

Using undecyl 3-bromopropionate and 4-((3-aminopropyl)amino)butan-1-ol as raw materials and according to step 2 of Example 3, diundecyl 3,3'-((3-((4-hydroxybutyl)(3-oxo-3-(undecyloxy)propyl)amino)propyl)azanediyl)dipropionate was obtained. The crude product was purified by column chromatography to obtain a target molecule.

ESI-MS *m*/*z:* cald for C₄₉H₉₇N₂O₇ [M+H]⁺: 825.7, found 825.7.

### Example 70

### Ditridecyl 3,3'-((3-((2-hydroxyethyl)(3-oxo-3-(tridecyloxy)propyl)amino)propyl)azanediyl)dipropionate

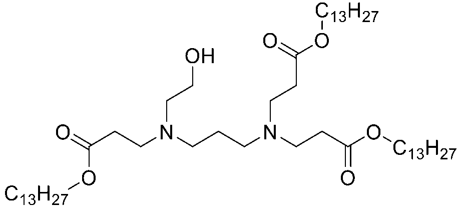

### Step 1: Synthesis of tridecyl 3-bromopropionate

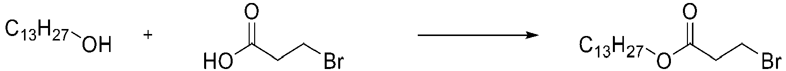

Using trideca-1-ol and 3-bromopropionic acid as raw materials and according to step 1 of Example 3, tridecyl 3-bromopropionate was obtained. The crude product was purified by column chromatography to obtain 1.8 g of a target molecule with a yield of 41.35%.

### Step 2: Ditridecyl 3,3'-((3-((2-hydroxyethyl)(3-oxo-3-(tridecyloxy)propyl)amino)propyl)azanediyl)dipropionate

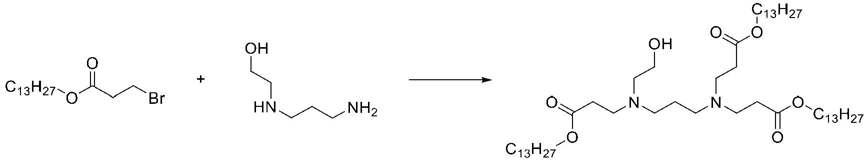

Using tridecyl 3-bromopropionate and 2-((3-aminopropyl)amino)ethane-1-ol as raw materials and according to step 2 of Example 3, ditridecyl 3,3'-((3-((2-hydroxyethyl)(3-oxo-3-(tridecyloxy)propyl)amino)propyl)azanediyl)dipropionate was obtained. The crude product was purified by column chromatography to obtain 0.18 g of a target molecule with a yield of 20.42%.

ESI-MS *m*/*z:* cald for C₅₃H₁₀₅N₂O₇ [M+H]⁺: 881.8, found 881.8.

¹H NMR (400 MHz, CDCl₃) δ 4.06 (m, 6H), 3.56 (t, J = 5.0 Hz, 2H), 2.90-2.69 (m, 6H), 2.65 - 2.53 (m, 2H), 2.52 - 2.35 (m, 10H), 1.73-1.50 (m, 8H), 1.45 - 1.16 (m, 61H), 0.88 (t, J = 6.8 Hz, 9H).

### Example 71

### Ditridecyl 3,3'-((3-((3-hydroxypropyl)(3-oxo-3-(tridecyloxy)propyl)amino)propyl)azanediyl)dipropionate

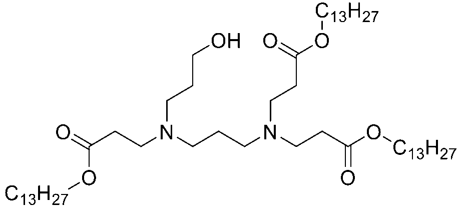

### Step 1: Synthesis of tridecyl 3-bromopropionate

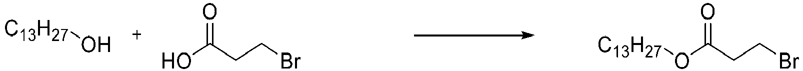

Using trideca-1-ol and 3-bromopropionic acid as raw materials and according to step 1 of Example 3, tridecyl 3-bromopropionate was obtained. The crude product was purified by column chromatography to obtain a target molecule.

### Step 3: Synthesis of ditridecyl 3,3'-((3-((3-hydroxypropyl)(3-oxo-3-(tridecyloxy)propyl)amino)propyl)azanediyl)dipropionate

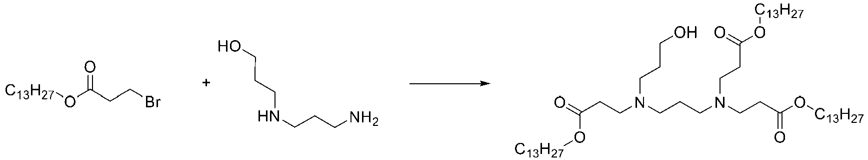

Using tridecyl 3-bromopropionate and 3-((3-aminopropyl)amino)propan-1-ol as raw materials and according to step 2 of Example 3, ditridecyl 3,3'-((3-((3-hydroxypropyl)(3-oxo-3-(tridecyloxy)propyl)amino)propyl)azanediyl)dipropionate was obtained. The crude product was purified by column chromatography to obtain a target molecule.

ESI-MS *m*/*z:* cald for C₅₄H₁₀₇N₂O₇ [M+H]⁺: 895.8, found 895.8.

### Example 72

### Ditridecyl 3,3'-((3-((4-hydroxybutyl)(3-oxo-3-(tridecyloxy)propyl)amino)propyl)azanediyl)dipropionate

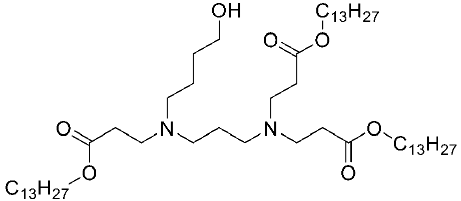

### Step 1: Synthesis of tridecyl 3-bromopropionate

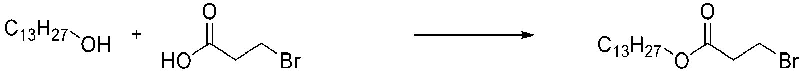

Using trideca-1-ol and 3-bromopropionic acid as raw materials and according to step 1 of Example 3, tridecyl 3-bromopropionate was obtained. The crude product was purified by column chromatography to obtain a target molecule.

### Step 3: Synthesis of ditridecyl 3,3'-((3-((4-hydroxybutyl)(3-oxo-3-(tridecyloxy)propyl)amino)propyl)azanediyl)dipropionate

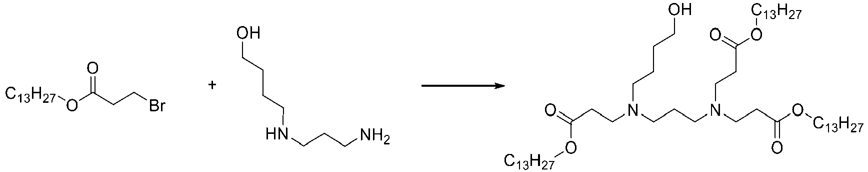

Using tridecyl 3-bromopropionate and 4-((3-aminopropyl)amino)butan-1-ol as raw materials and according to step 2 of Example 3, ditridecyl 3,3'-((3-((4-hydroxybutyl)(3-oxo-3-(tridecyloxy)propyl)amino)propyl)azanediyl)dipropionate was obtained. The crude product was purified by column chromatography to obtain a target molecule.

ESI-MS *m*/*z:* cald for C₅₅H₁₀₉N₂O₇ [M+H]⁺: 909.8, found 909.8.

### Example 73

### Diundecyl 2,2'-((3-((2-hydroxyethyl)(2-oxo-2-(undecyloxy)ethyl)amino)propyl)azanediyl)diacetate

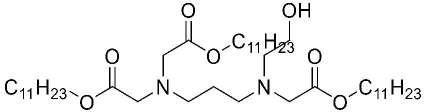

### Step 1: Synthesis of undecyl 2-bromoacetate

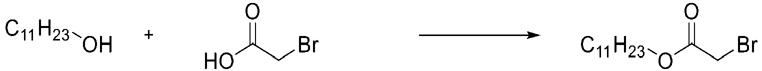

Using undeca-1-ol and 3-bromoacetic acid as raw materials and according to step 1 of Example 3, undecyl 2-bromoacetate was obtained. The crude product was purified by column chromatography to obtain a target molecule.

### Step 2: Synthesis of diundecyl 2,2'-((3-((2-hydroxyethyl)(2-oxo-2-(undecyloxy)ethyl)amino)propyl)azanediyl)diacetate

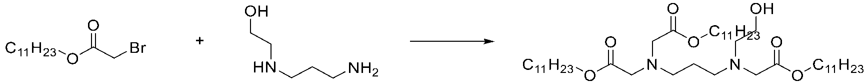

Using undecyl 2-bromoacetate and 2-((3-aminopropyl)amino)ethane-1-ol as raw materials and according to step 2 of Example 3, diundecyl 2,2'-((3-((2-hydroxyethyl)(2-oxo-2-(undecyloxy)ethyl)amino)propyl)azanediyl)diacetate was obtained. The crude product was purified by column chromatography to obtain a target molecule.

ESI-MS *m*/*z:* cald for C₄₄H₈₇N₂O₇ [M+H]⁺: 755.6, found 755.6.

### Example 74

### Diundecyl 2,2'-((3-((3-hydroxypropyl)(2-oxo-2-(undecyloxy)ethyl)amino)propyl)azanediyl)diacetate

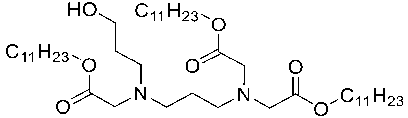

### Step 1: Synthesis of undecyl 2-bromoacetate

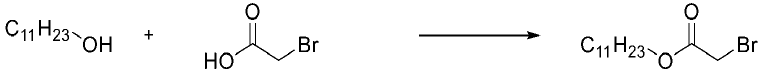

Using undeca-1-ol and 3-bromoacetic acid as raw materials and according to step 1 of Example 3, undecyl 2-bromoacetate was obtained. The crude product was purified by column chromatography to obtain a target molecule.

### Step 2: Synthesis of diundecyl 2,2'-((3-((2-hydroxyethyl)(2-oxo-2-(undecyloxy)ethyl)amino)propyl)azanediyl)diacetate

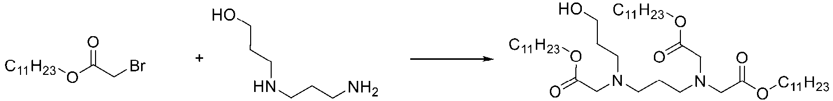

Using undecyl 2-bromoacetate and 3-((3-aminopropyl)amino)propan-1-ol as raw materials and according to step 2 of Example 3, diundecyl 2,2'-((3-((2-hydroxyethyl)(2-oxo-2-(undecyloxy)ethyl)amino)propyl)azanediyl)diacetate was obtained. The crude product was purified by column chromatography to obtain a target molecule.

ESI-MS *m*/*z:* cald for C₄₅H₈₉N₂O₇ [M+H]⁺: 769.6, found 769.6.

### Example 75

### Diundecyl 2,2'-((3-((4-hydroxybutyl)(2-oxo-2-(undecyloxy)ethyl)amino)propyl)azanediyl)diacetate

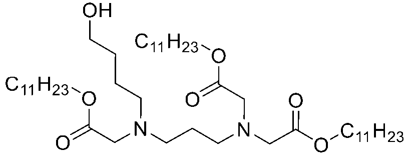

### Step 1: Synthesis of undecyl 2-bromoacetate

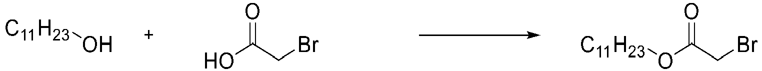

Using undeca-1-ol and 3-bromoacetic acid as raw materials and according to step 1 of Example 3, undecyl 2-bromoacetate was obtained. The crude product was purified by column chromatography to obtain a target molecule.

### Step 2: Synthesis of diundecyl 2,2'-((3-((4-hydroxybutyl)(2-oxo-2-(undecyloxy)ethyl)amino)propyl)azanediyl)diacetate

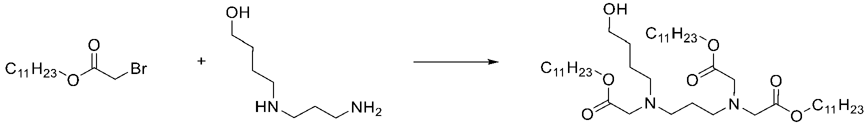

Using undecyl 2-bromoacetate and 4-((3-aminopropyl)amino)butan-1-ol as raw materials and according to step 2 of Example 3, diundecyl 2,2'-((3-((4-hydroxybutyl)(2-oxo-2-(undecyloxy)ethyl)amino)propyl)azanediyl)diacetate was obtained. The crude product was purified by column chromatography to obtain a target molecule.

ESI-MS *m*/*z:* cald for C₄₆H₉₁N₂O₇ [M+H]⁺: 783.6, found 783.6.

### Example 76

### Ditridecyl 2,2'-((3-((2-hydroxyethyl)(2-oxo-2-(tridecyloxy)ethyl)amino)propyl)azanediyl)diacetate

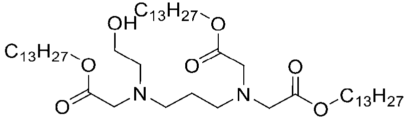

### Step 1: Synthesis of tridecyl 2-bromoacetate

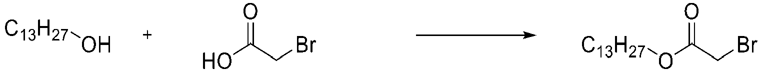

Using trideca-1-ol and 2-bromoacetic acid as raw materials and according to step 1 of Example 3, tridecyl 2-bromoacetate was obtained. The crude product was purified by column chromatography to obtain a target molecule.

### Step 2: Ditridecyl 2,2'-((3-((2-hydroxyethyl)(2-oxo-2-(tridecyloxy)ethyl)amino)propyl)azanediyl)diacetate

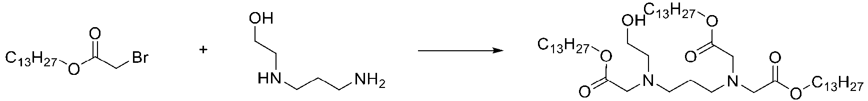

Using tridecyl 2-bromoacetate and 2-((3-aminopropyl)amino)ethane-1-ol as raw materials and according to step 2 of Example 3, ditridecyl 2,2'-((3-((2-hydroxyethyl)(2-oxo-2-(tridecyloxy)ethyl)amino)propyl)azanediyl)diacetate was obtained. The crude product was purified by column chromatography to obtain a target molecule.

ESI-MS *m*/*z:* cald for C₅₀H₉₉N₂O₇ [M+H]⁺: 834.7, found 834.7.

### Example 77

### Ditridecyl 2,2'-((3-((3-hydroxypropyl)(2-oxo-2-(tridecyloxy)ethyl)amino)propyl)azanediyl)diacetate

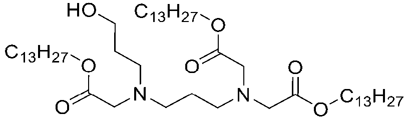

### Step 1: Synthesis of tridecyl 2-bromoacetate

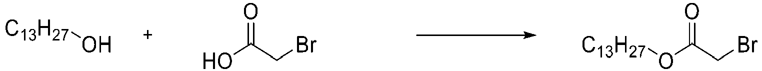

Using trideca-1-ol and 2-bromoacetic acid as raw materials and according to step 1 of Example 3, tridecyl 2-bromoacetate was obtained. The crude product was purified by column chromatography to obtain a target molecule.

### Step 2: Synthesis of ditridecyl 2,2'-((3-((3-hydroxypropyl)(2-oxo-2-(tridecyloxy)ethyl)amino)propyl)azanediyl)diacetate

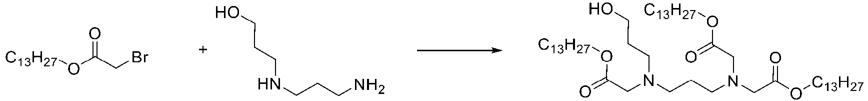

Using tridecyl 2-bromoacetate and 3-((3-aminopropyl)amino)propan-1-ol as raw materials and according to step 2 of Example 3, ditridecyl 2,2'-((3-((3-hydroxypropyl)(2-oxo-2-(tridecyloxy)ethyl)amino)propyl)azanediyl)diacetate was obtained. The crude product was purified by column chromatography to obtain a target molecule.

ESI-MS *m*/*z:* cald for C₅₁H₁₀₁N₂O₇ [M+H]⁺: 853.7, found 853.7.

### Example 78

### Ditridecyl 2,2'-((3-((4-hydroxybutyl)(2-oxo-2-(tridecyloxy)ethyl)amino)propyl)azanediyl)diacetate

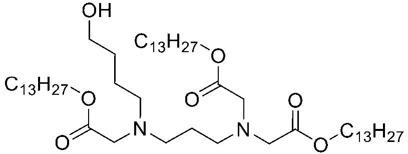

### Step 1: Synthesis of tridecyl 2-bromoacetate

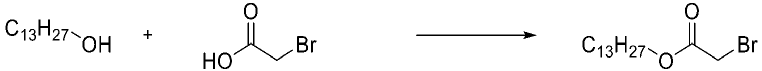

Using trideca-1-ol and 2-bromoacetic acid as raw materials and according to step 1 of Example 3, tridecyl 2-bromoacetate was obtained. The crude product was purified by column chromatography to obtain a target molecule.

### Step 2: Synthesis of ditridecyl 2,2'-((3-((4-hydroxybutyl)(2-oxo-2-(tridecyloxy)ethyl)amino)propyl)azanediyl)diacetate

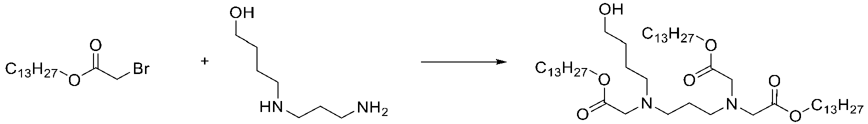

Using tridecyl 2-bromoacetate and 4-((3-aminopropyl)amino)butan-1-ol as raw materials and according to step 2 of Example 3, ditridecyl 2,2'-((3-((4-hydroxybutyl)(2-oxo-2-(tridecyloxy)ethyl)amino)propyl)azanediyl)diacetate was obtained. The crude product was purified by column chromatography to obtain a target molecule.

ESI-MS *m*/*z:* cald for C₅₂H₁₀₃N₂O₇ [M+H]⁺: 867.7, found 867.7.

### Example 79

### Di((9Z,12Z)-octadeca-9,12-dien-1-yl)8,8'-((3-(2-hydroxyethyl)(8-((9Z,12Z)-tetradec-9,13-dien-1-)oxy)-8-oxooctyl)amino)propyl)azadiyl)dioctanoic acid

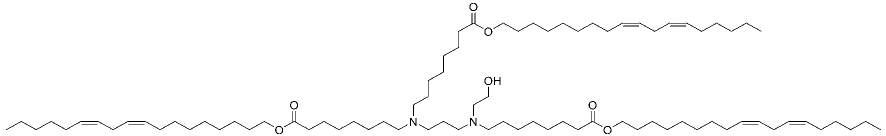

### Step 1: Synthesis of (9Z,12Z)-octadeca-9,12-dien-1-yl 8-bromooctanoic acid

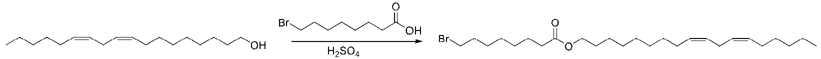

(9Z,12Z)-octadeca-9,12-dien-1-ol (1.0 g, 3.6 mmol) was mixed with 8-bromooctanoic acid (1.0 g, 4.5 mmol). The mixture was heated to 60 °C, concentrated sulfuric acid (0.1 mL) was added, and stirred at this temperature for 6 hours. TLC showed complete consumption of raw materials, the mixture was mixed directly, and purified by column chromatography with PE: EA=30:1 to obtain 1.6 g of a colorless oily product with a yield of 92.1%.

ESI-MS m/z: cald for C₂₆H₄₇BrO₂ [M+H]⁺: 471.6, found 471.6.

¹H-NMR (400 MHz, CDCl₃) δ5.43 - 5.27 (m, 4H), 4.05 (t, J = 6.7 Hz, 2H), 3.40 (t, J = 6.8 Hz, 2H), 2.77 (t, J = 6.4 Hz, 2H), 2.29 (t, J = 7.5 Hz, 2H), 2.09 - 1.99 (m, 4H), 1.90 - 1.79 (m, 2H), 1.67 - 1.58 (m, 4H), 1.48 - 1.39 (m, 2H), 1.37 - 1.26 (m, 20H), 0.89 (t, *J* = 6.8 Hz, 3H).

### Step 2: Di((9Z,12Z)-octadeca-9,12-dien-1-yl)8,8'-((3-(2-hydroxyethyl)(8-((9Z,12Z)-tetradec-9,13-dien-1-)oxy)-8-oxooctyl)amino)propyl)azadiyl)dioctanoic acid

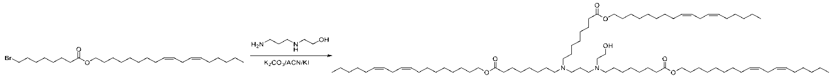

N-(2-hydroxyethyl)-1,3-propanediamine (60 mg, 0.5 mmol) was dissolved in acetonitrile (10 mL), and (9Z,12Z)-octadeca-9,12-dien-1-yl 8-bromooctanoic acid (1.0 g, 2.0 mmol), K2CO3 (0.4 g, 2.6 mmol), and KI (10 mg) were added respectively, and stirred at 85 °C for 16 hours. An appropriate amount of water was added to the mixture, then DCM (30 mL X 3) was added for extraction. The combined organic phases were dried and concentrated under reduced pressure. The crude product was purified by column chromatography with DCM: MeOH=30:1 to obtain 220 mg of a colorless oily product with a yield of 33.5 %.

ESI-MS m/z: cald for C₈₃H₁₅₂N₂O₇ [M+H]⁺: 1290.1, found 1290.1.

¹H-NMR (400 MHz, CDCl₃) δ 5.43 - 5.27 (m, 12H), 4.05 (t, J = 6.8 Hz, 6H), 3.54 (t, J = 5.2 Hz, 2H), 2.77 (t, J = 6.4 Hz, 6H), 2.57 - 2.34 (m, 11H), 2.28 (t, J = 7.6 Hz, 6H), 2.08 - 2.01 (m, 12H), 1.61 (t, J = 7.1 Hz, 12H), 1.47 - 1.39 (m, 6H), 1.36 - 1.24 (m, 70H), 0.89 (t, J = 6.8 Hz, 9H).

### Example 80

### Di((9Z,12Z)-octadeca-9,12-dien-1-yl)4,4'-((3-((2-hydroxyethyl)(4-(((9Z,12Z)-octadeca-9,12-dien-1-yl)oxo)-4-oxobutyl)amino)propyl)azanediyl)dibutanoate

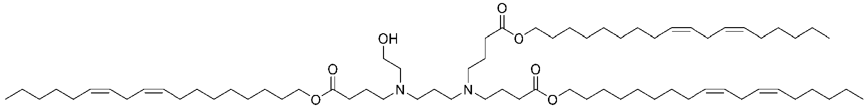

### Step 1: Synthesis of (10Z,13Z)-octadeca-10,13-dien-1-yl 4-bromobutanoate

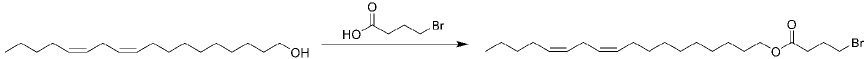

Using (10Z,13Z)-octadeca-10,13-dien-1-ol and 4-bromobutyric acid as raw materials and according to step 1 of Example 3, (10Z,13Z)-octadeca-10,13-dien-1-yl 4-bromobutanoate was obtained. The crude product was purified by column chromatography to obtain 4.2 g of a target molecule with a yield of 88.7%.

¹H NMR (400 MHz, CDCl₃) δ 5.44 - 5.28 (m, 4H), 4.07 (t, J = 6.7 Hz, 2H), 3.47 (t, J = 6.5 Hz, 2H), 2.77 (t, J = 6.5 Hz, 2H), 2.50 (t, J = 7.2 Hz, 2H), 2.25 - 2.11 (m, 2H), 2.10 - 1.98 (m, 4H), 1.67 - 1.58 (m, 2H), 1.37 - 1.27 (m, 16H), 0.89 (t, J = 6.8 Hz, 3H).

### Step 2: Synthesis of di((9Z,12Z)-octadeca-9,12-dien-1-yl)4,4'-((3-((2-hydroxyethyl)(4-(((9Z,12Z)-octadeca-9,12-dien-1-yl)oxo)-4-oxobutyl)amino)propyl)azanediyl)dibutanoate

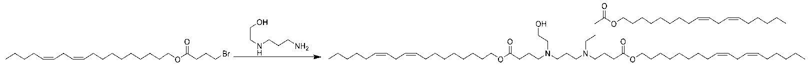

Using (10Z,13Z)-octadeca-10,13-dien-1-yl 4-bromobutanoate as raw material and according to step 2 of Example 3, di((9Z,12Z)-octadeca-9,12-dien-1-yl)4,4'-((3-((2-hydroxyethyl)(4-(((9Z,12Z)-octadeca-9,12-dien-1-yl)oxo)-4-oxobutyl)amino)propyl)azanediyl)dibutanoate was obtained. The crude product was purified by column chromatography to obtain 82 mg of a target molecule with a yield of 14.3%.

### ESI-MS m/z: cald for C₇₁H₁₂₈N₂O₇ [M+H]⁺: 1121.8, found 1121.9.

¹H NMR (400 MHz, CDCl₃) δ 5.45 - 5.22 (m, 12H), 4.09 - 3.98 (m, 6H), 3.54 (t, J = 5.2 Hz, 2H), 2.77 (t, J = 6.5 Hz, 6H), 2.56 (t, J = 5.2 Hz, 2H), 2.47 (t, J = 7.2 Hz, 4H), 2.41 (t, J = 7.2 Hz, 6H), 2.35 - 2.25 (m, 6H), 2.18 - 1.92 (m, 12H), 1.80 - 1.69 (m, 6H), 1.65 - 1.54 (m, 8H), 1.35 - 1.26 (m, 49H), 0.89 (t, J = 6.7 Hz, 9H).

### Example 81

### Di((9Z,12Z)-octadeca-9,12-dien-1-yl)4,4'-((3-((3-hydroxypropyl)(4-(((9Z,12Z)-octadeca-9,12-dien-1-yl)oxo)-4-oxobutyl)amino)propyl)azanediyl)dibutanoate

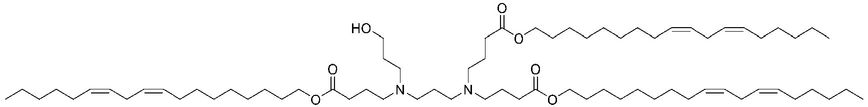

### Step 1: Synthesis of di((9Z,12Z)-octadeca-9,12-dien-1-yl)4,4'-((3-((4-(((9Z,12Z)-octadeca-9,12-dien-1-yl)oxo)-4-oxobutyl)amino)propyl)azanediyl)dibutanoate

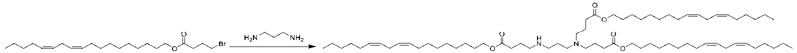

Using (10Z,13Z)-octadeca-10,13-dien-1-yl 4-bromobutanoate and 1,3-propanediamine as raw materials and according to step 2 of Example 18, di((9Z,12Z)-octadeca-9,12-dien-1-yl)4,4'-((3-((4-(((9Z,12Z)-octadeca-9,12-dien-1-yl)oxo)-4-oxobutyl)amino)propyl)azanediyl)dibutanoate was obtained. The crude product was purified by column chromatography to obtain a target molecule.

### ESI-MS m/z: cald for C₆₉H₁₂₄N₂O₆ [M+H]⁺: 1077.8, found 1077.9.

### Step 3: Synthesis of di((9Z,12Z)-octadeca-9,12-dien-1-yl)4,4'-((3-((3-hydroxypropyl)(4-(((9Z,12Z)-octadeca-9,12-dien-1-yl)oxo)-4-oxobutyl)amino)propyl)azanediyl)dibutanoate

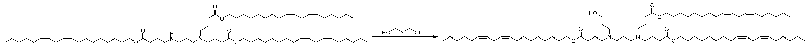

Using di((9Z,12Z)-octadeca-9,12-dien-1-yl)4,4'-((3-((4-(((9Z,12Z)-octadeca-9,12-dien-1-yl)oxo)-4-oxobutyl)amino)propyl)azanediyl)dibutanoate and 3-chloro-1-propanol as raw materials and according to step 3 of Example 18, di((9Z,12Z)-octadeca-9,12-dien-1-yl)4,4'-((3-((3-hydroxypropyl)(4-(((9Z,12Z)-octadeca-9,12-dien-1-yl)oxo)-4-oxobutyl)amino)propyl)azanediyl)dibutanoate was obtained. The crude product was purified by column chromatography to obtain a target molecule.

ESI-MS m/z: cald for C₇₂H₁₃₀N₂O₇ [M+H]⁺: 1135.8, found 1135.9.

¹H NMR (400 MHz, CDCl₃) δ 5.46 - 5.25 (m, 12H), 4.12 - 3.99 (m, 6H), 3.78 (t, J = 5.2 Hz, 2H), 2.77 (t, J = 6.5 Hz, 6H), 2.64 (t, J = 5.7 Hz, 2H), 2.53 - 2.35 (m, 10H), 2.31 (t, J = 7.4 Hz, 6H), 2.05 (q, J = 6.8 Hz, 12H), 1.86 - 1.66 (m, 11H), 1.64 - 1.59 (m, 6H), 1.42 - 1.23 (m, 48H), 0.89 (t, J = 6.8 Hz, 9H).

### Example 82

### Diundecyl 4,4'-((4-((3-hydroxypropyl)(4-oxo-4-(undecyloxy)butyl)amino)butyl)azanediyl)dibutanoate

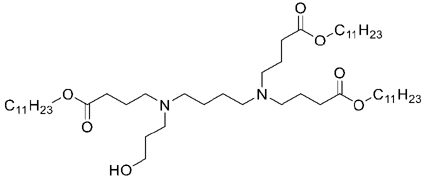

### Step 1: Synthesis of undecyl 4-bromobutanoate

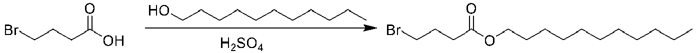

4-bromobutyric acid (4.6 g, 27.8 mmol) and 1-undecanol (4.0 g, 23.2 mmol) were dissolved after being stirred at 60 °C for 0.5 hours, and 2 drops of concentrated sulfuric acid were added. The mixture was reacted at 60 °C for 6 hours. TLC showed complete consumption of raw materials, the mixture was mixed directly, and purified by column chromatography with PE:EA=20:1 to obtain 6.7 g of a colorless oily product with a yield of 89.3%.

### Step 2: Synthesis of diundecyl 4,4'-((4-((4-oxo-4-(undecyloxy)butyl)amino)butyl)azanediyl)dibutanoate

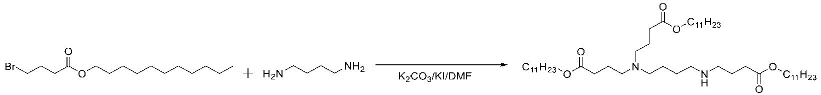

Butane-1,4-diamine (274 mg, 3.1 mmol) was dissolved in ACN (40 mL), and undecyl 4-bromobutanoate (3.0 g, 9.3 mmol), K2CO3 (2.1 g, 15.6 mmol), and KI (50 mg) were added respectively, and stirred at 85 °C for 16 hours. An appropriate amount of water was added to the mixture, then EA (80 mL * 3) was added for extraction. The combined organic phases were dried and concentrated under reduced pressure. The crude product was purified by column chromatography with DCM (containing 5% EA):MeOH=50:1 to 10:1 to obtain 390 mg of a colorless oily product with a yield of 15.6 %.

ESI-MS *m*/*z:* cald for C₄₉H₉₆N₂O₆ [M+H]⁺: 809.3, found 809.3.

¹H NMR (400 MHz, CDCl₃) δ 4.05 (t, J = 6.8 Hz, 6H), 2.85 - 2.65 (m, 4H), 2.52 - 2.44 (m, 5H), 2.43 - 2.37 (m, 3H), 2.32 (t, J = 7.2 Hz, 4H), 1.90 - 1.85 (m, 2H), 1.80 - 1.68 (m, 5H), 1.64 - 1.55 (m, 6H), 1.56 - 1.48 (m, 2H), 1.32 - 1.21 (m, 50H), 0.88 (t, J = 6.8 Hz, 9H).

### Step 3: Diundecyl 4,4'-((4-((3-hydroxypropyl)(4-oxo-4-(undecyloxy)butyl)amino)butyl)azanediyl)dibutanoate

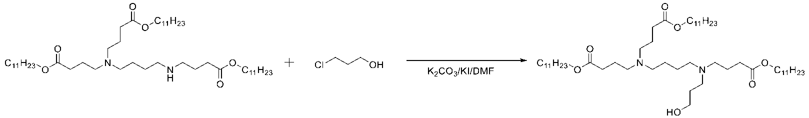

Diundecyl 4,4'-((4-((4-oxo-4-(undecyloxy)butyl)amino)butyl)azanediyl)dibutanoate (390 mg, 0.5 mmol) was dissolved in DMF (8 mL), and 3-chloro-1-propanol (137 mg, 1.4 mmol), K2CO3 (333 mg, 2.4 mmol), and KI (20 mg) were added respectively, and stirred at 90 °C for 16 hours. Then EA (80 mL * 3) was added for extraction. The combined organic phases were dried and concentrated under reduced pressure. The crude product was purified by column chromatography with DCM (containing 5% EA):MeOH=50:1 to 10:1 to obtain 82 mg of a colorless oily product with a yield of 19.3 %.

ESI-MS *m*/*z:* cald for C₅₂H₁₀₂N₂O₇ [M+H]⁺: 867.4, found 867.9.

¹H NMR (400 MHz, CDCl₃) δ 4.06 (td, J = 6.8, 2.5 Hz, 6H), 3.78 (t, J = 5.2 Hz, 2H), 2.67 (s, 2H), 2.45 (s, 9H), 2.32 (t, J = 7.3 Hz, 6H), 1.87 - 1.66 (m, 10H), 1.61 (t, J = 7.1 Hz, 6H), 1.43 (d, J = 6.0 Hz, 4H), 1.28 (d, J = 16.9 Hz, 48H), 0.88 (t, J = 6.7 Hz, 9H).

### Example 83

### Dinonyl 4,4'-(5-((3-hydroxypropyl)(4-oxo-4-(undecyloxy)butyl)amino)pentyl)azahexyl)dibutanoate

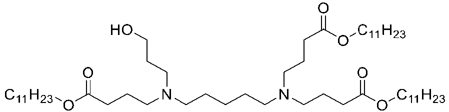

### Step 1: Synthesis of undecyl 4-bromobutanoate

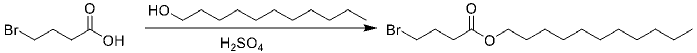

4-bromobutyric acid (10 g, 59.88 mmol) and 1-undecanol (10.32 g, 59.88 mmol) were dissolved after stirring at 60 °C for 0.5 hours, and 2 drops of concentrated sulfuric acid were added. The mixture was reacted at 60 °C for 6 hours. TLC showed complete consumption of raw materials, the mixture was mixed directly, and purified by column chromatography with PE:EA=20:1 to obtain 15 g of a colorless oily product with a yield of 78%.

### Step 2: Synthesis of dinonyl 4,4'-(5-((4-oxo-4-(undecyloxy)butyl)amino)pentyl)azahexyl)dibutanoate

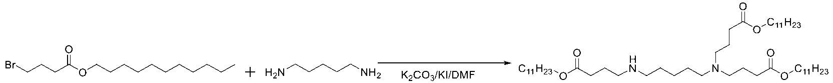

1,5-diaminopentane (787.2 mg, 7.7 mmol) was dissolved in DMF (50 mL), and undecyl 4-bromobutanoate (7.426 g, 23.11 mmol), K2CO3 (4.79 g, 34.66 mmol), and KI (50 mg) were added respectively, and the mixture was stirred at 60 °C for 6 hours. An appropriate amount of water was added to the mixture, then EA (120 mL * 3) was added for extraction. The combined organic phases were dried and concentrated under reduced pressure. The crude product was purified by column chromatography with DCM (containing 5% EA):MeOH=50:1 to 10:1 to obtain 650 mg of a colorless oily product with a yield of 10.25 %.

ESI-MS *m*/*z:* cald for C₅₀H₉₈N₂O₆ [M+H]⁺: 824.3, found 824.3.

### Step 3: dinonyl 4,4'-(5-((3-hydroxypropyl)(4-oxo-4-(undecyloxy)butyl)amino)pentyl)azahexyl)dibutanoate

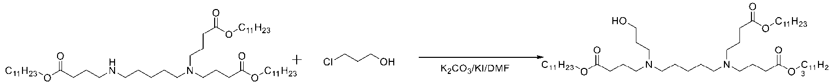

Dinonyl 4,4'-(5-((4-oxo-4-(undecyloxy)butyl)amino)pentyl)azahexyl)dibutanoate (650 mg, 0.789 mmol) was dissolved in DMF (10 mL), and 3-chloro-1-propanol (147 mg, 1.5 mmol), K2CO3 (414.6 mg, 3 mmol), and KI (20 mg) were added respectively, and stirred at 90 °C for 16 hours. Then EA (80 mL * 3) was added for extraction. The combined organic phases were dried and concentrated under reduced pressure. The crude product was purified by column chromatography with DCM (containing 5% EA):MeOH=50:1 to 10:1 to obtain 120 mg of a colorless oily product with a yield of 17.25 %.

ESI-MS *m*/*z:* cald for C₅₃H₁₀₄N₂O₇ [M+H]⁺: 882.4, found 882.4.

¹H NMR (400 MHz, CDCl₃) δ 4.10 (m, 6H), 3.83 (t, J = 5.2 Hz, 2H), 2.69 (t, J = 5.6 Hz, 2H), 2.54 - 2.27 (m, 15H), 1.91 - 1.58 (m, 20H), 1.43-1.22 (m, 50H), 0.92 (t, J = 6.7 Hz, 9H).

### Example 84

### Azetidinyl 4,4'-(4-((4-(dodecyloxy)-4-oxobutyl)(3-hydroxypropyl)amino)butyl)dibutanoate

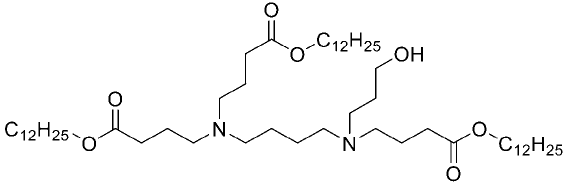

### Step 1: didodecyl 4,4'-(4-((4-(dodecyloxy)-4-oxobutyl)amino)butyl)azetidinyl)dibutanoate

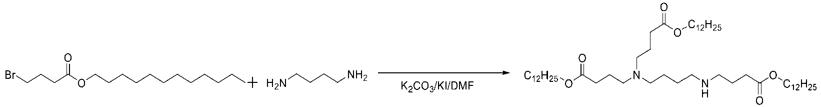

Using dodecyl 4-bromobutanoate and 1,4-butanediamine as raw materials and according to step 2 of Example 82, didodecyl 4,4'-(4-((4-(dodecyloxy)-4-oxobutyl)amino)butyl)azetidinyl)dibutanoate was obtained. The crude product was purified by column chromatography to obtain a target molecule.

ESI-MS *m*/*z:* cald for C₅₂H₁₀₃N₂O₆ [M+H]⁺: 852.4, found 852.6.

### Step 2: Synthesis of azetidinyl 4,4'-(4-((4-(dodecyloxy)-4-oxobutyl)(3-hydroxypropyl)amino)butyl)dibutanoate

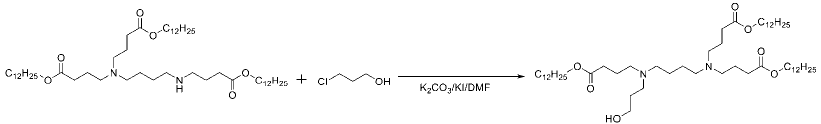

Using didodecyl 4,4'-(4-((4-(dodecyloxy)-4-oxobutyl)amino)butyl)azetidinyl)dibutanoate and 3-chloro-1-propanol as raw materials and according to step 3 of Example 82, azetidinyl 4,4'-(4-((4-(dodecyloxy)-4-oxobutyl)(3-hydroxypropyl)amino)butyl)dibutanoate was obtained. The crude product was purified by column chromatography to obtain a target molecule.

ESI-MS *m*/*z:* cald for C₅₅H₁₀₉N₂O₇ [M+H]⁺: 909.5, found 909.7.

### Example 85

### 4,4'-((4-((3-hydroxypropyl)(4-oxo-4-(tridecyloxy)butyl)amino)butyl)azetidinyl)dibutyric acid diester

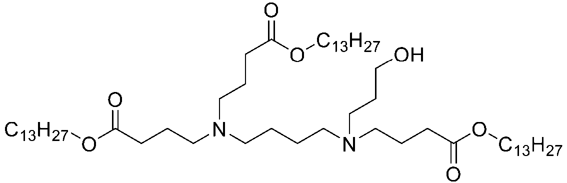

### Step 1: 4,4'-(4-(4-oxo-4-(tridecyloxy)butyl)amino)butyl)azetidinyl)dibutyric acid diester

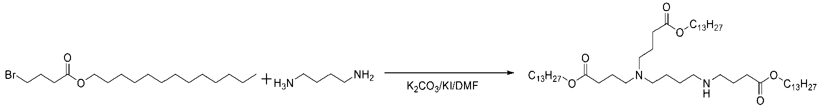

Using tridecyl 4-bromobutanoate and 1,4-butanediamine as raw materials and according to step 2 of Example 82, 4,4'-(4-(4-oxo-4-(tridecyloxy)butyl)amino)butyl)azetidinyl)dibutyric acid diester was obtained. The crude product was purified by column chromatography to obtain a target molecule.

ESI-MS *m*/*z:* cald for C₅₅H₁₀₉N₂O₆ [M+H]⁺: 893.5, found 893.7.

### Step 2: Synthesis of 4,4'-((4-((3-hydroxypropyl)(4-oxo-4-(tridecyloxy)butyl)amino)butyl)azetidinyl)dibutyric acid diester

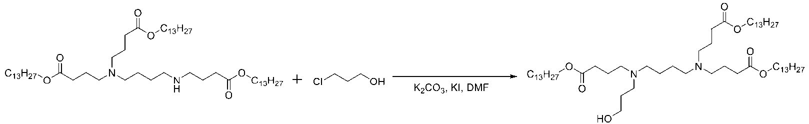

Using 4,4'-(4-(4-oxo-4-(tridecyloxy)butyl)amino)butyl)azetidinyl)dibutyric acid diester and 3-chloro-1-propanol as raw materials and according to step 3 of Example 82, 4,4'-((4-((3-hydroxypropyl)(4-oxo-4-(tridecyloxy)butyl)amino)butyl)azetidinyl)dibutyric acid diester was obtained. The crude product was purified by column chromatography to obtain a target molecule.

ESI-MS *m*/*z:* cald for C₅₈H₁₁₅N₂O₇ [M+H]⁺: 951.6, found 951.8.

### Example 86

### Didecyl 4,4'-((4-(decyloxy)-4-oxobutyl)(3-hydroxypropyl)amino)butyl)azadiyl)dibutanoate

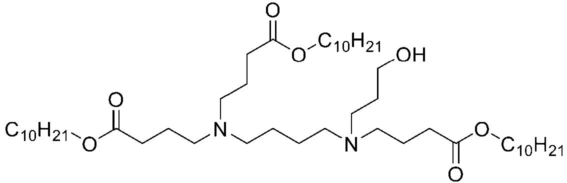

### Step 1: didecyl 4,4'-(4-((4-(decyloxy)-4-oxobutyl)amino)butyl)azadiyl)dibutanoate

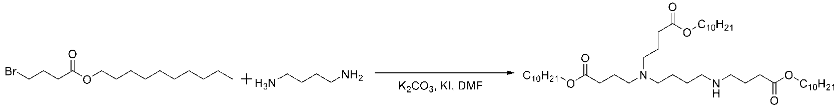

Using decyl 4-bromobutanoate and 1,4-butanediamine as raw materials and according to step 2 of Example 82, didecyl 4,4'-(4-((4-(decyloxy)-4-oxobutyl)amino)butyl)azadiyl)dibutanoate was obtained. The crude product was purified by column chromatography to obtain a target molecule.

ESI-MS *m*/*z:* cald for C₄₆H₉₁N₂O₆ [M+H]⁺: 767.2, found 767.4.

### Step 2: Synthesis of didecyl 4,4'-((4-(decyloxy)-4-oxobutyl)(3-hydroxypropyl)amino)butyl)azadiyl)dibutanoate

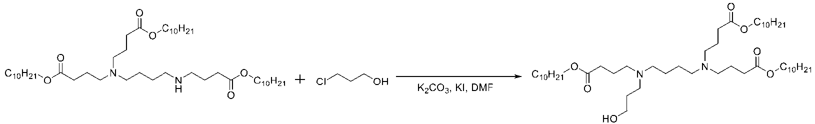

Using didecyl 4,4'-(4-((4-(decyloxy)-4-oxobutyl)amino)butyl)azadiyl)dibutanoate and 3-chloro-1-propanol as raw materials and according to step 3 of Example 82, 4,4'-((4-(decyloxy)-4-oxobutyl)(3-hydroxypropyl)amino)butyl)azadiyl)didecyl dibutanoate was obtained. The crude product was purified by column chromatography to obtain a target molecule.

ESI-MS *m*/*z:* cald for C₄₉H₉₇N₂O₇ [M+H]⁺: 825.3, found 825.5.

### Example 87

### Dinonyl 4,4'-((4-((2-hydroxyethyl)(4-oxo-4-(undecyloxy)butyl)amino)butyl)azadiyl)dibutanoate

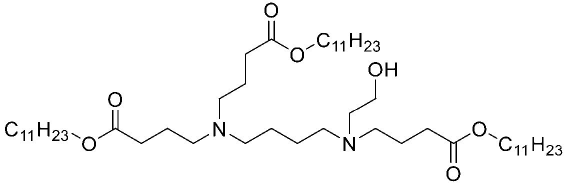

### Step 1: Synthesis of undecyl 4-bromobutanoate

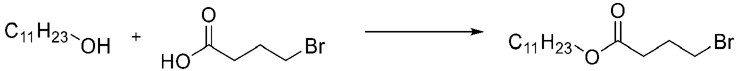

Using undecyl-1-ol and 4-bromobutyric acid as raw materials and according to step 1 of Example 19, undecyl 6-bromohexanoate was obtained. The crude product was purified by column chromatography to obtain 3.3 g of a target molecule with a yield of 88.5%.

¹H NMR (400 MHz, CDCl₃) δ 4.07 (t, J = 6.7 Hz, 2H), 3.46 (t, J = 6.4 Hz, 2H), 2.49 (t, J = 7.2 Hz, 2H), 2.22 - 2.11 (m, 2H), 1.66 - 1.59 (m, 2H), 1.35 - 1.23 (m, 16H), 0.88 (t, J = 6.7 Hz, 3H).

### Step 2: Synthesis of dinonyl 4,4'-(3-((4-oxo-4-(undecyloxy)butyl)amino)propyl)azetidinyl)dibutanoate

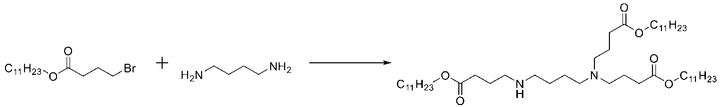

Using undecyl 4-bromobutanoate and 1,4-butanediamine as raw materials and according to step 2 of Example 19, dinonyl 4,4'-(3-((4-oxo-4-(undecyloxy)butyl)amino)propyl)azetidinyl)dibutanoate was obtained. The crude product was purified by column chromatography to obtain 390 mg of a target molecule with a yield of 16.0%.

### ESI-MS m/z: cald for C₄₉H₉₇N₂O₆ [M+H]⁺: 809.7, found 809.2.

¹H NMR (400 MHz, CDCl₃) δ 4.05 (t, J = 6.8 Hz, 6H), 2.85 -2.65 (m, 4H), 2.52 - 2.44 (m, 5H), 2.40 (m, 3H), 2.32 (m, 4H), 2.04 - 1.88 (m, 2H), 1.80 - 1.68 (m, 5H), 1.64 - 1.57 (m, 6H), 1.55 - 1.47 (m, 2H), 1.32 - 1.21 (m, 50H), 0.88 (t, J = 6.8 Hz, 9H).

### Step 3: Synthesis of dinonyl 4,4'-((4-((2-hydroxyethyl)(4-oxo-4-(undecyloxy)butyl)amino)butyl)azetidinyl)dibutanoate

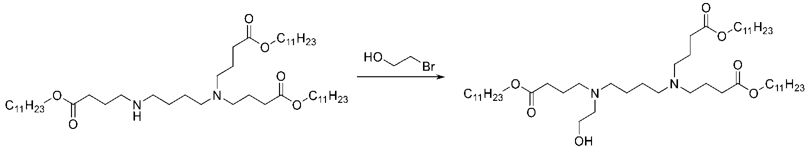

Using dinonyl 4,4'-(3-((4-oxo-4-(undecyloxy)butyl)amino)propyl)azetidinyl)dibutanoate and bromoethanol as raw materials and according to step 3 of Example 19, 4,4'-((4-((2-hydroxyethyl)(4-oxo-4-(undecyloxy)butyl)amino)butyl)azetidinyl)dinonyl dibutanoate was obtained. The crude product was purified by column chromatography to obtain 63 mg of a target molecule with a yield of 17.1%.

ESI-MS *m*/*z:* cald for C₅₁H₁₀₁N₂O₇ [M+H]⁺: 853.7, found 853.2.

¹H NMR (400 MHz, CDCl₃) δ 4.05 (t, J = 6.8 Hz, 6H), 3.54 (t, J = 5.4 Hz, 2H), 2.59 (m, 2H), 2.52 - 2.37 (m, 11H), 2.31 (m, 7H), 1.83 - 1.67 (m, 7H), 1.61 (m, 7H), 1.40 (m, 4H), 1.27 (m, 45H), 0.88 (t, J = 6.8 Hz, 9H).

### Example 88

### Dinonyl 5,5'-((4-((3-hydroxypropyl)(5-oxo-5-(undecyloxy)pentyl)amino)butyl)dipentanoate

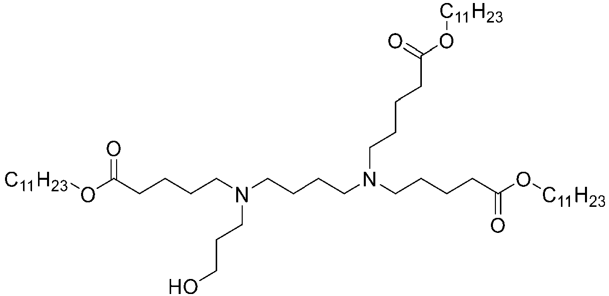

### Step 1: Synthesis of undecyl 5-bromopentanoate

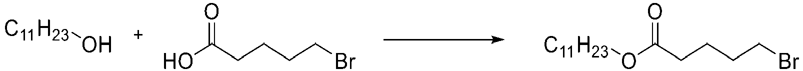

Using undeca-1-ol and 5-bromovaleric acid as raw materials and according to step 1 of Example 19, undecyl 5-bromopentanoate was obtained. The crude product was purified by column chromatography to obtain 1.8 g of a target molecule with a yield of 92.8%.

¹H NMR (400 MHz, CDCl₃) δ 4.06 (t, J = 6.7 Hz, 2H), 3.41 (t, J = 6.6 Hz, 2H), 2.34 (t, J = 7.3 Hz, 2H), 1.95 - 1.85 (m, 2H), 1.84 - 1.73 (m, 2H), 1.62 (t, J = 7.1 Hz, 2H), 1.33 - 1.23 (m, 16H), 0.88 (t, J = 6.7 Hz, 3H).

### Step 2: Synthesis of (4-((3-(tertbutyldimethylsilyl)oxy)propyl)amino)butyl)tert-butyl carbamate

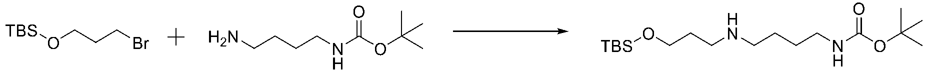

(3-bromopropoxy)(tert-butyl)dimethylsilane (2.0 g, 7.9 mmol) was dissolved in acetonitrile (40 mL), and (4-aminobutyl)tert-butyl carbamate (1.8 g, 9.5 mmol), K2CO3 (3.3 g, 23.7 mmol), and KI (130 mg, 0.8 mmol) were added respectively, and stirred at 85 °C for 16 hours. An appropriate amount of water was added to the mixture, then DCM (50 mL * 3) was added for extraction. The combined organic phases were dried and concentrated under reduced pressure. The crude product was purified by column chromatography with DCM:MeOH=20:1 to obtain 1.5 g of a colorless oily product with a yield of 54.1 %.

ESI-MS *m*/*z:* cald for C₁₈H₄₁N₂O₃Si [M+H]⁺: 361.6, found 361.8.

¹H NMR (400 MHz, CDCl₃) δ 3.68 (t, J = 6.1 Hz, 2H), 3.11 (m, 2H), 2.69 (t, J = 6.9 Hz, 2H), 2.64 - 2.58 (m, 2H), 1.79 - 1.64 (m, 4H), 1.54 - 1.48 (m, 4H), 1.43 (s, 9H), 0.88 (d, J = 1.1 Hz, 9H), 0.04 (d, J = 1.1 Hz, 6H).

### Step 3: Synthesis of undecyl 5-((4-(tert-butoxyoxo)amino)butyl)(3-((tertbutyldimethylsilyl)oxy)propyl)amino)pentanoate

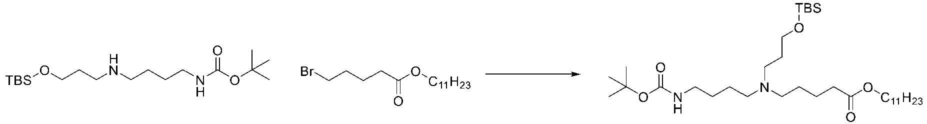

(4-((3-((tert-butyldimethylsilyl)oxy)propyl)amino)butyl)tert-butyl carbamate (300 mg, 0.8 mmol) was dissolved in acetonitrile (10 mL), and undecyl 5-bromopentanoate (279 mg, 0.8 mmol), potassium carbonate (345 mg, 2.5 mmol), and KI (13.8 mg, 0.08 mmol) were added respectively, and stirred at 85 °C for 16 hours. An appropriate amount of water was added to the mixture, then DCM (20 mL * 3) was added for extraction. The combined organic phases were dried and concentrated under reduced pressure. The crude product was purified by column chromatography with DCM:MeOH=30:1 to obtain 0.5 g of a colorless oily product with a yield of 97.7 %.

ESI-MS *m*/*z:* cald for C₃₄H₇₁N₂O₅Si [M+H]⁺: 616.0, found 616.1.

¹H NMR (400 MHz, CDCl₃) δ 4.05 (t, J = 6.8 Hz, 2H), 3.62 (t, J = 6.3 Hz, 2H), 3.16 - 3.04 (m, 2H), 2.43 (d, J = 29.8 Hz, 5H), 2.30 (t, J = 7.4 Hz, 2H), 1.62 (m, 8H), 1.45 (m, 14H), 1.28 (m, 17H), 0.88 (s, 12H), 0.04 (s, 6H).

### Step 4: Synthesis of undecyl 5-((4-aminobutyl)(3-hydroxypropyl)amino)pentanoate

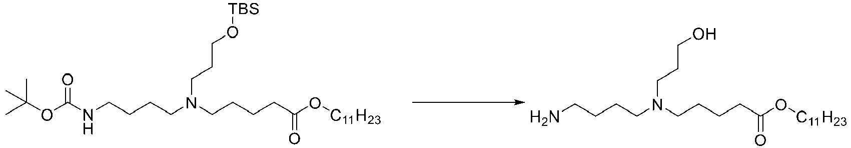

Undecyl 5-((4-(tert-butoxyoxo)amino)butyl)(3-((tertbutyldimethylsilyl)oxy)propyl)amino)pentanoate (500 mg, 0.8 mmol) was dissolved in DCM (20 mL), TMSOTf (368 mg, 1.6 mmol) was added, stirred at room temperature for 1.5 hours, saturated sodium bicarbonate solution was added dropwise under ice bath to neutralize, an appropriate amount of water was added, extracted with DCM (20 mL * 3), the organic phase was combined, dried and evaporated to dryness, the mixture was mixed and passed through the column, and eluted with DCM:MeOH(NH3)=10:1, to obtain 256 mg of a colorless oily product with a yield of 78.6%.

ESI-MS *m*/*z:* cald for C₂₃H₄₉N₂O₃ [M+H]⁺: 401.6, found401.8.

¹H NMR (400 MHz, CDCl₃) δ 4.05 (t, J = 6.8 Hz, 2H), 3.83 - 3.74 (m, 2H), 2.71 (t, J = 6.7 Hz, 2H), 2.63 (t, J = 5.6 Hz, 2H), 2.47 - 2.38 (m, 5H), 2.32 (t, J = 7.2 Hz, 2H), 1.70 - 1.65 (m, 2H), 1.64 - 1.57 (m, 4H), 1.54 - 1.41 (m, 6H), 1.35 - 1.23 (m, 18H), 0.87 (t, J = 6.8 Hz, 3H).

### Step 5: Synthesis of dinonyl 5,5'-((4-((3-hydroxypropyl)(5-oxo-5-(undecyloxy)pentyl)amino)butyl)azanediyl)dipentanoatepentanoate

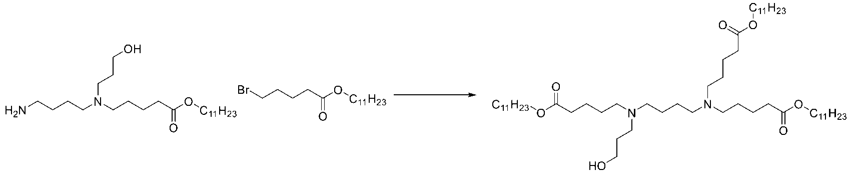

Undecyl 5-((4-aminobutyl)(3-hydroxypropyl)amino)pentanoatepentanoate (256 mg, 0.6 mmol) was dissolved in acetonitrile (10 mL), and undecyl 5-bromopentanoate (536 mg, 1.9 mmol), potassium carbonate (265 mg, 1.9 mmol), and KI (10.6 mg, 0.06 mmol) were added respectively, and stirred at 85 °C for 16 hours. An appropriate amount of water was added to the mixture, then DCM (20 mL * 3) was added for extraction. The combined organic phases were dried and concentrated under reduced pressure. The crude product was purified by column chromatography with DCM:MeOH=30:1 to obtain 210 mg of a colorless oily product with a yield of 36.1 %.

ESI-MS *m*/*z:* cald for C₅₅H₁₀₉N₂O₇ [M+H]⁺: 909.8, found 909.8.

¹H NMR (400 MHz, CDCl₃) δ 4.10 - 4.00 (m, 6H), 3.78 (t, J = 5.1 Hz, 2H), 2.63 (t, J = 5.6 Hz, 2H), 2.46 - 2.35 (m, 10H), 2.34 - 2.28 (m, 6H), 1.68 - 1.57 (m, 17H), 1.52 - 1.37 (m, 10H), 1.27 (d, J = 8.3 Hz, 46H), 0.88 (t, J = 6.8 Hz, 9H).

### Example 89

### Di((9Z,12Z)-octadeca-9,12-dien-1-yl)4,4'-(4-(3-hydroxypropyl)(4-(((9Z,12Z)-octadeca-9,12-dien-1-yl)oxy)-4-oxobutyl)amino)butyl)azanediyl)dibutanoate

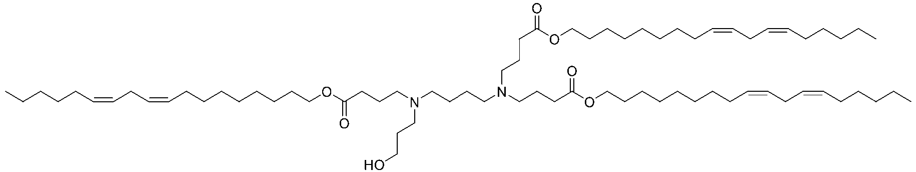

### Step 1: Synthesis of di((9Z,12Z)-octadeca-9,12-dien-1-yl)4,4'-(4-(4-(((9Z,12Z)-octadeca-9,12-dien-1-yl)oxy)-4-oxobutyl)amino)butyl)azanediyl)dibutanoate

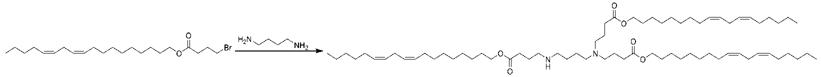

Using (10Z,13Z)-octadeca-10,13-dien-1-yl 4-bromobutanoate and 1,4-butanediamine as raw materials and according to step 2 of Example 82, di((9Z,12Z)-octadeca-9,12-dien-1-yl)4,4'-(4-(4-(((9Z,12Z)-octadeca-9,12-dien-1-yl)oxy)-4-oxobutyl)amino)butyl)azanediyl)dibutanoate was obtained. The crude product was purified by column chromatography to obtain a target molecule.

ESI-MS *m*/*z:* cald for C₇₀H₁₂₇N₂O₆ [M+H]⁺: 1091.8, found 1091.6.

### Step 2: Synthesis of di((9Z,12Z)-octadeca-9,12-dien-1-yl)4,4'-(4-(3-hydroxypropyl)(4-(((9Z,12Z)-octadeca-9,12-dien-1-yl)oxy)-4-oxobutyl)amino)butyl)azanediyl)dibutanoate

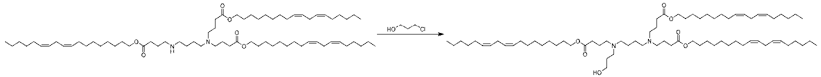

Using di((9Z,12Z)-octadeca-9,12-dien-1-yl)4,4'-(4-(4-(((9Z,12Z)-octadeca-9,12-dien-1-yl)oxy)-4-oxobutyl)amino)butyl)azanediyl)dibutanoate and 3-chloro-1-propanol as raw materials and according to step 3 of Example 82, di((9Z,12Z)-octadeca-9,12-dien-1-yl)4,4'-(4-(3-hydroxypropyl)(4-(((9Z,12Z)-octadeca-9,12-dien-1-yl)oxy)-4-oxobutyl)amino)butyl)azanediyl)dibutanoate was obtained. The crude product was purified by column chromatography to obtain a target molecule.

ESI-MS *m*/*z:* cald for C₇₃H₁₃₃N₂O₇ [M+H]⁺: 1150.9, found 1150.7.

¹H NMR (400 MHz, CDCl₃) δ 5.41 - 5.29 (m, 12H), 4.07 - 4.03 (m, 6H), 3.78 (t, J = 5.2 Hz, 2H), 2.76 (t, J = 6.5 Hz, 6H), 2.64 (t, J = 5.8 Hz, 2H), 2.52 - 2.36 (m, 10H), 2.32 (t, J = 7.4 Hz, 6H), 2.15-2.0 (m, 13H), 1.86 - 1.5 (m, 20H), 1.31-1.25 (m, 46H), 0.86 (t, J = 6.8 Hz, 9H).

### Example 90

### Tridecyl 8-(29-hydroxy-15-oxo-23-(8-oxo-8-(tridecyloxy)octyl)-23,27-diaza-14-oxanonacosane-27-yl)octanoate

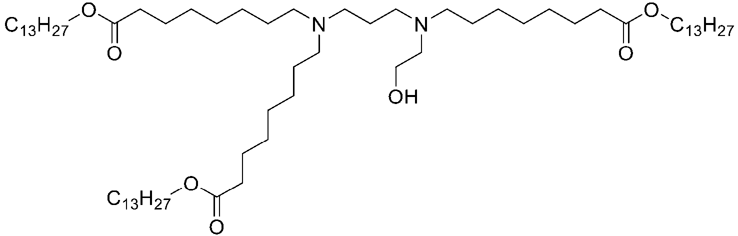

### Step 1: Synthesis of tridecyl 8-bromooctanoate

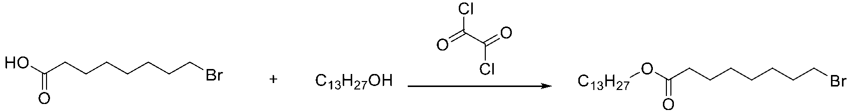

8-bromooctanoic acid (5 g, 22.41 mmol) was dissolved in 100 mL of DCM, oxalyl chloride (8.53 g, 67.23 mmol) was slowly added to the mixture, refluxed for 4 hours, and then concentrated under reduced pressure to obtain 12 g of crude product. The crude product was dissolved in DCM (150 mL) and TEA (3.28 g, 32.4 mmol), 1-tridecyl alcohol (4.49 g, 22.41 mmol) was added, and stirred at 40 °C for 4 hours. TLC showed complete consumption of raw materials, the mixture was mixed directly, and purified by column chromatography with PE:EA=10:1 to obtain 2.44 g of a colorless oily product with a yield of 26.8%.

### Step 2: Synthesis of tridecyl 8-(29-hydroxy-15-oxo-23-(8-oxo-8-(tridecyloxy)octyl)-23,27-diaza-14-oxanonacosane-27-yl)octanoate

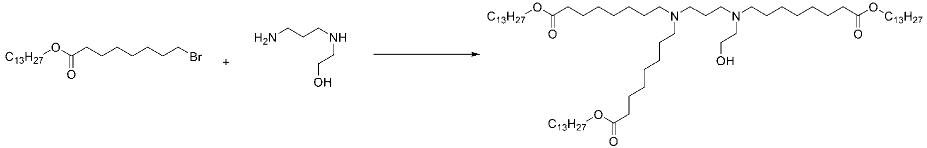

2-((3-aminopropyl)amino)ethan-1-ol (0.1 g, 846.17 umol) was dissolved in 20 mL MeCN, and tridecyl 8-bromooctanoate (1.2 g, 2.96 mmol), potassium carbonate (0.82 g, 5.92 mmol), and KI (10 mg) were added, and stirred overnight at 85°C. 50 mL of water and 50 mL of EA were added, the solution was separated, the organic phase was washed with saturated saline solution, dried with anhydrous sodium sulfate, and then concentrated under reduced pressure and purified by column chromatography with DCM (containing 5% EA):MeOH=50:1 to 5:1 to obtain 340 mg of a light yellow oily product with a yield of 36.8%.

ESI-MS *m*/*z:* cald for C₆₈H₁₃₅N₂O₇ [M+H]⁺: 1092.0, found 1092.1.

### Example 91

### Undecyl 10-(29-hydroxy-13-oxo-23-(10-oxo-10-(undecyl)oxydecyl)-23,27-diaza-12-oxanonacosane-27-yl)caprate

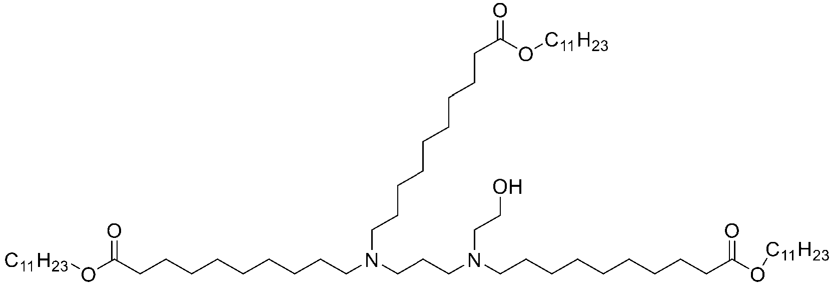

### Step 1: Synthesis of undecyl 10-bromocaprate

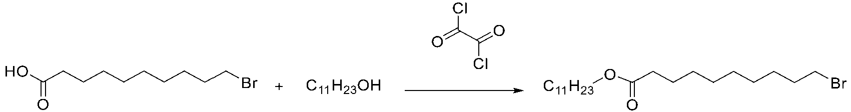

10-bromodecanoic acid (5 g, 19.91 mmol) was dissolved in 100 mL of DCM, oxalyl chloride (7.58 g, 59.723 mmol) was slowly added to the mixture, refluxed for 4 hours, and then concentrated under reduced pressure to obtain 12.3 g of crude product. The crude product was dissolved in DCM (150 mL) and TEA (5 g), 1-undecanol (3.43 g, 19.91 mmol) was added, and stirred at 40 °C for 4 hours. TLC showed complete consumption of raw materials, the mixture was mixed directly, and purified by column chromatography with PE:EA=10:1 to obtain 1.96 g of a colorless oily product with a yield of 24.3%.

### Step 2: Synthesis of undecyl 10-(29-hydroxy-13-oxo-23-(10-oxo-10-(undecyloxy)decyl)-23,27-diaza-12-oxanonacosane-27-yl)caprate

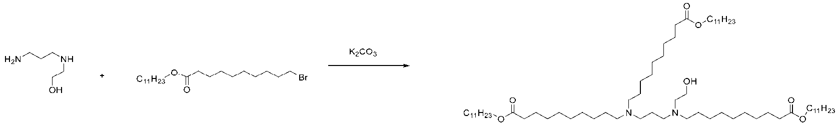

2-((3-aminopropyl)amino)ethan-1-ol (0.1 g, 846.17 umol) was dissolved in 20 mL MeCN, and undecyl 10-bromocaprate (1.2 g, 2.96 mmol), potassium carbonate (0.82 g, 5.92 mmol), and KI (10 mg) were added, and stirred overnight at 85 °C. 50 mL of water and 50 mL of EA were added, the solution was separated, the organic phase was washed with saturated saline solution, dried with anhydrous sodium sulfate, and then concentrated under reduced pressure and purified by column chromatography with DCM (containing 5% EA):MeOH=50:1 to 5:1 to obtain 310 mg of a light yellow oily product with a yield of 33.6%.

ESI-MS *m*/*z:* cald for C₆₈H₁₃₅N₂O₇ [M+H]⁺: 1092.0, found 1092.1.

### Example 92

### 10-((7Z)-27-hydroxy-21-(10-(((2Z)-non-2-enyl)oxy)-10-oxodecyl)-11-oxo-21,25-diaza-10-oxaheptacosane-7-en-25-yl)decanoic acid-(2Z)-non-2-en-1-yl ester

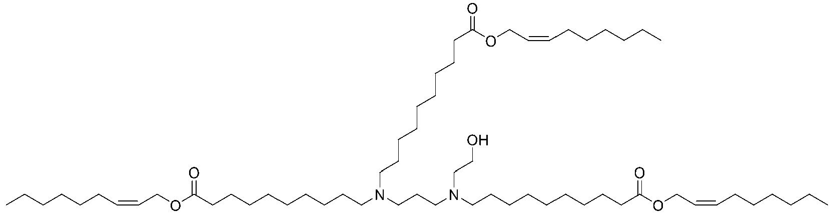

### Step 1: Synthesis of (2Z)-non-2-en-1-yl 10-bromocaprate

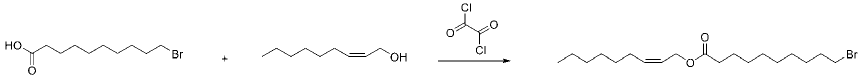

10-bromodecanoic acid (5 g, 19.91 mmol) was dissolved in 100 mL of DCM, oxalyl chloride (7.58 g, 59.723 mmol) was slowly added to the mixture, refluxed for 4 hours, and then concentrated under reduced pressure to obtain 12.3 g of crude product. The crude product was dissolved in DCM (150 mL) and TEA (5 g), (2Z)-non-2-en-1-ol (2.83 g, 19.91 mmol) was added, and stirred at 40 °C for 4 hours. TLC showed complete consumption of raw materials, the mixture was mixed directly, and purified by column chromatography with PE:EA=10:1 to obtain 3.3 g of a colorless oily product with a yield of 52.6%.

### Step 2: Synthesis of 10-((7Z)-27-hydroxy-21-(10-(((2Z)-non-2-enyl)oxy)-10-oxodecyl)-11-oxo-21,25-diaza-10-oxaheptacosane-7-en-25-yl)decanoic acid-(2Z)-non-2-en-1-yl ester

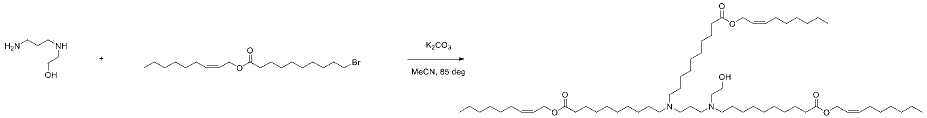

2-((3-aminopropyl)amino)ethan-1-ol (0.1 g, 846.17 umol) was dissolved in 20 mL MeCN, and (2Z)-non-2-en-1-yl 10-bromocaprate (1.11 g, 2.96 mmol), potassium carbonate (0.82 g, 5.92 mmol), and KI (10 mg) were added, and stirred overnight at 85 °C. 50 mL of water and 50 mL of EA were added, the solution was separated, the organic phase was washed with saturated saline solution, dried with anhydrous sodium sulfate, and then concentrated under reduced pressure and purified by column chromatography with DCM (containing 5% EA):MeOH=50:1 to 5:1 to obtain 190 mg of a light yellow oily product with a yield of 22.4%.

ESI-MS *m*/*z:* cald for C₆₂H₁₁₇N₂O₇ [M+H]⁺: 1001.9, found 1001.9.

### Example 93

### Decyl 4-(20-(2-hydroxyethyl)-12,24-dioxo-16,20-diaza-11,25-dioxapentatriacontane-16-yl)butanoate

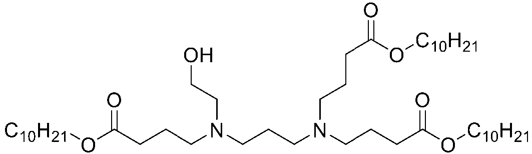

### Step 1: Synthesis of decyl 4-bromobutanoate

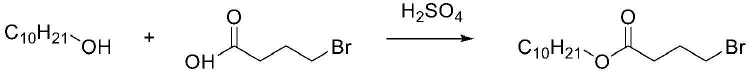

Decane-1-ol (10 g, 63.18 mmol) was mixed with 4-bromobutyric acid (11.61 g, 69.49 mmol), and heated to 65 °C. After the reaction mixture was completely melted, 3 drops of concentrated sulfuric acid were added to perform continue stirring at 65 °C overnight. Stop heating after the reaction was complete, 100 mL of EA and 100 mL of saturated NaHCO₃ solution were added for washing. The aqueous phase was removed, the organic phase was washed with saturated saline solution, dried with anhydrous sodium sulfate, and then concentrated under reduced pressure and purified by column chromatography with PE:EA=10:1 to obtain 15.2 g of a colorless oily product with a yield of 78.3%.

### Step 2: Synthesis of decyl 4-(20-(2-hydroxyethyl)-12,24-dioxo-16,20-diaza-11,25-dioxapentatriacontane-16-yl)butanoate

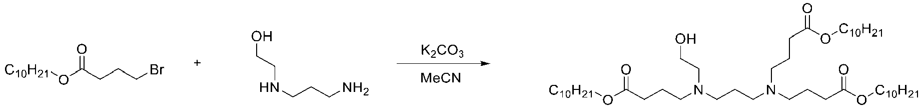

2-((3-aminopropyl)amino)ethan-1-ol (1 g, 8.46 mmol) was dissolved in 50 mL MeCN, and decyl 4-bromobutanoate (9.10 g, 29.62 mmol), potassium carbonate (7.02 g, 50.77 mmol), and KI (20 mg) were added, and stirred overnight at 85 °C. 100 mL of water and 100 mL of EA were added, the solution was separated, the organic phase was washed with saturated saline solution, dried with anhydrous sodium sulfate, and then concentrated under reduced pressure and purified by column chromatography with DCM (containing 5% EA):MeOH=50:1 to 5:1 to obtain 302.4 mg of a light yellow oily product with a yield of 4.44%.

ESI-MS _{m/z}: cald for C₄₇H₉₃N₂O₇ [M+H]⁺: 797.7, found 798.45.

### Example 94

### Dodecyl 4-(22-(2-hydroxyethyl)-14,26-dioxo-18,22-diaza-13,27-dioxanonatriacontane-18-yl)butanoate

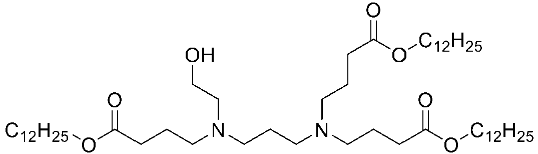

### Step 1: Synthesis of dodecyl 4-bromobutanoate

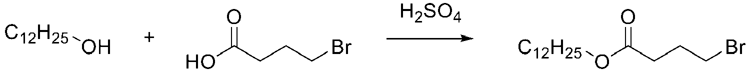

Dodecan-1-ol (12.0 mL, 53.67 mmol) was mixed with 4-bromobutyric acid (9.86 g, 59.03 mmol), and heated to 65 °C. After the reaction mixture was completely melted, 3 drops of concentrated sulfuric acid were added to perform continue stirring at 65 °C overnight. Stop heating after the reaction was complete, 100 mL of EA and 100 mL of saturated NaHCO₃ solution were added for washing. The aqueous phase was removed, the organic phase was washed with saturated saline solution, dried with anhydrous sodium sulfate, and then concentrated under reduced pressure and purified by column chromatography with PE:EA=10:1 to obtain 17.8 g of a colorless oily product with a yield of 98.9%.

### Step 2: dodecyl 4-(22-(2-hydroxyethyl)-14,26-dioxo-18,22-diaza-13,27-dioxanonatriacontane-18-yl)butanoate

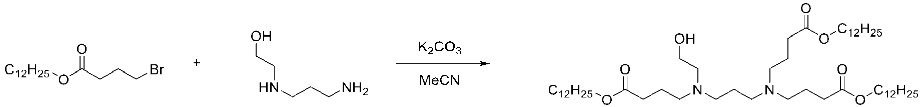

2-((3-aminopropyl)amino)ethan-1-ol (1 g, 8.46 mmol) was dissolved in 50 mL MeCN, and dodecyl 4-bromobutanoate (9.93 g, 29.62 mmol), potassium carbonate (7.02 g, 50.77 mmol), and KI (20 mg) were added, and stirred overnight at 85 °C. 100 mL of water and 100 mL of EA were added, the solution was separated, the organic phase was washed with saturated saline solution, dried with anhydrous sodium sulfate, and then concentrated under reduced pressure and purified by column chromatography with DCM (containing 5% EA):MeOH=50:1 to 5:1 to obtain 3.64 g of a light yellow oily product with a yield of 48.45%.

ESI-MS *m*/*z:* cald for C₅₃H₁₀₅N₂O₇ [M+H]⁺: 881.8, found 881.8.

### Example 95

### 8-((6Z,10Z,50Z,53Z)-32-(3-hydroxypropyl)-20,40-dioxo-28,32-diaza-19,41-dioxanonapentacontane-6,9,50,53-tetraen-28-yl)(10Z,12Z)-octadeca-9,12-dien-1-yl octanoate

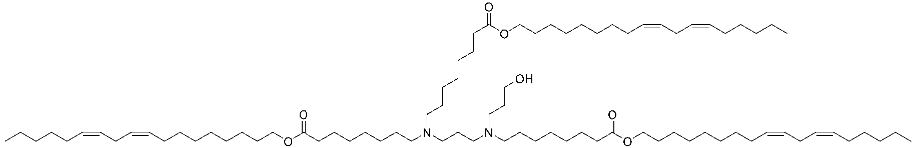

### Step 1: Synthesis of (10Z,12Z)-octadeca-9,12-dien-1-yl 8-bromooctanoate

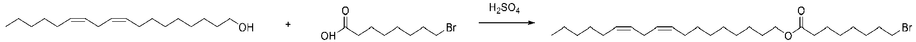

(10Z,12Z)-octadeca-9,12-dien-1-ol (2 g, 7.51 mmol) was mixed with 8-bromooctanoic acid (1.67 g, 8.26 mmol), and heated to 65 °C. After the reaction mixture was completely melted, 3 drops of concentrated sulfuric acid were added to perform continue stirring at 65 °C overnight. Stop heating after the reaction was complete, 100 mL of EA and 100 mL of saturated NaHCO₃ solution were added for washing. The aqueous phase was removed, the organic phase was washed with saturated saline solution, dried with anhydrous sodium sulfate, and then concentrated under reduced pressure and purified by column chromatography with PE:EA=10:1 to obtain 1.98 g of a colorless oily product with a yield of 55.9%.

### Step 2: Synthesis of 8-((30Z,33Z)-8-(8-((10Z,12Z)-octadeca-9,12-dienyl)oxy)-8-oxooctyl)-2,2,3,3-tetramethyl-20-oxo-8,12-diaza-4,21-dioxa-3-silanonatriacontane-30,33-dien-12-yl)(10Z,12Z)-octadeca-9,12-dien-1-yl octanoate

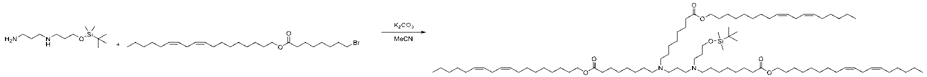

3-((2,2,3,3-tetramethyl-4-oxa-3-silahept-7-yl)amino)propan-1-amine (200 mg, 0.81 mmol) was dissolved in 20 mL MeCN, and (10Z,12Z)-octadeca-9,12-dien-1-yl 8-bromooctanoate (1.3 g, 2.84 mmol), potassium carbonate (672.5 mg, 4.87 mmol), and KI (10 mg) were added, and stirred overnight at 85°C. 50 mL of water and 50 mL of EA were added, the solution was separated, the organic phase was washed with saturated saline solution, dried with anhydrous sodium sulfate, and then concentrated under reduced pressure and purified by column chromatography with DCM (containing 5% EA):MeOH=50:1 to 5:1 to obtain 759.2 mg of a light yellow oily product with a yield of 66%.

### Step 3: Synthesis of 8-((6Z,10Z,50Z,532)-32-(3-hydroxypropyl)-20,40-dioxo-28,32-diaza-19,41-dioxanonapentacontane-6,9,50,53-tetraen-28-yl)(10Z, 12Z)-octadeca-9,12-dien-1-yl octanoate

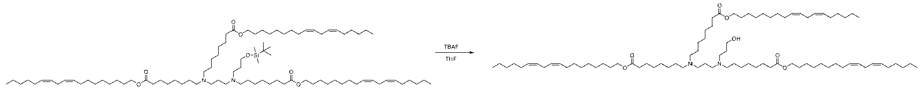

8-((30Z,33Z)-8-(8-((10Z,12Z)-octadeca-9,12-dienyl)oxy}-8-oxooctyl)-2,2,3,3-tetramethyl-20-oxo-8,12-diaza-4,21-dioxa-3-silanonatriacontane-30,33-dien-12-yl)(10Z,12Z)-octadeca-9,12-dien-1-yl octanoate (759.2 mg, 0.54 mmol) was dissolved in 10 mL THF, TBAF (198.42 mg, 0.76 mmol) was added, and stirred overnight at room temperature. The mixture was purified by column chromatography with DCM (containing 5% EA):MeOH=50:1 to 5:1 to obtain 566 mg of a light yellow oily product with a yield of 80.9%.

ESI-MS *m*/*z:* cald for C₈₄H₁₅₅N₂O₇ [M+H]⁺: 1304.2, found 1304.65.

### Example 96

### 8-((6Z,10Z,46Z,49Z)-30-(3-hydroxypropyl)-20,36-dioxo-26,30-diaza-19,37-dioxapentatriacontane-6,9,46,49-tetraen-26-yl)(7Z,10Z)-hexadec-7,10-dien-1-yl octanoate

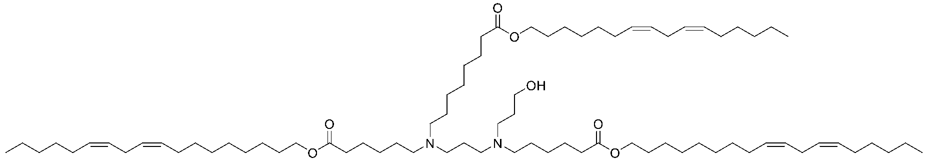

### Step 1: Synthesis of (10Z,12Z)-octadeca-9,12-dien-1-yl 6-bromohexanoate

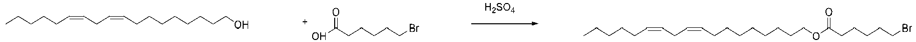

(10Z,12Z)-octadeca-9,12-dien-1-ol (2 g, 7.51 mmol) was mixed with 6-bromohexanoic acid (1.61 g, 8.26 mmol), and heated to 65 °C. After the reaction mixture was completely melted, 3 drops of concentrated sulfuric acid were added to perform continue stirring at 65 °C overnight. Stop heating after the reaction was complete, 100 mL of EA and 100 mL of saturated NaHCO₃ solution were added for washing. The aqueous phase was removed, the organic phase was washed with saturated saline solution, dried with anhydrous sodium sulfate, and then concentrated under reduced pressure and purified by column chromatography with PE:EA=10:1 to obtain 1.67 g of a colorless oily product with a yield of 51%.

### Step 2: Synthesis of 8-((28Z,31Z)-8-(6-((10Z,12Z)-octadeca-9,12-dienyl)oxy)-6-oxohexyl)-2,2,3,3-tetramethyl-18-oxo-8,12-diaza-4,19-dioxa-3-silaheptatriacontan-28,31-dien-12-yl)(7Z,10Z)-hexadec-7,10-dien-1-yl octanoate

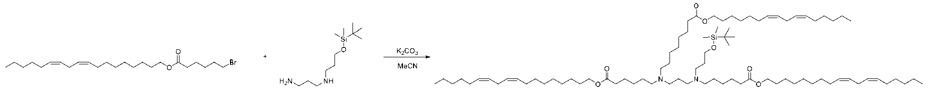

3-((2,2,3,3-tetramethyl-4-oxa-3-silahept-7-yl)amino)propan-1-amine (200 mg, 0.81 mmol) was dissolved in 20 mL MeCN, and (10Z,12Z)-octadeca-9,12-dien-1-yl 6-bromohexanoate (1.3 g, 2.84 mmol), potassium carbonate (672.5 mg, 4.87 mmol), and KI (10 mg) were added, and stirred overnight at 85°C. 50 mL of water and 50 mL of EA were added, the solution was separated, the organic phase was washed with saturated saline solution, dried with anhydrous sodium sulfate, and then concentrated under reduced pressure and purified by column chromatography with DCM (containing 5% EA):MeOH=50:1 to 5:1 to obtain 675 mg of a light yellow oily product with a yield of 62.5%.

### Step 3: Synthesis of 8-(6Z,10Z,46Z,49Z)-30-(3-hydroxypropyl)-20,36-dioxo-26,30-diaza-19,37-dioxapentatriacontane-6,9,46,49-tetraen-26-yl)(7Z,10Z)-hexadec-7,10-dien-1-yl octanoate

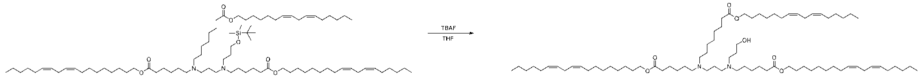

8-(((28Z,31Z)-8-(6-((10Z,12Z)-octadeca-9,12-dienyl)oxy)-6-oxohexyl)-2,2,3,3-tetramethyl-18-oxo-8,12-diaza-4,19-dioxa-3-silaheptatriacontan-28,31-dien-12-yl)(7Z,10Z)-hexadec-7,10-dien-1-yl octanoate (675 mg, 0.51 mmol) was dissolved in 10 mL THF, TBAF (198 mg, 0.76 mmol) was added, and stirred overnight at room temperature. The mixture was purified by column chromatography with DCM (containing 5% EA):MeOH=50:1 to 5:1 to obtain 457 mg of a light yellow oily product with a yield of 73.8%.

ESI-MS *m*/*z:* cald for C₇₈H₁₄₃N₂O₇ [M+H]⁺: 1220.1, found 1220.75.

### Example 97

### 8,8'-(3-((2-hydroxyethyl)(8-oxo-8-((Z)-pent-2-en-1-oxy)oxy)octyl)amino)propyl)azadiyl)dioctanoate

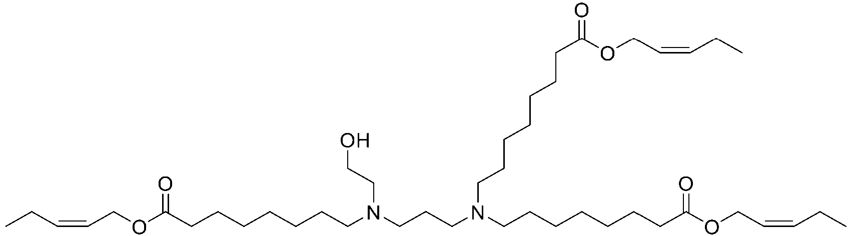

### Step 1: Synthesis of (Z)-pent-2-en-1-yl 8-bromooctanoate

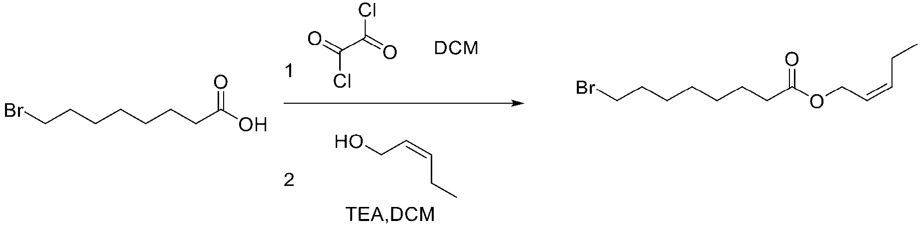

8-bromooctanoic acid (7.23 g, 32.4 mmol) was dissolved in 100 mL of DCM, oxalyl chloride (4.94 g, 38.89 mmol) was slowly added to the mixture, refluxed for 4 hours, and then concentrated under reduced pressure to obtain a crude product. Half of the crude product was dissolved in DCM (150 ml) and TEA (3.28 g, 32.4 mmol), cis-2-penten-1-ol (1.40 g, 16.2 mmol) was added, and stirred at 40°C for 4 hours. TLC showed complete consumption of raw materials, the mixture was mixed directly, and purified by column chromatography with PE:EA=15:1 to obtain 4.0 g of a target molecule with a yield of 84.78%.

### Step 2: Synthesis of 8,8'-(3-((2-hydroxyethyl)(8-oxo-8-((Z)-pent-2-en-1-oxy)oxy)octyl)amino)propyl)azadiyl)dioctanoate

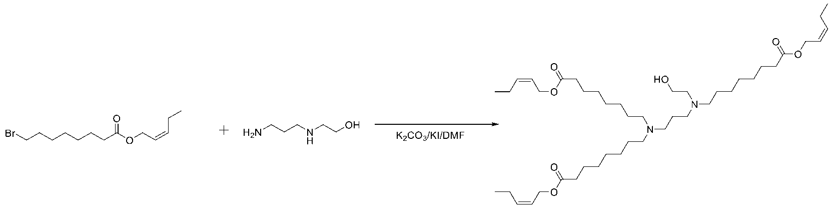

N-(2-hydroxyethyl)-1,3-propanediamine (59.1 mg, 0.5 mmol) was dissolved in DMF (5 mL), and (Z)-pent-2-en-1-yl 8-bromooctanoate (509 mg, 1.75 mmol), potassium carbonate (345 mg, 2.5 mmol), and KI (13 mg) were added respectively, and stirred at 85°C for 16 hours. An appropriate amount of water was added to the mixture, then EA (50 mL * 3) was added for extraction. The combined organic phases were dried and concentrated under reduced pressure. The crude product was purified by column chromatography with DCM (containing 5% EA):MeOH=50:1 to 5:1 to obtain 0.1 g of a target molecule with a yield of 26.7 %.

ESI-MS *m*/*z:* cald for C₄₄H₈₁N₂O₇ [M+H]⁺: 749.6, found 749.45.

¹H NMR (400 MHz, CDCl₃) δ 5.71 - 5.59 (m, 3H), 5.55 - 5.43 (m, 3H), 4.62 (m, 6H), 3.67 (t, J = 5.0 Hz, 2H), 2.99-2.90 (m, 1H), 2.87-2.78 (m, 4H), 2.74 - 2.65 (m, 4H), 2.61 - 2.51 (m, 2H), 2.31 (t, J = 7.5 Hz, 6H), 2.17-2.07 (m, 6H), 2.02-1.88 (m, 2H), 1.70-1.56 (m, 10H), 1.54-1.44 (m, 2H), 1.39-1.24 (m, 20H), 1.00 (t, J = 7.5 Hz, 9H).

### Example 98

### Tetraundecyl 8,8',8",8"-(((3-hydroxypropyl)azanediyl)di(propane-3,1-diyl))di(azanetriyl)tetraoctanoate

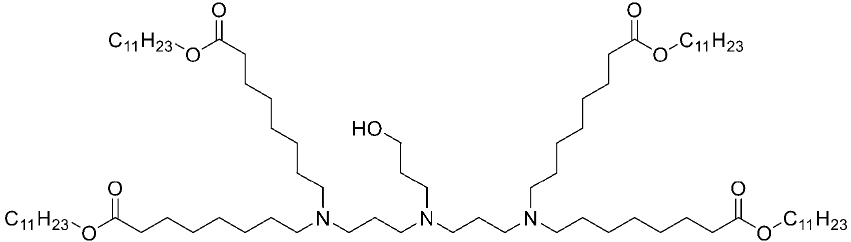

### Step 1: Synthesis of 8,8',8",8"-((azanediyldi(propan-3,1-diyl))di(azanetriyl))tetraundecyl tetraoctanoate

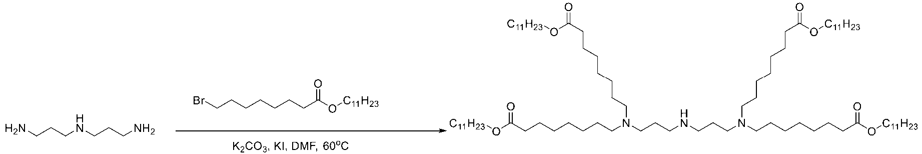

Di(3-aminopropyl)amine (191.32 mg, 1.46 mmol) was dissolved in DMF (5 mL), and undecyl 8-bromooctanoate (2.2 g, 5.83 mmol), potassium carbonate (1 g, 7.29 mmol), and KI (50 mg) were added respectively, and stirred at 60 °C for 6 hours. An appropriate amount of water was added to the mixture, then EA (100 mL * 3) was added for extraction. The combined organic phases were dried and concentrated under reduced pressure. The crude product was purified by column chromatography with DCM (containing 5% EA):MeOH=50:1 to 5:1 to obtain 0.62 g of a colorless oily product with a yield of 32.28 %.

ESI-MS *m*/*z:* cald for C₈₂H₁₆₂N₃O₈ [M+H]⁺: 1317.2, found 1317.2.

### Step 2: Synthesis of tetraundecyl 8,8',8",8"-(((3-hydroxypropyl)azanediyl)di(propane-3,1-diyl))di(azanetriyl)tetraoctanoate

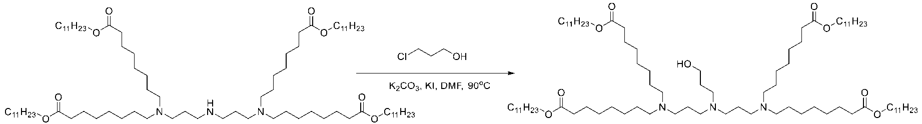

8,8',8",8"-((azadiyldi(propan-3,1-diyl))di(azanetriyl)tetraundecyl tetraoctanoate (620 mg, 0.47 mmol) was dissolved in DMF (5 mL), and 3-chloro-1-propanol (88.87 mg, 0.94 mmol), potassium carbonate (276.4 mg, 2 mmol), and KI (50 mg) were added respectively, and stirred at 90°C for 16 hours. Then EA (80 mL * 3) was added for extraction. The combined organic phases were dried and concentrated under reduced pressure. The crude product was purified by column chromatography with DCM (containing 5% EA):MeOH=50:1 to 5:1 to obtain 0.14 g of a target molecule with a yield of 21.66 %.

ESI-MS *m*/*z:* cald for C₈₅H₁₆₈N₃O₉ [M+H]⁺: 1375.3, found 1375.15.

¹H NMR (400 MHz, CDCl₃) δ 4.04 (t, J = 6.8 Hz, 8H), 3.80-3.65 (m, 3H), 2.83 - 2.67 (m, 9H), 2.83 - 2.37 (m, 12H), 2.28 (t, J = 7.5 Hz, 8H), 1.91-1.71 (m, 5H), 1.67-1.52 (m, 20H), 1.50 - 1.40 (m, 3H), 1.44 - 1.17 (m, 87H), 0.87 (t, J = 6.7 Hz, 12H).

### Example 99

### Tetratridecyl 8,8',8",8"-(((3-hydroxypropyl)azadiyl)di(propane-3,1-diyl))di(azanetriyl)tetraoctanoate

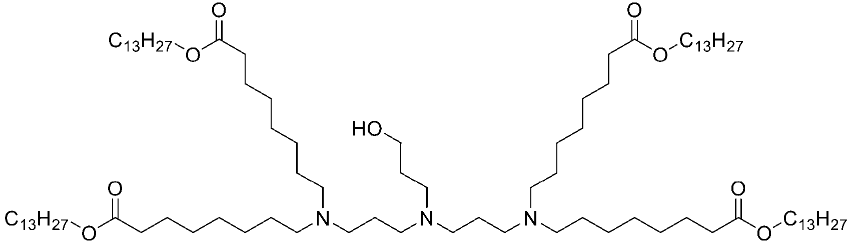

### Step 1: Synthesis of tetratridecyl 8,8',8",8"-((azadiyldi(propane-3,1-diyl))di(azanetriyl))tetraoctanoate

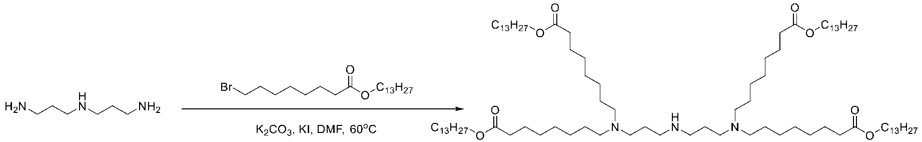

Di(3-aminopropyl)amine (243 mg, 1.85 mmol) was dissolved in DMF (8 mL), and tridecyl 8-bromooctanoate (3 g, 7.4 mmol), potassium carbonate (1.28 g, 9.25 mmol), and KI (50 mg) were added respectively, and stirred at 60 °C for 6 hours. An appropriate amount of water was added to the mixture, then EA (100 mL * 3) was added for extraction. The combined organic phases were dried and concentrated under reduced pressure. The crude product was purified by column chromatography with DCM (containing 5% EA):MeOH=50:1 to 5:1 to obtain 0.76 g of a target molecule with a yield of 28.74 %.

ESI-MS *m*/*z:* cald for C₉₀H₁₇₈N₃O₈ [M+H]⁺: 1429.4, found 1429.4.

### Step 2: Synthesis of tetratridecyl 8,8',8",8"-(((3-hydroxypropyl)azanediyl)di(propane-3,1-diyl))di(azanetriyl)tetraoctanoate

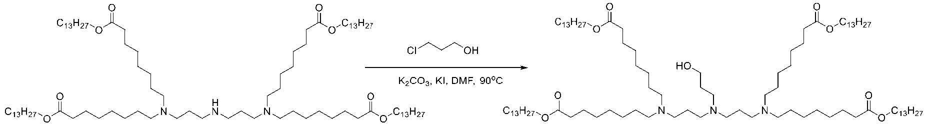

Tetratridecyl 8,8',8",8"-((azadiyldi(propane-3,1-diyl))di(azanetriyl))tetraoctanoate (760 mg, 0.53 mmol) was dissolved in DMF (5 mL), and 3-chloro-1-propanol (100.2 mg, 1.06 mmol), potassium carbonate (276.4 mg, 2 mmol), and KI (50 mg) were added respectively, and stirred at 90°C for 16 hours. Then EA (80 mL * 3) was added for extraction. The combined organic phases were dried and concentrated under reduced pressure. The crude product was purified by column chromatography with DCM (containing 5% EA):MeOH=50:1 to 5:1 to obtain 0.16 g of a target molecule with a yield of 20.29 %.

ESI-MS *m*/*z:* cald for C₉₃H₁₈₄N₃O₉ [M+H]⁺: 1487.4, found 1488.4.

¹H NMR (400 MHz, CDCl₃) δ 4.04 (t, J = 6.8 Hz, 8H), 3.77 (t, J = 5.1 Hz, 2H), 2.62 (t, J = 5.5 Hz, 3H), 2.50-2.32 (m, 16H), 2.28 (t, J = 7.5 Hz, 8H), 1.74-1.52 (m, 22H), 1.51-1.35 (m, 10H), 1.34-1.20 (m, 102H),0.87 (t, J = 6.7 Hz, 12H).

Example 100

### 4,4'-ditridecyl((5-((3-hydroxypropyl)(4-oxo-4-(tridecyloxy)butyl)amino)pentyl)azetidinyl)dibutanoate

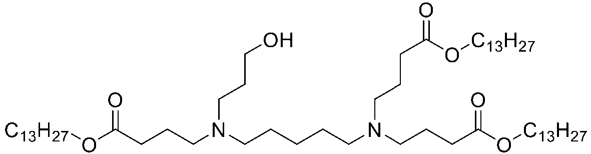

### Step 1: Synthesis of tridecyl 4-bromobutanoate

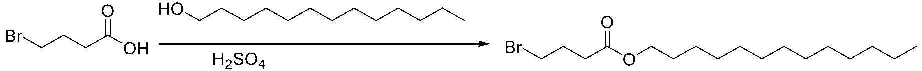

Using trideca-1-ol and 4-bromobutyric acid as raw materials and according to step 1 of Example 3, tridecyl 4-bromobutanoate was obtained. The crude product was purified by column chromatography to obtain 14 g of a target molecule with a yield of 66.9%.

### Step 2: Synthesis of 4,4'-ditridecyl((5-(4-oxo-4-(tridecyloxy)butyl)amino)pentyl)azetidinyl)dibutanoate

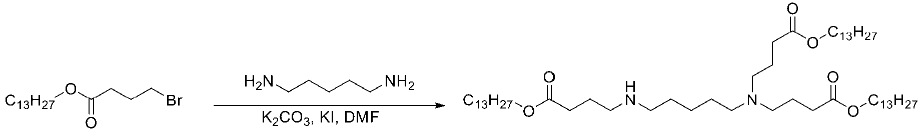

1,5-pentanediamine (633.7 mg, 6.2 mmol) was dissolved in DMF (35 mL), and tridecyl 4-bromobutanoate (6.5 g, 18.6 mmol), potassium carbonate (3.73 g, 26.96 mmol), and KI (50 mg) were added respectively, and stirred at 60 °C for 6 hours. An appropriate amount of water was added to the mixture, then EA (100 mL * 3) was added for extraction. The combined organic phases were dried and concentrated under reduced pressure. The crude product was purified by column chromatography with DCM (containing 5% EA):MeOH=50:1 to 5:1 to obtain 565 mg of a target product with a yield of 10.04 %.

ESI-MS *m*/*z:* cald for C₅₆H₁₁₁N₂O₆ [M+H]⁺: 907.8, found 907.8.

### Step 3: Synthesis of 4,4'-ditridecyl((5-((3-hydroxypropyl)(4-oxo-4-(tridecyloxy)butyl)amino)pentyl)azetidinyl)dibutanoate

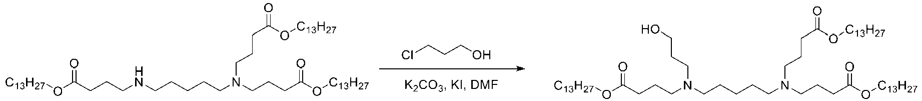

4,4'-ditridecyl((5-(4-oxo-4-(tridecyloxy)butyl)amino)pentyl)azetidinyl)dibutanoate (565 mg, 0.62 mmol) was dissolved in DMF (5 mL), 3-chloro-1-propanol (118 mg, 1.25 mmol), potassium carbonate (258 mg, 1.87 mmol), and KI (50 mg) were added respectively, and stirred at 90°C for 16 hours. Then EA (80 mL * 3) was added for extraction. The combined organic phases were dried and concentrated under reduced pressure. The crude product was purified by column chromatography with DCM (containing 5% EA):MeOH=50:1 to 5:1 to obtain 0.06 g of a target product with a yield of 9.97 %.

ESI-MS *m*/*z:* cald for C₅₉H₁₁₇N₂O₇ [M+H]⁺: 965.9, found 965.8.

¹H NMR (400 MHz, CDCl₃) δ 4.11-4.00 (m, 6H), 3.82-3.64 (m, 2H), 2.68-2.62 (m, 1H), 2.52-2.32 (m, 10H), 2.31-2.20 (m, 6H), 2.10-1.87 (m, 2H), 1.85-1.66 (m, 9H), 1.65-1.55 (m, 6H), 1.38-1.16 (m, 65H), 0.88 (t, J = 6.7 Hz, 9H).

### Example 101

### 4,4'-ditridecyl((4-((3-hydroxypropyl)(4-oxo-4-(tridecyloxy)butyl)amino)butyl)azetidinyl)dibutanoate

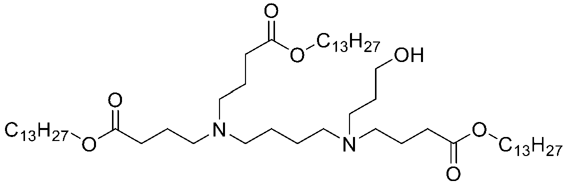

### Step 1: Synthesis of 4,4'-ditridecyl((4-(4-oxo-4-(tridecyloxy)butyl)amino)butyl)azetidinyl)dibutanoate

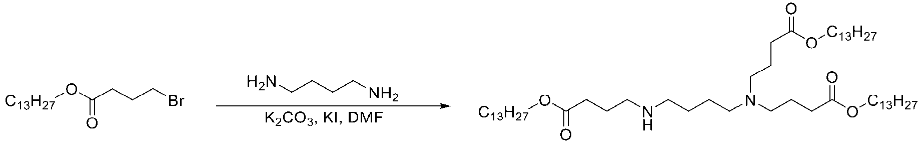

Using tridecyl 4-bromobutanoate and 1,4-butanediamine as raw materials and according to step 2 of Example 19, 4,4'-ditridecyl((4-(4-oxo-4-(tridecyloxy)butyl)amino)butyl)azetidinyl)dibutanoate was obtained. The crude product was purified by column chromatography to obtain 1 g of a target molecule with a yield of 14.84%.

ESI-MS *m*/*z:* cald for C₅₅H₁₀₉N₂O₆ [M+H]⁺: 893.8, found 893.8.

### Step 2: Synthesis of 4,4'-ditridecyl((4-((3-hydroxypropyl)(4-oxo-4-(tridecyloxy)butyl)amino)butyl)azetidinyl)dibutanoate

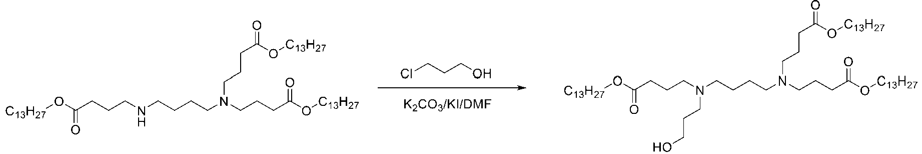

Using 4,4'-ditridecyl((4-(4-oxo-4-(tridecyloxy)butyl)amino)butyl)azetidinyl)dibutanoate and 3-chloro-1-propanol as raw materials and according to step 3 of Example 19, 4,4'-ditridecyl((4-((3-hydroxypropyl)(4-oxo-4-(tridecyloxy)butyl)amino)butyl)azetidinyl)dibutanoate was obtained. The crude product was purified by column chromatography to obtain 0.06 g of a target molecule with a yield of 6.3%.

ESI-MS *m*/*z:* cald for C₅₈H₁₁₅N₂O₇ [M+H]⁺: 951.9, found 951.8.

¹H NMR (400 MHz, CDCl₃) δ 4.12-3.94 (m, 6H), 3.78 (t, J = 5.2 Hz, 2H), 2.73 - 2.59 (m, 2H), 2.51-2.36 (m, 9H), 2.31 (t, J = 7.4 Hz, 6H), 1.89 - 1.78 (m, 2H), 1.77 - 1.65 (m, 6H), 1.64 - 1.54 (m, 6H), 1.50 - 1.35 (m, 6H), 1.35 - 1.17 (m, 60H), 0.88 (t, J = 6.7 Hz, 9H).

### Example 102

### 4,4'-didecyl((4-(decyloxy)-4-oxobutyl)(3-hydroxypropyl)amino)butyl)azetidinyl)dibutanoate

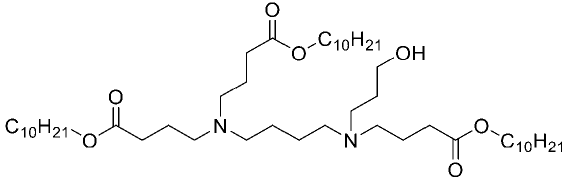

### Step 1: Synthesis of decyl 4-bromobutanoate

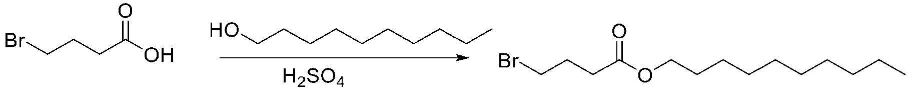

Using decane-1-ol and 4-bromobutyric acid as raw materials and according to step 1 of Example 3, decyl 4-bromobutanoate was obtained. The crude product was purified by column chromatography to obtain 0.06 g of a target molecule with a yield of 6.3%.

### Step 2: Synthesis of 4,4'-ditridecyl((5-(4-oxo-4-(tridecyloxy)butyl)amino)pentyl)azetidinyl)dibutanoate

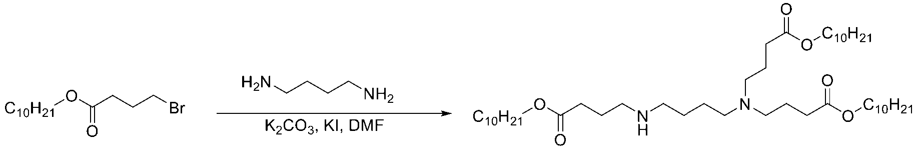

Using decyl 4-bromobutanoate and 1,4-butanediamine as raw materials and according to step 2 of Example 19, 4,4'-ditridecyl((5-(4-oxo-4-(tridecyloxy)butyl)amino)pentyl)azetidinyl)dibutanoate was obtained. The crude product was purified by column chromatography to obtain 700 mg of a target molecule with a yield of 9.35%.

### ESI-MS m/z: cald for C₄₆H₉₁N₂O₆ [M+H]⁺: 767.7, found 767.7.

### Step 3: Synthesis of 4,4'-didecyl((4-(decyloxy)-4-oxobutyl)(3-hydroxypropyl)amino)butyl)azetidinyl)dibutanoate

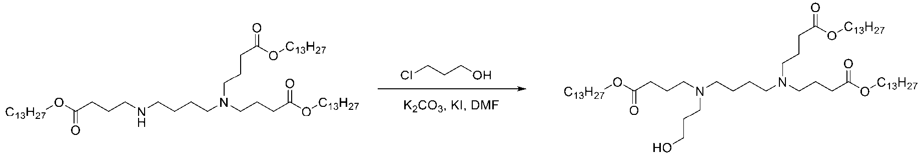

Using 4,4'-ditridecyl((5-(4-oxo-4-(tridecyloxy)butyl)amino)pentyl)azetidinyl)dibutanoate and 3-chloro-1-propanol as raw materials and according to step 3 of Example 19, 4,4'-didecyl((4-(decyloxy)-4-oxobutyl)(3-hydroxypropyl)amino)butyl)azetidinyl)dibutanoate was obtained. The crude product was purified by column chromatography to obtain 0.1 g of a target molecule with a yield of 13.29%.

ESI-MS *m*/*z:* cald for C₅₈H₁₁₅N₂O₇ [M+H]⁺: 951.9, found 951.9.

¹H NMR (400 MHz, CDCl₃) δ 4.05 (m, 2.7 Hz, 6H), 3.78 (t, J = 5.2 Hz, 2H), 2.71 - 2.62 (m, 2H), 2.52 - 2.36 (m, 10H), 2.31 (t, J = 7.3 Hz, 6H), 1.85 - 1.78 (m, 2H), 1.78 - 1.66 (m, 6H), 1.66 - 1.56 (m, 6H), 1.49 - 1.39 (m, 4H), 1.39 - 1.18 (m, 61H), 0.88 (t, J = 6.7 Hz, 9H).

### Example 103

### 4,4'-didodecyl((4-(dodecoxy)-4-oxobutyl)(3-hydroxypropyl)amino)butyl)azetidinyl)dibutanoate

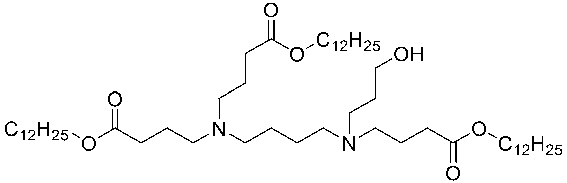

### Step 1: Synthesis of dodecyl 4-bromobutanoate

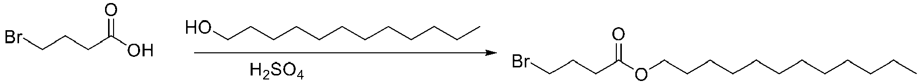

Using dodecan-1-ol and 4-bromobutyric acid as raw materials and according to step 1 of Example 3, dodecyl 4-bromobutanoate was obtained. The crude product was purified by column chromatography to obtain 10.25 g of a target molecule with a yield of 91.87%.

### Step 2: Synthesis of 4,4'-didodecyl((4-(dodecoxy)-4-oxobutyl)amino)butyl)azetidinyl)dibutanoate

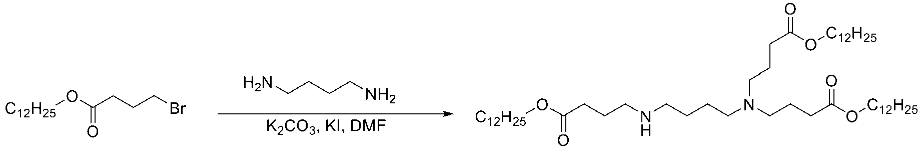

Using dodecyl 4-bromobutanoate and 1,4-butanediamine as raw materials and according to step 2 of Example 19, 4,4'-didodecyl((4-(dodecyloxy)-4-oxobutyl)amino)butyl)azetidinyl)dibutanoate was obtained. The crude product was purified by column chromatography to obtain 900 mg of a target molecule with a yield of 10.37%.

ESI-MS *m*/*z:* cald for C₅₂H₁₀₃N₂O₆ [M+H]⁺: 851.8, found 851.8.

### Step 3: Synthesis of 4,4'-didodecyl((4-(dodecoxy)-4-oxobutyl)(3-hydroxypropyl)amino)butyl)azetidinyl)dibutanoate

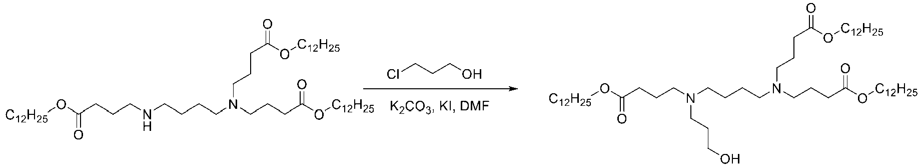

Using 4,4'-didodecyl((4-(dodecoxy)-4-oxobutyl)amino)butyl)azetidinyl)dibutanoate and 3-chloro-1-propanol as raw materials and according to step 3 of Example 19, 4,4'-didodecyl((4-(dodecoxy)-4-oxobutyl)(3-hydroxypropyl)amino)butyl)azetidinyl)dibutanoate was obtained. The crude product was purified by column chromatography to obtain 85 mg of a target molecule with a yield of 8.84%.

ESI-MS *m*/*z:* cald for C₅₅H₁₀₉N₂O₇ [M+H]⁺: 909.8, found 909.8.

¹H NMR (400 MHz, CDCl₃) δ 4.12-3.93 (m, 6H), 3.78 (t, J = 5.2 Hz, 2H), 2.73 - 2.62 (m, 2H), 2.56 - 2.37 (m, 9H), 2.35 - 2.27 (m, 7H), 1.89 - 1.65 (m, 8H), 1.66 - 1.54 (m, 6H), 1.51-1.36 (m, 4H), 1.37 - 1.17 (m, 55H), 0.88 (t, J = 6.8 Hz, 9H).

### Example 104

### Di((9Z,12Z)-octadeca-9,12-dien-1-yl)4,4'-((4-(3-hydroxypropyl)(4-(((9Z,12Z)-octadeca-9,12-dien-1-yl)oxy)-4-oxobutyl)amino)butyl)azetidinyl)dibutanoate

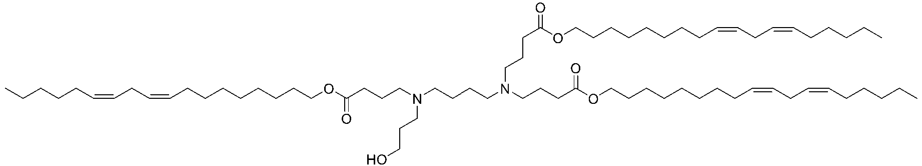

### Step 1: Synthesis of (9Z,12Z)-octadeca-9,12-dien-1-yl 4-bromobutanoate

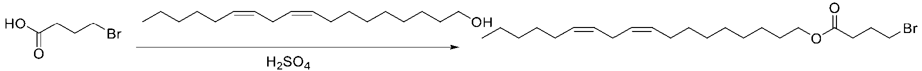

4-bromobutyric acid (12.5 g, 74.85 mmol) and (9Z,12Z)-octadeca-9,12-dien-1-ol (19.95 g, 74.85 mmol) were dissolved after stirring at 60 °C for 0.5 hours, and 3 drops of concentrated sulfuric acid were added. The mixture was reacted at 60 °C for 6 hours. TLC showed complete consumption of raw materials, the mixture was mixed directly, and purified by column chromatography with PE:EA=15:1 to obtain 23 g of a target product with a yield of 76.55%.

### Step 2: Synthesis of (4-((3-(tertbutyldimethylsilyl)oxy)propyl)amino)butyl)tert-butyl carbamate

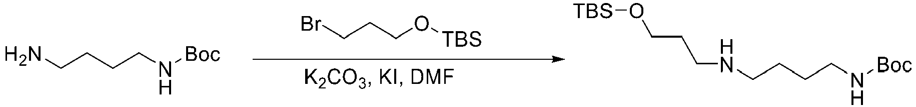

2-methylpropan-2-yl[(4-aminobutyl)amino]methyl formate (4.13 g, 21.96 mmol) was dissolved in DMF (100 mL), and 7-bromo-2,2,3,3-tetramethyl-4-oxo-3-silaheptane (5.56 g, 21.96 mmol), potassium carbonate (6.07 g, 43.91 mmol), and KI (0.36 g, 2.20 mmol) were added respectively, and stirred at 80 °C for 18 hours. 500 mL of water was added to the mixture, then EA (100 mL * 3) was added for extraction. The combined organic phases were dried and concentrated under reduced pressure. The crude product was purified by column chromatography with DCM (containing 5% EA):MeOH=50:1 to 5:1 to obtain 2.74 g of a target product with a yield of 34.6%.

ESI-MS *m*/*z:* cald for C₁₈H₄₁N₂O₃Si [M+H]⁺: 361.3, found 361.3.

### Step 3: Synthesis of (4-((3-(tertbutyldimethylsilyl)oxy)propyl)amino)butyl)tert-butyl carbamate

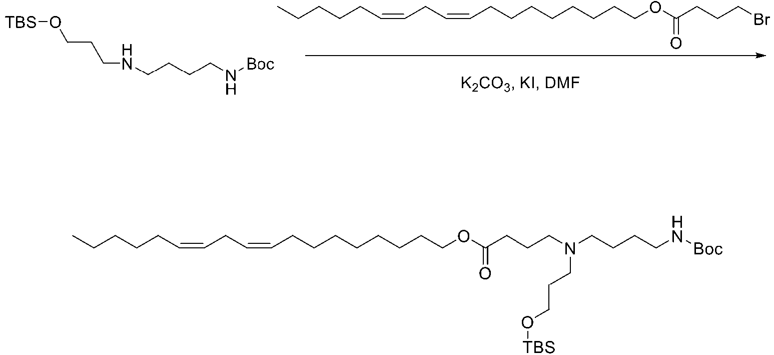

(4-((3-((tert-butyldimethylsilyl)oxy)propyl)amino)butyl)tert-butyl carbamate (5.33 g, 14.78 mmol) was dissolved in DMF (70 mL), and (9Z,12Z)-octadeca-9,12-dien-1-yl 4-bromobutanoate (6.14 g, 14.78 mmol), potassium carbonate (4.09 g, 29.56 mmol), and KI (250 mg, 1.478 mmol) were added respectively, and stirred at 85°C for 16 hours. After cooling to room temperature, 200 mL of water was added for dilution, then EA (100 mL * 3) was added for extraction. The combined organic phases were dried and concentrated under reduced pressure. The crude product was purified by column chromatography with DCM (containing 5% EA):MeOH=50:1 to 5:1 to obtain 7.81 g of a colorless oily product with a yield of 76.0 %.

ESI-MS *m*/*z:* cald for C₄₀H₇₉N₂O₅Si [M+H]⁺: 695.6, found 695.6.

### Step 4: Synthesis of (9Z,12Z)-octadeca-9,12-dien-1-yl-4-((4-aminobutyl)(3-hydroxypropyl)amino)butanoate

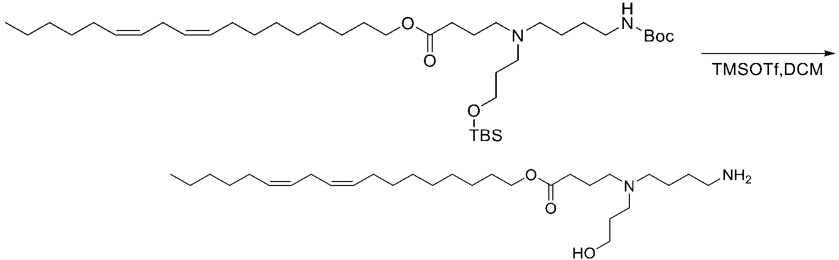

(9Z,12Z)-octadeca-9,12-dien-1-yl-4-((4-((tert-butoxyoxo)amino)butyl)(3-(((tert-butyldimethylsilyl)oxy)propyl)amino)butanoate (7.81 g, 11.23 mmol) was dissolved in DCM (50 mL), then trimethylsilyl trifluoromethanesulfonate (4.99 g, 22.46 mmol) was added. The reaction solution was stirred at room temperature for 16 hours. The mixture was diluted with 50 mL of DCM, and washed twice with saturated sodium bicarbonate solution. The combined organic phases were dried and concentrated under reduced pressure. The crude product was purified by column chromatography with DCM (containing 5% EA):MeOH=50:1 to 5:1 to obtain 2.58 g of a target product with a yield of 47.8%.

ESI-MS *m*/*z:* cald for C₂₉H₅₇N₂O₃ [M+H]⁺: 481.4, found 481.4.

### Step 5: Synthesis of di((9Z,12Z)-octadeca-9,12-dien-1-yl)4,4'-((4-(3-hydroxypropyl)(4-(((9Z,12Z)-octadeca-9,12-dien-1-yl)oxy)-4-oxobutyl)amino)butyl)azetidinyl)dibutanoate

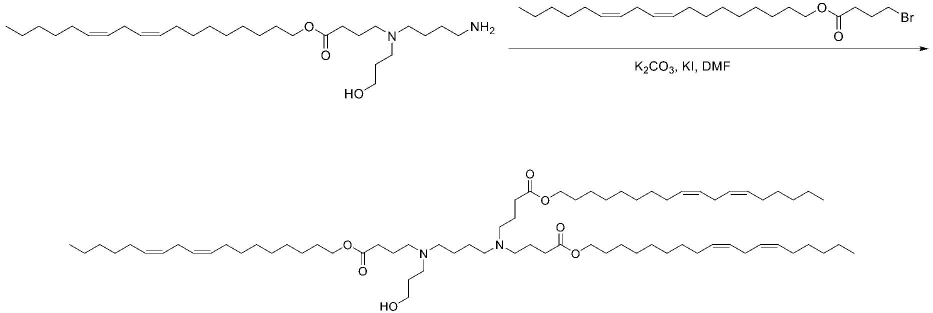

(9Z,12Z)-octadeca-9,12-dien-1-yl-4-((4-aminobutyl)(3-hydroxypropyl)amino)butanoate (2.58 g, 5.366 mmol) and (9Z,12Z)-octadeca-9,12-dien-1-yl 4-bromobutanoate (5.57 g, 13.4 mmol) were added into a 100 mL single-necked flask, and then 30 mL of DMF, potassium carbonate (2.22 g, 16.1 mmol), and potassium iodide (50 mg) were added. The reaction solution was stirred at 85°C for 16 hours. After cooling to room temperature, 100 mL of water was added for dilution, and then EA (100 mL * 3) was added for extraction three times. The organic phase was combined, and washed twice with saturated sodium bicarbonate solution. The combined organic phases were dried and concentrated under reduced pressure. The crude product was purified by column chromatography with DCM (containing 5% EA):MeOH=50:1 to 5:1 to obtain 2.5 g of a target product with a yield of 40.5 %.

ESI-MS *m*/*z:* cald for C₇₃H₁₃₃N₂O₇ [M+H]⁺: 1150.0, found 1150.0.

¹H NMR (400 MHz, CDCl₃) δ 5.56 - 5.16 (m, 12H), 4.17 - 3.95 (m, 6H), 3.78 (t, J = 5.2 Hz, 2H), 2.77 (t, J = 6.5 Hz, 6H), 2.64 (t, J = 5.7 Hz, 2H), 2.53-2.36 (m, 10H), 2.31 (t, J = 7.4 Hz, 6H), 2.05 (q, J = 6.9 Hz, 12H), 1.84-1.64 (m, 10H), 1.64 - 1.56 (m, 6H), 1.52-1.19 (m, 51H), 0.89 (t, J = 6.7 Hz, 9H).

### Example 105

### 4,4'-(3-(((2-hydroxyethyl)((E)-4-oxo-4-(undecyloxy)but-2-en-1-yl)amino)propyl)azadiyl)(2E,2'E)-di(but-2-enoic acid)

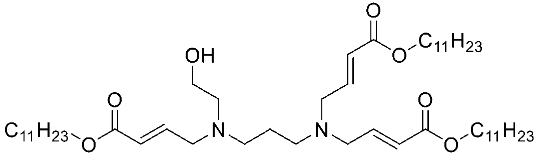

### Step 1: Synthesis of undecyl (E) -4-bromobut-2-enoic acid

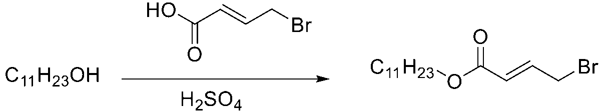

4-bromocrotonic acid (4.79 g, 29 mmol) and 1-undecanol (5 g, 29 mmol) were dissolved after stirring at 60 °C for 0.5 hours, and 3 drops of concentrated sulfuric acid were added. The mixture was reacted at 60°C for 6 hours. TLC showed complete consumption of raw materials, the mixture was mixed directly, and purified by column chromatography with PE:EA=15:1 to obtain 7.7 g of a colorless oily product with a yield of 83.11%.

### Step 2: Synthesis of di(2-hexyldecyl)4,4'-((3-(di(2-hydroxyethyl)amino)propyl)azadiyl)dibutanoate

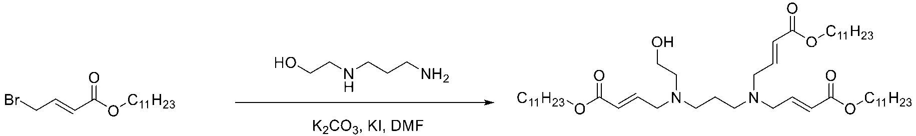

Undecyl(E)-4-bromobut-2-enoic acid (4.225 g, 13.327 mmol) was dissolved in DMF (15 mL), 2-(3-aminopropyl)amino)ethane-1-ol (350 mg, 2.96 mmol), potassium carbonate (2.05 g, 14.8 mmol), and KI (50 mg) were added respectively, and stirred at 85°C for 5 hours. Then EA (80 mL * 3) was added for extraction. The combined organic phases were dried and concentrated under reduced pressure. The crude product was purified by column chromatography with a PE: EA gradient to 50% to obtain 0.185 g of a light yellow oily product with a yield of 7.5%.

ESI-MS *m*/*z:* cald for C₅₆H₉₃N₂O₇ [M+H]⁺: 833.7, found 833.7.

¹H NMR (400 MHz, CDCl₃) δ 6.90 (m, 2H), 5.98 (m, 2H), 4.17 - 3.98 (m, 6H), 3.98 - 3.89 (m, 1H), 3.87 - 3.76 (m, 1H), 3.70 - 3.55 (m, 1H), 3.21 (m, 3H), 2.84-2.71 (m, 1H), 2.70 - 2.57 (m, 1H), 2.57 - 2.43 (m, 2H), 2.42 - 2.38 (m, 1H), 2.38 - 2.20 (m, 2H), 2.17 - 2.00 (m, 1H), 1.90 - 1.78 (m, 1H), 1.78 - 1.68 (m, 3H), 1.67 - 1.46 (m, 8H), 1.45 - 1.05 (m, 50H), 0.87 (t, J = 6.8 Hz, 9H).

### Example 106

### 4,4'-(3-((3-hydroxypropyl)((E)-4-oxo-4-(undecyloxy)but-2-en-1-yl)amino)propyl)azadiyl)(2E,2'E)-di(but-2-enoic acid)

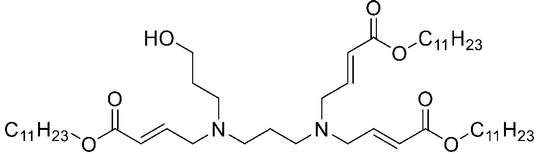

### Step 1: Synthesis of undecyl(E)-2,2,3,3-tetramethyl-8,12-di((E)-4-oxo-4-(undecyloxy)but-2-en-1-yl)-4-oxy-8,12-diaza-3-silane-hexadectetradecenoate

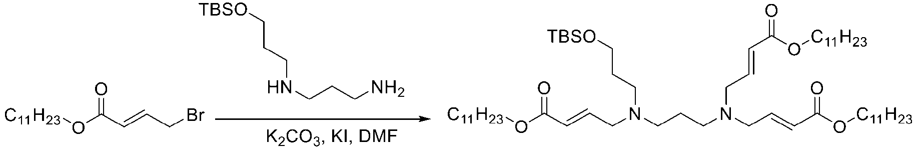

Undecyl(E)-4-bromobut-2-enoic acid (3.5 g, 10.96 mmol) was dissolved in DMF (45 mL), N1-(3-((tert-butyldimethylsilyl)oxy)propyl)propane-1,3-diamine (600 mg, 2.43 mmol), potassium carbonate (1.68 g, 12.17 mmol), and KI (40 mg) were added respectively, and stirred at 85°C for 5 hours. Then EA (80 mL * 3) was added for extraction. The combined organic phases were dried and concentrated under reduced pressure. The crude product was purified by column chromatography with a PE: EA gradient to 40% to obtain 1.4 g of a target product with a yield of 59.82 %.

ESI-MS *m*/*z:* cald for C₅₇H₁₀₉N₂O₇Si [M+H]⁺: 961.8, found 961.8.

¹H NMR (400 MHz, CDCl₃) δ 6.99-6.77 (m, 3H), 5.98 (d, J = 15.9 Hz, 3H), 4.34 - 4.00 (m, 6H), 3.62 (t, J = 6.2 Hz, 2H), 3.28 - 3.15 (m, 6H), 2.58-2.23 (m, 6H), 1.72 - 1.61 (m, 9H), 1.42-1.17 (m, 49H), 0.97 - 0.80 (m, 18H), 0.04 (s, 6H).

### Step 3: Synthesis of 4,4'-(3-((3-hydroxypropyl)((E)-4-oxo-4-(undecyloxy)but-2-en-1-yl)amino)propyl)azadiyl)(2E, 2'E)-di(but-2-enoic acid)

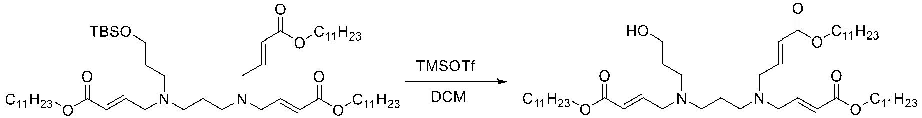

Undecyl(E)-2,2,3,3-tetramethyl-8,12-di((E)-4-oxo-4-(undecyloxy)but-2-en-1-yl)-4-oxy-8,12-diaza-3-silane-hexadectetradecenoate was dissolved in DCM (25 mL), then TMSOTf (416 mg, 1.87 mmol) was added, and stirred at room temperature for 1 hour. The mixture was diluted with DCM, and washed with saturated sodium bicarbonate. The combined organic phases were dried and concentrated under reduced pressure. The crude product was purified by column chromatography with a PE: EA gradient to 60% to obtain 0.72 g of a light yellow oily product with a yield of 90.79 %.

ESI-MS *m*/*z:* cald for C₅₁H₉₅N₂O₇ [M+H]⁺: 847.7, found 847.7.

¹H NMR (400 MHz, CDCl₃) δ 6.91 (m, 3H), 6.32-5.68 (m, 3H), 4.12 (t, J = 6.8 Hz, 6H), 3.75 (t, J = 5.3 Hz, 2H), 3.7 - 3.3 (m, 6H), 2.64 (t, J = 5.9 Hz, 2H), 2.47 (m, 4H), 1.85-1.44 (m, 13H), 1.43 - 1.13 (m, 46H), 0.87 (t, J = 6.7 Hz, 9H).

### Example 107

### Dinonyl 5,5'-((3-((2-hydroxyethyl)(5-oxo-5-(undecyloxy)pentyl)amino)propyl)azadiyl)dipentanoate

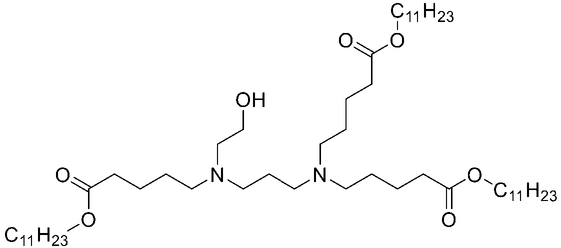

### Step 1: Synthesis of undecyl 5-bromopentanoate

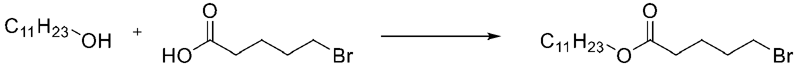

After stirring and dissolving 5-bromovaleric acid (1.3 g, 7.0 mmol) and 1-undecanol (1.0 g, 5.8 mmol) at 60 °C, 1 drop of concentrated sulfuric acid was added, and reacted at 60 °C for 6 hours. TLC showed complete consumption of raw materials, the mixture was mixed directly, and purified by column chromatography with PE:EA=30:1 to obtain 1.8 g of a colorless oily product with a yield of 92.8%.

¹H NMR (400 MHz, CDCl₃) δ 4.06 (t, J = 6.7 Hz, 2H), 3.41 (t, J = 6.6 Hz, 2H), 2.34 (t, J = 7.3 Hz, 2H), 1.95 - 1.85 (m, 2H), 1.84 - 1.73 (m, 2H), 1.62 (m, 2H), 1.33 - 1.23 (m, 16H), 0.88 (t, J = 6.7 Hz, 3H).

### Step 2: Synthesis of dinonyl 5,5'-((3-((2-hydroxyethyl)(5-oxo-5-(undecyloxy)pentyl)amino)propyl)azadiyl)dipentanoate

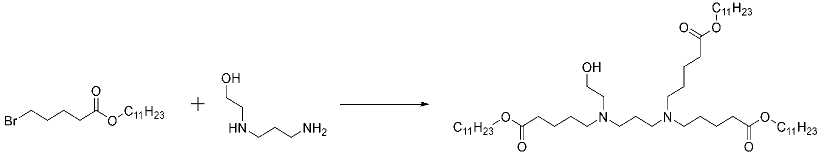

Undecyl 5-bromopentanoate (684 mg, 2.0 mmol) was dissolved in MeCN (10 mL), and 2-((3-aminopropyl)amino)ethane-1-ol (60 mg, 0.5 mmol), potassium carbonate (352 mg, 2.6 mmol), and KI (8.3 mg, 0.05 mmol) were added respectively, and stirred at 85°C for 16 hours. Then DCM (30 mL * 3) was added for extraction. The combined organic phases were dried and concentrated under reduced pressure. The crude product was purified by column chromatography with DCM (containing 5% EA):MeOH=50:1 to 5:1 to obtain 114 mg of a colorless oily product with a yield of 25.3 %.

ESI-MS *m*/*z:* cald for C₅₃H₁₀₅N₂O₇ [M+H]⁺: 881.8, found 881.6.

¹H NMR (400 MHz, CDCl₃) δ 4.05 (t, J = 6.8 Hz, 6H), 3.54 (t, J = 5.2 Hz, 2H), 2.55 (t, J = 5.2 Hz, 2H), 2.51 - 2.35 (m, 10H), 2.31 (t, J = 7.6 Hz, 6H), 1.65 - 1.55 (m, 14H), 1.50 - 1.41 (m, 6H), 1.33 - 1.23 (m, 49H), 1.06 - 0.71 (m, 9H).

### Example 108

### Di((9Z,12Z)-octadeca-9,12-dien-1-yl)4,4'-(3-(3-hydroxypropyl)(4-((9Z,12Z)-octadeca-9,12-dien-1-yl)oxy)-4-oxobutyl)amino)propyl)azanediyl)dibutanoate

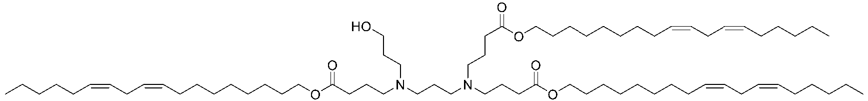

### Step 1: Synthesis of (9Z,12Z)-octadeca-9,12-dien-1-yl 4-bromobutanoate:

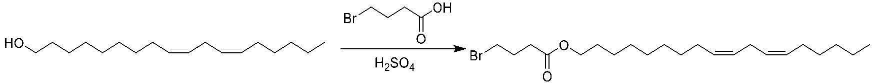

(9Z,12Z)-octadeca-9,12-dien-1-ol (10.0 g, 37.5 mmol) was mixed with 4-bromobutyric acid (7.5 g, 45.0 mmol). The mixture was heated to 60 °C, concentrated sulfuric acid (0.1 mL) was added, and stirred at this temperature for 16 hours. TLC showed basically complete consumption of raw materials, an appropriate amount of water was added, extracted with EA (30 mL * 3), and the organic phase was combined. After evaporating the solvent, the mixture was mixed directly, passed through a column, and eluted with PE:EA=30:1 to obtain 7.9 g of a colorless oily product with a yield of 50.7%.

¹H NMR (400 MHz, CDCl₃) δ 5.46 - 5.27 (m, 4H), 4.08 (t, J = 6.7 Hz, 2H), 3.47 (t, J = 6.5 Hz, 2H), 2.77 (t, J = 6.4 Hz, 2H), 2.50 (t, J = 7.2 Hz, 2H), 2.24 - 2.11 (m, 2H), 2.09 - 2.00 (m, 4H), 1.67 - 1.58 (m, 2H), 1.41 - 1.24 (m, 16H), 0.89 (t, J = 6.7 Hz, 3H).

### Step 2: Synthesis of N1-(3-((tert-butyldimethylsilyl)oxy)propyl)propane-1,3-diamine:

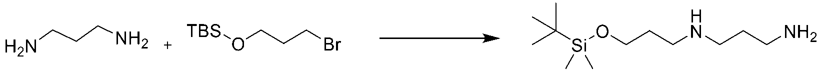

Propane-1,3-diamine (2.9 g, 39.5 mmol) was dissolved in methanol (30 mL), (3-bromopropoxy)(tert-butyl)dimethylsilane (2.0 g, 7.9 mL) dissolved in methanol (20 mL) was added dropwise with stirring, and stirred at room temperature for 18 hours. After evaporating the solvent, the mixture was mixed directly, passed through a column, and eluted with DCM:MeOH(NH₃)=10:1 to obtain 1.0 g of a colorless oily product with a yield of 50.4%.

ESI-MS *m*/*z:* cald for C₁₂H₃₁N₂OSi [M+H]⁺: 247.2, found 247.4.

¹H NMR (400 MHz, CDCl₃) 3.68 (t, J = 6.1 Hz, 2H), 2.76 (t, J = 6.9 Hz, 2H), 2.72 - 2.62 (m, 4H), 1.74 - 1.59 (m, 4H), 1.53 (s, 3H), 0.88 (s, 9H), 0.04 (s, 6H).

### Step 3: Synthesis of (9Z,12Z)-octadeca-9,12-dien-1-yl 2,2,3,3-tetramethyl-8-(4-(((6Z,9Z)-heptadec-6,9-dien-1-yl)oxy)-4-oxobutyl)-12-(4-((9Z,12Z)-octadeca-9,12-dien-1-yl)oxy)-4-oxobutyl)-4-oxo-8,12-diaza-3-silahexadecane-16-acid ester

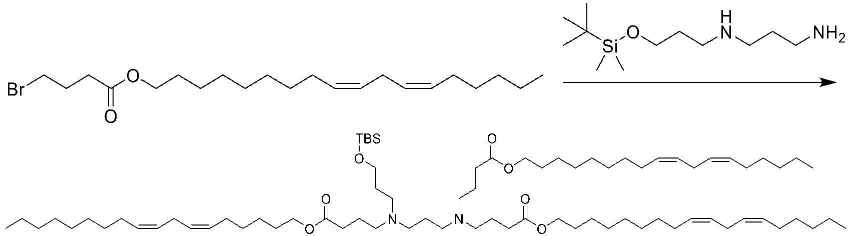

N1-(3-((tert-butyldimethylsilyl)oxy)propyl)propane-1,3-diamine (0.5 g, 1.9 mmol) was dissolved in MeCN (27 mL), and (9Z,12Z)-octadeca-9,12-dien-1-yl 4-bromobutanoate (2.7 g, 6.5 mmol), potassium carbonate (1.6 g, 11.2 mmol), and KI (31 mg, 0.2 mmol) were added respectively, and stirred at 85°C for 16 hours. An appropriate amount of water was added to the mixture, then EA (20 mL * 3) was added for extraction. The combined organic phases were dried and concentrated under reduced pressure. The crude product was purified by column chromatography with A:B=30% (A:PE/EA=5/1, B:DCM/MeOH(NH₃)=10/1) to obtain 1.5 g of a colorless oily product with a yield of 64.3 %.

ESI-MS *m*/*z:* cald for C₇₈H₁₄₅N₂O₇ Si[M+H]⁺: 1250.1, found 1250.3.

¹H NMR (400 MHz, CDCl₃) δ 5.45 - 5.26 (m, 12H), 4.04 (t, J = 6.8 Hz, 6H), 3.62 (t, J = 6.3 Hz, 2H), 2.77 (t, J = 6.5 Hz, 6H), 2.54 - 2.36 (m, 11H), 2.33 - 2.27 (m, 6H), 2.08 - 2.01 (m, 12H), 1.78 - 1.67 (m, 7H), 1.64 - 1.57 (m, 8H), 1.37 - 1.26 (m, 50H), 0.96 - 0.82 (m, 18H), 0.04 (s, 6H).

### Step 4: Synthesis of di((9Z,12Z)-octadeca-9,12-dien-1-yl)4,4'-((3-(3-hydroxypropyl)(4-(((6Z,9Z)-heptadec-6,9-dien-1-yloxy)-4-oxobutyl)amino)propyl)azanediyl)dibutanoate

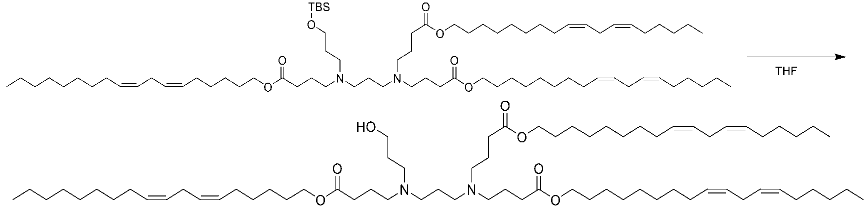

(9Z,12Z)-octadeca-9,12-dien-1-yl 2,2,3,3-tetramethyl-8-(4-(((6Z,9Z)-heptadec-6,9-dien-1-yl)oxy)-4-oxobutyl)-12-(4-((9Z,12Z)-octadeca-9,12-dien-1-yl)oxy)-4-oxobutyl)-4-oxo-8,12-diaza-3-silahexadecane-16-acid ester (3.0 g, 2.4 mmol) was dissolved in THF (20 mL), and then 1.0 M TBAF tetrahydrofuran solution (4.8 mL, 4.8 mmol) was added, and stirred at room temperature for 3 hours. An appropriate amount of water was added, extracted with EA (20 mL * 3), the organic phase was combined, dried and evaporated to dryness, the mixture was mixed and passed through the column, and eluted with A:B=10%-30% (A:PE/EA=5/1, B:DCM/MeOH(NH3)=10/1), to obtain 0.8 g of a colorless oily product with a yield of 27.9%.

ESI-MS *m*/*z:* cald for C₇₂H₁₃₁N₂O₇ [M+H]⁺: 1136.0, found 1136.2.

¹H NMR (400 MHz, CDCl₃) δ 5.46 - 5.25 (m, 12H), 4.12 - 3.99 (m, 6H), 3.78 (t, J = 5.2 Hz, 2H), 2.77 (t, J = 6.5 Hz, 6H), 2.64 (m, 2H), 2.53 - 2.35 (m, 10H), 2.31 (t, J = 7.4 Hz, 6H), 2.11 - 1.98 (m, 12H), 1.86 - 1.66 (m, 11H), 1.64 - 1.59 (m, 6H), 1.42 - 1.23 (m, 48H), 0.89 (t, J = 6.8 Hz, 9H).

### Example 109

### 4-(3-((2-hydroxyethyl)(4-oxo-4-(undecyloxy)butyl)amino)propyl)(4-oxido-4-(undecyloxy)butyl)amino)-4-oxobutanoate

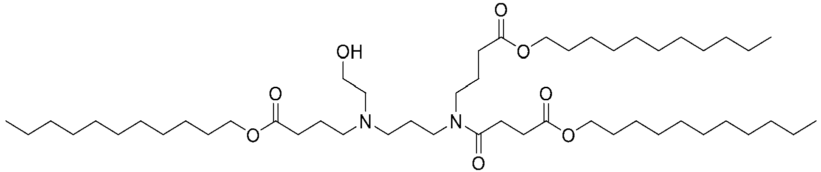

### Step 1: Synthesis of 4-oxo-4-(undecyloxy)butyric acid

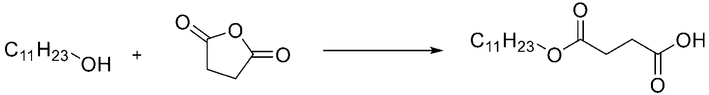

Dihydrofuran-2,5-dione (1.0 g, 10.0 mmol) and 1-undecanol (1.7 g, 10.0 mmol) were dissolved in DMF (10 mL), then DMAP (1.2 g, 10.0 mmol) was added, and stirred at room temperature for 16 hours. An appropriate amount of 1N hydrochloric acid was added, then DCM (30 mL * 3) was added for extraction. The combined organic phases were dried and concentrated under reduced pressure to obtain 2.7 g of a crude product with a yield of 99.2%.

¹H NMR (400 MHz, CDCl₃) δ 4.08 (t, J = 6.7 Hz, 2H), 2.70 - 2.64 (m, 2H), 2.57 - 2.63 (m, 2H), 1.67 - 1.55 (m, 2H), 1.33 - 1.22 (m, 16H), 0.87 (t, J = 6.8 Hz, 3H).

### Step 2: Synthesis of 3-((2-((tertbutyldimethylsilyl)oxy)ethyl)amino)propyl)tert-butyl carbamate

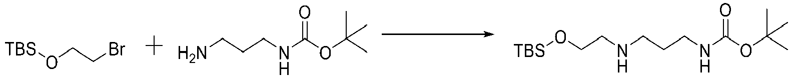

(2-bromoethoxy)(tert-butyl)dimethylsilane (4.8 g, 20.1 mmol) was dissolved in acetonitrile (100 mL), and (3-aminopropyl)tert-butyl carbamate (3.5 g, 20.1 mmol), and potassium carbonate (5.5 g, 40.2 mmol) were added respectively, and stirred at 80°C for 18 hours. An appropriate amount of water was added to the mixture, then DCM (30 mL * 3) was added for extraction. The combined organic phases were dried and concentrated under reduced pressure. The crude product was purified by column chromatography with DCM:MeOH=20:1 to obtain 2.8 g of a colorless oily product with a yield of 41.9 %.

ESI-MS *m*/*z:* cald for C₁₆H₃₇N₂O₃Si [M+H]⁺: 333.3, found 333.1.

¹H NMR (400 MHz, CDCl₃) δ 5.18 (br.s, 1H), 3.75 (t, J = 5.3 Hz, 2H), 3.22 (d, J = 6.5 Hz, 2H), 2.77 - 2.69 (m, 4H), 1.75 - 1.67 (m, 2H), 1.44 (s, 9H), 0.90 (s, 9H), 0.07 (s, 6H).

### Step 4: Synthesis of undecyl 4-((3-((tert-butoxyoxo)amino)propyl)(2-((tertbutyldimethylsilyl)oxy)ethyl)amino)butanoate

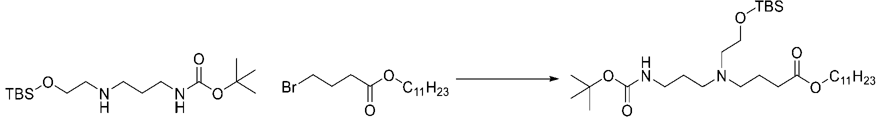

(3-((2-((tert-butyldimethylsilyl)oxy)ethyl)amino)propyl)tert-butyl carbamate (1.5 g, 4.5 mmol) was dissolved in acetonitrile (60 mL), and undecyl 4-bromobutanoate (2.2 g, 6.8 mmol), potassium carbonate (1.2 g, 9.0 mmol), and KI (83 mg, 0.5 mmol) were added respectively, and stirred at 85°C for 16 hours. An appropriate amount of water was added to the mixture, then DCM (20 mL * 3) was added for extraction. The combined organic phases were dried and concentrated under reduced pressure. The crude product was purified by column chromatography with DCM:MeOH=30:1 to obtain 2.1 g of a colorless oily product with a yield of 81.3 %.

ESI-MS *m*/*z:* cald for C₃₁H₆₅N₂O₅Si [M+H]⁺: 573.5, found 573.7.

¹H NMR (400 MHz, CDCl₃) δ 4.09 - 4.02 (m, 2H), 3.65 (t, J = 6.5 Hz, 2H), 3.17 (d, J = 6.3 Hz, 2H), 2.58 - 2.40 (m, 6H), 2.32 (t, J = 7.4 Hz, 2H), 1.81 - 1.69 (m, 2H), 1.68 - 1.54 (m, 6H), 1.43 (s, 9H), 1.26 (s, 15H), 0.92 - 0.86 (m, 12H), 0.05 (s, 6H).

### Step 4: Synthesis of undecyl 4-(3-aminopropyl)(2-((tertbutyldimethylsilyl)oxy)ethyl)amino)butanoate

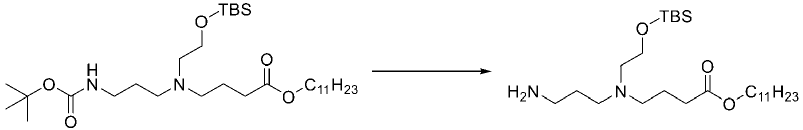

Undecyl 4-((3-((tert-butoxyoxo)amino)propyl)(2-((tertbutyldimethylsilyl)oxy)ethyl)amino)butanoate (1.5 g, 2.6 mmol) was dissolved in DCM (50 mL), TMSOTf (0.6 g, 2.6 mmol) was added, stirred at room temperature for 0.5 hours, saturated sodium bicarbonate solution was added dropwise under ice bath to neutralize, an appropriate amount of water was added, extracted with DCM (20 mL * 3), the organic phase was combined, dried and evaporated to dryness, the mixture was mixed and passed through the column, and eluted with DCM:MeOH(NH₃)=20:1, to obtain 1.1 g of a colorless oily product with a yield of 88.9%.

ESI-MS *m*/*z:* cald for C₂₆H₅₇N₂O₃ Si[M+H]⁺: 473.4, found 473.6.

¹H NMR (400 MHz, CDCl₃) δ 4.04 (t, J = 6.8 Hz, 2H), 3.64 (m, 2H), 2.73 (t, J = 6.7 Hz, 1H), 2.60 - 2.43 (m, 6H), 2.31 (t, J = 7.4 Hz, 2H), 1.95 (m, 2H) , 1.79 - 1.69 (m, 2H), 1.66 - 1.52 (m, 4H), 1.38 - 1.16 (m, 17H), 0.90 - 0.84 (m, 12H), 0.05 (s, 6H).

### Step 5: Synthesis of 2,2,3,3-tetramethyl-7-(4-oxo-4-(undecyloxy)butyl)-4-oxo-7,11-diaza-3-silapentatriacontane-15-undecyl ester

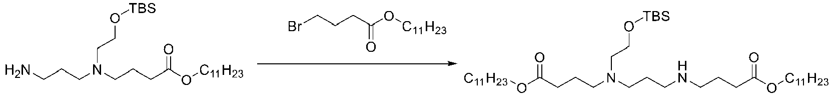

Undecyl 4-(3-Aminopropyl)(2-(tertbutyldimethylsilyl)oxy)ethyl)amino)butanoate (1.1 g, 2.3 mmol) was dissolved in acetonitrile (50 mL), and undecyl 4-bromobutanoate (0.8 g, 2.3 mmol), potassium carbonate (0.6 g, 4.6 mmol), and KI (10.6 mg, 0.06 mmol) were added respectively, and stirred at 85°C for 16 hours. An appropriate amount of water was added to the mixture, then DCM (30 mL * 3) was added for extraction. The combined organic phases were dried and concentrated under reduced pressure. The crude product was purified by column chromatography with DCM:MeOH=30:1 to obtain 800 mg of a colorless oily product with a yield of 48.2 %.

ESI-MS *m*/*z:* cald for C₄₁H₈₅N₂O₅Si [M+H]⁺: 713.6, found 713.4.

¹H NMR (400 MHz, CDCl₃) δ 4.07 - 4.01 (m, 4H), 3.69 - 3.60 (m, 2H), 2.72 - 2.63 (m, 3H), 2.61 - 2.44 (m, 6H), 2.39 - 2.27 (m, 4H), 1.88 - 1.71 (m, 3H), 1.69 - 1.53 (m, 6H), 1.41 - 1.18 (m, 35H), 0.90 - 0.85 (m, 15H) , 0.05 (s, 6H).

### Step 6: Synthesis of 2,2,3,3-tetramethyl-12-oxo-7,11-di(4-oxo-4-(undecyloxy)butyl)-4-oxo-7,11-diaza-3-silapentatriacontane-15-acid undecyl ester

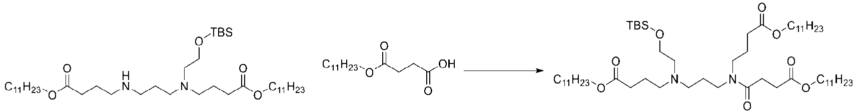

2,2,3,3-tetramethyl-7-(4-oxo-4-(undecyloxy)butyl)-4-oxo-7,11-diaza-3-silapentatriacontane-15-undecyl ester (300 mg, 0.4 mmol) was dissolved in DMF (20 mL), and 4-oxo-4-(undecyloxy)butyric acid (120 mg, 0.4 mmol), DIEA (163 mg, 1.3 mmol), and HATU (176 mg, 0.5 mmol) were added respectively, and stirred at room temperature for 2 hours. An appropriate amount of water was added to the mixture, then DCM (30 mL * 3) was added for extraction. The combined organic phases were dried and concentrated under reduced pressure. The crude product was purified by column chromatography with DCM:MeOH=30:1 to obtain 250 mg of a colorless oily product with a yield of 61.4 %.

ESI-MS *m*/*z:* cald for C₅₆H₁₁₁N₂O₈Si [M+H]⁺: 967.8, found 967.6.

¹H NMR (400 MHz, CDCl₃) δ 4.10 - 3.99 (m, 6H), 3.67 - 3.59 (m, 2H), 3.38 - 3.25 (m, 4H), 2.69 - 2.52 (m, 6H), 2.51 - 2.40 (m, 4H), 2.36 - 2.24 (m, 4H), 1.95 - 1.78 (m, 2H), 1.77 - 1.67 (m, 3H), 1.65 - 1.57 (m, 8H), 1.31 - 1.23 (m, 47H), 0.90 - 0.85 (m, 18H), 0.04 (s, 6H).

### Step 7: Synthesis of 4-(3-((2-hydroxyethyl)(4-oxo-4-(undecyloxy)butyl)amino)propyl)(4-oxido-4-(undecyloxy)butyl)amino)-4-oxobutanoate

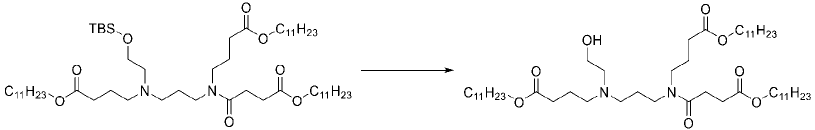

2,2,3,3-tetramethyl-12-oxo-7,11-di(4-oxo-4-(undecyloxy)butyl)-4-oxo-7,11-diaza-3-silapentatriacontane-15-acid undecyl ester (240 mg, 0.3 mmol) was dissolved in THF (5 mL), TBAF (97 mg, 0.4 mmol) was added, stirred at room temperature for 2 hours, saturated sodium bicarbonate solution was added dropwise under ice bath to neutralize, an appropriate amount of water was added, extracted with DCM (20 mL * 3), the organic phase was combined, dried and evaporated to dryness, the mixture was mixed and passed through the column, and eluted with DCM:MeOH(NH₃)=25:1, to obtain 150 mg of a colorless oily product with a yield of 70.9%.

ESI-MS *m*/*z:* cald for C₅₀H₉₇N₂O₈ [M+H]⁺: 853.7, found 853.5.

¹H NMR (400 MHz, CDCl₃) δ 4.13 - 3.98 (m, 6H), 3.60 - 3.49 (m, 2H), 3.40 - 3.28 (m, 4H), 2.72 - 2.52 (m, 6H), 2.52 - 2.41 (m, 4H), 2.39 - 2.25 (m, 4H), 2.01 - 1.91 (m, 1H), 1.91 - 1.85 (m, 1H), 1.85 - 1.76 (m, 3H), 1.75 - 1.58 (m, 8H), 1.32 - 1.24 (m, 48H), 0.88 (t, *J* = 6.7 Hz, 9H).

### Example 110

### Azetidinyl 4,4'-(4-((4-hydroxybutyl)(4-oxo-4-(undecyloxy)butyl)amino)butyl)dibutanoate

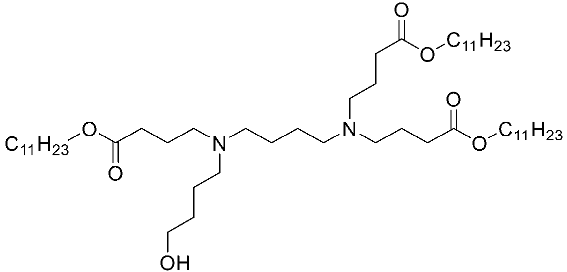

### Step 1: Synthesis of azetidinyl 4,4'-(4-((4-hydroxybutyl)(4-oxo-4-(undecyloxy)butyl)amino)butyl)dibutanoate

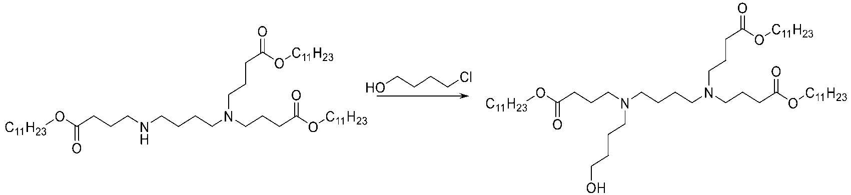

Dinonyl 4,4'-(3-((4-oxo-4-(undecyloxy)butyl)amino)propyl)azetidinyl)dibutanoate (350 mg, 0.4 mmol) was dissolved in DMF (8 mL), 1-chloro-4-butanol (98 mg, 0.9 mmol), potassium carbonate (179 mg, 1.3 mmol), and KI (21.6 mg, 0.1 mmol) were added respectively, and stirred at 85°C for 16 hours. Then DCM (50 mL * 3) was added for extraction. The combined organic phases were dried and concentrated under reduced pressure. The crude product was purified by column chromatography with DCM (containing 5% EA):MeOH=50:1 to 5:1 to obtain 142 mg of a colorless oily product with a yield of 40.3 %.

ESI-MS *m*/*z:* cald for C₅₃H₁₀₅N₂O₇ [M+H]+: 881.8, found 881.6.

¹H NMR (400 MHz, CDCl₃) δ 4.12 - 3.98 (m, 6H), 3.55 (t, J = 4.8 Hz, 2H), 2.49 - 2.36 (m, 12H), 2.31 (t, J = 7.4 Hz, 6H), 1.88 - 1.80 (m, 2H), 1.80 - 1.72 (m, 5H), 1.72 - 1.57 (m, 12H), 1.54 - 1.45 (m, 2H), 1.45 - 1.23 (m, 47H), 0.88 (t, *J =* 6.7 Hz, 9H).

### Example 111

### Di((9Z,12Z)-octadeca-9,12-dien-1-yl)5,5'-((3-((3-hydroxypropyl)(5-((9Z,12Z)-octadeca-9,12-dien-1-yl)oxy)-5-oxopentyl)amino)propyl)azanediyl)dipentanoatepentanoate

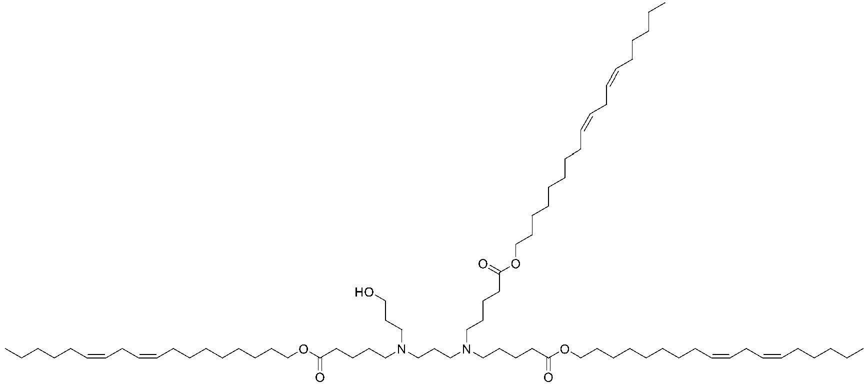

### Step 1: Synthesis of (9Z,12Z)-octadeca-9,12-dien-1-yl 5-bromopentanoate:

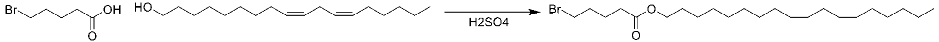

(9Z,12Z)-octadeca-9,12-dien-1-ol (5.0 g, 18.8 mmol) was mixed with 5-bromovaleric acid (3.7 g, 20.6 mmol). The mixture was heated to 60 °C, 2 drops of concentrated sulfuric acid were added, and stirred at this temperature for 16 hours. TLC showed basically complete consumption of raw materials, an appropriate amount of water was added, extracted with EA (30 mL * 3), and the organic phase was combined. After evaporating to dryness, the mixture was mixed, passed through a column, and eluted with PE:EA=30:1 to obtain 7.2 g of a colorless oily product with a yield of 89.3%.

¹H NMR (400 MHz, CDCl₃) δ 5.43 - 5.28 (m, 4H), 4.06 (t, J = 6.7 Hz, 2H), 3.41 (t, J = 6.6 Hz, 2H), 2.77 (t, J = 6.4 Hz, 2H), 2.34 (t, J = 7.2 Hz, 2H), 2.10 - 1.99 (m, 4H), 1.95 - 1.85 (m, 2H), 1.84 - 1.73 (m, 2H), 1.67 - 1.58 (m, 2H), 1.37 - 1.26 (m, 16H), 0.89 (t, J = 6.7 Hz, 3H).

### Step 2: Synthesis of (9Z,12Z)-octadeca-9,12-dien-1-yl 2,2,3,3-tetramethyl-8-(5-(((6Z,9Z)-octa-6,9-dien-1-yl)oxy)-5-oxopentyl)-12-(5-((((9Z,12Z)-heptadec-9,12-dien-1-yl(oxy))-5-oxopentyl)-4-oxo-8,12-diaza-3-silaheptadecane-17-acid ester:

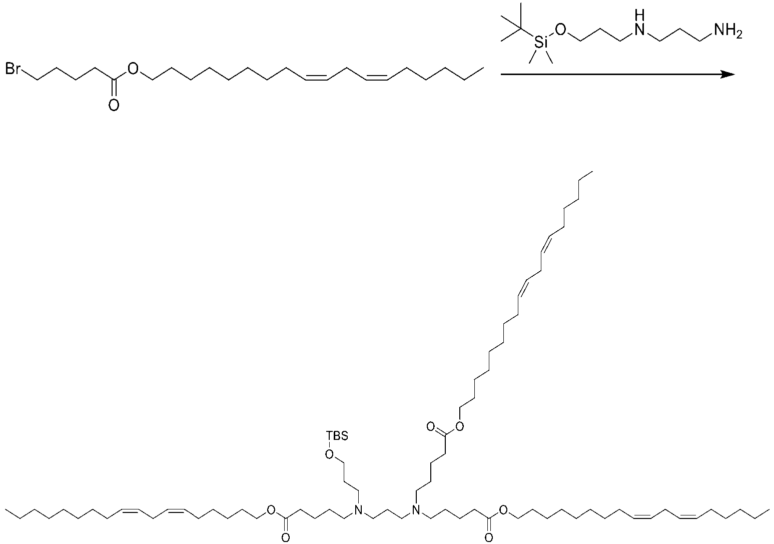

N1-(3-((tert-butyldimethylsilyl)oxy)propyl)propane-1,3-diamine (100 mg, 0.4 mmol) was dissolved in MeCN (15 mL), and (9Z,12Z)-octadeca-9,12-dien-1-yl 5-bromopentanoate (575 mg, 1.3 mmol), potassium carbonate (280 mg, 2.0 mmol), and KI (10 mg, 0.04 mmol) were added respectively, and stirred at 85°C for 16 hours. An appropriate amount of water was added to the mixture, then EA (20 mL * 3) was added for extraction. The combined organic phases were dried and concentrated under reduced pressure. The crude product was purified by column chromatography with A:B=30% (A:PE/EA=5/1, B:DCM/MeOH(NH₃)=10/1) to obtain 400 mg of a colorless oily product with a yield of 76.3 %.

ESI-MS *m*/*z:* cald for C₈₁H₁₅₁N₂O₇ Si[M+H]⁺: 1292.1, found 1292.3.

¹H NMR (400 MHz, CDCl₃) δ 5.48 - 5.23 (m, 12H), 4.05 (t, J = 6.8 Hz, 6H), 3.62 (t, J = 6.4 Hz, 2H), 2.77 (t, J = 6.5 Hz, 6H), 2.50 - 2.34 (m, 11H), 2.30 (t, J = 7.5 Hz, 6H), 2.10 - 1.98 (m, 12H), 1.70 - 1.53 (m, 18H), 1.49 - 1.40 (m, 6H), 1.37 - 1.26 (m, 47H), 0.93 - 0.83 (m, 18H), 0.04 (s, 6H).

### Step 3: Synthesis of di((9Z,12Z)-octadeca-9,12-dien-1-yl)5,5'-((3-((3-hydroxypropyl)(5-((9Z,12Z)-octadeca-9,12-dien-1-yl)oxy)-5-oxopentyl)amino)propyl)azanediyl)dipentanoate

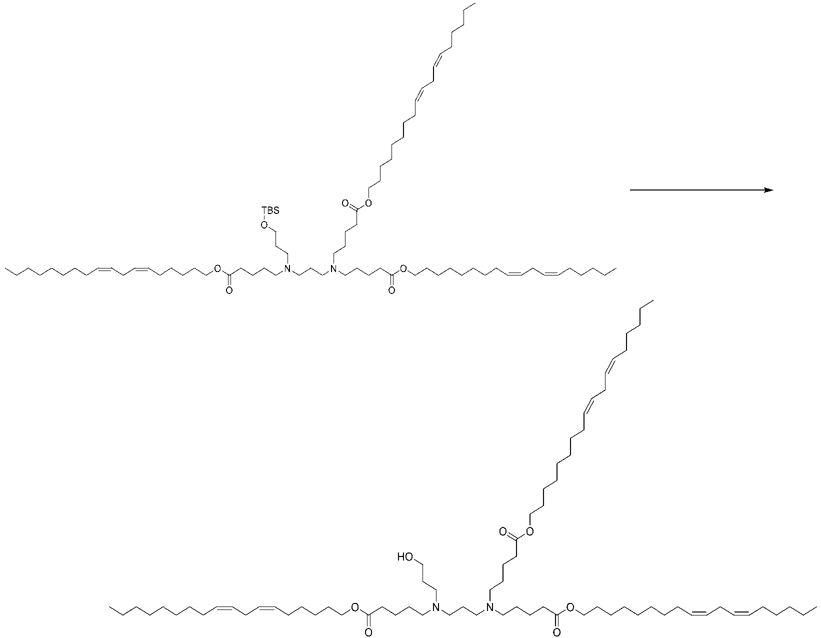

(9Z,12Z)-octadeca-9,12-dien-1-yl 2,2,3,3-tetramethyl-8-(5-(((6Z,9Z)-octa-6,9-dien-1-yl)oxy)-5-oxopentyl)-12-(5-(((9Z,12Z)-heptadec-9,12-dien-1-yl)oxy))-5-oxopentyl)-4-oxo-8,12-diaza-3-silaheptadecane-17-acid ester (400 mg, 0.3 mmol) was dissolved in THF (10 mL), then TBAF (196 mg, 0.6 mmol) was added, and stirred at room temperature for 2 hours. An appropriate amount of water was added, extracted with EA (20 mL * 3), the organic phase was combined, dried and evaporated to dryness, the mixture was mixed and passed through the column, and eluted with A:B=10%-30% (A:PE/EA=5/1, B:DCM/MeOH(NH3)=10/1), to obtain 200 mg of a colorless oily product with a yield of 54.9 %.

ESI-MS *m*/*z:* cald for C₇₅H₁₃₇N₂O₇ [M+H]⁺: 1178.0, found 1178.2.

¹H NMR (400 MHz, CDCl₃) δ 5.45 - 5.26 (m, 12H), 4.05 (t, J = 6.8 Hz, 6H), 3.77 (t, J = 5.2 Hz, 2H), 2.77 (t, J = 6.5 Hz, 6H), 2.63 (t, J = 5.7 Hz, 2H), 2.50 - 2.35 (m, 10H), 2.34 - 2.28 (m, 6H), 2.10 - 1.99 (m, 13H), 1.75 - 1.69 (m, 2H), 1.69 - 1.59 (m, 16H), 1.59 - 1.44 (m, 7H), 1.37 - 1.29 (m, 45H), 0.89 (t, J = 6.7 Hz, 9H).

### Example 112

### 4,4'-didodecyl((3-((4-(dodecoxy)-4-oxobutyl)(3-hydroxypropyl)amino)propyl)azetidinyl)dibutanoate

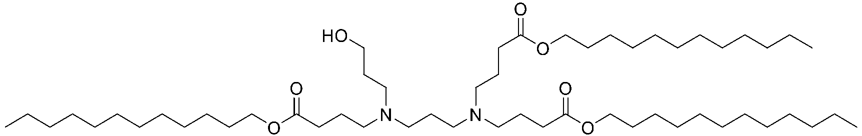

### Step 1: Synthesis of 2,2,3,3-tetramethyl-8,12-di(5-oxo-5-(undecyloxy)pentyl)-4-oxo-8,12-diaza-3-silaheptadecane-17-undecyl ester

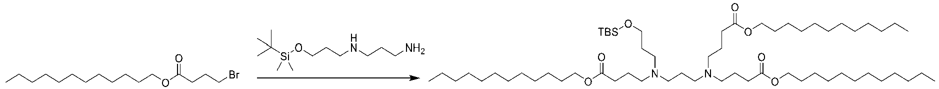

N1-(3-((tert-butyldimethylsilyl)oxy)propyl)propane-1,3-diamine (100 mg, 0.4 mmol) was dissolved in MeCN (15 mL), and dodecyl 4-bromobutanoate (449 mg, 1.3 mmol), potassium carbonate (280 mg, 2.0 mmol), and KI (10 mg, 0.04 mmol) were added respectively, and stirred at 85°C for 16 hours. An appropriate amount of water was added to the mixture, then EA (20 mL * 3) was added for extraction. The combined organic phases were dried and concentrated under reduced pressure. The crude product was purified by column chromatography with A:B=30% (A:PE/EA=5/1, B:DCM/MeOH(NH3)=10/1) to obtain 300 mg of a colorless oily product with a yield of 73.2 %.

ESI-MS *m*/*z:* cald for C₆₀H₁₂₁N₂O₇ Si[M+H]⁺: 1009.9, found 1009.7.

¹H NMR (400 MHz, CDCl₃) δ 4.04 (t, J = 6.8 Hz, 6H), 3.62 (t, J = 6.3 Hz, 2H), 2.54 - 2.47 (m, 2H), 2.47 - 2.35 (m, 10H), 2.34 - 2.25 (m, 6H), 1.80 - 1.67 (m, 10H), 1.70 - 1.60 (m, 9H), 1.60 - 1.51 (m, 2H), 1.30 - 1.24 (m, 49H), 0.89 - 0.85 (m, 18H), 0.04 (s, 6H).

### Step 2: Synthesis of 4,4'-didodecyl((3-((4-(dodecoxy)-4-oxobutyl)(3-hydroxypropyl)amino)propyl)azetidinyl)dibutanoate

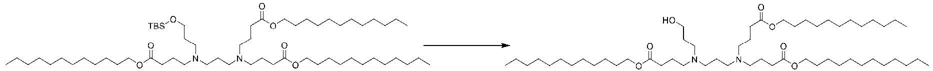

2,2,3,3-tetramethyl-8,12-di(5-oxo-5-(undecyloxy)pentyl)-4-oxo-8,12-diaza-3-silaheptadecane-17-undecyl ester (300 mg, 0.3 mmol) was dissolved in THF (10 mL), then TBAF (155 mg, 0.6 mmol) was added, and stirred at room temperature for 2 hours. An appropriate amount of water was added, extracted with EA (20 mL * 3), the organic phase was combined, dried and evaporated to dryness, the mixture was mixed and passed through the column, and eluted with A:B=10%-30% (A:PE/EA=5/1, B:DCM/MeOH(NH₃)=10/1), to obtain 210 mg of a colorless oily product with a yield of 78.9%.

ESI-MS *m*/*z:* cald for C₅₄H₁₀₇N₂O₇ [M+H]⁺: 895.8, found 895.6.

¹H NMR (400 MHz, CDCl₃) δ 4.05 (t, J = 6.8, Hz, 6H), 3.78 (t, J = 5.2 Hz, 2H), 2.64 (t, J = 5.7 Hz, 2H), 2.50 - 2.36 (m, 10H), 2.34 - 2.27 (m, 6H), 1.88 - 1.80 (m, 2H), 1.80 - 1.69 (m, 8H), 1.69 - 1.56 (m, 9H), 1.32 - 1.24 (m, 52H), 0.88 (t, J = 6.7 Hz, 9H).

### Example 113

### Didecyl 4,4'-((3-((4-(decyloxy)-4-oxobutyl)(3-hydroxypropyl)amino)propyl)azetidinyl)dibutanoate

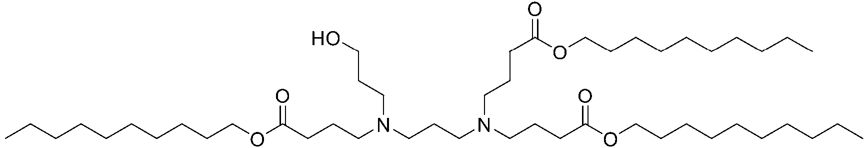

### Step 1: Synthesis of 1,12-di(4-(decyloxy)-4-oxobutyl)-2,2,3,3-tetramethyl-4-oxo-8,12-diaza-3-silahexadecane-16-caprate

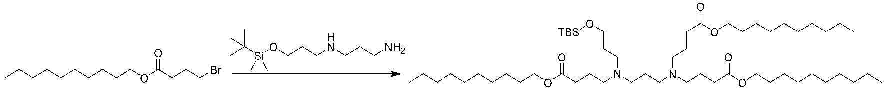

N1-(3-((tert-butyldimethylsilyl)oxy)propyl)propane-1,3-diamine (100 mg, 0.4 mmol) was dissolved in MeCN (15 mL), and dodecyl 4-bromobutanoate (374 mg, 1.2 mmol), potassium carbonate (280 mg, 2.0 mmol), and KI (10 mg, 0.04 mmol) were added respectively, and stirred at 85°C for 16 hours. An appropriate amount of water was added to the mixture, then EA (20 mL * 3) was added for extraction. The combined organic phases were dried and concentrated under reduced pressure. The crude product was purified by column chromatography with A:B=30% (A:PE/EA=5/1, B:DCM/MeOH(NH₃)=10/1) to obtain 295 mg of a colorless oily product with a yield of 78.6 %.

ESI-MS *m*/*z:* cald for C₅₄H₁₀₉N₂O₇ Si[M+H]⁺: 925.8, found 925.6.

¹H NMR (400 MHz, CDCl₃) δ 4.04 (t, J = 6.8 Hz, 6H), 3.62 (t, J = 6.3 Hz, 2H), 2.56 - 2.48 (m, 2H), 2.48 - 2.34 (m, 10H), 2.33 - 2.26 (m, 6H), 1.85 - 1.70 (m, 9H), 1.70 - 1.60 (m, 9H), 1.60 - 1.50 (m, 2H), 1.33 - 1.25 (m, 38H), 1.05 - 0.68 (m, 18H), 0.04 (s, 6H).

### Step 2: Synthesis of didecyl 4,4'-((3-((4-(decyloxy)-4-oxobutyl)(3-hydroxypropyl)amino)propyl)azetidinyl)dibutanoate

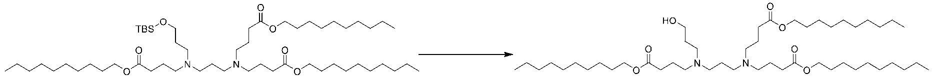

8,12-di(4-(decyloxy)-4-oxobutyl)-2,2,3,3-tetramethyl-4-oxo-8,12-diaza-3-silahexadecane-16-caprate (295 mg, 0.3 mmol) was dissolved in THF (10 mL), then TBAF (167 mg, 0.6 mmol) was added, and stirred at room temperature for 2 hours. An appropriate amount of water was added, extracted with EA (20 mL * 3), the organic phase was combined, dried and evaporated to dryness, the mixture was mixed and passed through the column, and eluted with A:B=10%-30% (A:PE/EA=5/1, B:DCM/MeOH(NH₃)=10/1), to obtain 190 mg of a colorless oily product with a yield of 73.5%.

ESI-MS *m*/*z:* cald for C₄₈H₉₅N₂O₇ [M+H]⁺: 811.7, found 811.5.

¹H NMR (400 MHz, CDCl₃) δ 4.05 (t, J = 6.8, Hz, 6H), 3.78 (t, J = 5.2 Hz, 2H), 2.64 (t, J = 5.7 Hz, 2H), 2.53 - 2.36 (m, 10H), 2.35 - 2.26 (m, 6H), 1.84 - 1.67 (m, 9H), 1.64 - 1.56 (m, 8H), 1.33 - 1.24 (m, 42H), 0.90 - 0.84 (m, 9H).

### Example 114

### Di((9Z,12Z)-octadeca-9,12-dien-1-yl)7,7'-((3-(3-hydroxypropyl)(7-(((9Z,12Z)-octadeca-9,12-dien-1-yl)oxy)-7-oxoheptyl)amino)propyl)azepan)diheptanoate

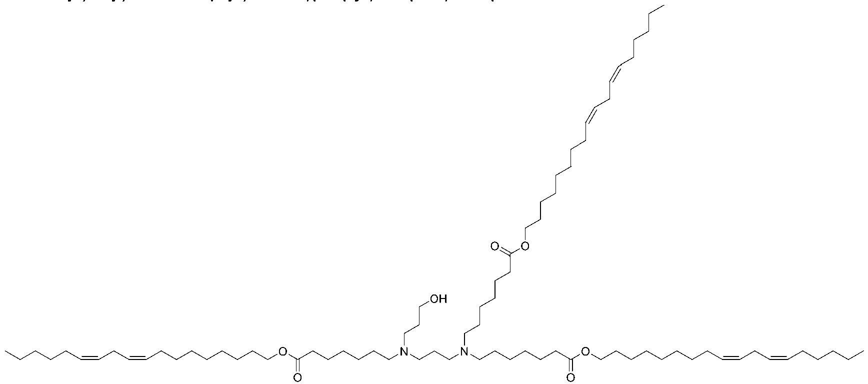

### Step 1: Synthesis of (9Z,12Z)-octadeca-9,12-dien-1-yl 7-bromoheptanoate:

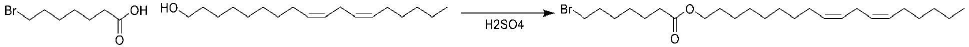

(9Z,12Z)-octadeca-9,12-dien-1-ol (2.0 g, 7.5 mmol) was mixed with 7-bromoheptanoic acid (1.7 g, 8.3 mmol). The mixture was heated to 60 °C, 2 drops of concentrated sulfuric acid were added, and stirred at this temperature for 16 hours. TLC showed basically complete consumption of raw materials, an appropriate amount of water was added, extracted with EA (30 mL * 3), and the organic phase was combined. After evaporating to dryness, the mixture was mixed, passed through a column, and eluted with PE:EA=30:1 to obtain 3.2 g of a colorless oily product with a yield of 93.2%.

¹H NMR (400 MHz, CDCl₃) δ 5.45 - 5.27 (m, 4H), 4.06 (t, J = 6.8 Hz, 2H), 3.40 (t, J = 6.8 Hz, 2H), 2.77 (t, J = 6.6 Hz, 2H), 2.30 (t, J = 7.5 Hz, 2H), 2.10 - 1.99 (m, 4H), 1.91 - 1.80 (m, 2H), 1.69 - 1.58 (m, 4H), 1.50 - 1.41 (m, 2H), 1.38 - 1.28 (m, 18H), 0.89 (t, J = 6.8 Hz, 3H).

### Step 2: Synthesis of (9Z,12Z)-octadeca-9,12-dien-1-yl 2,2,3,3-tetramethyl-8,12-di(7-(((9Z,12Z)octa-9,112-dien-1-yl)oxy)-7-oxoheptyl)-4-oxo-8,12-aza-3-silanone-19-acid ester

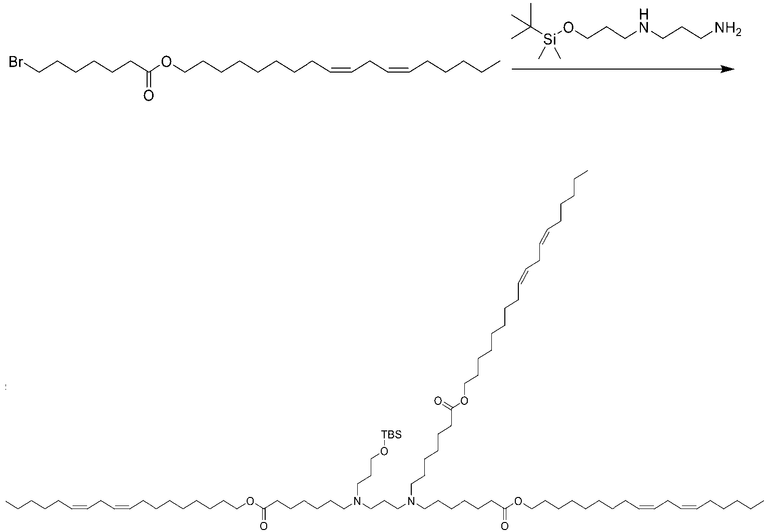

N1-(3-((tert-butyldimethylsilyl)oxy)propyl)propane-1,3-diamine (100 mg, 0.4 mmol) was dissolved in MeCN (30 mL), and (9Z,12Z)-octadeca-9,12-dien-1-yl 7-bromoheptanoate (650 mg, 1.4 mmol), potassium carbonate (280 mg, 2.0 mmol), and KI (10 mg, 0.04 mmol) were added respectively, and stirred at 85°C for 16 hours. An appropriate amount of water was added to the mixture, then EA (20 mL * 3) was added for extraction. The combined organic phases were dried and concentrated under reduced pressure. The crude product was purified by column chromatography with A:B=30% (A:PE/EA=5/1, B:DCM/MeOH(NH₃)=10/1) to obtain 300 mg of a colorless oily product with a yield of 53.7 %.

ESI-MS *m*/*z:* cald for C₈₇H₁₆₃N₂O₇ Si[M+H]⁺: 1376.2, found 1376.4.

¹H NMR (400 MHz, CDCl₃) δ 5.46 - 5.32 (m, 12H), 4.08 (t, J = 6.8 Hz, 6H), 3.66 (t, J = 6.3 Hz, 2H), 2.81 (t, J = 6.5 Hz, 6H), 2.51 (m, 2H), 2.41 (m, 9H), 2.32 (t, J = 7.5 Hz, 6H), 2.18 - 1.98 (m, 12H), 1.73 - 1.55 (m, 21H), 1.52 - 1.31 (m, 62H), 1.03 - 0.77 (m, 18H), 0.08 (s, 6H).

### Step 3: Synthesis of Di((9Z,12Z)-octadeca-9,12-dien-1-yl)7,7'-((3-(3-hydroxypropyl)(7-(((9Z,12Z)-octadeca-9,12-dien-1-yl)oxy)-7-oxoheptyl)amino)propyl)azepan)diheptanoate

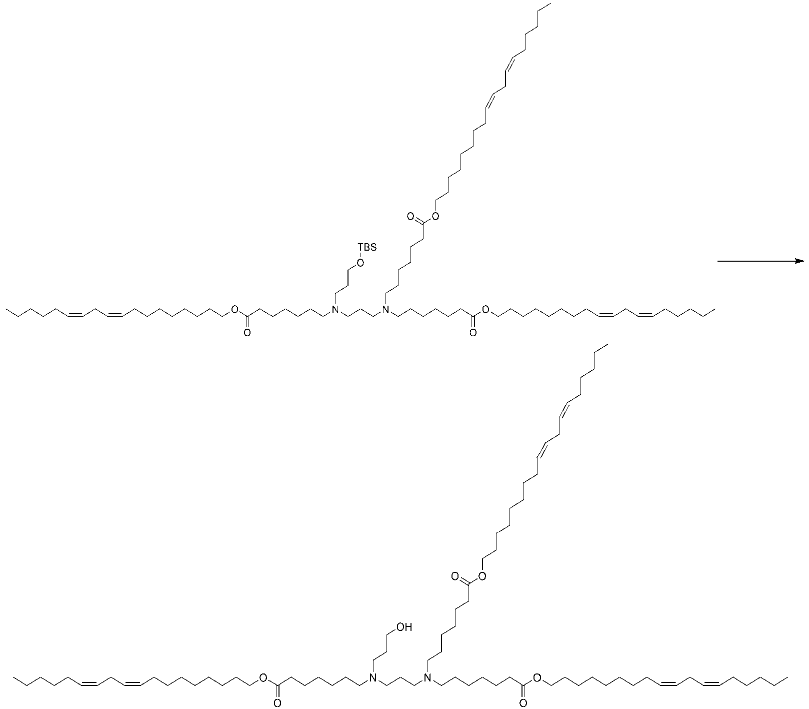

(9Z,12Z)-octadeca-9,12-dien-1-yl 2,2,3,3-tetramethyl-8,12-di(7-(((9Z,12Z)-octa-9,112-dien-1-yl)oxy)-7-oxoheptyl)-4-oxo-8,12-aza-3-silanone-19-acid ester (300 mg, 0.2 mmol) was dissolved in THF (10 mL), then TBAF (114 mg, 0.4 mmol) was added, and stirred at room temperature for 3 hours. An appropriate amount of water was added, extracted with EA (20 mL * 3), the organic phase was combined, dried and evaporated to dryness, the mixture was mixed and passed through the column, and eluted with A:B=10%-30% (A:PE/EA=5/1, B:DCM/MeOH(NH₃)=10/1), to obtain 132 mg of a colorless oily product with a yield of 48.0%.

ESI-MS *m*/*z:* cald for C₈₁H₁₄₉N₂O₇ [M+H]⁺: 1262.1, found 1262.3.

¹H NMR (400 MHz, CDCl₃) δ 5.48 - 5.23 (m, 12H), 4.05 (t, J = 6.8 Hz, 6H), 3.78 (t, J = 5.1 Hz, 2H), 2.77 (t, *i* = 6.5 Hz, 6H), 2.63 (t, J = 5.5 Hz, 2H), 2.48 - 2.34 (m, 10H), 2.29 (t, J = 7.5 Hz, 7H), 2.13 - 1.97 (m, 14H), 1.72 - 1.54 (m, 20H), 1.50 - 1.40 (m, 6H), 1.34 - 1.27 (m, 54H), 0.89 (t, J = 6.7 Hz, 9H).

### Example II : Preparation and detection of a lipid nanoparticle (LNP preparation)

The ionizable lipid compound and positive control 1 from Example I were dissolved in ethanol at a molar ratio of 50:10:48.25:1.25 with DOPE, cholesterol, and DMG-PEG2000 (all purchased from A.V.T (Shanghai) Pharmaceutical Co., Ltd.) to prepare an ethanol lipid solution. The enhanced green fluorescent protein (eGFP) mRNA was diluted in 20 mM citrate buffer (pH=6.1) to obtain an mRNA aqueous solution. By using a microfluidic device (Micro&Nano Biologics Co., Ltd., model: INano ^{™} L) mix ethanol lipid solution and mRNA aqueous solution in a volume ratio of 1:3 to prepare a lipid nanoparticle with a total lipid to mRNA N/P ratio of 12. The medium was replaced by ultrafiltration, ethanol was removed and volumed with DPBS. Finally, the lipid nanoparticle was filtered through a 0.2 µm sterile filter to obtain an LNP preparation encapsulating eGFP mRNA using ionizable lipid/DOPE/cholesterol/DMG-PEG2000 (50/10/48.25/1.25 mol%), with an mRNA content of 0.002 mg/ml-0.5 mg/mL.

Using Malvern Zetasizer Nano ZS (Malvern UK), the size and polydispersity index (PDI) of the lipid nanoparticle were determined by dynamic light scattering in 173° backscatter detection mode, and the encapsulation efficiency was measured by Ribogreen method. The test results were shown in Table 1.

### Example III: In vitro transfection experiment of a lipid nanoparticle (LNP preparation) into cells

This example used mouse bone marrow-derived dendritic cells DC2.4 (purchased from Millipore) as the validation cell line for a lipid nanoparticle transfection experiment. DC2.4 cells adhered to the wall for growth. All operational steps of cell culture strictly followed the prescribed procedures, using sterile reagents for aseptic operations under a sterile workbench. The main operational steps involved were as follows:
1) Cell recovery: the preserved cell cryopreservation solution was removed from the -80 °C freezer and immediately placed in a 37 °C water bath for rapid and continuous shaking, allowing it to completely melt within one minute. Fresh culture medium was added to a 15 mL centrifuge tube in advance, and the cell fluid was added to the centrifuge tube in a biosafety cabinet. A centrifuge tube of the same mass was taken, placed symmetrically in the centrifuge, and centrifuged at low speed for 5 minutes at 1200 rpm. After centrifugation, the upper clear liquid was removed, and an appropriate amount of culture medium was slowly added along the tube wall. After the cells were gently blown and dispersed evenly, the cell suspension was drawn and added to the prepared culture bottle. The state and distribution of cells in the early stage of recovery were observed under a microscope. The culture bottle was incubated in a 5% CO₂ incubator at 37 °C, and the recovery time was recorded.
2) Cell passaging: Under a microscope, if the cell density in the field of view reached 80%-90%, cells could be passaged and cultured. Adherent cells required trypsin digestion. In order to avoid residual serum in the culture medium reducing trypsin activity, the original culture medium was removed, and DPBS solution was added to wash once. After suctioning out the DPBS solution, trypsin was added to degrade the protein at the intercellular junction, thereby separating the cells and facilitating further cultivation. The mixture was gently shaken well and placed into the incubator for digestion for 2-3 minutes. After digestion was complete, an appropriate amount of culture medium was immediately added to the original culture dish to terminate digestion. Finally, a pipette was used to carefully blow off the cells and blow them until they were evenly dispersed, and then the cells were inoculated and subcultured in a certain proportion according to experimental requirements. Suspended cells did not require trypsin digestion steps.

Transfection of DC2.4 cells
1) DC2.4 cell recovery: the frozen DC2.4 cells were taken out from the -80 °C refrigerator and immediately put into a 37 °C water bath for rapid thawing. After melting, they were added to the preheated culture medium, and centrifuged at 1200 rpm for 5 minutes. The supernatant was discarded, and an appropriate amount of DMEM medium containing 10% FBS was added to resuspend the recovered DC2.4 cells, which were placed in a 37 °C, 5% CO₂ incubator for activation culture.
2) According to experimental requirements, DC2.4 cells were transfected into 24 well plates one day in advance. By counting cells, the number and viability of cells were recorded, and a cell suspension with 2 × 10⁵ cells per well were taken. Then, a certain volume of DMEM medium containing 10% FBS was added and mixed evenly, wherein 1 mL was added per well for subsequent transfection experiments.
3) After 24 hours, the culture medium was gently aspirated with a pipette 30 minutes in advance, and each well was washed with 1 mL of DPBS and aspirated. Then, 300 µL of serum-free Opti-MEM medium was added to each well for subsequent transfection experiments.
4) The LNP preparation prepared in Example II was diluted with Opti-MEM medium to a mRNA concentration of 1 ug/ml.
5) 200 ul of diluted mixture was added evenly above each well, which contains 200 ng of mRNA per well. The mixture was placed in a 37 °C, 5% CO₂ incubator for activation cultivation.
6) After 24 hours, cell viability (%), eGFP positivity rate (%), and mean fluorescence intensity data were obtained through cell fluorescence imaging and flow cytometry.

### Experimental results

Table 1 showed the characteristics of the lipid nanoparticle prepared in Example II , as well as the expression level of an eGFP-mRNA on DC2.4 after 24 hours, normalized based on Example 11.

**Table 1**

| Sample number | Particl e size | PDI | Encapsulation rate, % | Cell viability, % | Positive rate, % | Mean fluorescenc e intensity |
|---|---|---|---|---|---|---|
| Positive control 1 | 114.7 4 | 0.148 | 89.81 | 95.55 | 59.22 | 0.26 |
| Example 1 | 59.34 | 0.18 | 96.32 | 92.0 | 91.23 | 0.78 |
| Example 2 | 192.8 0 | 0.24 | 97.27 | 82.9 | 73.48 | 0.22 |
| Example 3 | 92.13 | 0.12 | 95.96 | 68.6 | 93.43 | 0.12 |
| Example 4 | 189.2 3 | 0.25 | 60.89 | 75.9 | 78.26 | 0.24 |
| Example 5 | 111.4 3 | 0.16 | 94.14 | 86.6 | 83.41 | 0.13 |
| Example 6 | 114.6 6 | 0.13 | 92.30 | 80.5 | 85.29 | 0.17 |
| Example 7 | 167.8 2 | 0.09 | 86.64 | 76.9 | 75.68 | 0.16 |
| Example 8 | 153.9 7 | 0.21 | 80.59 | 63.2 | 82.12 | 0.18 |
| Example 9 | 178.6 1 | 0.22 | 78.69 | 55.6 | 83.84 | 0.14 |
| Example 10 | 90.73 | 0.14 | 89.02 | 95.0 | 95.39 | 0.18 |
| Example 11 | 97.96 | 0.17 | 90.99 | 84.3 | 95.68 | 1 |
| Example 12 | 88.51 | 0.22 | 90.54 | 81.2 | 97.78 | 0.7 |
| Example 13 | 101.5 1 | 0.21 | 96.34 | 91.3 | 93.23 | 0.46 |
| Example 14 | 98.45 | 0.21 | 97.23 | 97.9 | 95.61 | 0.6 |
| Example 15 | 103.0 2 | 0.20 | 90.28 | 84.4 | 91.39 | 0.57 |
| Example 16 | 99.65 | 0.18 | 86.73 | 84.6 | 94.18 | 0.46 |
| Example 17 | 115.8 5 | 0.26 | 90.04 | 71.1 | 95.88 | 0.34 |
| Example 18 | 133.6 3 | 0.25 | 88.82 | 90.6 | 96.79 | 0.39 |
| Example 19 | 105.8 7 | 0.26 | 92.54 | 95.6 | 97.44 | 0.51 |
| Example 20 | 89.98 | 0.18 | 79.02 | 83.8 | 93.27 | 0.78 |
| Example 22 | 120.3 0 | 0.11 | 78.46 | 95.1 | 98.21 | 0.75 |
| Example 23 | 124.0 4 | 0.26 | 92.58 | 87.5 | 92.1 | 0.69 |
| Example 24 | 182.6 6 | 0.15 | 97.73 | 76.0 | 92.27 | 0.95 |
| Example 25 | 105.8 7 | 0.17 | 98.27 | 76.6 | 94.29 | 0.86 |
| Example 26 | 95.80 | 0.09 | 99.66 | 98.1 | 95.84 | 0.8 |
| Example 27 | 88.43 | 0.12 | 99.65 | 98.2 | 97.36 | 0.83 |
| Example 28 | 101.6 1 | 0.17 | 99.47 | 92.9 | 97.51 | 0.82 |
| Example 29 | 103.6 1 | 0.16 | 99.55 | 96.7 | 95.33 | 0.8 |
| Example 30 | 102.4 6 | 0.09 | 99.66 | 96.9 | 96.41 | 0.79 |
| Example 31 | 84.05 | 0.15 | 99.36 | 94.4 | 97.43 | 0.69 |
| Example 32 | 83.34 | 0.13 | 99.30 | 96.6 | 95.28 | 0.88 |
| Example 33 | 102.0 0 | 0.13 | 98.96 | 96.4 | 95.18 | 0.67 |
| Example 34 | 82.74 | 0.16 | 99.39 | 95.0 | 93.19 | 0.78 |
| Example 35 | 92.18 | 0.12 | 99.50 | 97.2 | 98.59 | 0.69 |
| Example 36 | 89.30 | 0.16 | 99.39 | 94.2 | 93.6 | 0.84 |
| Example 37 | 77.57 | 0.15 | 99.26 | 96.6 | 89.37 | 0.86 |
| Example 38 | 65.19 | 0.10 | 99.04 | 94.1 | 90.28 | 0.46 |
| Example 39 | 89.31 | 0.13 | 97.58 | 96.9 | 91.26 | 0.34 |
| Example 40 | 63.23 | 0.14 | 99.29 | 97.7 | 99.14 | 0.49 |
| Example 41 | 71.17 | 0.13 | 96.67 | 97.2 | 96.42 | 0.52 |
| Example 42 | 98.73 | 0.11 | 98.13 | 94.1 | 96.69 | 0.61 |
| Example 43 | 129.7 7 | 0.14 | 87.08 | 88.2 | 97.48 | 0.73 |
| Example 44 | 102.7 4 | 0.20 | 99.38 | 91.9 | 94.2 | 0.49 |
| Example 45 | 84.66 | 0.20 | 99.60 | 95.0 | 95.29 | 0.31 |
| Example 46 | 72.75 | 0.22 | 99.86 | 97.4 | 96.64 | 0.39 |
| Example 47 | 83.85 | 0.26 | 99.77 | 88.5 | 97.39 | 0.42 |
| Example 48 | 76.18 | 0.16 | 99.52 | 94.0 | 97.26 | 0.48 |
| Example 49 | 74.58 | 0.15 | 99.79 | 91.6 | 97.38 | 0.64 |
| Example 50 | 76.74 | 0.20 | 99.63 | 90.4 | 99.74 | 0.5 |
| Example 51 | 75.07 | 0.18 | 99.28 | 92.0 | 99.64 | 0.74 |
| Example 52 | 69.63 | 0.17 | 99.18 | 82.9 | 85.37 | 0.63 |
| Example 53 | 91.34 | 0.09 | 97.67 | 68.6 | 86.07 | 0.36 |
| Example 54 | 72.45 | 0.14 | 98.91 | 75.9 | 93.13 | 0.51 |
| Example 55 | 71.95 | 0.10 | 98.32 | 86.6 | 92.64 | 0.87 |
| Example 56 | 64.31 | 0.14 | 99.68 | 80.5 | 93.64 | 0.27 |
| Example 57 | 68.32 | 0.17 | 99.36 | 76.9 | 97.36 | 0.19 |
| Example 58 | 79.13 | 0.16 | 99.17 | 63.2 | 87.44 | 0.39 |
| Example 59 | 104.1 2 | 0.08 | 91.15 | 55.6 | 96.89 | 0.34 |
| Example 60 | 65.40 | 0.10 | 99.46 | 72.3 | 97.14 | 0.26 |
| Example 61 | 82.47 | 0.13 | 97.38 | 71.2 | 95.85 | 0.34 |
| Example 62 | 99.80 | 0.16 | 99.32 | 59.0 | 96.24 | 0.47 |
| Example 63 | 84.74 | 0.15 | 97.68 | 63.4 | 92.85 | 0.17 |
| Example 64 | 90.58 | 0.19 | 98.93 | 64.6 | 99.18 | 0.28 |
| Example 65 | 90.10 | 0.20 | 96.31 | 60.3 | 96.80 | 0.19 |
| Example 66 | 104.2 2 | 0.14 | 94.12 | 76.7 | 98.63 | 0.21 |
| Example 67 | 86.08 | 0.20 | 66.08 | 95.8 | 98.32 | 0.59 |
| Example 68 | 104.6 8 | 0.18 | 95.99 | 94.9 | 98.77 | 0.1 |
| Example 69 | 157.6 9 | 0.13 | 85.47 | 91.9 | 97.10 | 0.19 |
| Example 70 | 142.4 4 | 0.11 | 97.26 | 91.2 | 99.07 | 0.22 |
| Example 71 | 98.30 | 85.88 | 98.30 | 97.3 | 98.99 | 0.12 |
| Example 72 | 99.70 | 65.62 | 99.70 | 84.0 | 81.19 | 0.63 |
| Example 73 | 85.54 | 182.3 7 | 85.54 | 84.1 | 97.94 | 0.62 |
| Example 74 | 96.91 | 117.4 9 | 96.91 | 71.0 | 91.81 | 0.46 |
| Example 75 | 99.22 | 60.95 | 99.22 | 90.3 | 91.29 | 0.59 |
| Example 76 | 74.60 | 228.0 0 | 96.23 | 95.0 | 98.01 | 0.37 |
| Example 77 | 99.78 | 70.33 | 99.78 | 83.9 | 90.71 | 0.62 |
| Example 78 | 99.86 | 53.66 | 99.86 | 91.7 | 81.35 | 0.56 |
| Example 79 | 99.80 | 70.67 | 99.80 | 95.0 | 95.05 | 0.67 |
| Example 80 | 99.14 | 147.2 2 | 99.14 | 87.3 | 94.60 | 0.79 |
| Example 81 | 99.10 | 124.0 0 | 99.10 | 76.4 | 93.39 | 0.61 |
| Example 82 | 99.74 | 92.91 | 99.74 | 76.0 | 96.72 | 0.89 |
| Example 83 | 99.79 | 73.54 | 99.79 | 84.2 | 97.05 | 0.84 |
| Example 84 | 89.72 | 0.11 | 97.58 | 90.2 | 96.42 | 0.93 |
| Example 85 | 100.2 4 | 0.10 | 93.32 | 95.9 | 96.2 | 0.69 |
| Example 86 | 88.62 | 0.13 | 94.50 | 93.0 | 94.18 | 0.71 |
| Example 87 | 72.55 | 0.12 | 92.26 | 97.5 | 96.15 | 0.78 |
| Example 88 | 93.15 | 0.17 | 91.31 | 92.5 | 91.33 | 0.56 |
| Example 89 | 96.38 | 0.09 | 94.22 | 94.0 | 92.26 | 1.08 |
| Example 90 | 84.42 | 0.14 | 94.33 | 91.6 | 92.38 | 1.34 |
| Example 91 | 134.2 4 | 0.24 | 79.63 | 91.4 | 62.74 | 0.5 |
| Example 92 | 156.2 2 | 0.28 | 76.22 | 92.6 | 73.64 | 0.34 |
| Example 93 | 98.21 | 0.17 | 93.28 | 82.6 | 85.37 | 0.93 |
| Example 94 | 100.1 4 | 0.19 | 96.63 | 98.6 | 88.07 | 0.86 |
| Example 95 | 110.2 5 | 0.11 | 92.93 | 95.9 | 93.13 | 0.91 |
| Example 96 | 89.25 | 0.13 | 93.34 | 96.6 | 94.64 | 0.97 |
| Example 97 | 150.3 5 | 0.34 | 67.68 | 90.5 | 83.64 | 0.53 |
| Example 98 | 89.51 | 0.14 | 91.32 | 96.9 | 92.36 | 1.19 |
| Example 99 | 120.2 3 | 0.26 | 69.17 | 93.2 | 77.44 | 0.49 |
| Example 100 | 111.2 0 | 0.16 | 92.32 | 99.0 | 93.24 | 0.97 |
| Example 101 | 113.5 4 | 0.14 | 92.68 | 93.4 | 94.85 | 0.97 |
| Example 102 | 94.58 | 0.12 | 93.93 | 94.6 | 93.18 | 1.28 |
| Example 103 | 94.10 | 0.14 | 94.31 | 95.3 | 93.80 | 1.19 |
| Example 104 | 111.2 2 | 0.16 | 95.12 | 96.7 | 92.63 | 1.21 |
| Example 105 | 144.0 8 | 0.24 | 66.08 | 95.8 | 78.32 | 0.39 |
| Example 106 | 176.6 8 | 0.28 | 65.99 | 93.9 | 78.77 | 0.26 |
| Example 107 | 127.6 9 | 0.13 | 95.47 | 92.9 | 92.10 | 1.19 |
| Example 108 | 95.34 | 0.13 | 92.30 | 92.3 | 93.99 | 0.32 |
| Example 109 | 159.7 0 | 0.33 | 79.70 | 89.0 | 71.17 | 1.13 |
| Example 110 | 84.27 | 0.11 | 90.54 | 89.1 | 92.96 | 0.92 |
| Example 111 | 96.92 | 0.12 | 92.91 | 91.0 | 95.82 | 1.16 |
| Example 112 | 89.42 | 0.11 | 91.22 | 92.3 | 95.39 | 1.29 |
| Example 113 | 88.62 | 0.14 | 91.23 | 93.2 | 92.51 | 1.17 |
| Example 114 | 89.25 | 0.16 | 92.78 | 93.4 | 91.74 | 1.22 |

### Example IV: In vivo transfection experiment of a lipid nanoparticle (LNP preparation)

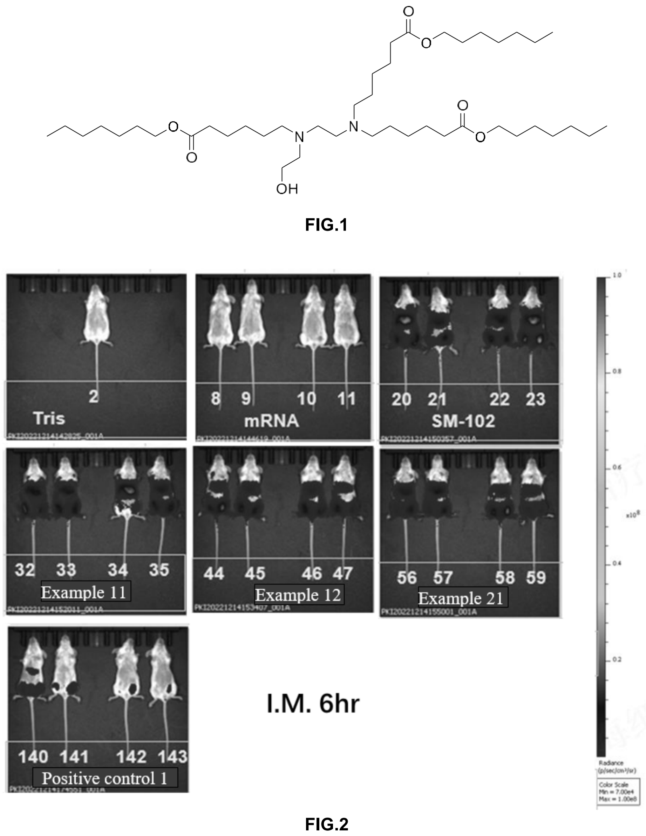

According to the method described in Example II , LNP lipid nanoparticles of Fluc-mRNA (Maxirna FLuc mRNA, 1 mg/mL), SM-102 (CAS number: 2089251-47-6, A.V.T (Shanghai) Pharmaceutical Co., Ltd.), and positive control 1 (US10195156B2, it was prepared according to the method described in the above embodiment, the main difference lay in replacing 2-((3-aminopropyl)amino)ethane-1-ol with 2-((2-aminoethyl)amino)ethane-1-ol, the structure was as shown in FIG. 1) were prepared respectively. The specific parameters were shown in Table 2. Cholesterol (Chol), DSPC, DOPE, and DMG-PEG2000 were purchased from A.V.T (Shanghai) Pharmaceutical Co., Ltd. Mice were randomly selected, and the lipid nanoparticles were injected intravenously and intramuscularly (4 mice per group) at a dosage of 0.5 mg/kg. Tris and Fluc-mRNA were directly injected as controls. After 6 hours, 200 ul of 10 mg/ml D-luciferin potassium salt was respectively injected into each mouse through the tail vein. After 10 minutes, the mice were placed under a live imaging system to observe the total fluorescence intensity of each mouse and take photos for recording. After 24 hours, the mice were placed under the live imaging system to observe the total fluorescence intensity of each mouse and take photos for recording.

**Table 2: Preparation parameters of each LNP**

| Sample number | Composition and proportion | Lipid/mRNA (N/P) |
|---|---|---|
| SM-102 | SM-102/Chol/DSPC/DMG-PEG2000=50/38.5/10/1.5 | 6/1 |
| Positive control 1 | Positive control 1/Chol/DOPE/DMG-PEG2000=50/38.75/10/1.25 | 12/1 |
| Example 11 | Lipid/Chol/DOPE/DMG-PEG2000=50/38.75/10/1.25 | 12/1 |
| Example 12 | Lipid/Chol/DOPE/DMG-PEG2000=50/38.75/10/1.25 | 12/1 |
| Example 21 | Lipid/Chol/DOPE/DMG-PEG2000=50/38.75/10/1.25 | 12/1 |

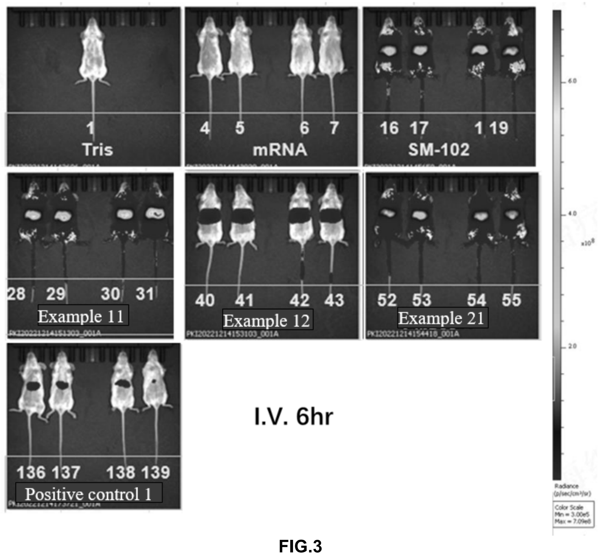

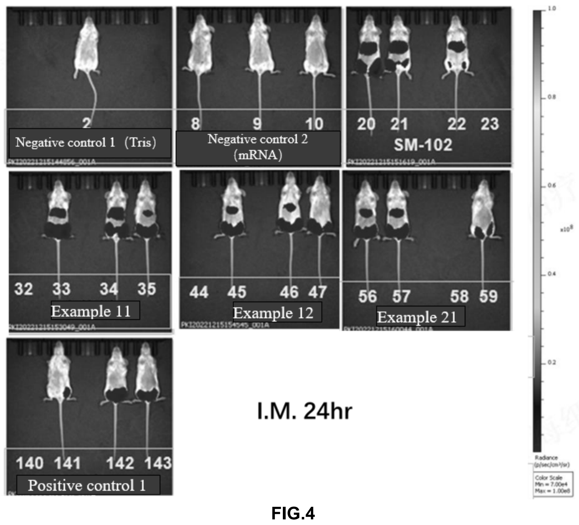

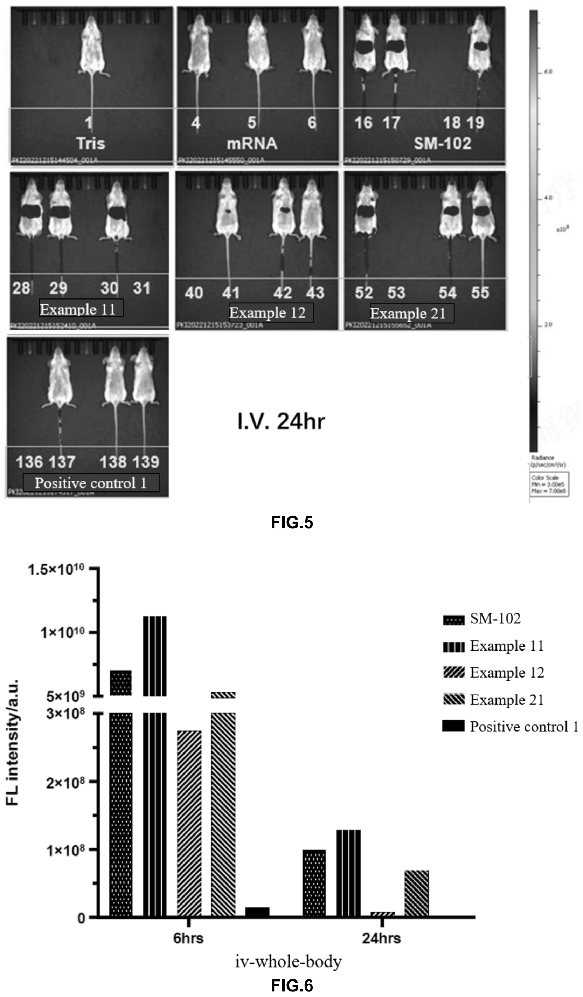

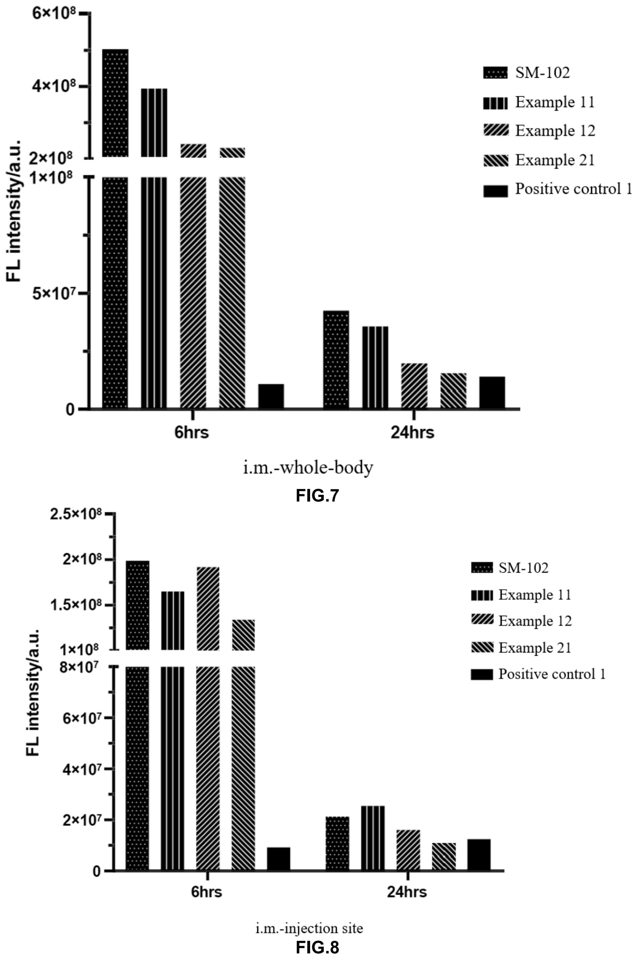

With intramuscular injection, the lipid nanoparticles will partially stay at the injection site, and the rest will mainly stay in the liver. By intravenous injection, all lipid nanoparticles were transferred to the chest and abdomen. As shown in FIGS. 4-7, 2-9, the fluorescence intensity of samples in Examples 11, 12, and 21 was stronger than that of positive control 1 and comparable to SM-102. Especially for intramuscular injection administration, Examples 11, 12, and 21 still maintain high fluorescence intensity, and the expression in the chest and abdomen in Examples 12 and 21 was much lower than that of SM-102.

LNP lipid nanoparticles of Fluc-mRNA were respectively prepared using the method described in Example II. The specific parameters were shown in Table 3. Mice were randomly selected, and the lipid nanoparticles were injected intramuscularly (2 mice per group) at a dosage of 0.5 mg/kg. Tris was directly injected as a control. After 6 hours, 200 ul of 10 mg/ml D-luciferin potassium salt was respectively injected into each mouse through the tail vein. After 10 minutes, the mice were placed under a live imaging system to observe the total fluorescence intensity of each mouse and take photos for recording. After 24 hours, the mice were placed under the live imaging system to observe the total fluorescence intensity of each mouse and take photos for recording.

**Table 3: Preparation parameters of each LNP**

| Sample number | Composition and proportion | Lipid/mRNA (N/P) |
|---|---|---|
| SM-102 | SM-102/Chol/DSPC/DMG-PEG2000=50/38.5/10/1.5 | 6/1 |
| Positive control 1 | Positive control 1/Chol/DSPC/DMG-PEG2000=50/38.75/10/1.25 | 12/1 |
| Example 1 | Lipid/Chol/DSPC/DMG-PEG2000=50/38.75/10/1.25 | 12/1 |
| Example 12 | Lipid/Chol/DSPC/DMG-PEG2000=50/38.75/10/1.25 | 12/1 |
| Example 64 | Lipid/Chol/DSPC/DMG-PEG2000=50/38.75/10/1.25 | 12/1 |
| Example 79 | Lipid/Chol/DSPC/DMG-PEG2000=50/38.75/10/1.25 | 12/1 |
| Example 81 | Lipid/Chol/DSPC/DMG-PEG2000=50/38.75/10/1.25 | 12/1 |

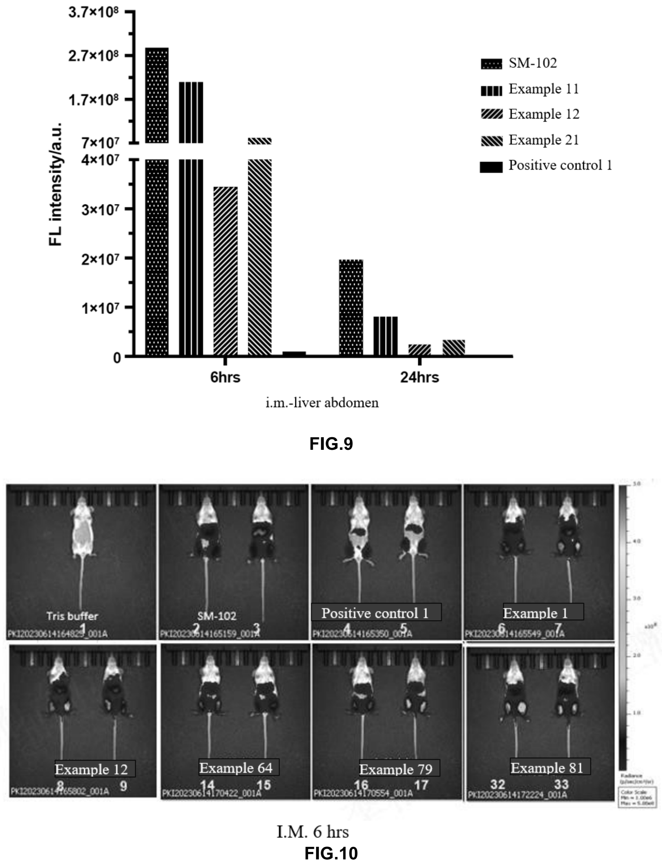

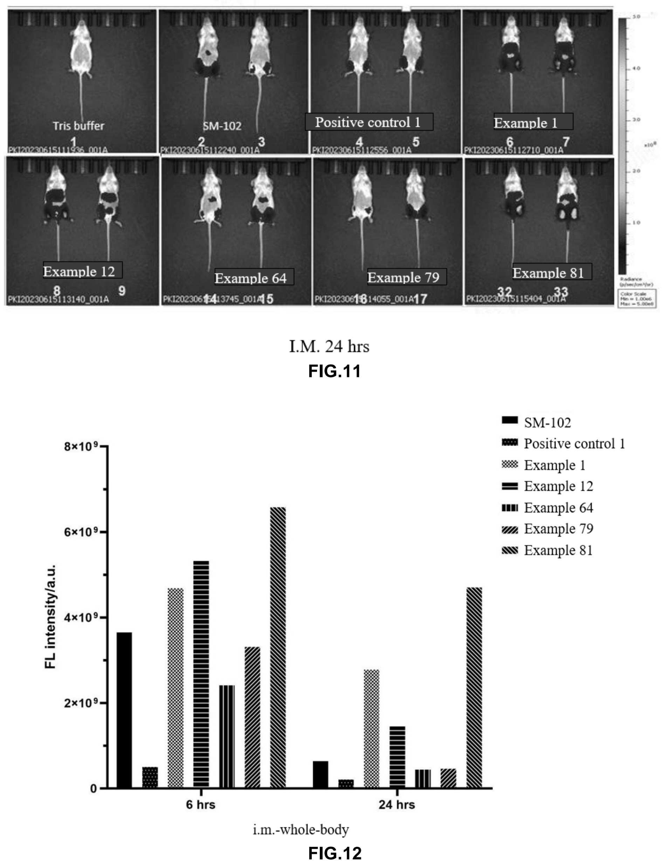

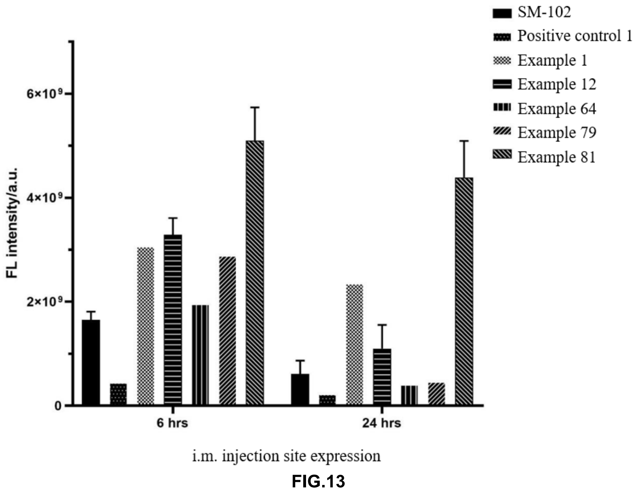

With intramuscular injection, the lipid nanoparticles will partially stay at the injection site, and the rest will mainly stay in the liver. As shown in FIGS. 10-13, the whole-body expression of the above embodiments was superior to that of positive control 1, and Examples 1, 12, and 81 were even better than SM-102. In terms of the injection site, compared with positive control 1 and SM-102, the embodiments had more advantages. Therefore, using DSPC instead of DOPE had superiority in the above embodiments, and could also explore process-related TFF and other aspects.

LNP lipid nanoparticles of Fluc-mRNA were respectively prepared using the method described in Example II . The specific parameters were shown in Table 4. Mice were randomly selected, and the lipid nanoparticles were injected intramuscularly (3 mice per group) at a dosage of 0.5 mg/kg. Tris was directly injected as a control. After 6 hours, 200 ul of 10 mg/ml D-luciferin potassium salt was respectively injected into each mouse through the tail vein. After 10 minutes, the mice were placed under a live imaging system to observe the total fluorescence intensity of each mouse and take photos for recording. After 24 hours, the mice were placed under the live imaging system to observe the total fluorescence intensity of each mouse and take photos for recording.

**Table 4: Preparation parameters of each LNP**

| Sample number | Composition and proportion | Lipid/mRNA (N/P) |
|---|---|---|
| SM-102 | SM-102/Chol/DSPC/DMG-PEG2000=50/38.5/10/1.5 | 6/1 |
| Positive control 1 | Positive control 1/Chol/DSPC/DMG-PEG2000=50/38.75/10/1.25 | 12/1 |
| Example 89 | Lipid/Chol/ DSPC /DMG-PEG2000=50/38.75/10/1.25 | 12/1 |

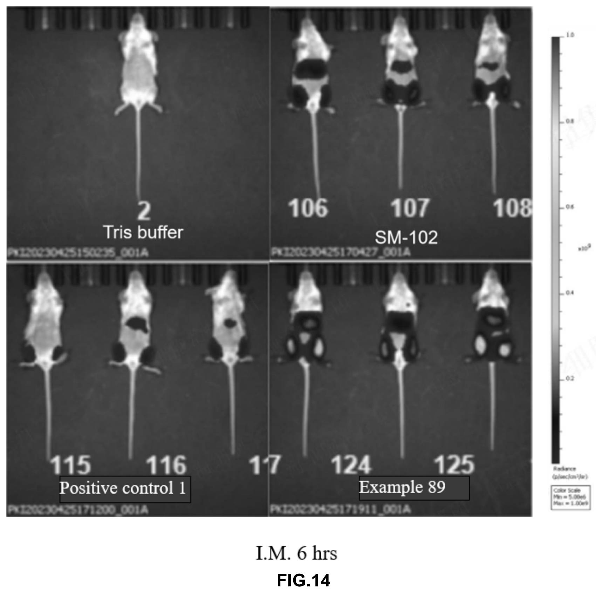

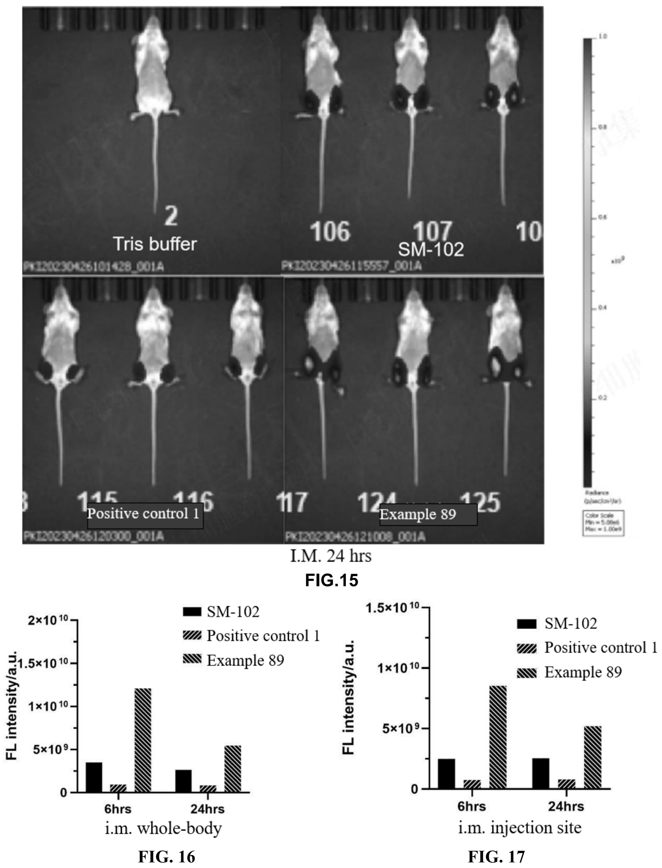

With intramuscular injection, the lipid nanoparticles will partially stay at the injection site, and the rest will mainly stay in the liver. As shown in FIGS. 14-17, the whole-body expression and injection site of Example 89 were superior to those of positive control 1 and SM-102.

## Claims

1. A compound of the following formula I, or a stereoisomer, tautomer, pharmaceutically acceptable salt, prodrug, or solvate thereof: 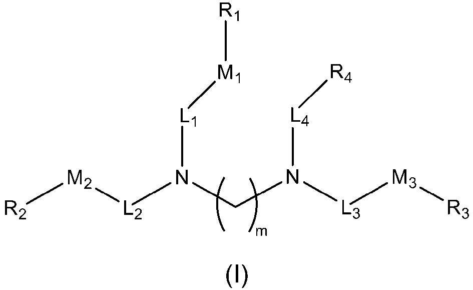 wherein,
L₁, L₂, and L₃ are each independently selected from optionally substituted alkylene, optionally substituted alkenylene, and optionally substituted alkynylene;
M₁, M₂, and M₃ are each independently selected from -C(O)O- and -OC(O)-;
R₁, R₂, and R₃ are each independently selected from optionally substituted alkyl, optionally substituted alkenyl, and optionally substituted alkynyl;
L₄ is selected from optionally substituted alkylene, optionally substituted alkenylene, and optionally substituted alkynylene;
R₄ is -OR₅, R₅ is selected from H, optionally substituted alkyl, optionally substituted alkenyl, and optionally substituted alkynyl; and
m is an integer from 3 to 6.

2. The compound or the stereoisomer, tautomer, pharmaceutically acceptable salt, prodrug, or solvate thereof according to claim 1, wherein,
L₁, L₂, and L₃ are each independently selected from an optionally substituted C₁₋₂₄ alkylene, an optionally substituted C₂₋₂₄ alkenylene, and an optionally substituted C₂₋₂₄alkynylene;
R₁, R₂, and R₃ are each independently selected from an optionally substituted C₁₋₂₄ alkyl, an optionally substituted C₂₋₂₄ alkenyl, and an optionally substituted C₂₋₂₄ alkynyl;
L₄ is selected from an optionally substituted C₁₋₆ alkylene, an optionally substituted C₂₋₆ alkenylene, and an optionally substituted C₂₋₆ alkynylene; and
R₅ is selected from H, an optionally substituted C₁₋₆ alkyl, an optionally substituted C₂₋₆ alkenyl, and an optionally substituted C₂₋₆ alkynyl.

3. The compound or the stereoisomer, tautomer, pharmaceutically acceptable salt, prodrug, or solvate thereof according to claim 1 or 2, wherein,
L₁, L₂, and L₃ are each independently selected from -(CH₂)ₙ-, wherein n is an integer from 1 to 24, preferably from 2 to 24, and more preferably from 3 to 15;
L₄ is selected from -(CH₂)ₒ-, wherein o is an integer from 1 to 6, preferably from 2 to 5, and more preferably from 2 to 4; and
R₅ is selected from H and a C₁₋₆ alkyl.

4. The compound or the stereoisomer, tautomer, pharmaceutically acceptable salt, prodrug, or solvate thereof according to any one of claims 1 to 3, wherein, R₁, R₂, and R₃ are each independently selected from a C₁₋₂₄ alkyl optionally substituted with one or more C₁₋₂₄ alkyls, a C₂₋₂₄ alkenyl optionally substituted with one or more C₁₋₂₄ alkyls, and preferably, the alkenyl contains 1-4 carbon-carbon double bonds.

5. The compound or the stereoisomer, tautomer, pharmaceutically acceptable salt, prodrug, or solvate thereof according to any one of claims 1 to 4, wherein, R₁, R₂, and R₃ are each independently selected from 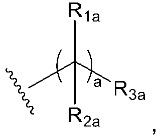 wherein, a is an integer from 1 to 24, and R₁ₐ and R₂ₐ are independently H or a C₁₋₂₄ alkyl at each occurrence; alternatively, each R₁ₐ is independently H or a C₁₋₂₄ alkyl, and R₂ₐ, together with the carbon atom to which it is bound is taken together with an adjacent R₂ₐ and the carbon atom to which it is bound to form a carbon-carbon double bond; and R₃ₐ is H or methyl.

6. The compound or the stereoisomer, tautomer, pharmaceutically acceptable salt, prodrug, or solvate thereof according to any one of claims 1 to 5, wherein, R₁, R₂, and R₃ are each independently selected from 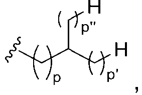 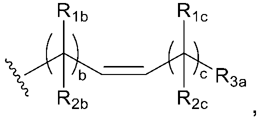 or 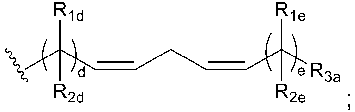
wherein, p is an integer from 0 to 2, p' is an integer from 1 to 24, and p" is an integer from 0 to 24;
b, c, d, and e are each independently an integer from 1 to 22;
R_{1b} and R_{2b} are independently H or a C₁₋₂₄ alkyl at each occurrence; alternatively, each R_{1b} is independently H or a C₁₋₁₂ alkyl, and at least one R_{2b}, together with the carbon atom to which it is bound is taken together with an adjacent R_{2b} and the carbon atom to which it is bound to form a carbon-carbon double bond;
R_{1c} and R_{2c} are independently H or a C₁₋₂₄ alkyl at each occurrence; alternatively, each R_{1c} is independently H or a C₁₋₁₂ alkyl, and at least one R_{2c}, together with the carbon atom to which it is bound is taken together with an adjacent R_{2c} and the carbon atom to which it is bound to form a carbon-carbon double bond;
R_{1d} and R_{2d} are independently H or a C₁₋₂₄ alkyl at each occurrence; alternatively, each R_{1d} is independently H or a C₁₋₁₂ alkyl, and at least one R_{2d}, together with the carbon atom to which it is bound is taken together with an adjacent R_{2d} and the carbon atom to which it is bound to form a carbon-carbon double bond; and
R₁ₑ and R₂ₑ are independently H or a C₁₋₂₄ alkyl at each occurrence; alternatively, each R₁ₑ is independently H or a C₁₋₁₂ alkyl, and at least one R₂ₑ, together with the carbon atom to which it is bound is taken together with an adjacent R₂ₑ and the carbon atom to which it is bound to form a carbon-carbon double bond.

7. The compound or the stereoisomer, tautomer, pharmaceutically acceptable salt, prodrug, or solvate thereof according to any one of claims 1 to 6, wherein the compound of formula I has a structure of the following formula la, Ib, Ic, or Id: 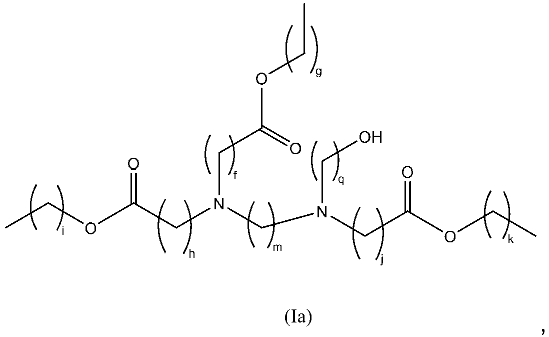 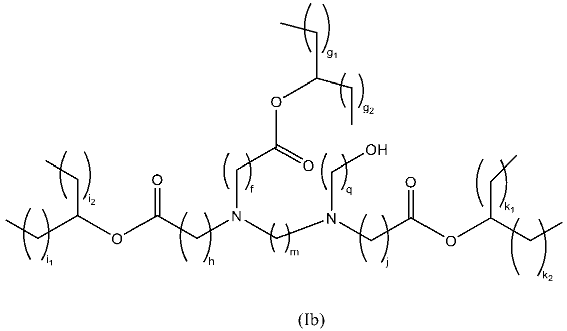 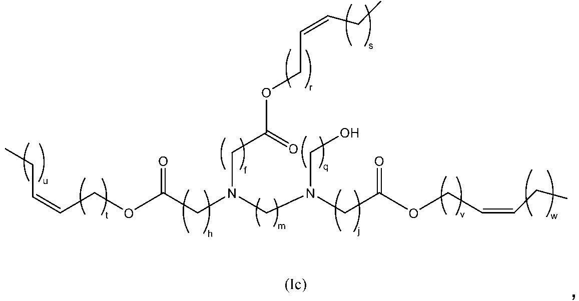 or 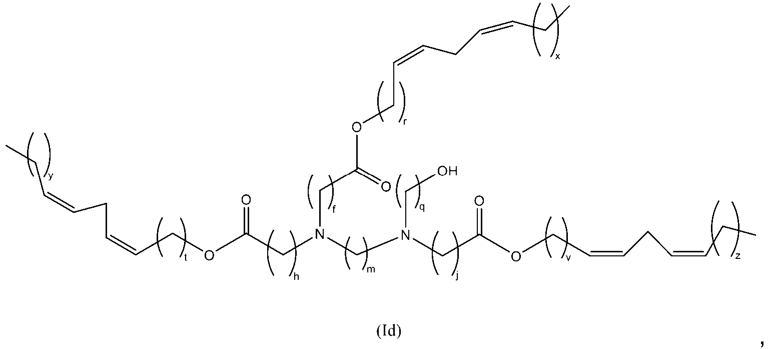 wherein, f, g, h, i, j, k, g1, g2, i1, i2, k1, k2, r, s, t, u, v, w, x, y, and z are each independently an integer from 1 to 24, and q is an integer from 1 to 6.

8. The compound or the stereoisomer, tautomer, pharmaceutically acceptable salt, prodrug, or solvate thereof according to claim 7, wherein,
f, h, and j are each independently an integer from 2 to 24, preferably an integer from 3 to 15,
g, g1, g2, i, i1, i2, k, k1, and k2 are each independently an integer from 3 to 24, preferably an integer from 3 to 15,
r, s, t, u, v, w, x, y, and z are each independently an integer from 1 to 15, preferably an integer from 1 to 10, and
q is an integer from 2 to 5, preferably an integer from 2 to 4;
preferably, in formula la, i and k are each independently an integer from 1 to 15, preferably each independently an integer from 3 to 15, and preferably, i and k are the same integer; h and j are each independently an integer from 2 to 11, preferably each independently an integer from 3 to 7, and preferably, h and j are the same integer; m is 3, 4, 5, or 6, preferably 3 or 4; f is an integer from 3 to 11, preferably an integer from 3 to 6; g is an integer from 3 to 15, preferably an integer from 6 to 12; and q is an integer from 2 to 5;
more preferably, in formula la, i, k, and g are each independently an integer from 1 to 15, preferably each independently an integer from 3 to 15, more preferably each independently an integer from 8 to 12, and preferably, i, k, and g are the same integer; f, h, and j are each independently an integer from 2 to 11, preferably each independently an integer from 3 to 7, and preferably, f, h, and j are the same integer; m is 3, 4, 5, or 6, preferably 3 or 4; and q is an integer from 2 to 5, preferably 2 or 3;
preferably, in formula Id, h and j are each independently an integer from 2 to 11, preferably each independently an integer from 3 to 8, and preferably, h and j are the same integer; m is 3, 4, 5, or 6, preferably 3 or 4; t and v are each independently an integer from 1 to 10, preferably each independently an integer from 5 to 10, and preferably, t and v are the same integer; y and z are each independently an integer from 1 to 10, preferably each independently an integer from 3 to 8, and preferably, y and z are the same integer; f is an integer from 3 to 11, preferably an integer from 3 to 8; r is an integer from 1 to 10, preferably an integer from 5 to 10; x is an integer from 1 to 10, preferably an integer from 3 to 8; and q is an integer from 2 to 5; and
more preferably, in formula Id, f, h, and j are each independently an integer from 2 to 11, preferably each independently an integer from 3 to 7, and preferably, f, h, and j are the same integer; m is 3, 4, 5, or 6, preferably 3 or 4; t, r, and v are each independently an integer from 1 to 10, preferably each independently an integer from 6 to 10, and preferably, t, r, and v are the same integer; x, y, and z are each independently an integer from 1 to 10, preferably each independently an integer from 3 to 7, and preferably, x, y, and z are the same integer; and q is an integer from 2 to 5.

9. A lipid nanoparticle, which comprises the compound or a stereoisomer, tautomer, pharmaceutically acceptable salt, prodrug or solvent thereof according to any one of claims 1 to 8; preferably, the lipid nanoparticle further comprises: one or more auxiliary lipid molecules, one or more cholesterol or cholesterol derivatives and/or one or more polymer-conjugated lipid molecules.

10. The lipid nanoparticle according to claim 9, wherein,
the auxiliary lipid molecule is a neutral lipid molecule, preferably selected from DSPC, DPPC, DMPC, DOPC, POPC, DOPE and SM, more preferably DOPE or DSPC; and/or
the polymer of the polymer-conjugated lipid molecule is polyethylene glycol; preferably, the polymer-conjugated lipid molecule is selected from PEG-DMG, PEG-DAG, PEG-PE, PEG-S-DAG, PEG-DSPE, PEG-cer, and PEG dialkoxypropyl carbamate, preferably PEG2000-DMG.

11. The lipid nanoparticle according to any one of claims 9 to 10, wherein, in the lipid nanoparticle, a molar ratio of the compound or the stereoisomer, tautomer, pharmaceutically acceptable salt, prodrug or solvate thereof to the auxiliary lipid molecule, the cholesterol or cholesterol derivative, and the polymer-conjugated lipid molecule is (60 to 5):(60 to 5):(50 to 5):(10 to 1), preferably (60 to 30):(30 to 10):(50 to 30):(5 to 1).

12. The lipid nanoparticle according to any one of claims 9 to 11, wherein the lipid nanoparticle further comprises a therapeutic agent or active agent; preferably, the therapeutic agent or active agent is a nucleic acid therapeutic agent or active agent; more preferably, the nucleic acid therapeutic agent or active agent is selected from: messenger RNA (mRNA), antisense oligonucleotide (ASO), small interfering RNA (siRNA), microRNA (miRNA), self-replicating RNA (saRNA), small guide RNA (sgRNA), ribozyme and aptamer.

13. A composition comprising the compound or the stereoisomer, tautomer, pharmaceutically acceptable salt, prodrug or solvate thereof according to any one of claims 1-8;
preferably, the composition is a pharmaceutical composition, which comprises the compound or the stereoisomer, tautomer, pharmaceutically acceptable salt, prodrug or solvate thereof according to any one of claims 1-8, a therapeutic agent or active agent, and one or more auxiliary lipid molecules, one or more cholesterol or cholesterol derivatives and/or one or more polymer-conjugated lipid molecules.

14. The composition according to claim 13, wherein,
the auxiliary lipid molecule is a neutral lipid molecule, preferably selected from DSPC, DPPC, DMPC, DOPC, POPC, DOPE and SM, more preferably DOPE or DSPC; and/or
the polymer of the polymer-conjugated lipid molecule is polyethylene glycol; preferably, the polymer-conjugated lipid molecule is selected from PEG-DMG, PEG-DAG, PEG-PE, PEG-S-DAG, PEG-DSPE, PEG-cer, and PEG dialkoxypropyl carbamate, preferably PEG2000-DMG; and/or
and/or the therapeutic agent or active agent is a nucleic acid therapeutic agent or active agent; preferably, the nucleic acid therapeutic agent or active agent is selected from: messenger RNA (mRNA), antisense oligonucleotide (ASO), small interfering RNA (siRNA), microRNA (miRNA), self-replicating RNA (saRNA), small guide RNA (sgRNA), ribozyme and aptamer.

15. Use selected from the following ones:
(1) use of the compound or the stereoisomer, tautomer, pharmaceutically acceptable salt, prodrug or solvate thereof according to any one of claims 1 to 8 in manufacture of a lipid nanoparticle or pharmaceutical composition for treatment or prevention of a disease or condition in a subject; or
(2) use of the compound or the stereoisomer, tautomer, pharmaceutically acceptable salt, prodrug or solvate thereof according to any one of claims 1 to 8 in manufacture of a reagent for the delivery of a therapeutic agent or active agent such as a nucleic acid; or
(3) use of the lipid nanoparticle according to any one of claims 9 to 12 in manufacture of drugs for treatment or prevention of a disease or condition in a subject; or
(4) use of the composition according to claim 13 or 14 in manufacture of drugs for treatment or prevention of a disease or condition in a subject.
